(19) Europäisches Patentamt
European Patent Office
Office européen des brevets

(11) EP 4 424 321 A2

(12) **EUROPEAN PATENT APPLICATION**

(43) Date of publication:
**04.09.2024 Bulletin 2024/36**

(21) Application number: **24159797.0**

(22) Date of filing: **25.09.2020**

(51) International Patent Classification (IPC):
***A61K 39/00*** *(2006.01)*

(52) Cooperative Patent Classification (CPC):
**C07K 16/2827; A61P 35/00; C07K 16/2803;**
A61K 2039/507; A61K 2039/545; Y02A 50/30

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Validation States:
**MA**

(30) Priority: **27.09.2019 US 201962907283 P**
**07.02.2020 US 202062971831 P**
**06.07.2020 US 202063048464 P**

(62) Document number(s) of the earlier application(s) in accordance with Art. 76 EPC:
**20789369.4 / 4 048 693**

(71) Applicant: **F. Hoffmann-La Roche AG**
**4070 Basel (CH)**

(72) Inventors:
- **MENG, Raymond D.**
  **South San Francisco, 94080-4990 (US)**
- **PATIL, Namrata Srivastava**
  **South San Francisco, 94080-4990 (US)**
- **FLANAGAN, William Michael**
  **South San Francisco, 94080-4990 (US)**

(74) Representative: **Klostermeyer-Rauber, Dörte et al**
**F. Hoffmann-La Roche AG**
**Corporate Law Patents (CLP)**
**Grenzacherstrasse 124**
**4070 Basel (CH)**

Remarks:
•The complete document including Reference Table(s) and the Sequence Listing(s) can be downloaded from the EPO website
•This application was filed on 26.02.2024 as a divisional application to the application mentioned under INID code 62.
•Claims filed after the date of filing of the application / after the date of receipt of the divisional application (Rule 68(4) EPC).

(54) **DOSING FOR TREATMENT WITH ANTI-TIGIT AND ANTI-PD-L1 ANTAGONIST ANTIBODIES**

(57) The invention provides methods of dosing for the treatment of cancers. In particular, provided are methods for treating human patients having lung cancer, such as non-small cell lung cancer (NSCLC), by administering a combination of an anti-TIGIT antagonist antibody and a PD-1 axis binding antagonist.

EP 4 424 321 A2

**Description**

## FIELD OF THE INVENTION

The present invention relates to the treatment of cancer (e.g., lung cancer). More specifically, the invention concerns the treatment of patients having cancer (e.g., lung cancer) by administering a combination of an anti-T-cell immunoreceptor with Ig and ITIM domains (TIGIT) antagonist antibody and a PD-1 axis binding antagonist (e.g., an anti-programmed death ligand-1 (PD-L1) antagonist antibody or an anti-programmed death-1 (PD-1) antagonist antibody).

## BACKGROUND OF THE INVENTION

Cancers are characterized by the uncontrolled growth of cell subpopulations. Cancers are the leading cause of death in the developed world and the second leading cause of death in developing countries, with over 14 million new cancer cases diagnosed and over eight million cancer deaths occurring each year. Cancer care thus represents a significant and ever-increasing societal burden.

Lung cancer, in particular, remains the leading cause of cancer deaths worldwide, accounting for approximately 13% of all new cancers in 2012. In 2017 in the United States, it was estimated that there were 222,500 new cases of lung cancer and 155,870 lung cancer deaths. Non-small cell lung cancer (NSCLC) is the predominant subtype, accounting for approximately 85% of all cases. The overall five-year survival rate for advanced disease is 2%-4%. Poor prognostic factors for survival in patients with NSCLC include advanced stage of disease at the time of initial diagnosis, poor performance status, and a history of unintentional weight loss. More than half of the patients with NSCLC are diagnosed with distant disease, which directly contributes to poor survival prospects.

Despite improvements in the first-line treatment of patients with advanced NSCLC that have resulted in longer survival times and reduced disease-related symptoms, nearly all patients experience disease progression. Cancer immunotherapies in particular offer the possibility of long-term disease control. In the metastatic NSCLC setting, PD-L1/PD-1 blocking antibodies (e.g., atezolizumab, nivolumab, and pembrolizumab) provided clinically meaningful benefit in either unselected or PD-L1-selected advanced NSCLC patients; however, a substantial proportion of patients still remained unresponsive or progressed on anti-PD-L1/PD-1 treatment, and the escape mechanisms to such treatment are poorly understood.

Thus, there is an unmet need in the field for the development of efficacious immunotherapies and methods of dosing the same for the treatment of cancers (e.g., lung cancer, e.g., NSCLC) that achieve a more favorable benefit-risk profile.

## SUMMARY OF THE INVENTION

The present invention relates to methods of treating a subject having cancer (e.g., lung cancer, e.g., NSCLC, e.g., squamous or non-squamous NSCLC, e.g., locally advanced unresectable NSCLC (e.g., Stage IIIB NSCLC), or recurrent or metastatic NSCLC (e.g., Stage IV NSCLC)) by administering a combination of an anti-TIGIT antagonist antibody (e.g., an anti-TIGIT antagonist antibody as disclosed herein, e.g., tiragolumab) and a PD-1 axis binding antagonist (e.g., an anti-PD-L1 antagonist antibody (e.g., atezolizumab)).

In a first aspect, the invention features a method for treating a subject having a lung cancer comprising administering to the subject one or more dosing cycles of an anti-TIGIT antagonist antibody (e.g., at a fixed dose of between about 30 mg to about 1200 mg every three weeks, at a fixed dose of between about 300 mg to about 800 mg every two weeks, or at a fixed dose of between about 700 mg to about 1000 mg every four weeks) and a PD-1 axis binding antagonist (e.g., an anti-PD-L1 antagonist antibody) (e.g., at a fixed dose of between about 80 mg to about 1600 mg every three weeks, at a fixed dose of between about 200 mg to about 1200 mg every two weeks, or at a fixed dose of between about 400 mg to about 2000 mg every four weeks), wherein the subject has been determined to have a PD-L1-positive tumor cell fraction of greater than, or equal to, 30% (e.g., greater than, or equal to, 50%), and the treatment results in (a) a complete response (CR) or a partial response (PR) and/or (b) an increase in progression-free survival (PFS) as compared to treatment with the PD-1 axis binding antagonist (e.g., anti-PD-L1 antagonist antibody) without the anti-TIGIT antagonist antibody.

In some embodiments of the first aspect, the method comprises administering to the subject an anti-TIGIT antagonist antibody at a fixed dose of between about 30 mg to about 600 mg every three weeks. In some embodiments, the method comprises administering to the subject an anti-TIGIT antagonist antibody at a fixed dose of about 600 mg every three weeks. In some embodiments of the first aspect, the method comprises administering to the subject an anti-TIGIT antagonist antibody at a fixed dose of between about 400 mg to about 500 mg every two weeks. In some embodiments, the method comprises administering to the subject an anti-TIGIT antagonist antibody at a fixed dose of about 420 mg every two weeks. In some embodiments of the first aspect, the method comprises administering to the subject an anti-TIGIT antagonist antibody at a fixed dose of between about 800 mg to about 900 mg every two weeks. In some embodiments, the method comprises administering to the subject an anti-TIGIT antagonist antibody at a fixed dose of about 840 mg every two weeks.

In some embodiments of the first aspect, the anti-TIGIT antagonist antibody comprises the following hypervariable regions (HVRs): an HVR-H1 sequence comprising the amino acid sequence of SNSAAWN (SEQ ID NO: 1); an HVR-H2 sequence comprising the amino acid sequence of KTYYRFKWYSDYAVSVKG (SEQ ID NO: 2); an HVR-H3 sequence comprising the amino acid sequence of ESTTYDLLAGPFDY (SEQ ID NO: 3); an HVR-L1 sequence comprising the amino acid sequence of KSSQTVLYSSNNKKYLA (SEQ ID NO: 4); an HVR-L2 sequence comprising the amino acid sequence of WASTRES (SEQ ID NO: 5); and an HVR-L3 sequence comprising the amino acid sequence of QQYYSTPFT (SEQ ID NO: 6). In some embodiments, the anti-TIGIT antagonist antibody further comprises the following light chain variable region framework regions (FRs): an FR-L1 comprising the amino acid sequence of DIVMTQSPDSLAVSLGERATINC (SEQ ID NO: 7); an FR-L2 comprising the amino acid sequence of WYQQKPGQPPNLLIY (SEQ ID NO: 8); an FR-L3 comprising the amino acid sequence of GVPDRFSGSGSGTDFTLTISSLQAEDVAVYYC (SEQ ID NO: 9); and an FR-L4 comprising the amino acid sequence of FGPGTKVEIK (SEQ ID NO: 10). In some embodiments, the anti-TIGIT antagonist antibody further comprises the following heavy chain variable region FRs: an FR-H1 comprising the amino acid sequence of $X_1$VQLQQSGPGLVKPSQTLSLTCAISGDSVS (SEQ ID NO: 11), wherein $X_1$ is Q or E; an FR-H2 comprising the amino acid sequence of WIRQSPSRGLEWLG (SEQ ID NO: 12); an FR-H3 comprising the amino acid sequence of

RITINPDTSKNQFSLQLNSVTPEDTAVFYCTR (SEQ ID NO: 13); and an FR-H4 comprising the amino acid sequence of WGQGTLVTVSS (SEQ ID NO: 14). In some embodiments, $X_1$ is Q. In some embodiments, $X_1$ is E.

In some embodiments of the first aspect, the anti-TIGIT antagonist antibody comprises: (a) a heavy chain variable (VH) domain comprising an amino acid sequence having at least 95% sequence identity to the amino acid sequence of SEQ ID NO: 17 or 18; (b) a light chain variable (VL) domain comprising an amino acid sequence having at least 95% sequence identity to the amino acid sequence of SEQ ID NO: 19; or (c) a VH domain as in (a) and a VL domain as in (b).

In some embodiments of the first aspect, the anti-TIGIT antagonist antibody comprises: a VH domain comprising the amino acid sequence of SEQ ID NO: 17 or 18 and a VL domain comprising the amino acid sequence of SEQ ID NO: 19.

In some embodiments of the first aspect, the anti-TIGIT antagonist antibody is a monoclonal antibody. In some embodiments, the anti-TIGIT antagonist antibody is a human antibody (e.g., a monoclonal human antibody).

In some embodiments of the first aspect, the anti-TIGIT antagonist antibody is a full-length antibody. In some embodiments of the first aspect, the anti-TIGIT antagonist antibody is tiragolumab.

In some embodiments of the first aspect, the anti-TIGIT antagonist antibody is an antibody fragment that binds TIGIT selected from the group consisting of Fab, Fab', Fab'-SH, Fv, single chain variable fragment (scFv), and $(Fab')_2$ fragments.

In some embodiments of the first aspect, the anti-TIGIT antagonist antibody is an IgG class antibody. In some embodiments, the IgG class antibody is an IgG1 subclass antibody.

In some embodiments of the first aspect, the method comprises administering to the subject a PD-1 axis binding antagonist (e.g., an anti-PD-L1 antibody) at a fixed dose of about 1200 mg every three weeks. In some embodiments of the first aspect, the method comprises administering to the subject a PD-1 axis binding antagonist (e.g., an anti-PD-L1 antibody) at a fixed dose of about 840 mg every two weeks. In some embodiments of the first aspect, the method comprises administering to the subject a PD-1 axis binding antagonist (e.g., an anti-PD-L1 antibody) at a fixed dose of about 1680 mg every four weeks.

In some embodiments of the first aspect, the PD-1 axis binding antagonist (e.g., anti-PD-L1 antagonist antibody) is atezolizumab (MPDL3280A), MSB0010718C, MDX-1105, or MEDI4736. In some embodiments, the anti-PD-L1 antagonist antibody is atezolizumab.

In some embodiments of the first aspect, the anti-PD-L1 antagonist antibody comprises the following HVRs: an HVR-H1 sequence comprising the amino acid sequence of GFTFSDSWIH (SEQ ID NO: 20); an HVR-H2 sequence comprising the amino acid sequence of AWISPYGGSTYYADSVKG (SEQ ID NO: 21); an HVR-H3 sequence comprising the amino acid sequence of RHWPGGFDY (SEQ ID NO: 22); an HVR-L1 sequence comprising the amino acid sequence of RASQDVSTAVA (SEQ ID NO: 23); an HVR-L2 sequence comprising the amino acid sequence of SASFLYS (SEQ ID NO: 24); and an HVR-L3 sequence comprising the amino acid sequence of QQYLYHPAT (SEQ ID NO: 25). In some embodiments, the anti-PD-L1 antagonist antibody comprises: (a) a heavy chain variable (VH) domain comprising an amino acid sequence having at least 95% sequence identity to the amino acid sequence of SEQ ID NO: 26; (b) a light chain variable (VL) domain comprising an amino acid sequence having at least 95% sequence identity to the amino acid sequence of SEQ ID NO: 27; or (c) a VH domain as in (a) and a VL domain as in (b).

In some embodiments of the first aspect, the anti-PD-L1 antagonist antibody comprises: a VH domain comprising the amino acid sequence of SEQ ID NO: 26 and a VL domain comprising the amino acid sequence of SEQ ID NO: 27.

In some embodiments of the first aspect, the PD-1 axis binding antagonist (e.g., anti-PD-L1 antagonist antibody)

is a monoclonal antibody. In some embodiments, the PD-1 axis binding antagonist (e.g., anti-PD-L1 antagonist antibody) is a humanized antibody (e.g., a monoclonal humanized antibody).

In some embodiments of the first aspect, the PD-1 axis binding antagonist (e.g., anti-PD-L1 antagonist antibody) is a full-length antibody.

In some embodiments of the first aspect, the PD-1 axis binding antagonist (e.g., anti-PD-L1 antagonist antibody) is an antibody fragment that binds PD-L1 selected from the group consisting of Fab, Fab', Fab'-SH, Fv, single chain variable fragment (scFv), and $(Fab')_2$ fragments.

In some embodiments of the first aspect, the PD-1 axis binding antagonist (e.g., anti-PD-L1 antagonist antibody) is an IgG class antibody. In some embodiments, the IgG class antibody is an IgG1 subclass antibody.

In some embodiments of the first aspect, the method comprises administering to the subject the anti-TIGIT antagonist antibody at a fixed dose of about 600 mg every three weeks and the PD-1 axis binding antagonist (e.g., anti-PD-L1 antagonist antibody) at a fixed dose of about 1200 mg every three weeks.

In some embodiments of the first aspect, the length of each of the one or more dosing cycles is 21 days.

In some embodiments of the first aspect, the method comprises administering to the subject the anti-TIGIT antagonist antibody and the PD-1 axis binding antagonist (e.g., anti-PD-L1 antagonist antibody) on about Day 1 of each of the one or more dosing cycles.

In some embodiments of the first aspect, the method comprises administering to the subject the anti-TIGIT antagonist antibody at a fixed dose of about 420 mg every two weeks and the PD-1 axis binding antagonist (e.g., anti-PD-L1 antagonist antibody) at a fixed dose of about 820 mg every two weeks. In some embodiments of the first aspect, the length of each of the one or more dosing cycles is 28 days. In some embodiments of the first aspect, the method comprises administering to the subject the anti-TIGIT antagonist antibody and the PD-1 axis binding antagonist (e.g., anti-PD-L1 antagonist antibody) on about Day 1 and Day 15 of each of the one or more dosing cycles.

In some embodiments of the first aspect, the method comprises administering to the subject the anti-TIGIT antagonist antibody at a fixed dose of about 840 mg every four weeks and the PD-1 axis binding antagonist (e.g., anti-PD-L1 antagonist antibody) at a fixed dose of about 1680 mg every four weeks. In some embodiments of the first aspect, the length of each of the one or more dosing cycles is 28 days. In some embodiments of the first aspect, the method comprises administering to the subject the anti-TIGIT antagonist antibody and the PD-1 axis binding antagonist (e.g., anti-PD-L1 antagonist antibody) on about Day 1 of each of the one or more dosing cycles.

In some embodiments of the first aspect, the method comprises administering to the subject the anti-TIGIT antagonist antibody before the PD-1 axis binding antagonist (e.g., anti-PD-L1 antagonist antibody). In some embodiments, the method comprises a first observation period following administration of the anti-TIGIT antagonist antibody and second observation period following administration of the PD-1 axis binding antagonist (e.g., anti-PD-L1 antagonist antibody). In some embodiments, the first observation period and the second observation period are each between about 30 minutes to about 60 minutes in length.

In some embodiments of the first aspect, the method comprises administering to the subject the PD-1 axis binding antagonist (e.g., anti-PD-L1 antagonist antibody) before the anti-TIGIT antagonist antibody. In some embodiments, the method comprises a first observation period following administration of the PD-1 axis binding antagonist (e.g., anti-PD-L1 antagonist antibody) and second observation period following administration of the anti-TIGIT antagonist antibody. In some embodiments, the first observation period and the second observation period are each between about 30 minutes to about 60 minutes in length.

In some embodiments of the first aspect, the method comprises administering to the subject the anti-TIGIT antagonist antibody and the PD-1 axis binding antagonist (e.g., anti-PD-L1 antagonist antibody) simultaneously.

In some embodiments of the first aspect, the method comprises administering to the subject the anti-TIGIT antagonist antibody and PD-1 axis binding antagonist (e.g., anti-PD-L1 antagonist antibody) intravenously. In some embodiments, the method comprises administering to the subject the anti-TIGIT antagonist antibody by intravenous infusion over 60 $\pm$ 10 minutes. In some embodiments, the method comprises administering to the subject the PD-1 axis binding antagonist (e.g., anti-PD-L1 antagonist antibody) by intravenous infusion over 60 $\pm$ 15 minutes.

In some embodiments of the first aspect, the PD-L1-positive tumor cell fraction has been determined by an immunohistochemical (IHC) assay. In some embodiments, the IHC assay uses anti-PD-L1 antibody SP263, 22C3, SP142, or 28-8. In some embodiments, the PD-L1-positive tumor cell fraction is determined by positive staining with an anti-PD-L1 antibody (e.g., SP263, 22C3, SP142, or 28-8). In some embodiments, the PD-L1-positive tumor cell fraction is greater than, or equal to, 50%, as determined by positive staining with the anti-PD-L1 antibody SP263 (e.g., wherein the PD-L1-positive tumor cell fraction is calculated using the Ventana SP263 IHC assay). In some embodiments, the PD-L1-positive tumor cell fraction is greater than, or equal to, 50%, as determined by positive staining with the anti-PD-L1 antibody 22C3 (e.g., wherein the PD-L1-positive tumor cell fraction is calculated using the pharmDx 22C3 IHC assay). In some embodiments, the PD-L1-positive tumor cell fraction is greater than, or equal to, 30%, as determined by positive staining with the anti-PD-L1 antibody SP142 (e.g., wherein the PD-L1-positive tumor cell fraction is calculated using the Ventana SP142 IHC assay). In some embodiments, the PD-L1-positive tumor cell fraction is greater than, or equal to, 50%, as determined by positive staining with the anti-PD-L1 antibody 28-8.

In some embodiments of the first aspect, a tumor sample obtained from the subject has been determined to have a detectable nucleic acid expression level of PD-L1. In some embodiments, the detectable nucleic acid expression level of PD-L1 has been determined by RNA-seq, RT-qPCR, qPCR, multiplex qPCR or RT-qPCR, microarray analysis, SAGE, MassARRAY technique, ISH, or a combination thereof.

In some embodiments of the first aspect, the lung cancer is a non-small cell lung cancer (NSCLC). In some embodiments, the NSCLC is a squamous NSCLC. In some embodiments, the NSCLC is a non-squamous NSCLC. In some embodiments, the NSCLC is a locally advanced unresectable NSCLC. In some embodiments, the NSCLC is a Stage IIIB NSCLC. In some embodiments, the NSCLC is a recurrent or metastatic NSCLC. In some embodiments, the NSCLC is a Stage IV NSCLC. In some embodiments, the subject has not been previously treated for Stage IV NSCLC.

In some embodiments of the first aspect, the subject does not have a sensitizing epidermal growth factor receptor *(EGFR)* gene mutation or anaplastic lymphoma kinase *(ALK)* gene rearrangement.

In some embodiments of the first aspect, the subject does not have a pulmonary lymphoepithelioma-like carcinoma subtype of NSCLC.

In some embodiments of the first aspect, the subject does not have an active Epstein-Barr virus (EBV) infection or a known or suspected chronic active EBV infection. In some embodiments, the subject is negative for EBV IgM or negative by EBV PCR. In some embodiments, the subject is negative for EBV IgM and negative by EBV PCR. In some embodiments, the subject is positive for EBV IgG or positive for Epstein-Barr nuclear antigen (EBNA). In some embodiments, the subject is positive for EBV IgG and positive for EBNA.

In some embodiments of the first aspect, the subject is negative for EBV IgG or negative for EBNA. In some embodiments, the subject is negative for EBV IgG and negative for EBNA.

In some embodiments, the subject is likely to have an increase in the PFS of the subject as compared to a reference PFS time. In some embodiments, the reference PFS time is the median PFS time of a population of subjects who have received a treatment comprising an PD-1 axis binding antagonist (e.g., anti-PD-L1 antagonist antibody) without an anti-TIGIT antagonist antibody.

In a second aspect, the invention features a method for treating a subject having a NSCLC comprising administering to the subject one or more dosing cycles of an anti-TIGIT antagonist antibody (e.g., at a fixed dose of about 600 mg every three weeks, at a fixed dose of about 420 mg every two weeks, or at a fixed dose of about 840 mg every four weeks) and a PD-1 axis binding antagonist (e.g., an anti-PD-L1 antagonist antibody) (e.g., at a fixed dose of about 1200 mg every three weeks, at a fixed dose of about 840 mg every two weeks, or at a fixed dose of about 1680 mg every four weeks), wherein the anti-TIGIT antagonist antibody comprises: a VH domain comprising the amino acid sequence of SEQ ID NO: 17 or 18 and a VL domain comprising the amino acid sequence of SEQ ID NO: 19, and wherein the subject has been determined to have a PD-L1-positive tumor cell fraction of greater than, or equal to, 30% (e.g., greater than, or equal to, 50%), and the treatment results in (a) a CR or a PR and/or (b) an increase in PFS as compared to treatment with atezolizumab without the anti-TIGIT antagonist antibody.

In a third aspect, the invention features a method for treating a subject having a NSCLC comprising (a) obtaining a tumor sample from the subject; (b) detecting the protein expression level of PD-L1 in the tumor sample by staining tumor cells from the tumor sample with anti-PD-L1 antibody SP263 and determining a PD-L1-positive tumor cell fraction therefrom, wherein the subject has been determined to have a PD-L1-positive tumor cell fraction of greater than, or equal to, 50%; and (c) administering to the subject a therapy comprising one or more dosing cycles of an anti-TIGIT antagonist antibody (e.g., at a fixed dose of 600 mg every three weeks, at a fixed dose of 420 mg every two weeks, or at a fixed dose of 840 mg every four weeks) and atezolizumab (e.g., at a fixed dose of 1200 mg every three weeks, at a fixed dose of 840 mg every two weeks, or at a fixed dose of 1680 mg every four weeks), wherein the anti-TIGIT antagonist antibody comprises: a VH domain comprising the amino acid sequence of SEQ ID NO: 17 or 18; and a VL domain comprising the amino acid sequence of SEQ ID NO: 19, and wherein the treatment results in (a) a CR or a PR and/or (b) an increase in PFS as compared to treatment with atezolizumab without the anti-TIGIT antagonist antibody.

In a fourth aspect, the invention features a method for treating a subject having a NSCLC comprising (a) obtaining a tumor sample from the subject; (b) detecting the protein expression level of PD-L1 in the tumor sample by staining tumor cells from the tumor sample with anti-PD-L1 antibody 22C3 and determining a PD-L1-positive tumor cell fraction therefrom, wherein the subject has been determined to have a PD-L1-positive tumor cell fraction of greater than, or equal to, 50%; and (c) administering to the subject a therapy comprising one or more dosing cycles of an anti-TIGIT antagonist antibody (e.g., at a fixed dose of 600 mg every three weeks, at a fixed dose of 420 mg every two weeks, or at a fixed dose of 840 mg every four weeks) and atezolizumab (e.g., at a fixed dose of 1200 mg every three weeks, at a fixed dose of 840 mg every two weeks, or at a fixed dose of 1680 mg every four weeks), wherein the anti-TIGIT antagonist antibody comprises: a VH domain comprising the amino acid sequence of SEQ ID NO: 17 or 18; and a VL domain comprising the amino acid sequence of SEQ ID NO: 19, and wherein the treatment results in (a) a CR or a PR and/or (b) an increase in PFS as compared to treatment with atezolizumab without the anti-TIGIT antagonist antibody.

In a fifth aspect, the invention features a method for treating a subject having a NSCLC comprising administering to the subject one or more dosing cycles of tiragolumab (e.g., at a fixed dose of 600 mg every three weeks, at a fixed dose of 420 mg every two weeks, or at a fixed dose of 840 mg every four weeks) and atezolizumab (e.g., at a fixed dose of 1200 mg every three weeks, at a fixed dose of 840 mg every two weeks, or at a fixed dose of 1680 mg every four weeks), wherein the subject has been determined to have a PD-L1-positive tumor cell fraction of greater than, or equal to, 30% (e.g., greater than, or equal to, 50%), and the treatment results in (a) a CR or a PR and/or (b) an increase in PFS as compared to treatment with atezolizumab without tiragolumab.

In a sixth aspect, the invention features a method for treating a subject having a NSCLC comprising (a) obtaining a tumor sample from the subject; (b) detecting the protein expression level of PD-L1 in the tumor sample by an IHC assay using anti-PD-L1 antibody SP263 and determining a PD-L1-positive tumor cell fraction therefrom, wherein the subject has been determined to have a PD-L1-positive tumor cell fraction of greater than, or equal to, 50%; and (c) administering to the subject a therapy comprising one or more dosing cycles of an anti-TIGIT antagonist antibody (e.g., at a fixed dose of 600 mg every three weeks, at a fixed dose of 420 mg every two weeks, or at a fixed dose of 840 mg every four weeks) and atezolizumab (e.g., at a fixed dose of 1200 mg every three weeks, at a fixed dose of 840 mg every two weeks, or at a fixed dose of 1680 mg every four weeks), wherein the anti-TIGIT antagonist antibody comprises: a VH domain comprising the amino acid sequence of SEQ ID NO: 17 or 18; and a VL domain comprising the amino acid sequence of SEQ ID NO: 19, and wherein the treatment results in (a) a CR or a PR and/or (b) an increase in PFS as compared to treatment with atezolizumab without the anti-TIGIT antagonist antibody.

In a seventh aspect, the invention features a method for treating a subject having a NSCLC comprising (a) obtaining a tumor sample from the subject; (b) detecting the protein expression level of PD-L1 in the tumor sample by an IHC assay using anti-PD-L1 antibody 22C3 and determining a PD-L1-positive tumor cell fraction therefrom, wherein the subject has been determined to have a PD-L1-positive tumor cell fraction of greater than, or equal to, 50%; and (c) administering to the subject a therapy comprising one or more dosing cycles of an anti-TIGIT antagonist antibody (e.g., at a fixed dose of 600 mg every three weeks, at a fixed dose of 420 mg every two weeks, or at a fixed dose of 840 mg every four weeks) and atezolizumab (e.g., at a fixed dose of 1200 mg every three weeks, at a fixed dose of 840 mg every two weeks, or at a fixed dose of 1680 mg every four weeks), wherein the anti-TIGIT antagonist antibody comprises: a VH domain comprising the amino acid sequence of SEQ ID NO: 17 or 18; and a VL domain comprising the amino acid sequence of SEQ ID NO: 19, and wherein the treatment results in (a) a CR or a PR and/or (b) an increase in PFS as compared to treatment with atezolizumab without the anti-TIGIT antagonist antibody.

In an eighth aspect, the invention features an anti-TIGIT antagonist antibody and a PD-1 axis binding antagonist (e.g., anti-PD-L1 antagonist antibody) for use in a method of treating a subject having a lung cancer, the method comprising administering to the subject one or more dosing cycles of the anti-TIGIT antagonist antibody (e.g., at a fixed dose of between about 30 mg to about 1200 mg every three weeks, at a fixed dose of between about 300 mg to about 800 mg every two weeks, or at a fixed dose of between about 700 mg to about 1000 mg every four weeks) and a PD-1 axis binding antagonist (e.g., an anti-PD-L1 antagonist antibody) (e.g., at a fixed dose of between about 80 mg to about 1600 mg every three weeks, at a fixed dose of between about 200 mg to about 1200 mg every two weeks, or at a fixed dose of between about 400 mg to about 2000 mg every four weeks), wherein the subject has been determined to have a PD-L1-positive tumor cell fraction of greater than, or equal to, 30% (e.g., greater than, or equal to, 50%), and the treatment results in (a) a CR or a PR and/or (b) an increase in PFS as compared to treatment with the PD-1 axis binding antagonist (e.g., anti-PD-L1 antagonist antibody) without the anti-TIGIT antagonist antibody.

In some embodiments of the eighth aspect, the anti-TIGIT antagonist antibody is to be administered to the subject at a fixed dose of between about 30 mg to about 600 mg every three weeks. In some embodiments, the anti-TIGIT antagonist antibody is to be administered to the subject at a fixed dose of about 600 mg every three weeks. In some embodiments, the anti-TIGIT antagonist antibody is to be administered at a fixed dose of between about 400 mg to about 500 mg every two weeks. In some embodiments, the anti-TIGIT antagonist antibody is to be administered at a fixed dose of about 420 mg every two weeks. In some embodiments, the anti-TIGIT antagonist antibody is to be administered at a fixed dose of between about 800 mg to about 900 mg every two weeks. In some embodiments, the anti-TIGIT antagonist antibody is to be administered at a fixed dose of about 840 mg every two weeks.

In some embodiments of the eighth aspect, the anti-TIGIT antagonist antibody comprises the following HVRs: an HVR-H1 sequence comprising the amino acid sequence of SNSAAWN (SEQ ID NO: 1); an HVR-H2 sequence comprising the amino acid sequence of KTYYRFKWYSDYAVSVKG (SEQ ID NO: 2); an HVR-H3 sequence comprising the amino acid sequence of ESTTYDLLAGPFDY (SEQ ID NO: 3); an HVR-L1 sequence comprising the amino acid sequence of KSSQTVLYSSNNKKYLA (SEQ ID NO: 4); an HVR-L2 sequence comprising the amino acid sequence of WASTRES (SEQ ID NO: 5); and an HVR-L3 sequence comprising the amino acid sequence of QQYYSTPFT (SEQ ID NO: 6). In some embodiments, the anti-TIGIT antagonist antibody further comprises the following light chain variable region FRs: an FR-L1 comprising the amino acid sequence of DIVMTQSPDSLAVSLGERATINC (SEQ ID NO: 7); an FR-L2 comprising the amino acid sequence of WYQQKPGQPPNLLIY (SEQ ID NO: 8); an FR-L3 comprising the amino acid sequence of GVPDRFSGSGSGTDFTLTISSLQAEDVAVYYC (SEQ ID NO: 9); and an FR-L4 comprising the amino acid sequence of FGPGTKVEIK (SEQ ID NO: 10). In some embodiments, the anti-TIGIT antagonist antibody further comprises the following heavy chain variable region FRs: an FR-H1 comprising the amino acid sequence of $X_1$VQLQQSGPGLVKPSQTLSLTCAISGDSVS (SEQ ID NO: 11), wherein $X_1$ is Q or E; an FR-H2 comprising the amino acid sequence of WIRQSPSRGLEWLG (SEQ ID NO: 12); an FR-H3 comprising the amino acid sequence of RITINPDTSKNQFSLQLNSVTPEDTAVFYCTR (SEQ ID NO: 13); and an FR-H4 comprising the amino acid sequence of WGQGTLVTVSS (SEQ ID NO: 14). In some embodiments, $X_1$ is Q. In some embodiments, $X_1$ is E.

In some embodiments of the eighth aspect, the anti-TIGIT antagonist antibody comprises: (a) a heavy chain variable (VH) domain comprising an amino acid sequence having at least 95% sequence identity to the amino acid sequence of SEQ ID NO: 17 or 18; (b) a light chain variable (VL) domain comprising an amino acid sequence having at least 95% sequence identity to the amino acid sequence of SEQ ID NO: 19; or (c) a VH domain as in (a) and a VL domain as in (b).

In some embodiments of the eighth aspect, the anti-TIGIT antagonist antibody comprises: a VH domain comprising the amino acid sequence of SEQ ID NO: 17 or 18 and a VL domain comprising the amino acid sequence of SEQ ID NO: 19.

In some embodiments of the eighth aspect, the anti-TIGIT antagonist antibody is a monoclonal antibody. In some embodiments, the anti-TIGIT antagonist antibody is a human antibody (e.g., a monoclonal human antibody).

In some embodiments of the eighth aspect, the anti-TIGIT antagonist antibody is a full-length antibody. In some embodiments of the eighteenth aspect, the anti-TIGIT antagonist antibody is tiragolumab.

In some embodiments of the eighth aspect, the anti-TIGIT antagonist antibody is an antibody fragment that binds TIGIT selected from the group consisting of Fab, Fab’, Fab’-SH, Fv, single chain variable fragment (scFv), and (Fab’)$_2$ fragments.

In some embodiments of the eighth aspect, the anti-TIGIT antagonist antibody is an IgG class antibody. In some embodiments, the IgG class antibody is an IgG1 subclass antibody.

In some embodiments of the eighth aspect, the PD-1 axis binding antagonist (e.g., anti-PD-L1 antagonist antibody) is to be administered to the subject at a fixed dose of about 1200 mg every three weeks. In other embodiments, the PD-1 axis binding antagonist (e.g., anti-PD-L1 antagonist antibody) is to be administered to the subject at a fixed dose of about 840 mg every two weeks. In other embodiments, the PD-1 axis binding antagonist (e.g., anti-PD-L1 antagonist antibody) is to be administered to the subject at a fixed dose of about 1680 mg every four weeks.

In some embodiments of the eighth aspect, the PD-1 axis binding antagonist is a PD-L1 binding antagonist or a PD-1 binding antagonist. In some embodiments, the PD-L1 binding antagonist is an anti-PD-L1 antagonist antibody (e.g., atezolizumab (MPDL3280A), MSB0010718C, MDX-1105, or MEDI4736). In some embodiments, the PD-L1 antagonist is atezolizumab. In some embodiments, the PD-1 binding antagonist is an anti-PD-1 antagonist antibody (e.g., nivolumab (MDX-1106) or pembrolizumab (formerly lambrolizumab (MK-3475))). In some embodiments, the PD-1 binding antagonist is AMP-224. In some embodiments of the eighth aspect, the anti-PD-L1 antagonist antibody comprises the following HVRs: an HVR-H1 sequence comprising the amino acid sequence of GFTFSDSWIH (SEQ ID NO: 20); an HVR-H2 sequence comprising the amino acid sequence of AWISPYGGSTYYADSVKG (SEQ ID NO: 21); an HVR-H3 sequence comprising the amino acid sequence of RHWPGGFDY (SEQ ID NO: 22); an HVR-L1 sequence comprising the amino acid sequence of RASQDVSTAVA (SEQ ID NO: 23); an HVR-L2 sequence comprising the amino acid sequence of SASFLYS (SEQ ID NO: 24); and an HVR-L3 sequence comprising the amino acid sequence of QQYLYHPAT (SEQ ID NO: 25). In some embodiments, the anti-PD-L1 antagonist antibody comprises: (a) a heavy chain variable (VH) domain comprising an amino acid sequence having at least 95% sequence identity to the amino acid sequence of SEQ ID NO: 26; (b) a light chain variable (VL) domain comprising an amino acid sequence having at least 95% sequence identity to the amino acid sequence of SEQ ID NO: 27; or (c) a VH domain as in (a) and a VL domain as in (b).

In some embodiments of the eighth aspect, the anti-PD-L1 antagonist antibody comprises: a VH domain comprising the amino acid sequence of SEQ ID NO: 26 and a VL domain comprising the amino acid sequence of SEQ ID NO: 27.

In some embodiments of the eighth aspect, the PD-1 axis binding antagonist (e.g., anti-PD-L1 antagonist antibody) is a monoclonal antibody. In some embodiments, the PD-1 axis binding antagonist (e.g., anti-PD-L1 antagonist antibody) is a humanized antibody (e.g., a monoclonal humanized antibody).

In some embodiments of the eighth aspect, PD-1 axis binding antagonist (e.g., anti-PD-L1 antagonist antibody) is a full-length antibody.

In some embodiments of the eighth aspect, the PD-1 axis binding antagonist (e.g., anti-PD-L1 antagonist antibody) is an antibody fragment that binds PD-L1 or PD-1 selected from the group consisting of Fab, Fab’, Fab’-SH, Fv, single chain variable fragment (scFv), and (Fab’)$_2$ fragments.

In some embodiments of the eighth aspect, the PD-1 axis binding antagonist (e.g., anti-PD-L1 antagonist antibody) is an IgG class antibody. In some embodiments, the IgG class antibody is an IgG1 subclass antibody.

In some embodiments of the eighth aspect, the anti-TIGIT antagonist antibody is to be administered to the subject at a fixed dose of about 600 mg every three weeks and the PD-1 axis binding antagonist (e.g., anti-PD-L1 antagonist antibody) is to be administered to the subject at a fixed dose of about 1200 mg every three weeks.

In some embodiments of the eighth aspect, the length of each of the one or more dosing cycles is 21 days.

In some embodiments of the eighth aspect, the anti-TIGIT antagonist antibody and PD-1 axis binding antagonist (e.g., anti-PD-L1 antagonist antibody) are to be administered to the subject on about Day 1 of each of the one or more dosing cycles.

In some embodiments, the anti-TIGIT antagonist antibody is to be administered at a fixed dose of about 420 mg every two weeks and the PD-1 axis binding antagonist (e.g., anti-PD-L1 antagonist antibody) is to be administered at a fixed dose of about 820 mg every two weeks. In some embodiments, the length of each of the one or more dosing cycles is 28 days. In some embodiments, the anti-TIGIT antagonist antibody and the PD-1 axis binding antagonist (e.g., anti-PD-L1 antagonist antibody) are to be administered on about Day 1 and Day 15 of each of the one or more dosing cycles.

In some embodiments, the anti-TIGIT antagonist antibody is to be administered at a fixed dose of about 840 mg every four weeks and the PD-1 axis binding antagonist (e.g., anti-PD-L1 antagonist antibody) is to be administered at a fixed dose of about 1680 mg every four weeks. In some embodiments, the length of each of the one or more dosing cycles is 28 days. In some embodiments, the anti-TIGIT antagonist antibody and the PD-1 axis binding antagonist (e.g., anti-PD-L1 antagonist antibody) are to be administered on about Day 1 of each of the one or more dosing cycles.

In some embodiments of the eighth aspect, the anti-TIGIT antagonist antibody is to be administered to the subject before the PD-1 axis binding antagonist (e.g., anti-PD-L1 antagonist antibody). In some embodiments, a first observation period is to follow administration of the anti-TIGIT antagonist antibody and second observation period is to follow administration of the PD-1 axis binding antagonist (e.g., anti-PD-L1 antagonist antibody). In some embodiments, the first observation period and the second observation period are each between about 30 minutes to about 60 minutes in length.

In some embodiments of the eighth aspect, the PD-1 axis binding antagonist (e.g., anti-PD-L1 antagonist antibody) is to be administered to the subject before the anti-TIGIT antagonist antibody. In some embodiments, a first observation period is to follow administration of the PD-1 axis binding antagonist (e.g., anti-PD-L1 antagonist antibody) and second observation period is to follow administration of the anti-TIGIT antagonist antibody. In some embodiments, the first observation period and the second observation period are each between about 30 minutes to about 60 minutes in length.

In some embodiments of the eighth aspect, the anti-TIGIT antagonist antibody is to be administered to the subject simultaneously with the PD-1 axis binding antagonist (e.g., anti-PD-L1 antagonist antibody).

In some embodiments of the eighth aspect, the anti-TIGIT antagonist antibody and PD-1 axis binding antagonist (e.g., anti-PD-L1 antagonist antibody) are to be administered to the subject intravenously. In some embodiments, the anti-TIGIT antagonist antibody is to be administered to the subject by intravenous infusion over 60 $\pm$ 10 minutes. In some embodiments, the PD-1 axis binding antagonist (e.g., anti-PD-L1 antagonist antibody) is to be administered to the subject by intravenous infusion over 60 $\pm$ 15 minutes.

In some embodiments of the eighth aspect, the PD-L1-positive tumor cell fraction has been determined by an immunohistochemical (IHC) assay. In some embodiments, the IHC assay uses anti-PD-L1 antibody SP263, 22C3, SP142, or 28-8. In some embodiments, the PD-L1-positive tumor cell fraction is determined by positive staining with an anti-PD-L1 antibody (e.g., SP263, 22C3, SP142, or 28-8). In some embodiments, the PD-L1-positive tumor cell fraction is greater than, or equal to, 50%, as determined by positive staining with the anti-PD-L1 antibody SP263 (e.g., wherein the PD-L1-positive tumor cell fraction is calculated using the Ventana SP263 IHC assay). In some embodiments, the PD-L1-positive tumor cell fraction is greater than, or equal to, 50%, as determined by positive staining with the anti-PD-L1 antibody 22C3 (e.g., wherein the PD-L1-positive tumor cell fraction is calculated using the pharmDx 22C3 IHC assay). In some embodiments, the PD-L1-positive tumor cell fraction is greater than, or equal to, 30%, as determined by positive staining with the anti-PD-L1 antibody SP142 (e.g., wherein the PD-L1-positive tumor cell fraction is calculated using the Ventana SP142 IHC assay). In some embodiments, the PD-L1-positive tumor cell fraction is greater than, or equal to, 50%, as determined by positive staining with the anti-PD-L1 antibody 28-8.

In some embodiments of the eighth aspect, the IHC assay uses anti-PD-L1 antibody SP263. In some embodiments, the IHC assay uses anti-PD-L1 antibody 22C3.

In some embodiments of the eighth aspect, a tumor sample obtained from the subject has been determined to have a detectable nucleic acid expression level of PD-L1. In some embodiments, the detectable nucleic acid expression level of PD-L1 has been determined by RNA-seq, RT-qPCR, qPCR, multiplex qPCR or RT-qPCR, microarray analysis, SAGE, MassARRAY technique, ISH, or a combination thereof.

In some embodiments of the eighth aspect, the lung cancer is a non-small cell lung cancer (NSCLC). In some embodiments, the NSCLC is a squamous NSCLC. In some embodiments, the NSCLC is a non-squamous NSCLC. In some embodiments, the NSCLC is a locally advanced unresectable NSCLC. In some embodiments, the NSCLC is a Stage IIIB NSCLC. In some embodiments, the NSCLC is a recurrent or metastatic NSCLC. In some embodiments, the NSCLC is a Stage IV NSCLC. In some embodiments, the subject has not been previously treated for Stage IV NSCLC.

In some embodiments of the eighth aspect, the subject does not have a sensitizing epidermal growth factor receptor *(EGFR)* gene mutation or anaplastic lymphoma kinase *(ALK)* gene rearrangement.

In some embodiments of the eighth aspect, the subject does not have a pulmonary lymphoepithelioma-like carcinoma subtype of NSCLC.

In some embodiments of the eighth aspect, the subject does not have an active EBV infection or a known or suspected chronic active EBV infection. In some embodiments, the subject is negative for EBV IgM or negative by EBV PCR. In some embodiments, the subject is negative for EBV IgM and negative by EBV PCR. In some embodiments, the subject is positive for EBV IgG or positive for EBNA. In some embodiments, the subject is positive for EBV IgG and positive for EBNA.

In some embodiments of the eighth aspect, the subject is negative for EBV IgG or negative for EBNA. In some embodiments, the subject is negative for EBV IgG and negative for EBNA.

In some embodiments of the eighth aspect, the PFS of the subject is increased as compared to a reference PFS time. In some embodiments, the reference PFS time is the median PFS time of a population of subjects who have received a treatment comprising a PD-1 axis binding antagonist (e.g., anti-PD-L1 antagonist antibody) without an anti-TIGIT antagonist antibody.

In a ninth aspect, the invention features an anti-TIGIT antagonist antibody and atezolizumab for use in a method of treating a subject having a NSCLC, the method comprising administering to the subject one or more dosing cycles of an anti-TIGIT antagonist antibody (e.g., at a fixed dose of 600 mg every three weeks, at a fixed dose of 420 mg every two weeks, or at a fixed dose of 840 mg every four weeks) and atezolizumab (e.g., at a fixed dose of 1200 mg every three weeks, at a fixed dose of 840 mg every two weeks, or at a fixed dose of 1680 mg every four weeks), wherein the anti-TIGIT antagonist antibody comprises: a VH domain comprising the amino acid sequence of SEQ ID NO: 17 or 18 and a VL domain comprising the amino acid sequence of SEQ ID NO: 19., and wherein the subject has been determined to have a PD-L1-positive tumor cell fraction of greater than, or equal to, 30% (e.g., greater than, or equal to, 50%), and the treatment results in (a) a CR or a PR and/or (b) an increase in PFS as compared to treatment with atezolizumab without the anti-TIGIT antagonist antibody

In a tenth aspect, the invention features tiragolumab and atezolizumab for use in a method of treating a subject having a NSCLC, the method comprising administering to the subject one or more dosing cycles of tiragolumab (e.g., at a fixed dose of 600 mg every three weeks, at a fixed dose of 420 mg every two weeks, or at a fixed dose of 840 mg every four weeks) and atezolizumab (e.g., at a fixed dose of 1200 mg every three weeks, at a fixed dose of 840 mg every two weeks, or at a fixed dose of 1680 mg every four weeks), and wherein the subject has been determined to have a PD-L1-positive tumor cell fraction of greater than, or equal to, 30% (e.g., greater than, or equal to, 50%), and the treatment results in (a) a CR or a PR and/or (b) an increase in PFS as compared to treatment with atezolizumab without tiragolumab.

In an eleventh aspect, the invention features a use of an anti-TIGIT antagonist antibody and a PD-1 axis binding antagonist (e.g., anti-PD-L1 antagonist antibody) in the manufacture of a medicament for use in a method of treating a subject having a lung cancer, the method comprising administering to the subject one or more dosing cycles of the medicament, wherein the medicament is formulated for administration of the anti-TIGIT antagonist antibody (e.g., at a fixed dose of between about 30 mg to about 1200 mg every three weeks, at a fixed dose of between about 300 mg to about 800 mg every two weeks, or at a fixed dose of between about 700 mg to about 1000 mg every four weeks) and a PD-1 axis binding antagonist (e.g., an anti-PD-L1 antagonist antibody) (e.g., at a fixed dose of between about 80 mg to about 1600 mg every three weeks, at a fixed dose of between about 200 mg to about 1200 mg every two weeks, or at a fixed dose of between about 400 mg to about 2000 mg every four weeks), wherein the subject has been determined to have a PD-L1-positive tumor cell fraction of greater than, or equal to, 30% (e.g., greater than, or equal to, 50%), and the treatment results in (a) a CR or a PR and/or (b) an increase in PFS as compared to treatment with the PD-1 axis binding antagonist (e.g., anti-PD-L1 antagonist antibody) without the anti-TIGIT antagonist antibody.

In a twelfth aspect, the invention features a use of an anti-TIGIT antagonist antibody in the manufacture of a medicament for use in a method of treating a subject having lung cancer, the method comprising administering to the subject one or more dosing cycles of the medicament and a PD-1 axis binding antagonist (e.g., anti-PD-L1 antagonist antibody), wherein the medicament is formulated for administration of the anti-TIGIT antagonist antibody (e.g., at a fixed dose of between about 30 mg to about 1200 mg every three weeks, at a fixed dose of between about 300 mg to about 800 mg every two weeks, or at a fixed dose of between about 700 mg to about 1000 mg every four weeks) and a PD-1 axis binding antagonist (e.g., an anti-PD-L1 antagonist antibody) (e.g., at a fixed dose of between about 80 mg to about 1600 mg every three weeks, at a fixed dose of between about 200 mg to about 1200 mg every two weeks, or at a fixed dose of between about 400 mg to about 2000 mg every four weeks), wherein the subject has been determined to have a PD-L1-positive tumor cell fraction of greater than, or equal to, 30% (e.g., greater than, or equal to, 50%), and the treatment results in (a) a CR or a PR and/or (b) an increase in PFS as compared to treatment with the PD-1 axis binding antagonist (e.g., anti-PD-L1 antagonist antibody) without the anti-TIGIT antagonist antibody.

In a thirteenth aspect, the invention features a use of a PD-1 axis binding antagonist (e.g., anti-PD-L1 antagonist antibody) in the manufacture of a medicament for use in a method of treating a subject having lung cancer, the method comprising administering to the subject one or more dosing cycles of the medicament and an anti-TIGIT antagonist antibody, wherein the medicament is formulated for administration of the PD-1 axis binding antagonist (e.g., anti-PD-L1 antagonist antibody) (e.g., at a fixed dose of between about 80 mg to about 1600 mg every three weeks, at a fixed dose of between about 200 mg to about 1200 mg every two weeks, or at a fixed dose of between about 400 mg to about 2000 mg every four weeks)and the anti-TIGIT antagonist antibody is to be administered (e.g., at a fixed dose of between about 30 mg to about 1200 mg every three weeks, at a fixed dose of between about 300 mg to about 800 mg every two weeks, or at a fixed dose of between about 700 mg to about 1000 mg every four weeks), wherein the subject has been determined to have a PD-L1-positive tumor cell fraction of greater than, or equal to, 30% (e.g., greater than, or equal to, 50%), and the treatment results in (a) a CR or a PR and/or (b) an increase in PFS as compared to treatment with the PD-1 axis binding antagonist (e.g., anti-PD-L1 antagonist antibody) without the anti-TIGIT antagonist antibody.

In some embodiments of any of the eleventh, twelfth, and thirteenth aspects, the anti-TIGIT antagonist antibody is to be administered to the subject at a fixed dose of between about 30 mg to about 600 mg every three weeks. In some embodiments, the anti-TIGIT antagonist antibody is to be administered to the subject at a fixed dose of about 600 mg every three weeks. In some embodiments, the anti-TIGIT antagonist antibody is to be administered at a fixed dose of between about 400 mg to about 500 mg every two weeks. In some embodiments, the anti-TIGIT antagonist antibody is to be administered at a fixed dose of about 420 mg every two weeks. In some embodiments, the anti-TIGIT antagonist antibody is to be administered at a fixed dose of between about 800 mg to about 900 mg every two weeks. In some embodiments, the anti-TIGIT antagonist antibody is to be administered at a fixed dose of about 840 mg every two weeks.

In some embodiments of any of the eleventh, twelfth, and thirteenth aspects, the anti-TIGIT antagonist antibody comprises the following hypervariable regions (HVRs): an HVR-H1 sequence comprising the amino acid sequence of SNSAAWN (SEQ ID NO: 1); an HVR-H2 sequence comprising the amino acid sequence of KTYYRFKWYSDYAVSVKG (SEQ ID NO: 2); an HVR-H3 sequence comprising the amino acid sequence of ESTTYDLLAGPFDY (SEQ ID NO: 3); an HVR-L1 sequence comprising the amino acid sequence of KSSQTVLYSSNNKKYLA (SEQ ID NO: 4); an HVR-L2 sequence comprising the amino acid sequence of WASTRES (SEQ ID NO: 5); and an HVR-L3 sequence comprising the amino acid sequence of QQYYSTPFT (SEQ ID NO: 6). In some embodiments, the anti-TIGIT antagonist antibody further comprises the following light chain variable region framework regions (FRs): an FR-L1 comprising the amino acid sequence of DIVMTQSPDSLAVSLGERATINC (SEQ ID NO: 7); an FR-L2 comprising the amino acid sequence of WYQQKPGQPPNLLIY (SEQ ID NO: 8); an FR-L3 comprising the amino acid sequence of GVPDRFSGSGSGTDFTLTISSLQAEDVAVYYC (SEQ ID NO: 9); and an FR-L4 comprising the amino acid sequence of FGPGTKVEIK (SEQ ID NO: 10). In some embodiments, the anti-TIGIT antagonist antibody further comprises the following heavy chain variable region FRs: an FR-H1 comprising the amino acid sequence of $X_1$VQLQQSGPGLVKPSQTLSLTCAISGDSVS (SEQ ID NO: 11), wherein $X_1$ is Q or E; an FR-H2 comprising the amino acid sequence of WIRQSPSRGLEWLG (SEQ ID NO: 12); an FR-H3 comprising the amino acid sequence of RITINPDTSKNQFSLQLNSVTPEDTAVFYCTR (SEQ ID NO: 13); and an FR-H4 comprising the amino acid sequence of WGQGTLVTVSS (SEQ ID NO: 14). In some embodiments, $X_1$ is Q. In some embodiments, $X_1$ is E.

In some embodiments of any of the eleventh, twelfth, and thirteenth aspects, the anti-TIGIT antagonist antibody comprises: (a) a heavy chain variable (VH) domain comprising an amino acid sequence having at least 95% sequence identity to the amino acid sequence of SEQ ID NO: 17 or 18; (b) a light chain variable (VL) domain comprising an amino acid sequence having at least 95% sequence identity to the amino acid sequence of SEQ ID NO: 19; or (c) a VH domain as in (a) and a VL domain as in (b).

In some embodiments of any of the eleventh, twelfth, and thirteenth aspects, the anti-TIGIT antagonist antibody is a monoclonal antibody. In some embodiments, the anti-TIGIT antagonist antibody is a human antibody (e.g., a monoclonal human antibody).

In some embodiments of any of the eleventh, twelfth, and thirteenth aspects, the anti-TIGIT antagonist antibody is a full-length antibody. In some embodiments of any of the twenty-first, twenty-second, and twenty-third aspects, the anti-TIGIT antagonist antibody is tiragolumab.

In some embodiments of any of the eleventh, twelfth, and thirteenth aspects, the anti-TIGIT antagonist antibody is an antibody fragment that binds TIGIT selected from the group consisting of Fab, Fab', Fab'-SH, Fv, single chain variable fragment (scFv), and $(Fab')_2$ fragments.

In some embodiments of any of the eleventh, twelfth, and thirteenth aspects, the anti-TIGIT antagonist antibody is an IgG class antibody. In some embodiments, the IgG class antibody is an IgG1 subclass antibody.

In some embodiments of any of the eleventh, twelfth, and thirteenth aspects, the PD-1 axis binding antagonist (e.g., anti-PD-L1 antagonist antibody) is to be administered to the subject at a fixed dose of about 1200 mg every three weeks. In some embodiments of any of the eleventh, twelfth, and thirteenth aspects, the PD-1 axis binding antagonist (e.g., anti-PD-L1 antagonist antibody) is to be administered to the subject at a fixed dose of about 840 mg every two weeks. In some embodiments of any of the eleventh, twelfth, and thirteenth aspects, the PD-1 axis binding antagonist (e.g., anti-PD-L1 antagonist antibody) is to be administered to the subject at a fixed dose of about 1680 mg every four weeks.

In some embodiments of any of the eleventh, twelfth, and thirteenth aspects, the PD-1 axis binding antagonist is a PD-L1 binding antagonist or a PD-1 binding antagonist. In some embodiments, the PD-L1 binding antagonist is an anti-PD-L1 antagonist antibody (e.g., atezolizumab (MPDL3280A), MSB0010718C, MDX-1105, or MEDI4736). In some embodiments, the PD-L1 antagonist is atezolizumab. In some embodiments, the PD-1 binding antagonist is an anti-PD-1 antagonist antibody (e.g., nivolumab (MDX-1106) or pembrolizumab (formerly lambrolizumab (MK-3475))). In some embodiments, the PD-1 binding antagonist is AMP-224.

In some embodiments of any of the eleventh, twelfth, and thirteenth aspects, the anti-PD-L1 antagonist antibody comprises the following HVRs: an HVR-H1 sequence comprising the amino acid sequence of GFTFSDSWIH (SEQ ID NO: 20); an HVR-H2 sequence comprising the amino acid sequence of AWISPYGGSTYYADSVKG (SEQ ID NO: 21); an HVR-H3 sequence comprising the amino acid sequence of RHWPGGFDY (SEQ ID NO: 22); an HVR-L1 sequence comprising the amino acid sequence of RASQDVSTAVA (SEQ ID NO: 23); an HVR-L2 sequence comprising the amino acid sequence of SASFLYS (SEQ ID NO: 24); and an HVR-L3 sequence comprising the amino acid sequence of QQYLYHPAT (SEQ ID NO: 25). In some embodiments, the anti-PD-L1 antagonist antibody comprises: (a) a heavy chain variable (VH) domain comprising an amino acid sequence having at least 95% sequence identity to the amino acid sequence of SEQ ID NO: 26; (b) a light chain variable (VL) domain comprising an amino acid sequence having at least 95% sequence identity to the amino acid sequence of SEQ ID NO: 27; or (c) a VH domain as in (a) and a VL domain as in (b). In some embodiments, the anti-PD-L1 antagonist antibody comprises: a VH domain comprising the amino acid sequence of SEQ ID NO: 26 and a VL domain comprising the amino acid sequence of SEQ ID NO: 27.

In some embodiments of any of the eleventh, twelfth, and thirteenth aspects, the anti-PD-L1 antagonist antibody is a monoclonal antibody. In some embodiments, the PD-1 axis binding antagonist (e.g., anti-PD-L1 antagonist antibody) is a humanized antibody (e.g., a monoclonal humanized antibody).

In some embodiments of any of the eleventh, twelfth, and thirteenth aspects, the PD-1 axis binding antagonist (e.g., anti-PD-L1 antagonist antibody) is a full-length antibody.

In some embodiments of any of the eleventh, twelfth, and thirteenth aspects, the PD-1 axis binding antagonist (e.g., anti-PD-L1 antagonist antibody) is an antibody fragment that binds PD-L1 or PD-1 selected from the group consisting of Fab, Fab', Fab'-SH, Fv, single chain variable fragment (scFv), and $(Fab')_2$ fragments.

In some embodiments of any of the eleventh, twelfth, and thirteenth aspects, the PD-1 axis binding antagonist (e.g., anti-PD-L1 antagonist antibody) is an IgG class antibody. In some embodiments, the IgG class antibody is an IgG1 subclass antibody.

In some embodiments of any of the eleventh, twelfth, and thirteenth aspects, the anti-TIGIT antagonist antibody is to be administered to the subject at a fixed dose of about 600 mg of every three weeks and the PD-1 axis binding antagonist (e.g., anti-PD-L1 antagonist antibody) is to be administered to the subject at a fixed dose of about 1200 mg every three weeks. In some embodiments of any of the eleventh, twelfth, and thirteenth aspects, the anti-TIGIT antagonist antibody is to be administered to the subject at a fixed dose of about 420 mg of every two weeks and the PD-1 axis binding antagonist (e.g., anti-PD-L1 antagonist antibody) is to be administered to the subject at a fixed dose of about 840 mg every two weeks. In some embodiments of any of the eleventh, twelfth, and thirteenth aspects, the anti-TIGIT antagonist antibody is to be administered to the subject at a fixed dose of about 840 mg of every two weeks and the PD-1 axis binding antagonist (e.g., anti-PD-L1 antagonist antibody) is to be administered to the subject at a fixed dose of about 1680 mg every three weeks.

In some embodiments of any of the eleventh, twelfth, and thirteenth aspects, the length of each of the one or more dosing cycles is 21 days.

In some embodiments of any of the eleventh, twelfth, and thirteenth aspects, the anti-TIGIT antagonist antibody and PD-1 axis binding antagonist (e.g., anti-PD-L1 antagonist antibody) are to be administered to the subject on about Day 1 of each of the one or more dosing cycles.

In some embodiments of any of the eleventh, twelfth, and thirteenth aspects, the anti-TIGIT antagonist antibody is to be administered to the subject before the PD-1 axis binding antagonist (e.g., anti-PD-L1 antagonist antibody). In some embodiments, a first observation period is to follow administration of the anti-TIGIT antagonist antibody and second observation period is to follow administration of the PD-1 axis binding antagonist (e.g., anti-PD-L1 antagonist antibody). In some embodiments, the first observation period and the second observation period are each between about 30 minutes to about 60 minutes in length.

In some embodiments of any of the eleventh, twelfth, and thirteenth aspects, the PD-1 axis binding antagonist (e.g., anti-PD-L1 antagonist antibody) is to be administered to the subject before the anti-TIGIT antagonist antibody. In some embodiments, a first observation period is to follow administration of the PD-1 axis binding antagonist (e.g., anti-PD-L1 antagonist antibody) and second observation period is to follow administration of the anti-TIGIT antagonist antibody. In some embodiments, the first observation period and the second observation period are each between about 30 minutes to about 60 minutes in length.

In some embodiments of the eleventh, the anti-TIGIT antagonist antibody is to be administered to the subject simultaneously with the PD-1 axis binding antagonist (e.g., anti-PD-L1 antagonist antibody).

In some embodiments of any of the eleventh, twelfth, and thirteenth aspects, the anti-TIGIT antagonist antibody and PD-1 axis binding antagonist (e.g., anti-PD-L1 antagonist antibody) are to be administered to the subject intravenously. In some embodiments, the anti-TIGIT antagonist antibody is to be administered to the subject by intravenous infusion over 60 ± 10 minutes. In some embodiments, the PD-1 axis binding antagonist (e.g., anti-PD-L1 antagonist antibody) is to be administered to the subject by intravenous infusion over 60 ± 15 minutes.

In some embodiments of any of the eleventh, twelfth, and thirteenth aspects, the anti-TIGIT antagonist antibody and PD-1 axis binding antagonist (e.g., anti-PD-L1 antagonist antibody) are to be administered to the subject subcutaneously.

In some embodiments of any of the eleventh, twelfth, and thirteenth aspects, the PD-L1-positive tumor cell fraction has been determined by an immunohistochemical (IHC) assay. In some embodiments, the IHC assay uses anti-PD-L1 antibody SP263, 22C3, SP142, or 28-8. In some embodiments, the PD-L1-positive tumor cell fraction is determined by positive staining with an anti-PD-L1 antibody (e.g., SP263, 22C3, SP142, or 28-8). In some embodiments, the PD-L1-positive tumor cell fraction is greater than, or equal to, 50%, as determined by positive staining with the anti-PD-L1 antibody SP263 (e.g., wherein the PD-L1-positive tumor cell fraction is calculated using the Ventana SP263 IHC assay). In some embodiments, the PD-L1-positive tumor cell fraction is greater than, or equal to, 50%, as determined by positive staining with the anti-PD-L1 antibody 22C3 (e.g., wherein the PD-L1-positive tumor cell fraction is calculated using the pharmDx 22C3 IHC assay). In some embodiments, the PD-L1-positive tumor cell fraction is greater than, or equal to, 30%, as determined by positive staining with the anti-PD-L1 antibody SP142 (e.g., wherein the PD-L1-positive tumor cell fraction is calculated using the Ventana SP142 IHC assay). In some embodiments, the PD-L1-positive tumor cell fraction is greater than, or equal to, 50%, as determined by positive staining with the anti-PD-L1 antibody 28-8.

In some embodiments of any of the eleventh, twelfth, and thirteenth aspects, the IHC assay uses anti-PD-L1 antibody SP263. In some embodiments, the IHC assay uses anti-PD-L1 antibody 22C3.

In some embodiments of any of the eleventh, twelfth, and thirteenth aspects, a tumor sample obtained from the subject has been determined to have a detectable nucleic acid expression level of PD-L1. In some embodiments, the detectable nucleic acid expression level of PD-L1 has been determined by RNA-seq, RT-qPCR, qPCR, multiplex qPCR or RT-qPCR, microarray analysis, SAGE, MassARRAY technique, ISH, or a combination thereof.

In some embodiments of any of the eleventh, twelfth, and thirteenth aspects, the lung cancer is a non-small cell lung cancer (NSCLC).

In some embodiments of any of the ninth, tenth, eleventh, twelfth, and thirteenth aspects, the NSCLC is a squamous NSCLC. In some embodiments, the NSCLC is a non-squamous NSCLC. In some embodiments, the NSCLC is a locally advanced unresectable NSCLC. In some embodiments, the NSCLC is a Stage IIIB NSCLC. In some embodiments, the NSCLC is a recurrent or metastatic NSCLC. In some embodiments, the NSCLC is a Stage IV NSCLC. In some embodiments, the subject has not been previously treated for Stage IV NSCLC.

In some embodiments of any of the ninth, tenth, eleventh, twelfth, and thirteenth aspects, the subject does not have a sensitizing epidermal growth factor receptor *(EGFR)* gene mutation or anaplastic lymphoma kinase *(ALK)* gene rearrangement.

In some embodiments of any of the ninth, tenth, eleventh, twelfth, and thirteenth aspects, the subject does not have a pulmonary lymphoepithelioma-like carcinoma subtype of NSCLC.

In some embodiments of any of the ninth, tenth, eleventh, twelfth, and thirteenth aspects, the subject does not have an active EBV infection or a known or suspected chronic active EBV infection. In some embodiments, the subject is negative for EBV IgM or negative by EBV PCR. In some embodiments, the subject is negative for EBV IgM and negative by EBV PCR. In some embodiments, the subject is positive for EBV IgG or positive for EBNA. In some embodiments, the subject is positive for EBV IgG and positive for EBNA.

In some embodiments of any of the ninth, tenth, eleventh, twelfth, and thirteenth aspects, the subject is negative for EBV IgG or negative for EBNA. In some embodiments, the subject is negative for EBV IgG and negative for EBNA.

In some embodiments of any of the eleventh, twelfth, and thirteenth aspects, the PFS of the subject is increased as compared to a reference PFS time. In some embodiments, the reference PFS time is the median PFS time of a population of subjects who have received a treatment comprising a PD-1 axis binding antagonist (e.g., anti-PD-L1 antagonist antibody) without an anti-TIGIT antagonist antibody.

In a fourteenth aspect, the invention features a use of an anti-TIGIT antagonist antibody and atezolizumab in the manufacture of a medicament for use in a method of treating a subject having a NSCLC, the method comprising administering to the subject one or more dosing cycles of the medicament, wherein the medicament is formulated for administration of the anti-TIGIT antagonist antibody (e.g., at a fixed dose of 600 mg every three weeks, at a fixed dose of 420 mg every two weeks, or at a fixed dose of 840 mg every four weeks) and atezolizumab (e.g., at a fixed dose of 1200 mg every three weeks, at a fixed dose of 840 mg every two weeks, or at a fixed dose of 1680 mg every four weeks), wherein the anti-TIGIT antagonist antibody comprises: a VH domain comprising the amino acid sequence of SEQ ID NO: 17 or 18 and a VL domain comprising the amino acid sequence of SEQ ID NO: 19, and wherein the subject has been determined to have a PD-L1-positive tumor cell fraction of greater than, or equal to, 30% (e.g., greater than, or equal to, 50%), and the treatment results in (a) a CR or a PR and/or (b) an increase in PFS as compared to treatment with atezolizumab without the anti-TIGIT antagonist antibody.

In a fifteenth aspect, the invention features a use of an anti-TIGIT antagonist antibody in the manufacture of a medicament for use in a method of treating a subject having a NSCLC, the method comprising administering to the subject one or more dosing cycles of the medicament and atezolizumab, wherein the medicament is formulated for administration of the anti-TIGIT antagonist antibody (e.g., at a fixed dose of 600 mg every three weeks, at a fixed dose of 420 mg every two weeks, or at a fixed dose of 840 mg every four weeks) and atezolizumab (e.g., at a fixed dose of 1200 mg every three weeks, at a fixed dose of 840 mg every two weeks, or at a fixed dose of 1680 mg every four weeks), wherein the anti-TIGIT antagonist antibody comprises: a VH domain comprising the amino acid sequence of SEQ ID NO: 17 or 18 and a VL domain comprising the amino acid sequence of SEQ ID NO: 19, and wherein the subject has been determined to have a PD-L1-positive tumor cell fraction of greater than, or equal to, 30% (e.g., greater than, or equal to, 50%), and the treatment results in (a) a CR or a PR and/or (b) an increase in PFS as compared to treatment with atezolizumab without the anti-TIGIT antagonist antibody.

In a sixteenth aspect, the invention features a use of atezolizumab in the manufacture of a medicament for use in a method of treating a subject having a NSCLC, the method comprising administering to the subject one or more dosing cycles of the medicament and an anti-TIGIT antagonist antibody, wherein the medicament is formulated for administration of atezolizumab (e.g., at a fixed dose of 1200 mg every three weeks, at a fixed dose of 840 mg every two weeks, or at a fixed dose of 1680 mg every four weeks) and the anti-TIGIT antagonist antibody is to be administered (e.g., at a fixed dose of 600 mg every three weeks, at a fixed dose of 420 mg every two weeks, or at a fixed dose of 840 mg every four weeks) , wherein the anti-TIGIT antagonist antibody comprises: a VH domain comprising the amino acid sequence of SEQ ID NO: 17 or 18 and a VL domain comprising the amino acid sequence of SEQ ID NO: 19, and wherein the subject has been determined to have a PD-L1-positive tumor cell fraction of greater than, or equal to, 30% (e.g., greater than, or equal to, 50%), and the treatment results in (a) a CR or a PR and/or (b) an increase in PFS as compared to treatment with atezolizumab without the anti-TIGIT antagonist antibody.

In a seventeenth aspect, the invention features a use of tiragolumab and atezolizumab in the manufacture of a medicament for use in a method of treating a subject having a NSCLC, the method comprising administering to the subject one or more dosing cycles of the medicament, wherein the medicament is formulated for administration of tiragolumab (e.g., at a fixed dose of 600 mg every three weeks, at a fixed dose of 420 mg every two weeks, or at a fixed dose of 840 mg every four weeks) and atezolizumab (e.g., at a fixed dose of 1200 mg every three weeks, at a fixed dose of 840 mg every two weeks, or at a fixed dose of 1680 mg every four weeks), and wherein the subject has been determined to have a PD-L1-positive tumor cell fraction of greater than, or equal to, 30% (e.g., greater than, or equal to, 50%), and the treatment results in (a) a CR or a PR and/or (b) an increase in PFS as compared to treatment with atezolizumab without tiragolumab.

In an eighteenth aspect, the invention features a use of tiragolumab in the manufacture of a medicament for use in a method of treating a subject having a NSCLC, the method comprising administering to the subject one or more dosing cycles of the medicament and atezolizumab, wherein the medicament is formulated for administration of tiragolumab (e.g., at a fixed dose of 600 mg every three weeks, at a fixed dose of 420 mg every two weeks, or at a fixed dose of 840 mg every four weeks) and atezolizumab is to be administered (e.g., at a fixed dose of 1200 mg every three weeks, at a fixed dose of 840 mg every two weeks, or at a fixed dose of 1680 mg every four weeks), and wherein the subject has been determined to have a PD-L1-positive tumor cell fraction of greater than, or equal to, 30% (e.g., greater than, or equal to, 50%), and the treatment results in (a) a CR or a PR and/or (b) an increase in PFS as compared to treatment with atezolizumab without tiragolumab.

In a nineteenth aspect, the invention features a use of atezolizumab in the manufacture of a medicament for use in a method of treating a subject having a NSCLC, the method comprising administering to the subject one or more dosing cycles of the medicament and tiragolumab, wherein the medicament is formulated for administration of atezolizumab (e.g., at a fixed dose of 1200 mg every three weeks, at a fixed dose of 840 mg every two weeks, or at a fixed dose of 1680 mg every four weeks)and tiragolumab is to be administered (e.g., at a fixed dose of 600 mg every three weeks, at a fixed dose of 420 mg every two weeks, or at a fixed dose of 840 mg every four weeks) , and wherein the subject has been determined to have a PD-L1-positive tumor cell fraction of greater than, or equal to, 30% (e.g., greater than, or equal to, 50%), and the treatment results in (a) a CR or a PR and/or (b) an increase in PFS as compared to treatment with atezolizumab without tiragolumab.

In some embodiments of any of the fourteenth, fifteenth, sixteenth, seventeenth, eighteenth, and nineteenth aspects, the subject does not have a pulmonary lymphoepithelioma-like carcinoma subtype of NSCLC.

In some embodiments of any of the fourteenth, fifteenth, sixteenth, seventeenth, eighteenth, and nineteenth aspects, the subject does not have a sensitizing epidermal growth factor receptor (*EGFR*) gene mutation or anaplastic lymphoma kinase (*ALK*) gene rearrangement.

In some embodiments of any of the fourteenth, fifteenth, sixteenth, seventeenth, eighteenth, and nineteenth aspects, the subject does not have an active EBV infection or a known or suspected chronic active EBV infection. In some embodiments, the subject is negative for EBV IgM or negative by EBV PCR. In some embodiments, the subject is negative for EBV IgM and negative by EBV PCR. In some embodiments, the subject is positive for EBV IgG or positive for EBNA. In some embodiments, the subject is positive for EBV IgG and positive for EBNA.

In some embodiments of any of the fourteenth, fifteenth, sixteenth, seventeenth, eighteenth, and nineteenth aspects, the subject is negative for EBV IgG or negative for EBNA. In some embodiments, the subject is negative for EBV IgG and negative for EBNA.

In some embodiments of any of the preceding aspects, the treatment results in an increase in PFS of at least about 3.1 months (e.g., at least about 4.9 months), as compared to treatment with atezolizumab without tiragolumab.

In some embodiments of any of the preceding aspects, the treatment results in an increase in OS of at least about 5.7 months (e.g., at least about 9 months), as compared to treatment with atezolizumab without tiragolumab.

In a twentieth aspect, the invention features a method for treating a subject having a lung cancer, the method comprising administering to the subject one or more dosing cycles of an anti-TIGIT antagonist antibody and a PD-1 axis binding antagonist, wherein the subject previously received concurrent chemoradiotherapy (cCRT) for lung cancer, and wherein the subject has not had disease progression after the cCRT (e.g., the subject has not had radiographic disease progression after the cCRT). In some embodiments, the subject previously received at least two cycles of the cCRT (e.g., at least three cycles of the cCRT, at least four cycles of the cCRT, at least five cycles of the cCRT, at least six cycles of the cCRT, or more). In some embodiments, the cCRT comprises a platinum-based chemotherapy (e.g., the cCRT comprises a concurrent platinum-based CRT, e.g., a concurrent CRT comprising administration of cisplatin (e.g., cisplatin-etoposide or cisplatin-vinorelbine) or a concurrent CRT comprising administration of carboplatin (e.g., carboplatin-paclitaxel)). In some embodiments, the cCRT comprises a thoracic radiotherapy. In some embodiments, the radiotherapy was administered to the subject at 60-66 Gy in 30-33 fractions. In some embodiments, the cCRT was administered with curative intent. In some embodiments, the cCRT was administered as a consolidation therapy.

In some embodiments, the anti-TIGIT antagonist antibody comprises the following hypervariable regions (HVRs): an HVR-H1 sequence comprising the amino acid sequence of SNSAAWN (SEQ ID NO: 1); an HVR-H2 sequence comprising the amino acid sequence of KTYYRFKWYSDYAVSVKG (SEQ ID NO: 2); an HVR-H3 sequence comprising the amino acid sequence of ESTTYDLLAGPFDY (SEQ ID NO: 3); an HVR-L1 sequence comprising the amino acid sequence of KSSQTVLYSSNNKKYLA (SEQ ID NO: 4); an HVR-L2 sequence comprising the amino acid sequence of WASTRES (SEQ ID NO: 5); and an HVR-L3 sequence comprising the amino acid sequence of QQYYSTPFT (SEQ ID NO: 6). In some embodiments, the anti-TIGIT antagonist antibody further comprises the following light chain variable region framework regions (FRs): an FR-L1 comprising the amino acid sequence of DIVMTQSPDSLAVSLGERATINC (SEQ ID NO: 7); an FR-L2 comprising the amino acid sequence of WYQQKPGQPPNLLIY (SEQ ID NO: 8); an FR-L3 comprising the amino acid sequence of GVPDRFSGSGSGTDFTLTISSLQAEDVAVYYC (SEQ ID NO: 9); and an FR-L4 comprising the amino acid sequence of FGPGTKVEIK (SEQ ID NO: 10). In some embodiments, the anti-TIGIT antagonist antibody further comprises the following heavy chain variable region FRs: an FR-H1 comprising the amino acid sequence of $X_1$VQLQQSGPGLVKPSQTLSLTCAISGDSVS (SEQ ID NO: 11), wherein $X_1$ is Q or E; an FR-H2 comprising the amino acid sequence of WIRQSPSRGLEWLG (SEQ ID NO: 12); an FR-H3 comprising the amino acid sequence of RITINPDTSKNQFSLQLNSVTPEDTAVFYCTR (SEQ ID NO: 13); and an FR-H4 comprising the amino acid sequence of WGQGTLVTVSS (SEQ ID NO: 14). In some embodiments, $X_1$ is Q. In other embodiments, $X_1$ is E. In some embodiments, the anti-TIGIT antagonist antibody comprises: (a) a heavy chain variable (VH) domain comprising an amino acid sequence having at least 95% sequence identity to the amino acid sequence of SEQ ID NO: 17 or 18; (b) a light chain variable (VL) domain comprising an amino acid sequence having at least 95% sequence identity to the amino acid sequence of SEQ ID NO: 19; or (c) a VH domain as in (a) and a VL domain as in (b). In some embodiments, the anti-TIGIT antagonist antibody comprises: a VH domain comprising the amino acid sequence of SEQ ID NO: 17 or 18; and a VL domain comprising the amino acid sequence of SEQ ID NO: 19.

In some embodiments, the anti-TIGIT antagonist antibody is a monoclonal antibody. In some embodiments, the anti-TIGIT antagonist antibody is a human antibody. In some embodiments, the anti-TIGIT antagonist antibody is a full-length antibody. In some embodiments, the anti-TIGIT antagonist antibody is tiragolumab.

In some embodiments, the anti-TIGIT antagonist antibody is an antibody fragment that binds TIGIT selected from the group consisting of Fab, Fab', Fab'-SH, Fv, single chain variable fragment (scFv), and $(Fab')_2$ fragments.

In some embodiments, the anti-TIGIT antagonist antibody is an IgG class antibody (e.g., an IgG1 subclass antibody).

In some embodiments, the PD-1 axis binding antagonist is a PD-L1 binding antagonist or a PD-1 binding antagonist. In some embodiments, the PD-L1 binding antagonist is an anti-PD-L1 antagonist antibody is atezolizumab (MPDL3280A), MSB0010718C, MDX-1105, or MEDI4736. In some embodiments, the anti-PD-L1 antagonist antibody is atezolizumab. In some embodiments, the PD-1 binding antagonist is an anti-PD-1 antagonist antibody. In some embodiments, the anti-PD-1 antagonist antibody is nivolumab (MDX-1106), pembrolizumab (MK-3475). In some embodiments, the PD-1 binding antagonist is AMP-224.

In some embodiments, the anti-PD-L1 antagonist antibody comprises the following HVRs: an HVR-H1 sequence comprising the amino acid sequence of GFTFSDSWIH (SEQ ID NO: 20); an HVR-H2 sequence comprising the amino acid sequence of AWISPYGGSTYYADSVKG (SEQ ID NO: 21); an HVR-H3 sequence comprising the amino acid sequence of RHWPGGFDY (SEQ ID NO: 22); an HVR-L1 sequence comprising the amino acid sequence of RASQDVSTAVA (SEQ ID NO: 23); an HVR-L2 sequence comprising the amino acid sequence of SASFLYS (SEQ ID NO: 24); and an HVR-L3 sequence comprising the amino acid sequence of QQYLYHPAT (SEQ ID NO: 25). In some embodiments, the anti-PD-L1 antagonist antibody comprises: (a) a heavy chain variable (VH) domain comprising an amino acid sequence having at least 95% sequence identity to the amino acid sequence of SEQ ID NO: 26; (b) a light chain variable (VL) domain comprising an amino acid sequence having at least 95% sequence identity to the amino acid sequence of SEQ ID NO: 27; or (c) a VH domain as in (a) and a VL domain as in (b). In some embodiments, the anti-PD-L1 antagonist antibody comprises: a VH domain comprising the amino acid sequence of SEQ ID NO: 26; and a VL domain comprising the amino acid sequence of SEQ ID NO: 27.

In some embodiments, the PD-1 axis binding antagonist is a monoclonal antibody. In some embodiments, the PD-1 axis binding antagonist is a humanized antibody. In some embodiments, the PD-1 axis binding antagonist is a full-length antibody.

In some embodiments, the PD-1 axis binding antagonist is an antibody fragment that binds PD-L1 selected from the group consisting of Fab, Fab', Fab'-SH, Fv, single chain variable fragment (scFv), and $(Fab')_2$ fragments. In some embodiments, the PD-1 axis binding antagonist is an IgG class antibody. In some embodiments, the IgG class antibody is an IgG1 subclass antibody.

In some embodiments, the method comprises administering to the subject the anti-TIGIT antagonist antibody at a fixed dose of between about 30 mg to about 1200 mg every three weeks, e.g., at a fixed dose of between about 30 mg to about 600 mg every three weeks, e.g., at a fixed dose of about 600 mg every three weeks. In some embodiments, the method comprises administering to the subject the PD-1 axis binding antagonist at a fixed dose of between about 80 mg to about 1600 mg every three weeks, e.g., at a fixed dose of about 1200 mg every three weeks. In some embodiments, the method comprises administering to the subject the anti-TIGIT antagonist antibody at a fixed dose of about 600 mg every three weeks and the PD-1 axis binding antagonist at a fixed dose of about 1200 mg every three weeks. In some embodiments, the length of each of the one or more dosing cycles is 21 days. In some embodiments, the method comprises administering to the subject the anti-TIGIT antagonist antibody and the PD-1 axis binding antagonist on about Day 1 of each of the one or more dosing cycles.

In some embodiments, the method comprises administering to the subject the anti-TIGIT antagonist antibody at a fixed dose of between about 300 mg to about 800 mg every two weeks, e.g., at a fixed dose of between about 400 mg to about 500 mg every two weeks, e.g., at a fixed dose of about 420 mg every two weeks. In some embodiments, the method comprises administering to the subject the PD-1 axis binding antagonist at a fixed dose of between about 200 mg to about 1200 mg every two weeks, e.g., at a fixed dose of about 840 mg every two weeks. In some embodiments, the method comprises administering to the subject the anti-TIGIT antagonist antibody at a fixed dose of about 420 mg every two weeks and the PD-1 axis binding antagonist at a fixed dose of about 840 mg every two weeks. In some embodiments, the length of each of the one or more dosing cycles is 28 days. In some embodiments, the method comprises administering to the subject the anti-TIGIT antagonist antibody and the PD-1 axis binding antagonist on about Days 1 and 15 of each of the one or more dosing cycles.

In some embodiments, the method comprises administering to the subject the anti-TIGIT antagonist antibody at a fixed dose of between about 700 mg to about 1000 mg every four weeks, e.g., at a fixed dose of between about 800 mg to about 900 mg every four weeks, e.g., at a fixed dose of about 840 mg every four weeks. In some embodiments, the method comprises administering to the subject the PD-1 axis binding antagonist at a fixed dose of between about 400 mg to about 2000 mg every four weeks, e.g., at a fixed dose of about 1680 mg every four weeks. In some embodiments, the method comprises administering to the subject the anti-TIGIT antagonist antibody at a fixed dose of about 840 mg every four weeks and the PD-1 axis binding antagonist at a fixed dose of about 1680 mg every four weeks. In some embodiments, the length of each of the one or more dosing cycles is 28 days. In some embodiments, the method comprises administering to the subject the anti-TIGIT antagonist antibody and the PD-1 axis binding antagonist on about Day 1 of each of the one or more dosing cycles.

In some embodiments, the method comprises administering to the subject the PD-1 axis binding antagonist before the anti-TIGIT antagonist antibody. In some embodiments, the method comprises a first observation period following administration of the PD-1 axis binding antagonist and second observation period following administration of the anti-TIGIT antagonist antibody. In some embodiments, the first observation period and the second observation period are each between about 30 minutes to about 60 minutes in length.

In some embodiments, the method comprises administering to the subject the anti-TIGIT antagonist antibody before the PD-1 axis binding antagonist. In some embodiments, the method comprises a first observation period following administration of the anti-TIGIT antagonist antibody and second observation period following administration of the PD-1 axis binding antagonist. In some embodiments, the first observation period and the second observation period are each between about 30 minutes to about 60 minutes in length.

In some embodiments, the method comprises administering to the subject the anti-TIGIT antagonist antibody and the PD-1 axis binding antagonist simultaneously.

In some embodiments, the method comprises administering to the subject the anti-TIGIT antagonist antibody and PD-1 axis binding antagonist intravenously, e.g., by intravenous infusion over 60 ± 10 minutes. In some embodiments, the method comprises administering to the subject the PD-1 axis binding antagonist by intravenous infusion over 60 ± 15 minutes.

In some embodiments, the method comprises administering to the subject the anti-TIGIT antagonist antibody and PD-1 axis binding antagonist subcutaneously.

In some embodiments, a PD-L1-positive tumor cell fraction of the subject is determined. In some embodiments, the PD-L1-positive tumor cell fraction is determined by positive staining with an anti-PD-L1 antibody, wherein the anti-PD-L1 antibody is SP263, 22C3, SP142, or 28-8. In some embodiments, the staining is part of an IHC assay. In some embodiments, the PD-L1-positive tumor cell fraction is greater than or equal to 1% tumor cell (TC), as determined by positive staining with an anti-PD-L1 antibody SP263 or 22C3. In some embodiments, the PD-L1-positive tumor cell fraction is less than 1% TC (e.g., from 0% to 1% TC, e.g., PD-L1-negative), as determined by positive staining with an anti-PD-L1 antibody SP263 or 22C3. In some embodiments, the PD-L1 expression is calculated using the Ventana SP263 IHC assay. In some embodiments, the PD-L1 expression is calculated using the pharmDx 22C3 IHC assay.

In some embodiments, a detectable nucleic acid expression level of PD-L1 has been determined by RNA-seq, RT-qPCR, qPCR, multiplex qPCR or RT-qPCR, microarray analysis, SAGE, MassARRAY technique, ISH, or a combination thereof.

In some embodiments, the lung cancer is a non-small cell lung cancer (NSCLC). In some embodiments, the NSCLC is a squamous NSCLC. In other embodiments, the NSCLC is a non-squamous NSCLC. In some embodiments, the NSCLC is a locally advanced unresectable NSCLC (e.g., a locally advanced unresectable NSCLC having a PD-L1-positive tumor cell fraction less than 1% TC or a locally advanced unresectable NSCLC having a PD-L1-positive tumor cell fraction greater than, or equal to, 1% TC). In some embodiments, the NSCLC is a Stage III NSCLC (e.g., Stage IIIA NSCLC, Stage IIIB NSCLC, or Stage IIIC NSCLC), e.g., a Stage III NSCLC having a PD-L1-positive tumor cell fraction less than 1% TC (e.g., a Stage IIIA NSCLC having a PD-L1-positive tumor cell fraction less than 1% TC, a Stage IIIB NSCLC having a PD-L1-positive tumor cell fraction less than 1% TC, or a Stage IIIC NSCLC having a PD-L1-positive tumor cell fraction less than 1% TC) or a Stage III NSCLC having a PD-L1-positive tumor cell fraction greater than, or equal to, 1% TC (e.g., a Stage IIIA NSCLC having a PD-L1-positive tumor cell fraction greater than, or equal to, 1% TC, a Stage IIIB NSCLC having a PD-L1-positive tumor cell fraction greater than, or equal to, 1% TC, or a Stage IIIC NSCLC having a PD-L1-positive tumor cell fraction greater than, or equal to, 1% TC). In some embodiments, the NSCLC (e.g., the squamous NSCLC, the non-squamous NSCLC, or the locally advanced unresectable NSCLC) is not a Stage IV NSCLC.

In some embodiments, the subject does not have a sensitizing epidermal growth factor receptor *(EGFR)* gene mutation or anaplastic lymphoma kinase *(ALK)* gene rearrangement. In some embodiments, the subject does not have an active Epstein-Barr virus (EBV) infection or a known or suspected chronic active EBV infection. In some embodiments, the subject is negative for EBV IgM or negative by EBV PCR. In some embodiments, the subject is negative for EBV IgM and negative by EBV PCR. In some embodiments, the subject is positive for EBV IgG or positive for Epstein-Barr nuclear antigen (EBNA). In some embodiments, the subject is positive for EBV IgG and positive for EBNA. In some embodiments, the subject is negative for EBV IgG or negative for EBNA. In some embodiments, the subject is negative for EBV IgG and negative for EBNA.

In some embodiments, the PFS is increased as compared to a reference PFS time, e.g., the median PFS time of a population of subjects who have received a treatment comprising a PD-1 axis binding antagonist (e.g., durvalumab) without an anti-TIGIT antagonist antibody.

In a twenty-first aspect, provided herein is a method for treating a subject having a NSCLC, the method comprising administering to the subject one or more dosing cycles of an anti-TIGIT antagonist antibody and atezolizumab, wherein the anti-TIGIT antagonist antibody comprises: a VH domain comprising the amino acid sequence of SEQ ID NO: 17 or 18; and a VL domain comprising the amino acid sequence of SEQ ID NO: 19, and wherein the subject previously received cCRT for lung cancer, and wherein the subject has not had disease progression after the cCRT, and wherein the treatment results in (a) a CR or a PR and/or (b) an increase in PFS as compared to treatment with durvalumab without the anti-TIGIT antagonist antibody. In some embodiments, the anti-TIGIT antagonist antibody is administered at a fixed dose of 600 mg every three weeks and atezolizumab is administered at a fixed dose of 1200 mg every three weeks. In other embodiments, the anti-TIGIT antagonist antibody is administered at a fixed dose of 420 mg every two weeks and atezolizumab is administered at a fixed dose of 840 mg every two weeks. In other embodiments, the anti-TIGIT antagonist antibody is administered at a fixed dose of 840 mg every four weeks and atezolizumab is administered at a fixed dose of 1680 mg every four weeks.

In some embodiments, the subject previously received at least two cycles of the cCRT. In some embodiments, the cCRT comprises a platinum-based chemotherapy. In some embodiments, the cCRT comprises a thoracic radiotherapy, e.g., a thoracic radiotherapy administered to the subject at 60-66 Gy in 30-33 fractions. In some embodiments, the cCRT was administered with curative intent. In some embodiments, the cCRT was administered as a consolidation therapy.

In a twenty-second aspect, the invention features a method for treating a subject having a NSCLC, the method comprising administering to the subject one or more dosing cycles of tiragolumab and atezolizumab, wherein the subject previously received cCRT for lung cancer, and wherein the subject has not had disease progression after the cCRT, and the treatment results in (a) a CR or a PR and/or (b) an increase in PFS as compared to treatment with durvalumab without tiragolumab. In some embodiments, tiragolumab is administered at a fixed dose of 600 mg every three weeks and atezolizumab is administered at a fixed dose of 1200 mg every three weeks. In other embodiments, tiragolumab is administered at a fixed dose of 420 mg every two weeks and atezolizumab is administered at a fixed dose of 840 mg every two weeks. In other embodiments, tiragolumab is administered at a fixed dose of 840 mg every four weeks and atezolizumab is administered at a fixed dose of 1680 mg every four weeks.

In some embodiments, the subject previously received at least two cycles of the cCRT. In some embodiments, the cCRT comprises a platinum-based chemotherapy. In some embodiments, the cCRT comprises a thoracic radiotherapy, e.g., a thoracic radiotherapy administered to the subject at 60-66 Gy in 30-33 fractions. In some embodiments, the cCRT was administered with curative intent. In some embodiments, the cCRT was administered as a consolidation therapy.

In a twenty-third aspect, provided herein is an anti-TIGIT antagonist antibody and an anti-PD-L1 antagonist antibody for use in a method of treating a subject having a lung cancer, wherein the method is according to any one of the preceding aspects.

In a twenty-fourth aspect, the invention features a use of an anti-TIGIT antagonist antibody in the manufacture of a medicament for treating a subject having a lung cancer in combination with an anti-PD-L1 antagonist antibody, wherein the treatment is according to the method of any one of the preceding aspects. In some embodiments, the anti-TIGIT antagonist antibody and the anti-PD-L1 antagonist antibody are formulated separately. In other embodiments, the anti-TIGIT antagonist antibody and the anti-PD-L1 antagonist antibody are formulated together.

In a twenty-fifth aspect, the invention features a use of an anti-PD-L1 antagonist antibody in the manufacture of a medicament for treating a subject having a lung cancer in combination with an anti-TIGIT antagonist antibody, wherein the treatment is according to the method of any one of the preceding aspects. In some embodiments, the anti-TIGIT antagonist antibody and the anti-PD-L1 antagonist antibody are formulated separately. In other embodiments, the anti-TIGIT antagonist antibody and the anti-PD-L1 antagonist antibody are formulated together.

In some embodiments of any of the preceding aspects, the subject is a human (e.g., an adult patient).

## BRIEF DESCRIPTION OF THE DRAWINGS

**FIG. 1** is a schematic diagram of the study design showing the parameters for the selection of subjects, randomization into treatment arms, and treatment endpoints.

**FIG. 2** is a table showing minor imbalances in sex, race, and ECOG in baseline demographics divided by TPS (TPS ≥ 50% and TPS 1-49%) at the interim analysis timepoint.

**FIG. 3** is a table showing differences in treatment outcomes and study discontinuations across the PD-L1 TPS ≥ 50% and PD-L1 TPS 1-49% populations and monotherapy and combination therapy arms at the interim analysis timepoint.

**FIG. 4** is a table showing the difference in best overall response (BOR) observed in the primary population (PD-L1 TPS ≥ 1%), the PD-L1 TPS ≥ 50% population, and the PD-L1 TPS 1-49% population receiving either an atezolizumab monotherapy or a combination therapy of tiragolumab and atezolizumab at the interim analysis timepoint.

**FIG. 5** is a series of tables showing an improved BOR in squamous cell cancer patients in the intent-to-treat (ITT) population at the interim analysis timepoint.

**FIG. 6** is a table and accompanying graph showing the relative frequency and type of adverse events recorded for patients receiving either an atezolizumab monotherapy or a combination therapy of tiragolumab and atezolizumab at the interim analysis timepoint. Adverse events with an asterisk were observed at higher frequency in the combination therapy arm than the monotherapy arm.

**FIG. 7** is a table showing observed treatment-related and immune-related adverse events (AEs) were imbalanced between the treatment arms due to rash and IRR at the interim analysis timepoint.

**FIGS. 8A and 8B** are a pair of tables showing subgroup analysis of the objective response rate (ORR) at the primary endpoint analysis timepoint.

**FIG. 9A** is a table showing the difference in ORR observed in the primary population (PD-L1 TPS ≥ 1%) receiving either an atezolizumab monotherapy or a combination therapy of tiragolumab and atezolizumab at the primary endpoint analysis timepoint.

**FIG. 9B** is a pair of tables showing the difference in ORR observed in the PD-L1 TPS ≥ 50% population and the PD-L1 TPS 1-49% population receiving either an atezolizumab monotherapy or a combination therapy of tiragolumab and atezolizumab at the primary endpoint analysis timepoint.

**FIGS. 10A and 10B** are a pair of tables showing subgroup analysis of progression-free survival (PFS) at the primary endpoint analysis timepoint.

**FIG. 11A** is a graph and accompanying table showing the difference in PFS observed in the primary population (PD-L1 TPS ≥ 1%) receiving either an atezolizumab monotherapy or a combination therapy of tiragolumab and atezolizumab at the primary endpoint analysis timepoint.

**FIG. 11B** is a graph and accompanying table showing the difference in PFS observed in the PD-L1 TPS ≥ 50% population receiving either an atezolizumab monotherapy or a combination therapy of tiragolumab and atezolizumab at the primary endpoint analysis timepoint.

**FIG. 11C** is a graph and accompanying table showing the difference in PFS observed in the PD-L1 TPS 1-49% population receiving either an atezolizumab monotherapy or a combination therapy of tiragolumab and atezolizumab at the primary endpoint analysis timepoint.

**FIGS. 12A and 12B** are a pair of tables showing subgroup analysis of overall survival (OS) at the primary endpoint analysis timepoint.

**FIG. 13A** is a graph and accompanying table showing the difference in OS observed in the primary population (PD-L1 TPS ≥ 1%) receiving either an atezolizumab monotherapy or a combination therapy of tiragolumab and atezolizumab at the primary endpoint analysis timepoint.

**FIG. 13B** is a graph and accompanying table showing the difference in OS observed in the PD-L1 TPS ≥ 50% population receiving either an atezolizumab monotherapy or a combination therapy of tiragolumab and atezolizumab at the primary endpoint analysis timepoint.

**FIG. 13C** is a graph and accompanying table showing the difference in OS observed in the PD-L1 TPS 1-49% population receiving either an atezolizumab monotherapy or a combination therapy of tiragolumab and atezolizumab at the primary endpoint analysis timepoint.

**FIGS. 14A-14D** are a series of waterfall plots showing the best percent change from baseline for the PD-L1 TPS ≥ 50% population and the PD-L1 TPS 1-49% population receiving either an atezolizumab monotherapy or a combination therapy of tiragolumab and atezolizumab at the primary endpoint analysis timepoint.

**FIGS. 15A-15D** are a series of graphs showing the percent change in sum of the longest diameters (SLD) of target lesions for the PD-L1 TPS ≥ 50% population and the PD-L1 TPS 1-49% population receiving either an atezolizumab monotherapy or a combination therapy of tiragolumab and atezolizumab at the primary endpoint analysis timepoint.

**FIG. 16** is a schematic diagram of the study design showing the parameters for the selection of subjects, randomization into treatment arms, and treatment endpoints. 1L = first-line; ALK = anaplastic lymphoma kinase (gene); ECOG = Eastern Cooperative Oncology Group; EGFR = epidermal growth factor receptor gene; IHC = immunohistochemistry; NSCLC = non-small cell lung cancer; PD-L1= programmed death-ligand 1; PS = Performance Status; Q3W = every 3 weeks; RECIST v1.1 = Response Evaluation Criteria in Solid Tumors, Version 1.1; TPS = tumor proportion score.

**FIG. 17** is a schematic diagram of a phase III study design showing the parameters for the selection of subjects, randomization into treatment arms, and treatment endpoints. ALK = anaplastic lymphoma kinase (gene); atezo = atezolizumab; durva = durvalumab; ECOG= Eastern Cooperative Oncology Group; EGFR= epidermal growth factor receptor (gene); iDMC= independent Data Monitoring Committee; NSCLC= non-small cell lung cancer; OS= overall survival; PD-L1= programmed death-ligand 1; PFS= progression-free survival; pos= positivity; PS = Performance Status; R = randomization; tira= tiragolumab.

**FIG. 18** is a schematic diagram of the dosing schedule for experimental and comparator arms of a phase III study. D = day; Q2W = every two weeks; Q4W = every four weeks.

## DETAILED DESCRIPTION OF THE INVENTION

## I. GENERAL TECHNIQUES

The techniques and procedures described or referenced herein are generally well understood and commonly employed using conventional methodology by those skilled in the art, such as, for example, the widely utilized methodologies described in Sambrook et al., Molecular Cloning: A Laboratory Manual 3d edition (2001) Cold Spring Harbor Laboratory Press, Cold Spring Harbor, N.Y.; Current Protocols in Molecular Biology (F.M. Ausubel, et al. eds., (2003)); the series Methods in Enzymology (Academic Press, Inc.): PCR 2: A Practical Approach (M.J. MacPherson, B.D. Hames and G.R. Taylor eds. (1995)), Harlow and Lane, eds. (1988) Antibodies, A Laboratory Manual, and Animal Cell Culture (R.I. Freshney, ed. (1987)); Oligonucleotide Synthesis (M.J. Gait, ed., 1984); Methods in Molecular Biology, Humana Press; Cell Biology: A Laboratory Notebook (J.E. Cellis, ed., 1998) Academic Press; Animal Cell Culture (R.I. Freshney), ed., 1987); Introduction to Cell and Tissue Culture (J.P. Mather and P.E. Roberts, 1998) Plenum Press; Cell and Tissue Culture: Laboratory Procedures (A. Doyle, J.B. Griffiths, and D.G. Newell, eds., 1993-8) J. Wiley and Sons; *Handbook of Experimental Immunology* (D.M. Weir and C.C. Blackwell, eds.); Gene Transfer Vectors for Mammalian Cells (J.M. Miller and M.P. Calos, eds., 1987); PCR: The Polymerase Chain Reaction, (Mullis et al., eds., 1994); Current Protocols in Immunology (J.E. Coligan et al., eds., 1991); Short Protocols in Molecular Biology (Wiley and Sons, 1999); Immunobiology (C.A. Janeway and P. Travers, 1997); Antibodies (P. Finch, 1997); Antibodies: A Practical Approach (D. Catty., ed., IRL Press, 1988-1989); Monoclonal Antibodies: A Practical Approach (P. Shepherd and C. Dean, eds., Oxford University Press, 2000); Using Antibodies: A Laboratory Manual (E. Harlow and D. Lane (Cold Spring Harbor Laboratory Press, 1999); The Antibodies (M. Zanetti and J. D. Capra, eds., Harwood Academic Publishers, 1995); and Cancer: Principles and Practice of Oncology (V.T. DeVita et al., eds., J.B. Lippincott Company, 1993).

## II. DEFINITIONS

It is to be understood that aspects and embodiments of the invention described herein include "comprising," "consisting," and "consisting essentially of' aspects and embodiments. As used herein, the singular form "a," "an," and "the" includes plural references unless indicated otherwise.

The term "about" as used herein refers to the usual error range for the respective value readily known to the skilled person in this technical field. Reference to "about" a value or parameter herein includes (and describes) embodiments that are directed to that value or parameter per se. For example, description referring to "about X" includes description of "X."

The "amount," "level," or "expression level," used herein interchangeably, of a biomarker is a detectable level in a biological sample. "Expression" generally refers to the process by which information (e.g., gene-encoded and/or epigenetic) is converted into the structures present and operating in the cell. Therefore, as used herein, "expression" may refer to transcription into a polynucleotide, translation into a polypeptide, or even polynucleotide and/or polypeptide modifications (e.g., posttranslational modification of a polypeptide). Fragments of the transcribed polynucleotide, the translated polypeptide, or polynucleotide and/or polypeptide modifications (e.g., posttranslational modification of a polypeptide) shall also be regarded as expressed whether they originate from a transcript generated by alternative splicing or a degraded transcript, or from a post-translational processing of the polypeptide, e.g., by proteolysis. "Expressed genes" include those that are transcribed into a polynucleotide as mRNA and then translated into a polypeptide, and also those that are transcribed into RNA but not translated into a polypeptide (for example, transfer and ribosomal RNAs). Expression levels can be measured by methods known to one skilled in the art and also disclosed herein. The expression level or amount of a biomarker (e.g., PD-L1) can be used to identify/characterize a subject having a cancer (e.g., lung cancer, e.g., NSCLC, e.g., squamous or non-squamous NSCLC, e.g., locally advanced unresectable NSCLC (e.g., Stage IIIB NSCLC), or recurrent or metastatic NSCLC (e.g., Stage IV NSCLC)) who may be likely to respond to, or benefit from, a particular therapy (e.g., a therapy comprising one or more dosing cycles of an anti-TIGIT antagonist antibody and a PD-1 axis binding antagonist, e.g., an anti-PD-L1 antagonist antibody).

The presence and/or expression level/amount of various biomarkers described herein in a sample can be analyzed by a number of methodologies, many of which are known in the art and understood by the skilled artisan, including, but not limited to, immunohistochemistry ("IHC"), Western blot analysis, immunoprecipitation, molecular binding assays, ELISA, ELIFA, fluorescence activated cell sorting ("FACS"), MassARRAY, proteomics, quantitative blood based assays (e.g., Serum ELISA), biochemical enzymatic activity assays, in situ hybridization, fluorescence in situ hybridization (FISH), Southern analysis, Northern analysis, whole genome sequencing, massively parallel DNA sequencing (e.g., next-generation sequencing), NANOSTRING®, polymerase chain reaction (PCR) including quantitative real time PCR (qRT-PCR) and other amplification type detection methods, such as, for example, branched DNA, SISBA, TMA and the like, RNA-seq, microarray analysis, gene expression profiling, and/or serial analysis of gene expression ("SAGE"), as well as any one of the wide variety of assays that can be performed by protein, gene, and/or tissue array analysis. Typical protocols for evaluating the status of genes and gene products are found, for example in Ausubel et al., eds., 1995, Current Protocols In Molecular Biology, Units 2 (Northern Blotting), 4 (Southern Blotting), 15 (Immunoblotting) and 18 (PCR Analysis). Multiplexed immunoassays such as those available from Rules Based Medicine or Meso Scale Discovery ("MSD") may also be used.

The term "TIGIT" or "T-cell immunoreceptor with Ig and ITIM domains" as used herein refers to any native TIGIT from any vertebrate source, including mammals such as primates (e.g., humans) and rodents (e.g., mice and rats), unless otherwise indicated. TIGIT is also known in the art as DKFZp667A205, FLJ39873, V-set and immunoglobulin domain-containing protein 9, V-set and transmembrane domain-containing protein 3, VSIG9, VSTM3, and WUCAM. The term encompasses "full-length," unprocessed TIGIT (e.g., full-length human TIGIT having the amino acid sequence of SEQ ID NO: 30), as well as any form of TIGIT that results from processing in the cell (e.g., processed human TIGIT without a signal sequence, having the amino acid sequence of SEQ ID NO: 31). The term also encompasses naturally occurring variants of TIGIT, e.g., splice variants or allelic variants. The amino acid sequence of an exemplary human TIGIT may be found under UniProt Accession Number Q495A1.

The term "PD-L1" or "Programmed Cell Death Ligand 1" refers herein to any native PD-L1from any vertebrate source, including mammals such as primates (e.g., humans) and rodents (e.g., mice and rats), unless otherwise indicated. PD-L1 is also known in the art as CD274 molecule, CD274 antigen, B7 homolog 1, PDCD1 Ligand 1, PDCD1LG1, PDCD1L1, B7H1, PDL1, programmed death ligand 1, B7-H1, and B7-H. The term also encompasses naturally occurring variants of PD-L1, e.g., splice variants, or allelic variants. The amino acid sequence of an exemplary human PD-L1 may be found under UniProt Accession Number Q9NZQ7 (SEQ ID NO: 32).

The term "antagonist" is used in the broadest sense, and includes any molecule that partially or fully blocks, inhibits, or neutralizes a biological activity of a native polypeptide disclosed herein. Suitable antagonist molecules specifically include antagonist antibodies or antibody fragments (e.g., antigen-binding fragments), fragments or amino acid sequence variants of native polypeptides, peptides, antisense oligonucleotides, small organic molecules, etc. Methods for identifying antagonists of a polypeptide may comprise contacting a polypeptide with a candidate antagonist molecule and measuring a detectable change in one or more biological activities normally associated with the polypeptide.

The term "PD-1 axis binding antagonist" refers to a molecule that inhibits the interaction of a PD-1 axis binding partner with either one or more of its binding partner, so as to remove T-cell dysfunction resulting from signaling on the PD-1 signaling axis, with a result being to restore or enhance T-cell function (e.g., proliferation, cytokine production, target cell killing). As used herein, a PD-1 axis binding antagonist includes a PD-1 binding antagonist, a PD-L1 binding antagonist, and a PD-L2 binding antagonist.

The term "PD-L1 binding antagonist" refers to a molecule that decreases, blocks, inhibits, abrogates, or interferes with signal transduction resulting from the interaction of PD-L1 with either one or more of its binding partners, such as PD-1 or B7-1. In some embodiments, a PD-L1 binding antagonist is a molecule that inhibits the binding of PD-L1 to its binding partners. In a specific aspect, the PD-L1 binding antagonist inhibits binding of PD-L1 to PD-1 and/or B7-1. In some embodiments, the PD-L1 binding antagonists include anti-PD-L1 antibodies, antigen-binding fragments thereof, immunoadhesins, fusion proteins, oligopeptides, and other molecules that decrease, block, inhibit, abrogate, or interfere with signal transduction resulting from the interaction of PD-L1 with one or more of its binding partners, such as PD-1 or B7-1. In one embodiment, a PD-L1 binding antagonist reduces the negative co-stimulatory signal mediated by or through cell surface proteins expressed on T lymphocytes mediated signaling through PD-L1 so as to render a dysfunctional T-cell less dysfunctional (e.g., enhancing effector responses to antigen recognition). In some embodiments, a PD-L1 binding antagonist is an anti-PD-L1 antagonist antibody. The term "anti-PD-L1 antagonist antibody" refers to an antibody or antigen-binding fragment or variant thereof that is capable of binding PD-L1 with sufficient affinity such that it substantially or completely inhibits the biological activity of PD-L1 (e.g., abrogates or interferes with signal transduction resulting from the interaction of PD-L1 with either one or more of its binding partners, such as PD-1, B7-1). For example, an anti-PD-L1 antagonist antibody may reduce the negative co-stimulatory signal mediated by or through cell surface proteins expressed on T lymphocytes mediated signaling through PD-L1 so as to render a dysfunctional T-cell less dysfunctional (e.g., enhancing effector responses to antigen recognition). In some embodiments, an anti-PD-L1 antagonist antibody is a molecule that inhibits the binding of PD-L1 to its binding partners. In a specific aspect, the anti-PD-L1 antagonist antibody inhibits binding of PD-L1 to PD-1 and/or B7-1. In one embodiment, the extent of binding of an anti-PD-L1 antagonist antibody to an unrelated, non-PD-L1 protein is less than about 10% of the binding of the antibody to PD-L1 as measured, e.g., by a radioimmunoassay (RIA). In certain embodiments, an anti-PD-L1 antagonist antibody that binds to PD-L1 has a dissociation constant ($K_D$) of $\leq 1\mu M$, $\leq 100$ nM, $\leq 10$ nM, $\leq 1$ nM, $\leq 0.1$ nM, $\leq 0.01$ nM, or $\leq 0.001$ nM (e.g., $10^{-8}$ M or less, e.g. from $10^{-8}$ M to $10^{-13}$ M, e.g., from $10^{-9}$ M to $10^{-13}$ M). In certain embodiments, an anti-PD-L1 antagonist antibody binds to an epitope of PD-L1 that is conserved among PD-L1 from different species. In some embodiments, the anti-PD-L1 antagonist antibody is MPDL3280A (atezolizumab), MDX-1105, MEDI4736 (durvalumab), or MSB0010718C (avelumab). In a specific aspect, an anti-PD-L1 antagonist antibody is atezolizumab, marketed as TECENTRIQ™ with a WHO Drug Information (International Nonproprietary Names for Pharmaceutical Substances), Recommended INN: List 74, Vol. 29, No. 3, 2015 (see page 387). In another aspect, the anti-PD-L1 antagonist antibody is MDX-1105. In another specific aspect, an anti PD-L1 antagonist antibody is MSB0015718C. In still another specific aspect, an anti-PD-L1 antagonist antibody is MEDI4736.

The term "PD-1 binding antagonist" refers to a molecule that decreases, blocks, inhibits, abrogates, or interferes with signal transduction resulting from the interaction of PD-1 with one or more of its binding partners, such as PD-L1, PD-L2. In some embodiments, the PD-1 binding antagonist is a molecule that inhibits the binding of PD-1 to one or more of its binding partners. In a specific aspect, the PD-1 binding antagonist inhibits the binding of PD-1 to PD-L1 and/or PD-L2. For example, PD-1 binding antagonists include anti-PD-1 antibodies, antigen-binding fragments thereof, immunoadhesins, fusion proteins, oligopeptides, and other molecules that decrease, block, inhibit, abrogate, or interfere with signal transduction resulting from the interaction of PD-1 with PD-L1 and/or PD-L2. In one embodiment, a PD-1 binding antagonist reduces the negative co-stimulatory signal mediated by or through cell surface proteins expressed on T lymphocytes mediated signaling through PD-1 so as render a dysfunctional T-cell less dysfunctional (e.g., enhancing effector responses to antigen recognition). In some embodiments, the PD-1 binding antagonist is an anti-PD-1 antagonist antibody.

The term "anti-PD-1 antagonist antibody" refers to an antibody or antigen-binding fragment or variant thereof that is capable of binding PD-1 with sufficient affinity such that it substantially or completely inhibits the biological activity of PD-1 (e.g., abrogates or interferes with signal transduction resulting from the interaction of PD-1 with either one or more of its binding partners, such as PD-L1). For example, an anti-PD-1 antagonist antibody may reduce the negative co-stimulatory signal mediated by or through cell surface proteins expressed on T lymphocytes mediated signaling through PD-1 so as to render a dysfunctional T-cell less dysfunctional (e.g., enhancing effector responses to antigen recognition). In some embodiments, an anti-PD-1 antagonist antibody is a molecule that inhibits the binding of PD-1 to its binding partners. In a specific aspect, the anti-PD-1 antagonist antibody inhibits binding of PD-1 to PD-L1. In one embodiment, the extent of binding of an anti-PD-1 antagonist antibody to an unrelated, non-PD-1 protein is less than about 10% of the binding of the antibody to PD-1 as measured, e.g., by a radioimmunoassay (RIA). In certain embodiments, an anti-PD-1 antagonist antibody that binds to PD-1 has a dissociation constant ($K_D$) of $\leq 1 \mu M$, $\leq 100$ nM, $\leq 10$ nM, $\leq 1$ nM, $\leq 0.1$ nM, $\leq 0.01$ nM, or $\leq 0.001$ nM (e.g., $10^{-8}$ M or less, e.g. from $10^{-8}$ M to $10^{-13}$ M, e.g., from $10^{-9}$ M to $10^{-13}$ M). In certain embodiments, an anti-PD-1 antagonist antibody binds to an epitope of PD-1 that is conserved among PD-1 from different species. In some embodiments, the anti-PD-1 antagonist antibody is nivolumab (MDX-1106) or pembrolizumab (formerly lambrolizumab (MK-3475). In some embodiments, the anti-PD-1 antagonist antibody is MDX-1106 (nivolumab). In some embodiments, the anti-PD-1 antagonist antibody is MK-3475 (pembrolizumab). In some embodiments, the anti-PD-1 antagonist antibody is MED1-0680. In some instances, the anti-PD-1 antagonist antibody is PDR001 (spartalizumab). In some instances, the anti-PD-1 antagonist antibody is REGN2810 (cemiplimab). In some instances, the anti-PD-1 antagonist antibody is BGB-108. In other instances, the anti-PD-1 antagonist antibody is prolgolimab, camrelizumab, sintilimab, tislelizumab, or toripalimab.

Further examples of PD-1 axis binding antagonists include cemiplimab, prolgolimab, camrelizumab, sintilimab, tislelizumab, toripalimab, dostarlimab, retifanlimab, spartalizumab, sasanlimab, penpulimab, CS1003, HLX10, SCT-I10A, SHR-1316, CS1001, envafolimab, TQB2450, ZKAB001, LP-002, zimberelimab, balstilimab, genolimzumab, BI 754091, cetrelimab, YBL-006, BAT1306, HX008, CX-072, IMC-001, KL-A167, budigalimab, AMG 404, CX-188, JTX-4014, 609A, Sym021, LZM009, F520, SG001, APL-502, cosibelimab, lodapolimab, GS-4224, INCB086550, FAZ053, TG-1501, BGB-A333, BCD-135, AK-106, LDP, GR1405, HLX20, MSB2311, MAX-10181, RC98, BION-004, AM0001, CB201, ENUM 244C8, ENUM 388D4, AUNP-012, STI-1110, ADG104, AK-103, LBL-006, hAb21, AVA-004, PDL-GEX, INCB090244, KD036, KY1003, LYN192, MT-6035, VXM10, YBL-007, ABSK041, GB7003, JS-003, and HS-636.

The term "anti-TIGIT antagonist antibody" refers to an antibody or an antigen-binding fragment or variant thereof that is capable of binding TIGIT with sufficient affinity such that it substantially or completely inhibits the biological activity of TIGIT. For example, an anti-TIGIT antagonist antibody may block signaling through PVR, PVRL2, and/or PVRL3 so as to restore a functional response by T-cells (e.g., proliferation, cytokine production, target cell killing) from a dysfunctional state to antigen stimulation. It will be understood by one of ordinary skill in the art that in some instances, an anti-TIGIT antagonist antibody may antagonize one TIGIT activity without affecting another TIGIT activity. For example, an anti-TIGIT antagonist antibody for use in certain of the methods or uses described herein is an anti-TIGIT antagonist antibody that antagonizes TIGIT activity in response to one of PVR interaction, PVRL3 interaction, or PVRL2 interaction, e.g., without affecting or minimally affecting any of the other TIGIT interactions. In one embodiment, the extent of binding of an anti-TIGIT antagonist antibody to an unrelated, non-TIGIT protein is less than about 10% of the binding of the antibody to TIGIT as measured, e.g., by a radioimmunoassay (RIA). In certain embodiments, an anti-TIGIT antagonist antibody that binds to TIGIT has a dissociation constant ($K_D$) of $\leq 1 \mu M$, $\leq 100$ nM, $\leq 10$ nM, $\leq 1$ nM, $\leq 0.1$ nM, $\leq 0.01$ nM, or $\leq 0.001$ nM (e.g., $10^{-8}$ M or less, e.g. from $10^{-8}$ M to $10^{-13}$ M, e.g., from $10^{-9}$ M to $10^{-13}$ M). In certain embodiments, an anti-TIGIT antagonist antibody binds to an epitope of TIGIT that is conserved among TIGIT from different species or an epitope on TIGIT that allows for cross-species reactivity. In one embodiment, the anti-TIGIT antagonist antibody is tiragolumab.

As used herein, "administering" is meant a method of giving a dosage of a compound (e.g., an anti-TIGIT antagonist antibody or a PD-1 axis binding antagonist (e.g., an anti-PD-L1 antagonist antibody)) or a composition (e.g., a pharmaceutical composition, e.g., a pharmaceutical composition including an anti-TIGIT antibody and/or a PD-1 axis binding antagonist (e.g., an anti-PD-L1 antibody)) to a subject. The compounds and/or compositions utilized in the methods described herein can be administered, for example, intravenously (e.g., by intravenous infusion), subcutaneously, intramuscularly, intradermally, percutaneously, intraarterially, intraperitoneally, intralesionally, intracranially, intraarticularly, intraprostatically, intrapleurally, intratracheally, intranasally, intravitreally, intravaginally, intrarectally, topically, intratumorally, peritoneally, subconjunctivally, intravesicularlly, mucosally, intrapericardially, intraumbilically, intraocularly, orally, topically, locally, by inhalation, by injection, by infusion, by continuous infusion, by localized perfusion bathing target cells directly, by catheter, by lavage, in cremes, or in lipid compositions. The method of administration can vary depending on various factors (e.g., the compound or composition being administered and the severity of the condition, disease, or disorder being treated).

A "fixed" or "flat" dose of a therapeutic agent (e.g., an anti-TIGIT antagonist antibody and/or a PD-1 axis binding antagonist (e.g., an anti-PD-L1 antagonist antibody)) herein refers to a dose that is administered to a patient without regard for the weight or body surface area (BSA) of the patient. The fixed or flat dose is therefore not provided as a mg/kg dose or a mg/m$^2$ dose, but rather as an absolute amount of the therapeutic agent (e.g., mg).

As used herein, the term "treatment" or "treating" refers to clinical intervention designed to alter the natural course of the individual or cell being treated during the course of clinical pathology. Desirable effects of treatment include delaying or decreasing the rate of disease progression, ameliorating or palliating the disease state, and remission or improved prognosis. For example, an individual is successfully "treated" if one or more symptoms associated with cancer are mitigated or eliminated, including, but are not limited to, reducing the proliferation of (or destroying) cancerous cells, decreasing symptoms resulting from the disease, increasing the quality of life of those suffering from the disease, decreasing the dose of other medications required to treat the disease, delaying the progression of the disease, and/or prolonging survival of individuals.

As used herein, "disease progression" refers to a worsening of a disease. A "subject has not had disease progression" if the disease has remained stable or improved. In some instances, disease progression is radiographic disease progression, e.g., as defined by growth of existing lesions, new lesions, or recurrence of previously resolved lesions. Disease progression (e.g., radiographic disease progression) can be determined by RECIST v1.1. In some embodiments, disease progression (or lack of disease progression) is confirmed by a confirmatory scan and/or pathology.

As used herein, "in conjunction with" refers to administration of one treatment modality in addition to another treatment modality. As such, "in conjunction with" refers to administration of one treatment modality before, during, or after administration of the other treatment modality to the individual.

A "disorder" or "disease" is any condition that would benefit from treatment including, but not limited to, disorders that are associated with some degree of abnormal cell proliferation, e.g., cancer, e.g., lung cancer, e.g., NSCLC, e.g., squamous or non-squamous NSCLC, e.g., locally advanced unresectable NSCLC (e.g., Stage IIIB NSCLC), or recurrent or metastatic NSCLC (e.g., Stage IV NSCLC)).

The term "dysfunction," in the context of immune dysfunction, refers to a state of reduced immune responsiveness to antigenic stimulation.

The term "dysfunctional," as used herein, also includes refractory or unresponsive to antigen recognition, specifically, impaired capacity to translate antigen recognition into downstream T-cell effector functions, such as proliferation, cytokine production (e.g., gamma interferon) and/or target cell killing.

The terms "cancer" and "cancerous" refer to or describe the physiological condition in mammals that is typically characterized by unregulated cell growth. Examples of cancer include, but are not limited to, carcinoma, lymphoma, blastoma, sarcoma, and leukemia or lymphoid malignancies. More particular examples of such cancers include, but are not limited to, lung cancer, such as non-small cell lung cancer (NSCLC), which includes squamous NSCLC or non-squamous NSCLC, including locally advanced unresectable NSCLC (e.g., Stage IIIB NSCLC), or recurrent or metastatic NSCLC (e.g., Stage IV NSCLC), adenocarcinoma of the lung, or squamous cell cancer (e.g., epithelial squamous cell cancer); esophageal cancer; cancer of the peritoneum; hepatocellular cancer; gastric or stomach cancer, including gastrointestinal cancer and gastrointestinal stromal cancer; pancreatic cancer; glioblastoma; cervical cancer; ovarian cancer; liver cancer; bladder cancer (e.g., urothelial bladder cancer (UBC), muscle invasive bladder cancer (MIBC), and BCG-refractory non-muscle invasive bladder cancer (NMIBC)); cancer of the urinary tract; hepatoma; breast cancer (e.g., HER2+ breast cancer and triple-negative breast cancer (TNBC), which are estrogen receptors (ER-), progesterone receptors (PR-), and HER2 (HER2-) negative); colon cancer; rectal cancer; colorectal cancer; endometrial or uterine carcinoma; salivary gland carcinoma; kidney or renal cancer (e.g., renal cell carcinoma (RCC)); prostate cancer; vulval cancer; thyroid cancer; hepatic carcinoma; anal carcinoma; penile carcinoma; melanoma, including superficial spreading melanoma, lentigo maligna melanoma, acral lentiginous melanomas, and nodular melanomas; multiple myeloma and B-cell lymphoma (including low grade/follicular non-Hodgkin's lymphoma (NHL)); small lymphocytic (SL) NHL; intermediate grade/follicular NHL; intermediate grade diffuse NHL; high grade immunoblastic NHL; high grade lymphoblastic NHL; high grade small non-cleaved cell NHL; bulky disease NHL; mantle cell lymphoma; AIDS-related lymphoma; and Waldenstrom's Macroglobulinemia); chronic lymphocytic leukemia (CLL); acute lymphoblastic leukemia (ALL); acute myologenous leukemia (AML); hairy cell leukemia; chronic myeloblastic leukemia (CML); post-transplant lymphoproliferative disorder (PTLD); and myelodysplastic syndromes (MDS), as well as abnormal vascular proliferation associated with phakomatoses, edema (such as that associated with brain tumors), Meigs' syndrome, brain cancer, head and neck cancer, and associated metastases.

The term "tumor" refers to all neoplastic cell growth and proliferation, whether malignant or benign, and all pre-cancerous and cancerous cells and tissues. The terms "cancer," "cancerous," "cell proliferative disorder," "proliferative disorder," and "tumor" are not mutually exclusive as referred to herein.

"Tumor immunity" refers to the process in which tumors evade immune recognition and clearance. Thus, as a therapeutic concept, tumor immunity is "treated" when such evasion is attenuated, and the tumors are recognized and attacked by the immune system. Examples of tumor recognition include tumor binding, tumor shrinkage, and tumor clearance.

As used herein, "metastasis" is meant the spread of cancer from its primary site to other places in the body. Cancer cells can break away from a primary tumor, penetrate into lymphatic and blood vessels, circulate through the bloodstream, and grow in a distant focus (metastasize) in normal tissues elsewhere in the body. Metastasis can be local or distant. Metastasis is a sequential process, contingent on tumor cells breaking off from the primary tumor, traveling through the bloodstream, and stopping at a distant site. At the new site, the cells establish a blood supply and can grow to form a life-threatening mass. Both stimulatory and inhibitory molecular pathways within the tumor cell regulate this behavior, and interactions between the tumor cell and host cells in the distant site are also significant.

The term "anti-cancer therapy" refers to a therapy useful in treating cancer (e.g., lung cancer, e.g., NSCLC, e.g., squamous or non-squamous NSCLC, e.g., locally advanced unresectable NSCLC (e.g., Stage IIIB NSCLC), or recurrent or metastatic NSCLC (e.g., Stage IV NSCLC)). Examples of anti-cancer therapeutic agents include, but are limited to, e.g., immunomodulatory agents (e.g., an immunomodulatory agent (e.g., an agent that decreases or inhibits one or more immune co-inhibitory receptors (e.g., one or more immune co-inhibitory receptors selected from TIGIT, PD-L1, PD-1, CTLA-4, LAG3, TIM3, BTLA, and/or VISTA), such as a CTLA-4 antagonist, e.g., an anti-CTLA-4 antagonist antibody (e.g., ipilimumab (YERVOY®)), an anti-TIGIT antagonist antibody, or a PD-1 axis binding antagonist (e.g., an anti-PD-L1 antagonist antibody), or an agent that increases or activates one or more immune co-stimulatory receptors (e.g., one or more immune co-stimulatory receptors selected from CD226, OX-40, CD28, CD27, CD137, HVEM, and/or GITR), such as an OX-40 agonist, e.g., an OX-40 agonist antibody), chemotherapeutic agents, growth inhibitory agents, cytotoxic agents, agents used in radiation therapy, anti-angiogenesis agents, apoptotic agents, anti-tubulin agents, and other agents to treat cancer. Combinations thereof are also included in the invention.

The term "cytotoxic agent" as used herein refers to a substance that inhibits or prevents a cellular function and/or causes cell death or destruction. Cytotoxic agents include, but are not limited to, radioactive isotopes (e.g., $At^{211}$, $I^{131}$, $I^{125}$, $Y^{90}$, $Re^{186}$, $Re^{188}$, $Sm^{153}$, $Bi^{212}$, $P^{32}$, $Pb^{212}$ and radioactive isotopes of Lu); chemotherapeutic agents or drugs (e.g., methotrexate, adriamicin, vinca alkaloids (vincristine, vinblastine, etoposide), doxorubicin, melphalan, mitomycin C, chlorambucil, daunorubicin or other intercalating agents); growth inhibitory agents; enzymes and fragments thereof such as nucleolytic enzymes; antibiotics; toxins such as small molecule toxins or enzymatically active toxins of bacterial, fungal, plant or animal origin, including fragments and/or variants thereof; and the various antitumor or anti-cancer agents disclosed below.

"Chemotherapeutic agent" includes chemical compounds useful in the treatment of cancer. Examples of chemotherapeutic agents include erlotinib (TARCEVA®, Genentech/OSI Pharm.), bortezomib (VELCADE®, Millennium Pharm.), disulfiram, epigallocatechin gallate , salinosporamide A, carfilzomib, 17-AAG (geldanamycin), radicicol, lactate dehydrogenase A (LDH-A), fulvestrant (FASLODEX®, AstraZeneca), sunitib (SUTENT®, Pfizer/Sugen), letrozole (FEMARA®, Novartis), imatinib mesylate (GLEEVEC®, Novartis), finasunate (VATALANIB®, Novartis), oxaliplatin (ELOXATIN®, Sanofi), 5-FU (5-fluorouracil), leucovorin, Rapamycin (Sirolimus, RAPAMUNE®, Wyeth), Lapatinib (TYKERB®, GSK572016, Glaxo Smith Kline), Lonafamib (SCH 66336), sorafenib (NEXAVAR®, Bayer Labs), gefitinib (IRESSA®, AstraZeneca), AG1478, alkylating agents such as thiotepa and CYTOXAN® cyclosphosphamide; alkyl sulfonates such as busulfan, improsulfan and piposulfan; aziridines such as benzodopa, carboquone, meturedopa, and uredopa; ethylenimines and methylamelamines including altretamine, triethylenemelamine, triethylenephosphoramide, triethylenethiophosphoramide and trimethylomelamine; acetogenins (especially bullatacin and bullatacinone); a camptothecin (including topotecan and irinotecan); bryostatin; callystatin; CC-1065 (including its adozelesin, carzelesin and bizelesin synthetic analogs); cryptophycins (particularly cryptophycin 1 and cryptophycin 8); adrenocorticosteroids (including prednisone and prednisolone); cyproterone acetate; 5$\alpha$-reductases including finasteride and dutasteride); vorinostat, romidepsin, panobinostat, valproic acid, mocetinostat dolastatin; aldesleukin, talc duocarmycin (including the synthetic analogs, KW-2189 and CB1-TM1); eleutherobin; pancratistatin; a sarcodictyin; spongistatin; nitrogen mustards such as chlorambucil, chlomaphazine, chlorophosphamide, estramustine, ifosfamide, mechlorethamine, mechlorethamine oxide hydrochloride, melphalan, novembichin, phenesterine, prednimustine, trofosfamide, uracil mustard; nitrosoureas such as carmustine, chlorozotocin, fotemustine, lomustine, nimustine, and ranimnustine; antibiotics such as the enediyne antibiotics (e.g., calicheamicin, especially calicheamicin $\gamma$1I and calicheamicin $\omega$1I (Angew Chem. Intl. Ed. Engl. 1994 33:183-186); dynemicin, including dynemicin A; bisphosphonates, such as clodronate; an esperamicin; as well as neocarzinostatin chromophore and related chromoprotein enediyne antibiotic chromophores), aclacinomysins, actinomycin, authramycin, azaserine, bleomycins, cactinomycin, carabicin, caminomycin, carzinophilin, chromomycinis, dactinomycin, daunorubicin, detorubicin, 6-diazo-5-oxo-L-norleucine, ADRIAMYCIN® (doxorubicin), morpholino-doxorubicin, cyanomorpholino-doxorubicin, 2-pyrrolino-doxorubicin and deoxydoxorubicin), epirubicin, esorubicin, idarubicin, marcellomycin, mitomycins such as mitomycin C, mycophenolic acid, nogalamycin, olivomycins, peplomycin, porfiromycin, puromycin, quelamycin, rodorubicin, streptonigrin, streptozocin, tubercidin, ubenimex, zinostatin, zorubicin; anti-metabolites such as methotrexate and 5-fluorouracil (5-FU); folic acid analogs such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine;

androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aldophosphamide glycoside; aminolevulinic acid; eniluracil; amsacrine; bestrabucil; bisantrene; edatraxate; defofamine; demecolcine; diaziquone; elfomithine; elliptinium acetate; an epothilone; etoglucid; gallium nitrate; hydroxyurea; lentinan; lonidainine; maytansinoids such as maytansine and ansamitocins; mitoguazone; mitoxantrone; mopidamnol; nitraerine; pentostatin; phenamet; pirarubicin; losoxantrone; podophyllinic acid; 2-ethylhydrazide; procarbazine; PSK® polysaccharide complex (JHS Natural Products, Eugene, Oreg.); razoxane; rhizoxin; sizofuran; spirogermanium; tenuazonic acid; triaziquone; 2,2',2"-trichlorotriethylamine; trichothecenes (especially T-2 toxin, verracurin A, roridin A and anguidine); urethan; vindesine; dacarbazine; mannomustine; mitobronitol; mitolactol; pipobroman; gacytosine; arabinoside ("Ara-C"); cyclophosphamide; thiotepa; taxoids, e.g., TAXOL (paclitaxel; Bristol-Myers Squibb Oncology, Princeton, N.J.), ABRAXANE® (Cremophor-free), albumin-engineered nanoparticle formulations of paclitaxel (American Pharmaceutical Partners, Schaumberg, III.), and TAXOTERE® (docetaxel, doxetaxel; Sanofi-Aventis); chloranmbucil; GEMZAR® (gemcitabine); 6-thioguanine; mercaptopurine; methotrexate; platinum analogs such as cisplatin and carboplatin; vinblastine; etoposide (VP-16); ifosfamide; mitoxantrone; vincristine; NAVELBINE® (vinorelbine); novantrone; teniposide; edatrexate; daunomycin; aminopterin; capecitabine (XELODA®); ibandronate; CPT-11; topoisomerase inhibitor RFS 2000; difluoromethylornithine (DMFO); retinoids such as retinoic acid; and pharmaceutically acceptable salts, acids and derivatives of any of the above.

Chemotherapeutic agent also includes (i) anti-hormonal agents that act to regulate or inhibit hormone action on tumors such as anti-estrogens and selective estrogen receptor modulators (SERMs), including, for example, tamoxifen (including NOLVADEX®; tamoxifen citrate), raloxifene, droloxifene, iodoxyfene , 4-hydroxytamoxifen, trioxifene, keoxifene, LY117018, onapristone, and FARESTON® (toremifine citrate); (ii) aromatase inhibitors that inhibit the enzyme aromatase, which regulates estrogen production in the adrenal glands, such as, for example, 4(5)-imidazoles, aminoglutethimide, MEGASE® (megestrol acetate), AROMASIN® (exemestane; Pfizer), formestanie, fadrozole, RIVISOR® (vorozole), FEMARA® (letrozole; Novartis), and ARIMIDEX® (anastrozole; AstraZeneca); (iii) anti-androgens such as flutamide, nilutamide, bicalutamide, leuprolide and goserelin; buserelin, tripterelin, medroxyprogesterone acetate, diethylstilbestrol, premarin, fluoxymesterone, all transretionic acid, fenretinide, as well as troxacitabine (a 1,3-dioxolane nucleoside cytosine analog); (iv) protein kinase inhibitors (e.g., an anaplastic lymphoma kinase (Alk) inhibitor, such as AF-802 (also known as CH-5424802 or alectinib)); (v) lipid kinase inhibitors; (vi) antisense oligonucleotides, particularly those which inhibit expression of genes in signaling pathways implicated in aberrant cell proliferation, such as, for example, PKC-alpha, Ralf and H-Ras; (vii) ribozymes such as VEGF expression inhibitors (e.g., ANGIOZYME®) and HER2 expression inhibitors; (viii) vaccines such as gene therapy vaccines, for example, ALLOVECTIN®, LEUVECTIN®, and VAXID®; PROLEUKIN®, rIL-2; a topoisomerase 1 inhibitor such as LURTOTECAN®; ABARELIX® rmRH; and (ix) pharmaceutically acceptable salts, acids and derivatives of any of the above.

Chemotherapeutic agent also includes antibodies such as alemtuzumab (Campath), bevacizumab (AVASTIN®, Genentech); cetuximab (ERBITUX®, Imclone); panitumumab (VECTIBIX®, Amgen), rituximab (RITUXAN®, Genentech/Biogen Idec), pertuzumab (OMNITARG®, 2C4, Genentech), trastuzumab (HERCEPTIN®, Genentech), tositumomab (Bexxar, Corixia), and the antibody drug conjugate, gemtuzumab ozogamicin (MYLOTARG®, Wyeth). Additional humanized monoclonal antibodies with therapeutic potential as agents in combination with the compounds of the invention include: apolizumab, aselizumab, atlizumab, bapineuzumab, bivatuzumab mertansine, cantuzumab mertansine, cedelizumab, certolizumab pegol, cidfusituzumab, cidtuzumab, daclizumab, eculizumab, efalizumab, epratuzumab, erlizumab, felvizumab, fontolizumab, gemtuzumab ozogamicin, inotuzumab ozogamicin, ipilimumab, labetuzumab, lintuzumab, matuzumab, mepolizumab, motavizumab, motovizumab, natalizumab, nimotuzumab, nolovizumab, numavizumab, ocrelizumab, omalizumab, palivizumab, pascolizumab, pecfusituzumab, pectuzumab, pexelizumab, ralivizumab, ranibizumab, reslivizumab, reslizumab, resyvizumab, rovelizumab, ruplizumab, sibrotuzumab, siplizumab, sontuzumab, tacatuzumab tetraxetan, tadocizumab, talizumab, tefibazumab, tocilizumab, toralizumab, tucotuzumab celmoleukin, tucusituzumab, umavizumab, urtoxazumab, ustekinumab, visilizumab, and the anti-interleukin-12 (ABT-874/J695, Wyeth Research and Abbott Laboratories) which is a recombinant exclusively human-sequence, full-length IgG1 $\lambda$ antibody genetically modified to recognize interleukin-12 p40 protein.

Chemotherapeutic agent also includes "EGFR inhibitors," which refers to compounds that bind to or otherwise interact directly with EGFR and prevent or reduce its signaling activity, and is alternatively referred to as an "EGFR antagonist." Examples of such agents include antibodies and small molecules that bind to EGFR. Examples of antibodies which bind to EGFR include MAb 579 (ATCC CRL HB 8506), MAb 455 (ATCC CRL HB8507), MAb 225 (ATCC CRL 8508), MAb 528 (ATCC CRL 8509) (see, US Patent No. 4,943, 533, Mendelsohn et al.) and variants thereof, such as chimerized 225 (C225 or Cetuximab; ERBUTIX®) and reshaped human 225 (H225) (see, WO 96/40210, Imclone Systems Inc.); IMC-11F8, a fully human, EGFR-targeted antibody (Imclone); antibodies that bind type II mutant EGFR (US Patent No. 5,212,290); humanized and chimeric antibodies that bind EGFR as described in US Patent No. 5,891,996; and human antibodies that bind EGFR, such as ABX-EGF or Panitumumab (see WO98/50433, Abgenix/Amgen); EMD 55900 (Stragliotto et al. Eur. J. Cancer 32A:636-640 (1996)); EMD7200 (matuzumab) a humanized EGFR antibody directed against EGFR that competes with both EGF and TGF-alpha for EGFR binding (EMD/Merck); human EGFR antibody, HuMax-EGFR (GenMab); fully human antibodies known as E1.1, E2.4, E2.5, E6.2, E6.4, E2.11, E6. 3 and E7.6. 3 and described in US 6,235,883; MDX-447 (Medarex Inc); and mAb 806 or humanized mAb 806 (Johns et al., J. Biol. Chem. 279(29):30375-30384 (2004)). The anti-EGFR antibody may be conjugated with a cytotoxic agent, thus generating an immunoconjugate (see, e.g., EP659,439A2, Merck Patent GmbH). EGFR antagonists include small molecules such as compounds described in US Patent Nos: 5,616,582, 5,457,105, 5,475,001, 5,654,307, 5,679,683, 6,084,095, 6,265,410, 6,455,534, 6,521,620, 6,596,726, 6,713,484, 5,770,599, 6,140,332, 5,866,572, 6,399,602, 6,344,459, 6,602,863, 6,391,874, 6,344,455, 5,760,041, 6,002,008, and 5,747,498, as well as the following PCT publications: WO98/14451, WO98/50038, WO99/09016, and WO99/24037. Particular small molecule EGFR antagonists include OSI-774 (CP-358774, erlotinib, TARCEVA® Genentech/OSI Pharmaceuticals); PD 183805 (CI 1033, 2-propenamide, N-[4-[(3-chloro-4-fluorophenyl)amino]-7-[3-(4-morpholinyl)propoxy]-6-quinazolinyl]-, dihydrochloride, Pfizer Inc.); ZD1839, gefitinib (IRESSA®) 4-(3'-Chloro-4'-fluoroanilino)-7-methoxy-6-(3-morpholinopropoxy)quinazoline, AstraZeneca); ZM 105180 ((6-amino-4-(3-methylphenyl-amino)-quinazoline, Zeneca); BIBX-1382 (N8-(3-chloro-4-fluoro-phenyl)-N2-(1-methyl-piperidin-4-yl)-pyrimido[5,4-d]pyrimidine-2,8-diamine, Boehringer Ingelheim); PKI-166 ((R)-4-[4-[(1-phenylethyl)amino]-1H-pyrrolo[2,3-d]pyrimidin-6-yl]-phenol); (R)-6-(4-hydroxyphenyl)-4-[(1-phenylethyl)amino]-7H-pyrrolo[2,3-d]pyrimidine); CL-387785 (N-[4-[(3-bromophenyl)amino]-6-quinazolinyl]-2-butynamide); EKB-569 (N-[4-[(3-chloro-4-fluorophenyl)amino]-3-cyano-7-ethoxy-6-quinolinyl]-4-(dimethylamino)-2-butenamide) (Wyeth); AG1478 (Pfizer); AG1571 (SU 5271; Pfizer); dual EGFR/HER2 tyrosine kinase inhibitors such as lapatinib (TYKERB®, GSK572016 or N-[3-chloro-4-[(3 fluorophenyl)methoxy]phenyl]-6[5[[[2methylsulfonyl)ethyl]amino]methyl]-2-furanyl]-4-quinazolinamine).

Chemotherapeutic agents also include "tyrosine kinase inhibitors" including the EGFR-targeted drugs noted in the preceding paragraph; inhibitors of insulin receptor tyrosine kinases, including anaplastic lymphoma kinase (Alk) inhibitors, such as AF-802 (also known as CH-5424802 or alectinib), ASP3026, X396, LDK378, AP26113, crizotinib (XALKORI®), and ceritinib (ZYKADIA®); small molecule HER2 tyrosine kinase inhibitor such as TAK165 available from Takeda; CP-724,714, an oral selective inhibitor of the ErbB2 receptor tyrosine kinase (Pfizer and OSI); dual-HER inhibitors such as EKB-569 (available from Wyeth) which preferentially binds EGFR but inhibits both HER2 and EGFR-overexpressing cells; lapatinib (GSK572016; available from Glaxo-SmithKline), an oral HER2 and EGFR tyrosine kinase inhibitor; PKI-166 (available from Novartis); pan-HER inhibitors such as canertinib (CI-1033; Pharmacia); Raf-1 inhibitors such as antisense agent ISIS-5132 available from ISIS Pharmaceuticals which inhibit Raf-1 signaling; non-HER targeted TK inhibitors such as imatinib mesylate (GLEEVEC®, available from Glaxo SmithKline); multi-targeted tyrosine kinase inhibitors such as sunitinib (SUTENT®, available from Pfizer); VEGF receptor tyrosine kinase inhibitors such as vatalanib (PTK787/ZK222584, available from Novartis/Schering AG); MAPK extracellular regulated kinase I inhibitor CI-1040 (available from Pharmacia); quinazolines, such as PD 153035,4-(3-chloroanilino) quinazoline; pyridopyrimidines; pyrimidopyrimidines; pyrrolopyrimidines, such as CGP 59326, CGP 60261 and CGP 62706; pyrazolopyrimidines, 4-(phenylamino)-7H-pyrrolo[2,3-d] pyrimidines; curcumin (diferuloyl methane, 4,5-bis (4-fluoroanilino)phthalimide); tyrphostines containing nitrothiophene moieties; PD-0183805 (Warner-Lamber); antisense molecules (e.g. those that bind to HER-encoding nucleic acid); quinoxalines (US Patent No. 5,804,396); tryphostins (US Patent No. 5,804,396); ZD6474 (Astra Zeneca); PTK-787 (Novartis/Schering AG); pan-HER inhibitors such as CI-1033 (Pfizer); Affinitac (ISIS 3521; Isis/Lilly); imatinib mesylate (GLEEVEC®); PKI 166 (Novartis); GW2016 (Glaxo SmithKline); CI-1033 (Pfizer); EKB-569 (Wyeth); Semaxinib (Pfizer); ZD6474 (AstraZeneca); PTK-787 (Novartis/Schering AG); INC-1C11 (Imclone), rapamycin (sirolimus, RAPAMUNE®); or as described in any of the following patent publications: US Patent No. 5,804,396; WO 1999/09016 (American Cyanamid); WO 1998/43960 (American Cyanamid); WO 1997/38983 (Warner Lambert); WO 1999/06378 (Warner Lambert); WO 1999/06396 (Warner Lambert); WO 1996/30347 (Pfizer, Inc); WO 1996/33978 (Zeneca); WO 1996/3397 (Zeneca) and WO 1996/33980 (Zeneca).

Chemotherapeutic agents also include dexamethasone, interferons, colchicine, metoprine, cyclosporine, amphotericin, metronidazole, alemtuzumab, alitretinoin, allopurinol, amifostine, arsenic trioxide, asparaginase, BCG live, bevacuzimab, bexarotene, cladribine, clofarabine, darbepoetin alfa, denileukin, dexrazoxane, epoetin alfa, elotinib, filgrastim, histrelin acetate, ibritumomab, interferon alfa-2a, interferon alfa-2b, lenalidomide, levamisole, mesna, methoxsalen, nandrolone, nelarabine, nofetumomab, oprelvekin, palifermin, pamidronate, pegademase, pegaspargase, pegfilgrastim, pemetrexed disodium, plicamycin, porfimer sodium, quinacrine, rasburicase, sargramostim, temozolomide, VM-26, 6-TG, toremifene, tretinoin, ATRA, valrubicin, zoledronate, and zoledronic acid, and pharmaceutically acceptable salts thereof.

Chemotherapeutic agents also include hydrocortisone, hydrocortisone acetate, cortisone acetate, tixocortol pivalate, triamcinolone acetonide, triamcinolone alcohol, mometasone, amcinonide, budesonide, desonide, fluocinonide, fluocinolone acetonide, betamethasone, betamethasone sodium phosphate, dexamethasone, dexamethasone sodium phosphate, fluocortolone, hydrocortisone-17-butyrate, hydrocortisone-17-valerate, aclometasone dipropionate, betamethasone valerate, betamethasone dipropionate, prednicarbate, clobetasone-17-butyrate, clobetasol-17-propionate, fluocortolone caproate, fluocortolone pivalate and fluprednidene acetate; immune selective anti-inflammatory peptides (ImSAIDs) such as phenylalanine-glutamine-glycine (FEG) and its D-isomeric form (feG) (IMULAN BioTherapeutics, LLC); anti-rheumatic drugs such as azathioprine, ciclosporin (cyclosporine A), D-penicillamine, gold salts, hydroxychloroquine, leflunomideminocycline, sulfasalazine, tumor necrosis factor alpha (TNFα) blockers such as etanercept (Enbrel), infliximab (Remicade), adalimumab (Humira), certolizumab pegol (Cimzia), golimumab (Simponi), Interleukin 1 (IL-1) blockers such as anakinra (Kineret), T cell costimulation blockers such as abatacept (Orencia), Interleukin 6 (IL-6) blockers such as tocilizumab (ACTEMERA®); Interleukin 13 (IL-13) blockers such as lebrikizumab; Interferon alpha (IFN) blockers such as Rontalizumab; Beta 7 integrin blockers such as rhuMAb Beta7; IgE pathway blockers such as Anti-M1 prime; Secreted homotrimeric LTa3 and membrane bound heterotrimer LTa1/β2 blockers such as Anti-lymphotoxin alpha (LTa); radioactive isotopes (e.g., At211, 1131, 1125, Y90, Re186, Re188, Sm153, Bi212, P32, Pb212 and radioactive isotopes of Lu); miscellaneous investigational agents such as thioplatin, PS-341, phenylbutyrate, ET-18- OCH3, or farnesyl transferase inhibitors (L-739749, L-744832); polyphenols such as quercetin, resveratrol, piceatannol, epigallocatechine gallate, theaflavins, flavanols, procyanidins, betulinic acid and derivatives thereof; autophagy inhibitors such as chloroquine; delta-9-tetrahydrocannabinol (dronabinol, MARINOL®); beta-lapachone; lapachol; colchicines; betulinic acid; acetylcamptothecin, scopolectin, and 9-aminocamptothecin); podophyllotoxin; tegafur (UFTORAL®); bexarotene (TARGRETIN®); bisphosphonates such as clodronate (for example, BONEFOS® or OSTAC®), etidronate (DIDROCAL®), NE-58095, zoledronic acid/zoledronate (ZOMETA®), alendronate (FOSAMAX®), pamidronate (AREDIA®), tiludronate (SKELID®), or risedronate (ACTONEL®); and epidermal growth factor receptor (EGF-R); vaccines such as THERATOPE® vaccine; perifosine, COX-2 inhibitor (e.g. celecoxib or etoricoxib), proteosome inhibitor (e.g. PS341); CCI-779; tipifarnib (R11577); orafenib, ABT510; Bcl-2 inhibitor such as oblimersen sodium (GENASENSE®); pixantrone; farnesyltransferase inhibitors such as lonafarnib (SCH 6636, SARASARTM); and pharmaceutically acceptable salts, acids or derivatives of any of the above; as well as combinations of two or more of the above such as CHOP, an abbreviation for a combined therapy of cyclophosphamide, doxorubicin, vincristine, and prednisolone; and FOLFOX, an abbreviation for a treatment regimen with oxaliplatin (ELOXATIN™) combined with 5-FU and leucovorin.

Chemotherapeutic agents also include non-steroidal anti-inflammatory drugs with analgesic, antipyretic and anti-inflammatory effects. NSAIDs include non-selective inhibitors of the enzyme cyclooxygenase. Specific examples of NSAIDs include aspirin, propionic acid derivatives such as ibuprofen, fenoprofen, ketoprofen, flurbiprofen, oxaprozin and naproxen, acetic acid derivatives such as indomethacin, sulindac, etodolac, diclofenac, enolic acid derivatives such as piroxicam, meloxicam, tenoxicam, droxicam, lornoxicam and isoxicam, fenamic acid derivatives such as mefenamic acid, meclofenamic acid, flufenamic acid, tolfenamic acid, and COX-2 inhibitors such as celecoxib, etoricoxib, lumiracoxib, parecoxib, rofecoxib, rofecoxib, and valdecoxib. NSAIDs can be indicated for the symptomatic relief of conditions such as rheumatoid arthritis, osteoarthritis, inflammatory arthropathies, ankylosing spondylitis, psoriatic arthritis, Reiter's syndrome, acute gout, dysmenorrhoea, metastatic bone pain, headache and migraine, postoperative pain, mild-to-moderate pain due to inflammation and tissue injury, pyrexia, ileus, and renal colic.

As used herein, the terms "chemoradiotherapy," "chemoradiation therapy," and "CRT" are used interchangeably to refer to a therapy that includes administration of a chemotherapeutic agent (e.g., a platinum-based chemotherapeutic agent) in combination with radiation therapy (RT). CRT can be concurrent CRT or sequential CRT.

The term "concurrent chemoradiotherapy," or "cCRT," is used herein to refer to administration of a chemotherapy and a radiotherapy, wherein at least part of the administration of the chemotherapy overlaps in time with at least part of the administration of the radiotherapy. Accordingly, concurrent chemoradiotherapy (cCRT) includes a chemotherapeutic dosing regimen in which the administration of one or more chemotherapeutic agent(s) continues after discontinuing the administration of a radiotherapy. Alternatively, cCRT includes a radiotherapy in which the administration of the radiotherapy continues after discontinuing the administration of the chemotherapy. Concurrent chemoradiotherapy is distinct from sequential chemoradiotherapy, which refers to administration of a chemotherapy which is initiated after administration of a radiotherapy is discontinued or, alternatively, administration of a radiotherapy which is initiated after administration of a chemotherapy is discontinued. An "effective amount" of a compound, for example, an anti-TIGIT antagonist antibody or PD-1 axis binding antagonist (e.g., anti-PD-L1 antagonist antibody), or a composition (e.g., pharmaceutical composition) thereof, is at least the minimum amount required to achieve the desired therapeutic result, such as a measurable increase in overall survival or progression-free survival of a particular disease or disorder (e.g., cancer, e.g., lung cancer, e.g., NSCLC, e.g., squamous or non-squamous NSCLC, e.g., locally advanced unresectable NSCLC (e.g., Stage IIIB NSCLC), or recurrent or metastatic NSCLC (e.g., Stage IV NSCLC)). An effective amount herein may vary according to factors such as the disease state, age, sex, and weight of the patient, and the ability of the antibody to elicit a desired response in the subject. An effective amount is also one in which any toxic or detrimental effects of the treatment are outweighed by the therapeutically beneficial effects. For prophylactic use, beneficial or desired results include results such as eliminating or reducing the risk, lessening the severity, or delaying the onset of the disease, including biochemical, histological and/or behavioral symptoms of the disease, its complications, and intermediate pathological phenotypes presenting during development of the disease. For therapeutic use, beneficial or desired results include clinical results such as decreasing one or more symptoms resulting from the disease (e.g., reduction or delay in cancer-related pain, symptomatic skeletal-related events (SSE), reduction in symptoms per the European Organization for Research and Treatment of Cancer Quality-of-Life Questionnaire (EORTC QLQ-C30, e.g., fatigue, nausea, vomiting, pain, dyspnea, insomnia, appetite loss, constipation, diarrhea, or general level of physical emotional, cognitive, or social functioning), reduction in pain as measured by, e.g., the 10-point pain severity (measured at its worst) numerical rating scale (NRS), and/or reduction in symptoms associated with lung cancer per the health-related quality of life (HRQoL) questionnaire as assessed by symptoms in lung cancer (SILC) scale (e.g., time to deterioration (TTD) in cough dyspenea and chest pain), increasing the quality of life of those suffering from the disease, decreasing the dose of other medications required to treat the disease, enhancing effect of another medication such as via targeting, delaying the progression of the disease (e.g. progression-free survival or radiographic progression-free survival (rPFS); delay of unequivocal clinical progression (e.g., cancer-related pain progression, symptomatic skeletal-related event, deterioration in Eastern Cooperative Group Oncology Group (ECOG) Performance Status (PS) (e.g., how the disease affects the daily living abilities of the patient), and/or initiation of next systemic anti-cancer therapy), and/or delaying time to lung-specific antigen progression), and/or prolonging survival. In the case of cancer or tumor, an effective amount of the drug may have the effect in reducing the number of cancer cells; reducing the tumor size; inhibiting (i.e., slow to some extent or desirably stop) cancer cell infiltration into peripheral organs; inhibit (i.e., slow to some extent and desirably stop) tumor metastasis; inhibiting to some extent tumor growth; and/or relieving to some extent one or more of the symptoms associated with the disorder. An effective amount can be administered in one or more administrations. For purposes of this invention, an effective amount of drug, compound, or pharmaceutical composition is an amount sufficient to accomplish prophylactic or therapeutic treatment either directly or indirectly. As is understood in the clinical context, an effective amount of a drug, compound, or pharmaceutical composition may or may not be achieved in conjunction with another drug, compound, or pharmaceutical composition. Thus, an "effective amount" may be considered in the context of administering one or more therapeutic agents, and a single agent may be considered to be given in an effective amount if, in conjunction with one or more other agents, a desirable result may be or is achieved.

"Immunogenicity" refers to the ability of a particular substance to provoke an immune response. Tumors are immunogenic and enhancing tumor immunogenicity aids in the clearance of the tumor cells by the immune response. Examples of enhancing tumor immunogenicity include but are not limited to treatment with a TIGIT and/or PD-L1 antagonist (e.g., anti-TIGIT antagonist antibodies and/or anti-PDL-1 antagonist antibodies).

"Individual response" or "response" can be assessed using any endpoint indicating a benefit to the subject, including, without limitation, (1) inhibition, to some extent, of disease progression (e.g., progression of cancer, e.g., lung cancer, e.g., NSCLC, e.g., squamous or non-squamous NSCLC, e.g., locally advanced unresectable NSCLC (e.g., Stage IIIB NSCLC), or recurrent or metastatic NSCLC (e.g., Stage IV NSCLC)), including slowing down and complete arrest; (2) a reduction in tumor size; (3) inhibition (i.e., reduction, slowing down or complete stopping) of cancer cell infiltration into adjacent peripheral organs and/or tissues; (4) inhibition (i.e. reduction, slowing down or complete stopping) of metastasis; (5) relief, to some extent, of one or more symptoms associated with the disease or disorder (e.g., cancer, e.g., lung cancer, e.g., NSCLC, e.g., squamous or non-squamous NSCLC, e.g., locally advanced unresectable NSCLC (e.g., Stage IIIB NSCLC), or recurrent or metastatic NSCLC (e.g., Stage IV NSCLC)); (6) increase or extend in the length of survival, including overall survival and progression-free survival; and/or (9) decreased mortality at a given point of time following treatment.

As used herein, "complete response" or "CR" refers to disappearance of all target lesions.

As used herein, "partial response" or "PR" refers to at least a 30% decrease in the sum of the longest diameters (SLD) of target lesions, taking as reference the baseline SLD.

As used herein, "objective response rate" (ORR) refers to the sum of complete response (CR) rate and partial response (PR) rate.

As used herein, "duration of objective response" (DOR) is defined as the time from the first occurrence of a documented objective response to disease progression, or death from any cause within 30 days of the last dose of a treatment, whichever occurs first.

"Sustained response" refers to the sustained effect on reducing tumor growth after cessation of a treatment. For example, the tumor size may remain to be the same or smaller as compared to the size at the beginning of the administration phase. In some embodiments, the sustained response has a duration at least the same as the treatment duration, at least 1.5x, 2.0x, 2.5x, or 3.0x length of the treatment duration.

As used herein, "survival" refers to the patient remaining alive, and includes overall survival as well as progression-free survival.

As used herein, "overall survival" (OS) refers to the percentage of subjects in a group who are alive after a particular duration of time, e.g., 1 year or 5 years from the time of diagnosis or treatment.

As used herein, "progression-free survival" (PFS) refers to the length of time during and after treatment during which the disease being treated (e.g., cancer, e.g., lung cancer, e.g., NSCLC, e.g., squamous or non-squamous NSCLC, e.g., locally advanced unresectable NSCLC (e.g., Stage IIIB NSCLC), or recurrent or metastatic NSCLC (e.g., Stage IV NSCLC)) does not get worse. Progression-free survival may include the amount of time patients have experienced a complete response or a partial response, as well as the amount of time patients have experienced stable disease.

As used herein, "stable disease" or "SD" refers to neither sufficient shrinkage of target lesions to qualify for PR, nor sufficient increase to qualify for PD, taking as reference the smallest SLD since the treatment started.

As used herein, "progressive disease" or "PD" refers to at least a 20% increase in the SLD of target lesions, taking as reference the smallest SLD recorded since the treatment started or the presence of one or more new lesions.

As used herein, "delaying progression" of a disorder or disease means to defer, hinder, slow, retard, stabilize, and/or postpone development of the disease or disorder (e.g., cancer, e.g., lung cancer, e.g., NSCLC, e.g., squamous or non-squamous NSCLC, e.g., locally advanced unresectable NSCLC (e.g., Stage IIIB NSCLC), or recurrent or metastatic NSCLC (e.g., Stage IV NSCLC)). This delay can be of varying lengths of time, depending on the history of the disease and/or subject being treated. As is evident to one skilled in the art, a sufficient or significant delay can, in effect, encompass prevention, in that the subject does not develop the disease. For example, in a late stage cancer, development of central nervous system (CNS) metastasis, may be delayed.

As used herein, the term "reducing or inhibiting cancer relapse" means to reduce or inhibit tumor or cancer relapse, or tumor or cancer progression.

By "reduce or inhibit" is meant the ability to cause an overall decrease of 20%, 30%, 40%, 50%, 60%, 70%, 75%, 80%, 85%, 90%, 95%, or greater. Reduce or inhibit can refer to the symptoms of the disorder being treated (e.g., cancer, e.g., lung cancer, e.g., NSCLC, e.g., squamous or non-squamous NSCLC, e.g., locally advanced unresectable NSCLC (e.g., Stage IIIB NSCLC), or recurrent or metastatic NSCLC (e.g., Stage IV NSCLC)), the presence or size of metastases, or the size of the primary tumor.

By "extending survival" is meant increasing overall or progression free survival in a treated patient relative to an untreated patient (e.g., relative to a patient not treated with the medicament), or relative to a patient who does not express a biomarker at the designated level, and/or relative to a patient treated with an approved anti-tumor agent. An objective response refers to a measurable response, including complete response (CR) or partial response (PR).

The terms "detecting" and "detection" are used herein in the broadest sense to include both qualitative and quantitative measurements of a target molecule. Detecting includes identifying the mere presence of the target molecule in a sample as well as determining whether the target molecule is present in the sample at detectable levels. Detecting may be direct or indirect.

As used herein, a "PD-L1-positive tumor cell fraction" is the percentage of viable tumor cells showing partial or complete membrane staining (exclusive of cytoplasmic staining) at any intensity relative to all viable tumor cells present in a sample, following staining of the sample in the context of an immunohistochemical (IHC) assay, e.g., an IHC assay staining for PD-L1 using the antibody SP263, 22C3, SP142, or 28-8. Accordingly, a PD-L1-positive tumor cell fraction may be calculated using the PD-L1 IHC SP263 (Ventana) assay, for example, by the formula PD-L1-positive tumor cell fraction = (number of PD-L1-positive tumor cells)/(total number of PD-L1-positive and PD-L1 negative tumor cells), wherein PD-L1 cytoplasmic staining of tumor cells and all non-tumor cells (e.g., tumor-infiltrating immune cells, normal cells, necrotic cells, and debris) are excluded from evaluation and scoring. It will be appreciated that any given diagnostic PD-L1 antibody may correspond with a particular IHC assay protocol and/or scoring terminology that can be used to derive a PD-L1-positive tumor cell fraction. For example, a PD-L1-positive tumor cell fraction can be derived from a tumor cell sample stained with SP263, 22C3, SP142, or 28-8 using OPTIVIEW® detection on Benchmark ULTRA, EnVision Flex on AutostainerLink 48, OPTIVIEW® detection and amplification on Benchmark ULTRA, or EnVision Flex on AutostainerLink 48, respectively. In another example, a PD-L1-positive tumor cell fraction may be calculated using the PD-L1 IHC 22C3 pharmDx assay (Dako) according to the formula above. A skilled artisan will appreciate that the sensitivities can vary between different PD-L1 antibodies used in IHC assays. For example, only about 64% of samples that meet a 1% TC or 25% TC threshold, as defined respectively by staining with 28-8 or 22C3 and SP263, meet the threshold when stained using SP142. Hirsch et al., Journal of Thoracic Oncology 2016, 12(2): 208-222. As used herein, the terms PD-L1-positive tumor cell fraction and "tumor proportion score" (TPS) are used interchangeably.

As used herein, the "Ventana SP263 IHC assay" is conducted according to the Ventana PD-L1 (SP263) Assay package insert (Tucson, AZ: Ventana Medical Systems, Inc.), which is incorporated herein by reference in its entirety.

As used herein, the "Ventana SP142 IHC assay" is conducted according to the Ventana PD-L1 (SP142) Assay package insert (Tucson, AZ: Ventana Medical Systems, Inc.), which is incorporated herein by reference in its entirety.

As used herein, the "pharmDx 22C3 IHC assay" is conducted according to the PD-L1 IHC 22C3 pharmDx package insert (Carpinteria, CA: Dako, Agilent Pathology Solutions), which is incorporated herein by reference in its entirety.

A "tumor-infiltrating immune cell," as used herein, refers to any immune cell present in a tumor or a sample thereof. Tumor-infiltrating immune cells include, but are not limited to, intratumoral immune cells, peritumoral immune cells, other tumor stroma cells (e.g., fibroblasts), or any combination thereof. Such tumor-infiltrating immune cells can be, for example, T lymphocytes (such as CD8+ T lymphocytes and/or CD4+ T lymphocytes), B lymphocytes, or other bone marrow-lineage cells, including granulocytes (e.g., neutrophils, eosinophils, and basophils), monocytes, macrophages, dendritic cells (e.g., interdigitating dendritic cells), histiocytes, and natural killer cells.

The term "biomarker" as used herein refers to an indicator, e.g., predictive, diagnostic, and/or prognostic, which can be detected in a sample. The biomarker may serve as an indicator of a particular subtype of a disease or disorder (e.g., cancer, e.g., lung cancer, e.g., NSCLC, e.g., squamous or non-squamous NSCLC, e.g., locally advanced unresectable NSCLC (e.g., Stage IIIB NSCLC), or recurrent or metastatic NSCLC (e.g., Stage IV NSCLC)) characterized by certain, molecular, pathological, histological, and/or clinical features. In some embodiments, a biomarker is a gene. Biomarkers include, but are not limited to, polypeptides, polynucleotides (e.g., DNA, and/or RNA), polynucleotide copy number alterations (e.g., DNA copy numbers), polypeptide and polynucleotide modifications (e.g., posttranslational modifications), carbohydrates, and/or glycolipid-based molecular markers. In some embodiments, the biomarker is PD-L1.

The term "antibody" includes monoclonal antibodies (including full-length antibodies which have an immunoglobulin Fc region), antibody compositions with polyepitopic specificity, multispecific antibodies (e.g., bispecific antibodies), diabodies, and single-chain molecules, as well as antibody fragments, including antigen-binding fragments, such as Fab, $F(ab')_2$, and Fv. The term "immunoglobulin" (Ig) is used interchangeably with "antibody" herein.

The basic 4-chain antibody unit is a heterotetrameric glycoprotein composed of two identical light (L) chains and two identical heavy (H) chains. An IgM antibody consists of 5 of the basic heterotetramer units along with an additional polypeptide called a J chain, and contains 10 antigen binding sites, while IgA antibodies comprise from 2-5 of the basic 4-chain units which can polymerize to form polyvalent assemblages in combination with the J chain. In the case of IgGs, the 4-chain unit is generally about 150,000 Daltons. Each L chain is linked to an H chain by one covalent disulfide bond, while the two H chains are linked to each other by one or more disulfide bonds depending on the H chain isotype. Each H and L chain also has regularly spaced intrachain disulfide bridges. Each H chain has at the N-terminus, a variable domain ($V_H$) followed by three constant domains ($C_H$) for each of the $\alpha$ and $\gamma$ chains and four $C_H$ domains for $\mu$ and $\varepsilon$ isotypes. Each L chain has at the N-terminus, a variable domain ($V_L$) followed by a constant domain at its other end. The $V_L$ is aligned with the $V_H$ and the $C_L$ is aligned with the first constant domain of the heavy chain ($C_H1$). Particular amino acid residues are believed to form an interface between the light chain and heavy chain variable domains. The pairing of a $V_H$ and $V_L$ together forms a single antigen-binding site. For the structure and properties of the different classes of antibodies, see, e.g., Basic and Clinical Immunology, 8th Edition, Daniel P. Sties, Abba I. Terr and Tristram G. Parsolw (eds), Appleton & Lange, Norwalk, CT, 1994, page 71 and Chapter 6. The L chain from any vertebrate species can be assigned to one of two clearly distinct types, called kappa and lambda, based on the amino acid sequences of their constant domains. Depending on the amino acid sequence of the constant domain of their heavy chains (CH), immunoglobulins can be assigned to different classes or isotypes. There are five classes of immunoglobulins: IgA, IgD, IgE, IgG and IgM, having heavy chains designated $\alpha$, $\delta$, $\varepsilon$, $\gamma$, and $\mu$, respectively. The $\gamma$ and $\alpha$ classes are further divided into subclasses on the basis of relatively minor differences in the CH sequence and function, e.g., humans express the following subclasses: IgG1, IgG2A, IgG2B, IgG3, IgG4, IgA1 and IgA2.

The term "hypervariable region" or "HVR" refers to the regions of an antibody variable domain which are hypervariable in sequence and/or form structurally defined loops. Generally, antibodies comprise six HVRs; three in the VH (H1, H2, H3), and three in the VL (L1, L2, L3). In native antibodies, H3 and L3 display the most diversity of the six HVRs, and H3 in particular is believed to play a unique role in conferring fine specificity to antibodies. See, e.g., Xu et al., Immunity 13:37-45 (2000); Johnson and Wu, in Methods in Molecular Biology 248:1-25 (Lo, ed., Human Press, Totowa, NJ, 2003). Indeed, naturally occurring camelid antibodies consisting of a heavy chain only are functional and stable in the absence of light chain. See, *e.g.,* Hamers-Casterman et al., Nature 363:446-448 (1993); Sheriff et al., Nature Struct. Biol. 3:733-736 (1996).

A number of HVR delineations are in use and are encompassed herein. The Kabat Complementarity Determining Regions (CDRs) are based on sequence variability and are the most commonly used (Kabat et al., Sequences of Proteins of Immunological Interest, 5th Ed. Public Health Service, National Institutes of Health, Bethesda, MD. (1991)). Chothia refers instead to the location of the structural loops (Chothia and Lesk, J. Mol. Biol. 196:901-917 (1987)). The AbM HVRs represent a compromise between the Kabat HVRs and Chothia structural loops, and are used by Oxford Molecular's AbM antibody modeling software. The "contact" HVRs are based on an analysis of the available complex crystal structures. The residues from each of these HVRs are noted below.

| Loop | Kabat | AbM | Chothia | Contact |
|---|---|---|---|---|
| L1 | L24-L34 | L24-L34 | L26-L32 | L30-L36 |
| L2 | L50-L56 | L50-L56 | L50-L52 | L46-L55 |
| L3 | L89-L97 | L89-L97 | L91-L96 | L89-L96 |
| H1 | H31-H35B | H26-H35B | H26-H32 | H30-H35B (Kabat numbering) |
| H1 | H31-H35 | H26-H35 | H26-H32 | H30-H35 (Chothia numbering) |
| H2 | H50-H65 | H50-H58 | H53-H55 | H47-H58 |
| H3 | H95-H102 | H95-H102 | H96-H101 | H93-H101 |

HVRs may comprise "extended HVRs" as follows: 24-36 or 24-34 (L1), 46-56 or 50-56 (L2) and 89-97 or 89-96 (L3) in the VL and 26-35 (H1), 50-65 or 49-65 (H2) and 93-102, 94-102, or 95-102 (H3) in the VH. The variable domain residues are numbered according to Kabat *et al., supra,* for each of these definitions.

The expression "variable-domain residue-numbering as in Kabat" or "amino-acid-position numbering as in Kabat," and variations thereof, refers to the numbering system used for heavy-chain variable domains or light-chain variable domains of the compilation of antibodies in Kabat *et al., supra.* Using this numbering system, the actual linear amino acid sequence may contain fewer or additional amino acids corresponding to a shortening of, or insertion into, a FR or HVR of the variable domain. For example, a heavy-chain variable domain may include a single amino acid insert (residue 52a according to Kabat) after residue 52 of H2 and inserted residues (*e.g.* residues 82a, 82b, and 82c, *etc.* according to Kabat) after heavy-chain FR residue 82. The Kabat numbering of residues may be determined for a given antibody by alignment at regions of homology of the sequence of the antibody with a "standard" Kabat numbered sequence.

The term "variable" refers to the fact that certain segments of the variable domains differ extensively in sequence among antibodies. The V domain mediates antigen binding and defines the specificity of a particular antibody for its particular antigen. However, the variability is not evenly distributed across the entire span of the variable domains. Instead, it is concentrated in three segments called hypervariable regions (HVRs) both in the light-chain and the heavy chain variable domains. The more highly conserved portions of variable domains are called the framework regions (FR). The variable domains of native heavy and light chains each comprise four FR regions, largely adopting a beta-sheet configuration, connected by three HVRs, which form loops connecting, and in some cases forming part of, the beta-sheet structure. The HVRs in each chain are held together in close proximity by the FR regions and, with the HVRs from the other chain, contribute to the formation of the antigen binding site of antibodies (see Kabat et al., Sequences of Immunological Interest, Fifth Edition, National Institute of Health, Bethesda, MD (1991)). The constant domains are not involved directly in the binding of antibody to an antigen, but exhibit various effector functions, such as participation of the antibody in antibody-dependent cellular toxicity.

The "variable region" or "variable domain" of an antibody refers to the amino-terminal domains of the heavy or light chain of the antibody. The variable domains of the heavy chain and light chain may be referred to as "VH" and "VL", respectively. These domains are generally the most variable parts of the antibody (relative to other antibodies of the same class) and contain the antigen binding sites.

"Framework" or "FR" refers to variable domain residues other than hypervariable region (HVR) residues. The FR of a variable domain generally consists of four FR domains: FR1, FR2, FR3, and FR4. Accordingly, the HVR and FR sequences generally appear in the following sequence in VH (or VL): FR1-H1(L1)-FR2-H2(L2)-FR3-H3(L3)-FR4.

The terms "full-length antibody," "intact antibody," and "whole antibody" are used interchangeably to refer to an antibody in its substantially intact form, as opposed to an antibody fragment. Specifically, whole antibodies include those with heavy and light chains including an Fc region. The constant domains may be native sequence constant domains (e.g., human native sequence constant domains) or amino acid sequence variants thereof. In some cases, the intact antibody may have one or more effector functions.

An "antibody fragment" comprises a portion of an intact antibody, preferably the antigen-binding and/or the variable region of the intact antibody. Examples of antibody fragments include Fab, Fab', $F(ab')_2$ and Fv fragments; diabodies; linear antibodies (see U.S. Patent 5,641,870, Example 2; Zapata et al., Protein Eng. 8(10): 1057-1062 [1995]); single-chain antibody molecules and multispecific antibodies formed from antibody fragments. Papain digestion of antibodies produced two identical antigen-binding fragments, called "Fab" fragments, and a residual "Fc" fragment, a designation reflecting the ability to crystallize readily. The Fab fragment consists of an entire L chain along with the variable region domain of the H chain ($V_H$), and the first constant domain of one heavy chain ($C_H1$). Each Fab fragment is monovalent with respect to antigen binding, i.e., it has a single antigen-binding site. Pepsin treatment of an antibody yields a single large $F(ab')_2$ fragment which roughly corresponds to two disulfide linked Fab fragments having different antigen-binding activity and is still capable of cross-linking antigen. Fab' fragments differ from Fab fragments by having a few additional residues at the carboxy terminus of the $C_H1$ domain including one or more cysteines from the antibody hinge region. Fab'-SH is the designation herein for Fab' in which the cysteine residue(s) of the constant domains bear a free thiol group. $F(ab')_2$ antibody fragments originally were produced as pairs of Fab' fragments which have hinge cysteines between them. Other chemical couplings of antibody fragments are also known.

The Fc fragment comprises the carboxy-terminal portions of both H chains held together by disulfides. The effector functions of antibodies are determined by sequences in the Fc region, the region which is also recognized by Fc receptors (FcR) found on certain types of cells.

"Functional fragments" of the antibodies of the invention comprise a portion of an intact antibody, generally including the antigen binding or variable region of the intact antibody or the Fc region of an antibody which retains or has modified FcR binding capability. Examples of antibody fragments include linear antibody, single-chain antibody molecules and multispecific antibodies formed from antibody fragments.

"Fv" is the minimum antibody fragment which contains a complete antigen-recognition and - binding site. This fragment consists of a dimer of one heavy- and one light-chain variable region domain in tight, non-covalent association. From the folding of these two domains emanate six hypervariable loops (3 loops each from the H and L chain) that contribute the amino acid residues for antigen binding and confer antigen binding specificity to the antibody. However, even a single variable domain (or half of an Fv comprising only three HVRs specific for an antigen) has the ability to recognize and bind antigen, although at a lower affinity than the entire binding site.

"Single-chain Fv" also abbreviated as "sFv" or "scFv" are antibody fragments that comprise the $V_H$ and $V_L$ antibody domains connected into a single polypeptide chain. Preferably, the sFv polypeptide further comprises a polypeptide linker between the $V_H$ and $V_L$ domains which enables the sFv to form the desired structure for antigen binding. For a review of the sFv, see Pluckthun in The Pharmacology of Monoclonal Antibodies, vol. 113, Rosenburg and Moore eds., Springer-Verlag, New York, pp. 269-315 (1994).

The term "Fc region" herein is used to define a C-terminal region of an immunoglobulin heavy chain, including native-sequence Fc regions and variant Fc regions. Although the boundaries of the Fc region of an immunoglobulin heavy chain might vary, the human IgG heavy-chain Fc region is usually defined to stretch from an amino acid residue at position Cys226, or from Pro230, to the carboxyl-terminus thereof. The C-terminal lysine (residue 447 according to the EU numbering system) of the Fc region may be removed, for example, during production or purification of the antibody, or by recombinantly engineering the nucleic acid encoding a heavy chain of the antibody. Accordingly, a composition of intact antibodies may comprise antibody populations with all K447 residues removed, antibody populations with no K447 residues removed, and antibody populations having a mixture of antibodies with and without the K447 residue. Suitable native-sequence Fc regions for use in the antibodies of the invention include human IgG1, IgG2 (IgG2A, IgG2B), IgG3 and IgG4. Unless otherwise specified herein, numbering of amino acid residues in the Fc region or constant region is according to the EU numbering system, also called the EU index, as described in Kabat et al., Sequences of Proteins of Immunological Interest, 5th Ed. Public Health Service, National Institutes of Health, Bethesda, MD, 1991.

The term "diabodies" refers to small antibody fragments prepared by constructing sFv fragments (see preceding paragraph) with short linkers (about 5-10) residues) between the $V_H$ and $V_L$ domains such that inter-chain but not intra-chain pairing of the V domains is achieved, thereby resulting in a bivalent fragment, i.e., a fragment having two antigen-binding sites. Bispecific diabodies are heterodimers of two "crossover" sFv fragments in which the $V_H$ and $V_L$ domains of the two antibodies are present on different polypeptide chains. Diabodies are described in greater detail in, for example, EP 404,097; WO 93/11161; Hollinger et al., Proc. Natl. Acad. Sci. USA 90: 6444-6448 (1993).

The monoclonal antibodies herein specifically include "chimeric" antibodies (immunoglobulins) in which a portion of the heavy and/or light chain is identical with or homologous to corresponding sequences in antibodies derived from a particular species or belonging to a particular antibody class or subclass, while the remainder of the chain(s) is(are) identical with or homologous to corresponding sequences in antibodies derived from another species or belonging to another antibody class or subclass, as well as fragments of such antibodies, so long as they exhibit the desired biological activity (U.S. Patent No. 4,816,567; Morrison et al., Proc. Natl. Acad. Sci. USA, 81:6851-6855 (1984)). Chimeric antibodies of interest herein include PRIMATIZED® antibodies wherein the antigen-binding region of the antibody is derived from an antibody produced by, *e.g.,* immunizing macaque monkeys with an antigen of interest. As used herein, "humanized antibody" is used a subset of "chimeric antibodies."

The "class" of an antibody refers to the type of constant domain or constant region possessed by its heavy chain. There are five major classes of antibodies: IgA, IgD, IgE, IgG, and IgM, and several of these may be further divided into subclasses (isotypes), e.g., $IgG_1$, $IgG_2$, $IgG_3$, $IgG_4$, $IgA_1$, and $IgA_2$. The heavy chain constant domains that correspond to the different classes of immunoglobulins are called $\alpha$, $\delta$, $\varepsilon$, $\gamma$, and $\mu$, respectively.

"Affinity" refers to the strength of the sum total of non-covalent interactions between a single binding site of a molecule (e.g., an antibody) and its binding partner (e.g., an antigen, e.g., TIGIT or PD-L1). Unless indicated otherwise, as used herein, "binding affinity" refers to intrinsic binding affinity which reflects a 1:1 interaction between members of a binding pair (e.g., antibody and antigen). The affinity of a molecule X for its partner Y can generally be represented by the dissociation constant ($K_D$). Affinity can be measured by common methods known in the art, including those described herein. Specific illustrative and exemplary embodiments for measuring binding affinity are described in the following.

"Fc receptor" or "FcR" describes a receptor that binds to the Fc region of an antibody. The preferred FcR is a native sequence human FcR. Moreover, a preferred FcR is one which binds an IgG antibody (a gamma receptor) and includes receptors of the FcγRI, FcγRII, and FcγRIII subclasses, including allelic variants and alternatively spliced forms of these receptors, FcγRII receptors include FcyRIIA (an "activating receptor") and FcγRIIB (an "inhibiting receptor"), which have similar amino acid sequences that differ primarily in the cytoplasmic domains thereof. Activating receptor FcyRIIA contains an immunoreceptor tyrosine-based activation motif (ITAM) in its cytoplasmic domain. Inhibiting receptor FcγRIIB contains an immunoreceptor tyrosine-based inhibition motif (ITIM) in its cytoplasmic domain. (see M. Daëron, Annu. Rev. Immunol. 15:203-234 (1997). FcRs are reviewed in Ravetch and Kinet, Annu. Rev. Immunol. 9: 457-92 (1991); Capel et al., Immunomethods 4: 25-34 (1994); and de Haas et al., J. Lab. Clin. Med. 126: 330-41 (1995). Other FcRs, including those to be identified in the future, are encompassed by the term "FcR" herein.

A "human antibody" is an antibody that possesses an amino-acid sequence corresponding to that of an antibody produced by a human and/or has been made using any of the techniques for making human antibodies as disclosed herein. This definition of a human antibody specifically excludes a humanized antibody comprising non-human antigen-binding residues. Human antibodies can be produced using various techniques known in the art, including phage-display libraries. Hoogenboom and Winter, J. Mol. Biol., 227:381 (1991); Marks et al., J. Mol. Biol., 222:581 (1991). Also available for the preparation of human monoclonal antibodies are methods described in Cole et al., Monoclonal Antibodies and Cancer Therapy, Alan R. Liss, p. 77 (1985); Boerner et al., J. Immunol., 147(1):86-95 (1991). See also van Dijk and van de Winkel, Curr. Opin. Pharmacol., 5: 368-74 (2001). Human antibodies can be prepared by administering the antigen to a transgenic animal that has been modified to produce such antibodies in response to antigenic challenge, but whose endogenous loci have been disabled, *e.g.,* immunized xenomice (see, *e.g.,* U.S. Pat. Nos. 6,075,181 and 6,150,584 regarding XENOMOUSE™ technology). See also, for example, Li et al., Proc. Natl. Acad. Sci. USA, 103:3557-3562 (2006) regarding human antibodies generated via a human B-cell hybridoma technology.

"Humanized" forms of non-human (*e.g.,* murine) antibodies are chimeric antibodies that contain minimal sequence derived from non-human immunoglobulin. In one embodiment, a humanized antibody is a human immunoglobulin (recipient antibody) in which residues from an HVR (hereinafter defined) of the recipient are replaced by residues from an HVR of a non-human species (donor antibody) such as mouse, rat, rabbit or non-human primate having the desired specificity, affinity, and/or capacity. In some instances, framework ("FR") residues of the human immunoglobulin are replaced by corresponding non-human residues. Furthermore, humanized antibodies may comprise residues that are not found in the recipient antibody or in the donor antibody. These modifications may be made to further refine antibody performance, such as binding affinity. In general, a humanized antibody will comprise substantially all of at least one, and typically two, variable domains, in which all or substantially all of the hypervariable loops correspond to those of a non-human immunoglobulin sequence, and all or substantially all of the FR regions are those of a human immunoglobulin sequence, although the FR regions may include one or more individual FR residue substitutions that improve antibody performance, such as binding affinity, isomerization, immunogenicity, *etc.* The number of these amino acid substitutions in the FR are typically no more than 6 in the H chain, and in the L chain, no more than 3. The humanized antibody optionally will also comprise at least a portion of an immunoglobulin constant region (Fc), typically that of a human immunoglobulin. For further details, see, *e.g.,* Jones et al., Nature 321 :522-525 (1986); Riechmann et al., Nature 332:323-329 (1988); and Presta, Curr. Op. Struct. Biol. 2:593-596 (1992). See also, for example, Vaswani and Hamilton, Ann. Allergy, Asthma & Immunol. 1:105-115 (1998); Harris, Biochem. Soc. Transactions 23:1035-1038 (1995); Hurle and Gross, Curr. Op. Biotech. 5:428-433 (1994); and U.S. Pat. Nos. 6,982,321 and 7,087,409.

The term an "isolated antibody" when used to describe the various antibodies disclosed herein, means an antibody that has been identified and separated and/or recovered from a cell or cell culture from which it was expressed. Contaminant components of its natural environment are materials that would typically interfere with diagnostic or therapeutic uses for the polypeptide, and can include enzymes, hormones, and other proteinaceous or non-proteinaceous solutes. In some embodiments, an antibody is purified to greater than 95% or 99% purity as determined by, for example, electrophoretic (e.g., SDS-PAGE, isoelectric focusing (IEF), capillary electrophoresis) or chromatographic (e.g., ion exchange or reverse phase HPLC). For a review of methods for assessment of antibody purity, see, e.g., Flatman et al., J. Chromatogr. B 848:79-87 (2007). In preferred embodiments, the antibody will be purified (1) to a degree sufficient to obtain at least 15 residues of N-terminal or internal amino acid sequence by use of a spinning cup sequenator, or (2) to homogeneity by SDS-PAGE under non-reducing or reducing conditions using Coomassie blue or, preferably, silver stain. Isolated antibody includes antibodies *in situ* within recombinant cells, because at least one component of the polypeptide natural environment will not be present. Ordinarily, however, isolated polypeptide will be prepared by at least one purification step.

The term "monoclonal antibody" as used herein refers to an antibody obtained from a population of substantially homogeneous antibodies, i.e., the individual antibodies comprising the population are identical except for possible naturally occurring mutations and/or post-translation modifications (e.g., isomerizations, amidations) that may be present in minor amounts. Monoclonal antibodies are highly specific, being directed against a single antigenic site. In contrast to polyclonal antibody preparations which typically include different antibodies directed against different determinants (epitopes), each monoclonal antibody is directed against a single determinant on the antigen. In addition to their specificity, the monoclonal antibodies are advantageous in that they are synthesized by the hybridoma culture, uncontaminated by other immunoglobulins. The modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by a variety of techniques, including, for example, the hybridoma method (e.g., Kohler and Milstein., Nature, 256:495-97 (1975); Hongo et al., Hybridoma, 14 (3): 253-260 (1995), Harlow et al., Antibodies: A Laboratory Manual, (Cold Spring Harbor Laboratory Press, 2nd ed. 1988); Hammerling et al., in: Monoclonal Antibodies and T-Cell Hybridomas 563-681 (Elsevier, N.Y., 1981)), recombinant DNA methods (see, e.g., U.S. Patent No. 4,816,567), phage-display technologies (see, e.g., Clackson et al., Nature, 352: 624-628 (1991); Marks et al., J. Mol. Biol. 222: 581-597 (1992); Sidhu et al., J. Mol. Biol. 338(2): 299-310 (2004); Lee etal., J. Mol. Biol. 340(5): 1073-1093 (2004); Fellouse, Proc. Natl. Acad. Sci. USA 101(34): 12467-12472 (2004); and Lee et al., J. Immunol. Methods 284(1-2): 119-132 (2004), and technologies for producing human or human-like antibodies in animals that have parts or all of the human immunoglobulin loci or genes encoding human immunoglobulin sequences (see, e.g., WO 1998/24893; WO 1996/34096; WO 1996/33735; WO 1991/10741; Jakobovits etal., Proc. Natl. Acad. Sci. USA 90: 2551 (1993); Jakobovits et al., Nature 362: 255-258 (1993); Bruggemann et al., Year in Immunol. 7:33 (1993); U.S. Patent Nos. 5,545,807; 5,545,806; 5,569,825; 5,625,126; 5,633,425; and 5,661,016; Marks et al., BiolTechnology 10: 779-783 (1992); Lonberg et al., Nature 368: 856-859 (1994); Morrison, Nature 368: 812-813 (1994); Fishwild et al., Nature Biotechnol. 14: 845-851 (1996); Neuberger, Nature Biotechnol. 14: 826 (1996); and Lonberg and Huszar, Intern. Rev. Immunol. 13: 65-93 (1995).

As used herein, the term "binds," "specifically binds to," or is "specific for" refers to measurable and reproducible interactions such as binding between a target and an antibody, which is determinative of the presence of the target in the presence of a heterogeneous population of molecules including biological molecules. For example, an antibody that specifically binds to a target (which can be an epitope) is an antibody that binds this target with greater affinity, avidity, more readily, and/or with greater duration than it binds to other targets. In one embodiment, the extent of binding of an antibody to an unrelated target is less than about 10% of the binding of the antibody to the target as measured, for example, by a radioimmunoassay (RIA). In certain embodiments, an antibody that specifically binds to a target has a dissociation constant ($K_D$) of ≤ 1μM, ≤ 100 nM, ≤ 10 nM, ≤ 1 nM, or ≤ 0.1 nM. In certain embodiments, an antibody specifically binds to an epitope on a protein that is conserved among the protein from different species. In another embodiment, specific binding can include, but does not require exclusive binding. The term as used herein can be exhibited, for example, by a molecule having a $K_D$ for the target of $10^{-4}$ M or lower, alternatively $10^{-5}$ M or lower, alternatively $10^{-6}$ M or lower, alternatively $10^{-7}$ M or lower, alternatively $10^{-8}$ M or lower, alternatively $10^{-9}$ M or lower, alternatively $10^{-10}$ M or lower, alternatively $10^{-11}$ M or lower, alternatively $10^{-12}$ M or lower or a $K_D$ in the range of $10^{-4}$ M to $10^{-6}$ M or $10^{-6}$ M to $10^{-10}$ M or $10^{-7}$ M to $10^{-9}$ M. As will be appreciated by the skilled artisan, affinity and $K_D$ values are inversely related. A high affinity for an antigen is measured by a low $K_D$ value. In one embodiment, the term "specific binding" refers to binding where a molecule binds to a particular polypeptide or epitope on a particular polypeptide without substantially binding to any other polypeptide or polypeptide epitope.

The phrase "substantially reduced" or "substantially different," as used herein, denotes a sufficiently high degree of difference between two numeric values (generally one associated with a molecule and the other associated with a reference/comparator molecule) such that one of skill in the art would consider the difference between the two values to be of statistical significance within the context of the biological characteristic measured by said values (e.g., $K_D$ values). The difference between said two values is, for example, greater than about 10%, greater than about 20%, greater than about 30%, greater than about 40%, and/or greater than about 50% as a function of the value for the reference/comparator molecule.

The term "substantially similar" or "substantially the same," as used herein, denotes a sufficiently high degree of similarity between two numeric values (for example, one associated with an antibody of the invention and the other associated with a reference/comparator antibody), such that one of skill in the art would consider the difference between the two values to be of little or no biological and/or statistical significance within the context of the biological characteristic measured by said values (e.g., $K_D$ values). The difference between said two values is, for example, less than about 50%, less than about 40%, less than about 30%, less than about 20%, and/or less than about 10% as a function of the reference/comparator value.

"Percent (%) amino acid sequence identity" with respect to a reference polypeptide sequence is defined as the percentage of amino acid residues in a candidate sequence that are identical with the amino acid residues in the reference polypeptide sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN or Megalign (DNASTAR) software. Those skilled in the art can determine appropriate parameters for aligning sequences, including any algorithms needed to achieve maximal alignment over the full length of the sequences being compared. For purposes herein, however, % amino acid sequence identity values are generated using the sequence comparison computer program ALIGN-2. The ALIGN-2 sequence comparison computer program was authored by Genentech, Inc., and the source code has been filed with user documentation in the U.S. Copyright Office, Washington D.C., 20559, where it is registered under U.S. Copyright Registration No. TXU510087. The ALIGN-2 program is publicly available from Genentech, Inc., South San Francisco, California, or may be compiled from the source code. The ALIGN-2 program should be compiled for use on a UNIX operating system, including digital UNIX V4.0D. All sequence comparison parameters are set by the ALIGN-2 program and do not vary.

In situations where ALIGN-2 is employed for amino acid sequence comparisons, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows:

100 times the fraction X/Y

where X is the number of amino acid residues scored as identical matches by the sequence alignment program ALIGN-2 in that program's alignment of A and B, and where Y is the total number of amino acid residues in B. It will be appreciated that where the length of amino acid sequence A is not equal to the length of amino acid sequence B, the % amino acid sequence identity of A to B will not equal the % amino acid sequence identity of B to A. Unless specifically stated otherwise, all % amino acid sequence identity values used herein are obtained as described in the immediately preceding paragraph using the ALIGN-2 computer program.

As used herein, "subject" or "individual" is meant a mammal, including, but not limited to, a human or non-human mammal, such as a bovine, equine, canine, ovine, or feline. In some embodiments, the subject is a human. Patients are also subjects herein.

The term "sample," as used herein, refers to a composition that is obtained or derived from a subject and/or individual of interest that contains a cellular and/or other molecular entity that is to be characterized and/or identified, for example based on physical, biochemical, chemical and/or physiological characteristics. For example, the phrase "tumor sample," "disease sample," and variations thereof refers to any sample obtained from a subject of interest that would be expected or is known to contain the cellular and/or molecular entity that is to be characterized. In some embodiments, the sample is a tumor tissue sample (e.g., a lung cancer tumor tissue sample, e.g., an NSCLC tumor tissue sample, e.g., squamous or non-squamous NSCLC tumor tissue sample, e.g., locally advanced unresectable NSCLC tumor tissue sample (e.g., Stage IIIB NSCLC tumor tissue sample), or recurrent or metastatic NSCLC tumor tissue sample (e.g., Stage IV NSCLC tumor tissue sample). Other samples include, but are not limited to, primary or cultured cells or cell lines, cell supernatants, cell lysates, platelets, serum, plasma, vitreous fluid, lymph fluid, synovial fluid, follicular fluid, seminal fluid, amniotic fluid, milk, whole blood, blood-derived cells, urine, cerebro-spinal fluid, saliva, sputum, tears, perspiration, mucus, stool, tumor lysates, and tissue culture medium, tissue extracts such as homogenized tissue, cellular extracts, and combinations thereof.

A "reference sample," "reference cell," "reference tissue," "control sample," "control cell," or "control tissue," as used herein, refers to a sample, cell, tissue, standard, or level that is used for comparison purposes. In one embodiment, a reference sample, reference cell, reference tissue, control sample, control cell, or control tissue is obtained from a healthy and/or non-diseased part of the body (e.g., tissue or cells) of the same subject. For example, healthy and/or non-diseased cells or tissue adjacent to the diseased cells or tissue (e.g., cells or tissue adjacent to a tumor). In another embodiment, a reference sample is obtained from an untreated tissue and/or cell of the body of the same subject. In yet another embodiment, a reference sample, reference cell, reference tissue, control sample, control cell, or control tissue is obtained from a healthy and/or non-diseased part of the body (e.g., tissues or cells) of a subject who is not the subject. In even another embodiment, a reference sample, reference cell, reference tissue, control sample, control cell, or control tissue is obtained from an untreated tissue and/or cell of the body of an individual who is not the subject.

The term "protein," as used herein, refers to any native protein from any vertebrate source, including mammals such as primates (e.g., humans) and rodents (e.g., mice and rats), unless otherwise indicated. The term encompasses "full-length," unprocessed protein as well as any form of the protein that results from processing in the cell. The term also encompasses naturally occurring variants of the protein, e.g., splice variants or allelic variants.

"Polynucleotide" or "nucleic acid," as used interchangeably herein, refers to polymers of nucleotides of any length, and include DNA and RNA. The nucleotides can be deoxyribonucleotides, ribonucleotides, modified nucleotides or bases, and/or their analogs, or any substrate that can be incorporated into a polymer by DNA or RNA polymerase, or by a synthetic reaction. Thus, for instance, polynucleotides as defined herein include, without limitation, single- and double-stranded DNA, DNA including single- and double-stranded regions, single- and double-stranded RNA, and RNA including single- and double-stranded regions, hybrid molecules comprising DNA and RNA that may be single-stranded or, more typically, double-stranded or include single- and double-stranded regions. In addition, the term "polynucleotide" as used herein refers to triple-stranded regions comprising RNA or DNA or both RNA and DNA. The strands in such regions may be from the same molecule or from different molecules. The regions may include all of one or more of the molecules, but more typically involve only a region of some of the molecules. One of the molecules of a triple-helical region often is an oligonucleotide. The terms "polynucleotide" and "nucleic acid" specifically includes mRNA and cDNAs.

A polynucleotide may comprise modified nucleotides, such as methylated nucleotides and their analogs. If present, modification to the nucleotide structure may be imparted before or after assembly of the polymer. The sequence of nucleotides may be interrupted by non-nucleotide components. A polynucleotide may be further modified after synthesis, such as by conjugation with a label. Other types of modifications include, for example, "caps," substitution of one or more of the naturally-occurring nucleotides with an analog, internucleotide modifications such as, for example, those with uncharged linkages (e.g., methyl phosphonates, phosphotriesters, phosphoamidates, carbamates, and the like) and with charged linkages (e.g., phosphorothioates, phosphorodithioates, and the like), those containing pendant moieties, such as, for example, proteins (e.g., nucleases, toxins, antibodies, signal peptides, poly-L-lysine, and the like), those with intercalators (e.g., acridine, psoralen, and the like), those containing chelators (e.g., metals, radioactive metals, boron, oxidative metals, and the like), those containing alkylators, those with modified linkages (e.g., alpha anomeric nucleic acids), as well as unmodified forms of the polynucleotide(s). Further, any of the hydroxyl groups ordinarily present in the sugars may be replaced, for example, by phosphonate groups, phosphate groups, protected by standard protecting groups, or activated to prepare additional linkages to additional nucleotides, or may be conjugated to solid or semi-solid supports. The 5' and 3' terminal OH can be phosphorylated or substituted with amines or organic capping group moieties of from 1 to 20 carbon atoms. Other hydroxyls may also be derivatized to standard protecting groups. Polynucleotides can also contain analogous forms of ribose or deoxyribose sugars that are generally known in the art, including, for example, 2'-O-methyl-, 2'-O-allyl-, 2'-fluoro-, or 2'-azido-ribose, carbocyclic sugar analogs, $\alpha$-anomeric sugars, epimeric sugars such as arabinose, xyloses or lyxoses, pyranose sugars, furanose sugars, sedoheptuloses, acyclic analogs, and abasic nucleoside analogs such as methyl riboside. One or more phosphodiester linkages may be replaced by alternative linking groups. These alternative linking groups include, but are not limited to, embodiments wherein phosphate is replaced by P(O)S ("thioate"), P(S)S ("dithioate"), "(O)$NR_2$ ("amidate"), P(O)R, P(O)OR', CO or $CH_2$ ("formacetal"), in which each R or R' is independently H or substituted or unsubstituted alkyl (1-20 C) optionally containing an ether (-O-) linkage, aryl, alkenyl, cycloalkyl, cycloalkenyl or araldyl. Not all linkages in a polynucleotide need be identical. The preceding description applies to all polynucleotides referred to herein, including RNA and DNA.

"Carriers" as used herein include pharmaceutically acceptable carriers, excipients, or stabilizers that are nontoxic to the cell or mammal being exposed thereto at the dosages and concentrations employed. Often the physiologically acceptable carrier is an aqueous pH buffered solution. Examples of physiologically acceptable carriers include buffers such as phosphate, citrate, and other organic acids; antioxidants including ascorbic acid; low molecular weight (less than about 10 residues) polypeptide; proteins, such as serum albumin, gelatin, or immunoglobulins; hydrophilic polymers such as polyvinylpyrrolidone; amino acids such as glycine, glutamine, asparagine, arginine or lysine; monosaccharides, disaccharides, and other carbohydrates including glucose, mannose, or dextrins; chelating agents such as EDTA; sugar alcohols such as mannitol or sorbitol; salt-forming counterions such as sodium; and/or nonionic surfactants such as TWEEN™, polyethylene glycol (PEG), and PLURONICS™.

The phrase "pharmaceutically acceptable" indicates that the substance or composition must be compatible chemically and/or toxicologically, with the other ingredients comprising a formulation, and/or the mammal being treated therewith.

The term "pharmaceutical formulation" refers to a preparation which is in such form as to permit the biological activity of an active ingredient contained therein to be effective, and which contains no additional components which are unacceptably toxic to a subject to which the formulation would be administered.

## III. THERAPEUTIC METHODS AND USES

Provided herein are methods and uses for treating cancer (e.g., lung cancer, e.g., non-small cell lung cancer (NSCLC), e.g., squamous or non-squamous NSCLC, e.g., locally advanced unresectable NSCLC (e.g., Stage IIIB NSCLC), or recurrent or metastatic NSCLC (e.g., Stage IV NSCLC)) in a subject comprising administering to the subject one or more dosing cycles of an effective amount of an anti-TIGIT antagonist antibody and a PD-1 axis binding antagonist (e.g., an anti-PD-L1 antagonist antibody).

### *Dosing Regimens and Administration*

The therapeutic methods and uses of the invention described herein include, in one aspect, administering to a subject having a cancer (e.g., lung cancer, e.g., non-small cell lung cancer (NSCLC), e.g., squamous or non-squamous NSCLC, e.g., locally advanced unresectable NSCLC (e.g., Stage IIIB NSCLC), or recurrent or metastatic NSCLC (e.g., Stage IV NSCLC)) who has been determined to have a PD-L1 tumor cell fraction of greater than, or equal to, 30% (e.g., greater than, or equal to, 50%) one or more dosing cycles of an effective amount of an anti-TIGIT antagonist antibody (e.g., an anti-TIGIT antagonist antibody as disclosed herein, e.g., tiragolumab) and an effective amount of a PD-1 axis binding antagonist (e.g., an anti-PD-L1 antagonist antibody (e.g., atezolizumab)), wherein the treatment results in a complete response (CR) or a partial response (PR) as compared to treatment with the PD-1 axis binding antagonist without the anti-TIGIT antagonist antibody, thereby treating the subject. In some instances, the anti-TIGIT antagonist antibody (e.g., an anti-TIGIT antagonist antibody as disclosed herein, e.g., tiragolumab) and the PD-1 axis binding antagonist (e.g., an anti-PD-L1 antagonist antibody (e.g., atezolizumab)) are administered every two weeks (e.g., on Days 1 and 15 of each 28-day dosing cycle), every three weeks (e.g., on Day 1 of each 21-day dosing cycle), or every four weeks (e.g., on Day 1 of each 28-day dosing cycle).

In some aspects, the therapeutic methods and uses of the invention described herein include administering to a subject having a cancer (e.g., lung cancer, e.g., non-small cell lung cancer (NSCLC), e.g., squamous or non-squamous NSCLC, e.g., Stage III NSCLC), who has previously received concurrent chemoradiotherapy (cCRT) for lung cancer, and wherein the subject has not had disease progression after the cCRT, one or more dosing cycles of an effective amount of an anti-TIGIT antagonist antibody (e.g., an anti-TIGIT antagonist antibody as disclosed herein, e.g., tiragolumab) and an effective amount of a PD-1 axis binding antagonist (e.g., an anti-PD-L1 antagonist antibody (e.g., atezolizumab)), thereby treating the subject. In some instances, the anti-TIGIT antagonist antibody (e.g., an anti-TIGIT antagonist antibody as disclosed herein, e.g., tiragolumab) and the PD-1 axis binding antagonist (e.g., an anti-PD-L1 antagonist antibody (e.g., atezolizumab)) are administered every two weeks (e.g., on Days 1 and 15 of each 28-day dosing cycle), every three weeks (e.g., on Day 1 of each 21-day dosing cycle), or every four weeks (e.g., on Day 1 of each 28-day dosing cycle).

In some instances, the effective amount of the anti-TIGIT antagonist antibody (e.g., an anti-TIGIT antagonist antibody as disclosed herein, e.g., tiragolumab) is a fixed dose of between about 30 mg to about 1200 mg (e.g., between about 30 mg to about 1100 mg, e.g., between about 60 mg to about 1000 mg, e.g., between about 100 mg to about 900 mg, e.g., between about 200 mg to about 800 mg, e.g., between about 300 mg to about 800 mg, e.g., between about 400 mg to about 800 mg, e.g., between about 400 mg to about 750 mg, e.g., between about 450 mg to about 750 mg, e.g., between about 500 mg to about 700 mg, e.g., between about 550 mg to about 650 mg, e.g., 600 mg ± 10 mg, e.g., 600 ± 6 mg, e.g., 600 ± 5 mg, e.g., 600 ± 3 mg, e.g., 600 ± 1 mg, e.g., 600 ± 0.5 mg, e.g., 600 mg) every three weeks. In some instances, the effective amount of the anti-TIGIT antagonist antibody (e.g., an anti-TIGIT antagonist antibody as disclosed herein, e.g., tiragolumab) is a fixed dose of between about 30 mg to about 600 mg (e.g., between about 50 mg to between 600 mg, e.g., between about 60 mg to about 600 mg, e.g., between about 100 mg to about 600 mg, e.g., between about 200 mg to about 600 mg, e.g., between about 200 mg to about 550 mg, e.g., between about 250 mg to about 500 mg, e.g., between about 300 mg to about 450 mg, e.g., between about 350 mg to about 400 mg, e.g., about 375 mg) every three weeks. In some instances, the effective amount of the anti-TIGIT antagonist antibody (e.g., an anti-TIGIT antagonist antibody as disclosed herein, e.g., tiragolumab) is a fixed dose of about 600 mg every three weeks. In some instances, effective amount of the anti-TIGIT antagonist antibody (e.g., an anti-TIGIT antagonist antibody as disclosed herein, e.g., tiragolumab) is a fixed dose of 600 mg every three weeks. In some instances, the fixed dose of the anti-TIGIT antagonist antibody (e.g., an anti-TIGIT antagonist antibody as disclosed herein, e.g., tiragolumab) administered in a combination therapy (e.g., a combination treatment with a PD-1 axis binding antagonist (e.g., an anti-PD-L1 antagonist antibody, e.g., atezolizumab)) may be reduced as compared to a standard dose of the anti-TIGIT antagonist antibody administered as a monotherapy.

In some instances, the effective amount of the anti-TIGIT antagonist antibody (e.g., an anti-TIGIT antagonist antibody as disclosed herein, e.g., tiragolumab) is a fixed dose of between about 10 mg to about 1000 mg (e.g., between about 20 mg to about 1000 mg, e.g., between about 50 mg to about 900 mg, e.g., between about 100 mg to about 850 mg, e.g., between about 200 mg to about 800 mg, e.g., between about 300 mg to about 600 mg, e.g., between about 400 mg to about 500 mg, e.g., between about 405 mg to about 450 mg, e.g., between about 410 mg to about 430 mg, e.g., about 420 mg) every two weeks (Q2W). In some instances, the effective amount of the anti-TIGIT antagonist antibody (e.g., an anti-TIGIT antagonist antibody as disclosed herein, e.g., tiragolumab) is a fixed dose of about 420 mg every two weeks (e.g., 420 mg ± 10 mg, e.g., 420 ± 6 mg, e.g., 420 ± 5 mg, e.g., 420 ± 3 mg, e.g., 420 ± 1 mg, e.g., 420 ± 0.5 mg, e.g., 420 mg every two weeks).

In some instances, the effective amount of the anti-TIGIT antagonist antibody (e.g., an anti-TIGIT antagonist antibody as disclosed herein, e.g., tiragolumab) is a fixed dose of between about 200 mg to about 2000 mg (e.g., between about 200 mg to about 1600 mg, e.g., between about 250 mg to about 1600 mg, e.g., between about 300 mg to about 1600 mg, e.g., between about 400 mg to about 1500 mg, e.g., between about 500 mg to about 1400 mg, e.g., between about 600 mg to about 1200 mg, e.g., between about 700 mg to about 1100 mg, e.g., between about 800 mg to about 1000 mg, e.g., between about 800 mg to about 900 mg, e.g., about 800, about 810, about 820, about 830, about 840, about 850, about 860, about 870, about 880, about 890, or about 900 mg) every four weeks (Q4W). In some instances, the effective amount of anti-TIGIT antagonist antibody (e.g., an anti-TIGIT antagonist antibody as disclosed herein, e.g., tiragolumab) is a fixed dose of about 840 mg every four weeks (e.g., 840 mg ± 10 mg, e.g., 840 ± 6 mg, e.g., 840 ± 5 mg, e.g., 840 ± 3 mg, e.g., 840 ± 1 mg, e.g., 840 ± 0.5 mg, e.g., 840 mg every four weeks).

In some instances, the effective amount of the PD-1 axis binding antagonist (e.g., anti-PD-L1 antagonist antibody (e.g., atezolizumab)) is a fixed dose of between about 80 mg to about 1600 mg (e.g., between about 100 mg to about 1600 mg, e.g., between about 200 mg to about 1600 mg, e.g., between about 300 mg to about 1600 mg, e.g., between about 400 mg to about 1600 mg, e.g., between about 500 mg to about 1600 mg, e.g., between about 600 mg to about 1600 mg, e.g., between about 700 mg to about 1600 mg, e.g., between about 800 mg to about 1600 mg, e.g., between about 900 mg to about 1500 mg, e.g., between about 1000 mg to about 1400 mg, e.g., between about 1050 mg to about 1350 mg, e.g., between about 1100 mg to about 1300 mg, e.g., between about 1150 mg to about 1250 mg, e.g., between about 1175 mg to about 1225 mg, e.g., between about 1190 mg to about 1210 mg, e.g., 1200 mg ± 5 mg, e.g., 1200 ± 2.5 mg, e.g., 1200 ± 1.0 mg, e.g., 1200 ± 0.5 mg, e.g., 1200 ) every three weeks. In some instances, the effective amount of the PD-1 axis binding antagonist (e.g., anti-PD-L1 antagonist antibody (e.g., atezolizumab)) is a fixed dose of about 1200 mg every three weeks. In some instances, the effective amount of the PD-1 axis binding antagonist (e.g., anti-PD-L1 antagonist antibody (e.g., atezolizumab)) is a fixed dose of 1200 mg every three weeks.

In some embodiments, the PD-1 axis binding antagonist is administered at a dose of about 80 mg to about 2000 mg every two weeks, three weeks, or four weeks (e.g., about 840 mg every two weeks, about 1200 mg every three weeks, or about 1680 mg every four weeks). In some embodiments, the PD-1 axis binding antagonist is administered at a dose of about 1680 mg every four weeks. In some embodiments, the anti-TIGIT antagonist antibody is administered at a dose of about 600 mg every three weeks and the PD-1 axis binding antagonist is administered at a dose of about 1680 mg every four weeks. In some embodiments, the PD-1 axis binding antagonist is administered at a dose of about 1200 mg every three weeks. In some embodiments, the anti-TIGIT antagonist antibody is administered at a dose of about 600 mg every three weeks and the PD-1 axis binding antagonist is administered at a dose of about 1200 mg every three weeks. In some embodiments, the PD-1 axis binding antagonist is administered at a dose of about 840 mg every two weeks. In some embodiments, the anti-TIGIT antagonist antibody is administered at a dose of about 600 mg every three weeks and the PD-1 axis binding antagonist is administered at a dose of about 840 mg every two weeks.

In some instances, the effective amount of the PD-1 axis binding antagonist (e.g., anti-PD-L1 antagonist antibody (e.g., atezolizumab)) is a fixed dose of about 840 mg every two weeks. In some instances, the effective amount of the PD-1 axis binding antagonist (e.g., anti-PD-L1 antagonist antibody (e.g., atezolizumab)) is a fixed dose of about 1200 mg every three weeks. In some instances, the effective amount of the PD-1 axis binding antagonist (e.g., anti-PD-L1 antagonist antibody (e.g., atezolizumab)) is a fixed dose of 1680 mg every four weeks.

In some instances, the effective amount of the PD-1 axis binding antagonist (e.g., anti-PD-L1 antagonist antibody (e.g., atezolizumab)) is a fixed dose of between about 20 mg to about 1600 mg (e.g., between about 40 mg to about 1500 mg, e.g., between about 200 mg to about 1400 mg, e.g., between about 300 mg to about 1400 mg, e.g., between about 400 mg to about 1400 mg, e.g., between about 500 mg to about 1300 mg, e.g., between about 600 mg to about 1200 mg, e.g., between about 700 mg to about 1100 mg, e.g., between about 800 mg to about 1000 mg, e.g., between about 800 mg to about 900 mg, e.g., about 800, about 810, about 820, about 830, about 840, about 850, about 860, about 870, about 880, about 890, or about 900 mg) every two weeks (Q2W). In some instances, the effective amount of the PD-1 axis binding antagonist is atezolizumab at a fixed dose of about 840 mg every two weeks (e.g., 840 mg ± 10 mg, e.g., 840 t 6 mg, e.g., 840 ± 5 mg, e.g., 840 ± 3 mg, e.g., 840 ± 1 mg, e.g., 840 ± 0.5 mg, e.g., 840 mg every two weeks). In some embodiments, the effective amount of the PD-1 axis binding antagonist is avelumab at a fixed dose of about 800 mg every two weeks. In some embodiments, the effective amount of the PD-1 axis binding antagonist is nivolumab at a fixed dose of about 240 mg every two weeks.

In some instances, the effective amount of the PD-1 axis binding antagonist (e.g., anti-PD-L1 antagonist antibody (e.g., atezolizumab)) is a fixed dose of between about 500 mg to about 3000 mg (e.g., between about 500 mg to about 2800 mg, e.g., between about 600 mg to about 2700 mg, e.g., between about 650 mg to about 2600 mg, e.g., between about 700 mg to about 2500 mg, e.g., between about 1000 mg to about 2400 mg, e.g., between about 1100 mg to about 2300 mg, e.g., between about 1200 mg to about 2200 mg, e.g., between about 1300 mg to about 2100 mg, e.g., between about 1400 mg to about 2000 mg, e.g., between about 1500 mg to about 1900 mg, e.g., between about 1600 mg to about 1800 mg, e.g., between about 1620 mg to about 1700 mg, e.g., between about 1640 mg to about 1690 mg, e.g., between about 1660 mg to about 1680 mg, about 1680 mg, e.g., about 1600 mg, about 1610 mg, about 1620 mg, about 1630 mg, about 1640 mg, about 1650 mg, about 1660 mg, about 1670 mg, about 1680 mg, about 1690 mg, or about 1700 mg) every four weeks (Q4W). In some instances, the effective amount of the PD-1 axis binding antagonist (e.g., anti-PD-L1 antagonist antibody (e.g., atezolizumab)) is a fixed dose of 1680 mg every four weeks (e.g., 1680 mg ± 10 mg, e.g., 1680 ± 6 mg, e.g., 1680 ± 5 mg, e.g., 1680 ± 3 mg, e.g., 1680 ± 1 mg, e.g., 1680 ± 0.5 mg, e.g., 1680 mg every four weeks). In some embodiments, the effective amount of the PD-1 axis binding antagonist is nivolumab at a fixed dose of about 480 mg every four weeks.

In some instances, the fixed dose of the PD-1 axis binding antagonist (e.g., anti-PD-L1 antagonist antibody (e.g., atezolizumab)) administered in a combination therapy (e.g., a combination treatment with an anti-TIGIT antagonist antibody, such as an anti-TIGIT antagonist antibody disclosed herein, e.g., tiragolumab) may be reduced as compared to a standard dose of the PD-1 axis binding antagonist administered as a monotherapy.

In some instances, the effective amount of the PD-1 axis binding antagonist (e.g., anti-PD-L1 antagonist antibody (e.g., atezolizumab)) is a dose of between about 0.01 mg/kg to about 50 mg/kg of the subject's body weight (e.g., between about 0.01 mg/kg to about 45 mg/kg, e.g., between about 0.1 mg/kg to about 40 mg/kg, e.g., between about 1 mg/kg to about 35 mg/kg, e.g., between about 2.5 mg/kg to about 30 mg/kg, e.g., between about 5 mg/kg to about 25 mg/kg, e.g., between about 10 mg/kg to about 20 mg/kg, e.g., between about 12.5 mg/kg to about 15 mg/kg, e.g., about 15 ± 2 mg/kg, about 15 t 1 mg/kg, about 15 ± 0.5 mg/kg, about 15 ± 0.2 mg/kg, or about 15 ± 0.1 mg/kg, e.g., about 15 mg/kg) every three weeks. In some instances, the effective amount of the PD-1 axis binding antagonist (e.g., anti-PD-L1 antagonist antibody (e.g., atezolizumab)) is a dose of between about 0.01 mg/kg to about 15 mg/kg of the subject's body weight (e.g., between about 0.1 mg/kg to about 15 mg/kg, e.g., between about 0.5 mg/kg to about 15 mg/kg, e.g.,

between about 1 mg/kg to about 15 mg/kg, e.g., between about 2.5 mg/kg to about 15 mg/kg, e.g., between about 5 mg/kg to about 15 mg/kg, e.g., between about 7.5 mg/kg to about 15 mg/kg, e.g., between about 10 mg/kg to about 15 mg/kg, e.g., between about 12.5 mg/kg to about 15 mg/kg, e.g., between about 14 mg/kg to about 15 mg/kg, e.g., about 15 ± 1 mg/kg, e.g., about 15 ± 0.5 mg/kg, e.g., about 15 ± 0.2 mg/kg, e.g., about 15 ± 0.1 mg/kg, e.g., about 15 mg/kg) every two weeks, every three weeks, or every four weeks. In some instances, the effective amount of PD-1 axis binding antagonist (e.g., anti-PD-L1 antagonist antibody (e.g., atezolizumab)) is a dose of about 15 mg/kg administered every three weeks. In some instances, the effective amount of PD-1 axis binding antagonist (e.g., anti-PD-L1 antagonist antibody (e.g., atezolizumab)) is a dose of about 10 mg/kg administered every two weeks. In some instances, the effective amount of PD-1 axis binding antagonist (e.g., anti-PD-L1 antagonist antibody (e.g., atezolizumab)) is a dose of about 20 mg/kg administered every two weeks. In some instances, the dose of the PD-1 axis binding antagonist (e.g., anti-PD-L1 antagonist antibody (e.g., atezolizumab)) administered in a combination therapy (e.g., a combination treatment with an anti-TIGIT antagonist antibody, such as an anti-TIGIT antagonist antibody disclosed herein, e.g., tiragolumab) may be reduced as compared to a standard dose of the PD-1 axis binding antagonist administered as a monotherapy.

In any of the methods and uses of the invention, the anti-TIGIT antagonist antibody (e.g., an anti-TIGIT antagonist antibody as disclosed herein, e.g., tiragolumab) and the PD-1 axis binding antagonist (e.g., anti-PD-L1 antagonist antibody (e.g., atezolizumab)) may be administered in one or more dosing cycles (e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 or more dosing cycles). In some instances, the dosing cycles of the anti-TIGIT antagonist antibody (e.g., an anti-TIGIT antagonist antibody as disclosed herein, e.g., tiragolumab) and the PD-1 axis binding antagonist (e.g., anti-PD-L1 antagonist antibody (e.g., atezolizumab)) continue until there is a loss of clinical benefit (e.g., confirmed disease progression, drug resistance, death, or unacceptable toxicity). In some instances, the length of each dosing cycle is about 14 to 28 days (e.g., 14 days, 15 days, 16 days, 17 days, 18 days, 19 days, 20 days, 21 days, 22 days, 23 days, 24 days, 25 days, 26 days, 27 days, or 28 days).

In some instances, the length of each dosing cycle is about 21 days. In some instances, the anti-TIGIT antagonist antibody (e.g., an anti-TIGIT antagonist antibody as disclosed herein, e.g., tiragolumab) is administered on about Day 1 (e.g., Day 1 ± 3 days) of each dosing cycle. For example, the anti-TIGIT antagonist antibody (e.g., an anti-TIGIT antagonist antibody as disclosed herein, e.g., tiragolumab) is administered intravenously at a fixed dose of about 600 mg on Day 1 of each 21-day cycle (i.e., at a fixed dose of about 600 mg every three weeks). Similarly, in some instances, the PD-1 axis binding antagonist (e.g., anti-PD-L1 antagonist antibody (e.g., atezolizumab)) is administered on about Day 1 (e.g., Day 1 ± 3 days) of each dosing cycle. For example, in some instances, the PD-1 axis binding antagonist (e.g., anti-PD-L1 antagonist antibody (e.g., atezolizumab)) is administered intravenously at a fixed dose of about 1200 mg on Day 1 of each 21-day cycle (i.e., at a fixed dose of about 1200 mg every three weeks). In some instances, both the anti-TIGIT antagonist antibody (e.g., an anti-TIGIT antagonist antibody as disclosed herein, e.g., tiragolumab) and the PD-1 axis binding antagonist (e.g., anti-PD-L1 antagonist antibody (e.g., atezolizumab)) are administered on about Day 1 (e.g., Day 1 ± 3 days) of each dosing cycle. For example, the anti-TIGIT antagonist antibody (e.g., an anti-TIGIT antagonist antibody as disclosed herein, e.g., tiragolumab) is administered intravenously at a fixed dose of about 600 mg on Day 1 of each 21-day cycle (i.e., at a fixed dose of about 600 mg every three weeks), and the PD-1 axis binding antagonist (e.g., anti-PD-L1 antagonist antibody (e.g., atezolizumab)) is administered intravenously at a fixed dose of about 1200 mg on Day 1 of each 21-day cycle (i.e., at a fixed dose of about 1200 mg every three weeks).

In some instances, the length of each dosing cycle is about 28 days. In some instances, the anti-TIGIT antagonist antibody (e.g., an anti-TIGIT antagonist antibody as disclosed herein, e.g., tiragolumab) is administered on about Day 1 (e.g., Day 1 ± 3 days) of each dosing cycle. For example, the anti-TIGIT antagonist antibody (e.g., an anti-TIGIT antagonist antibody as disclosed herein, e.g., tiragolumab) is administered intravenously at a fixed dose of about 420 mg on Day 1 and Day 15 of each 28-day cycle (i.e., at a fixed dose of about 420 mg every two weeks). Similarly, in some instances, the PD-1 axis binding antagonist (e.g., anti-PD-L1 antagonist antibody (e.g., atezolizumab)) is administered on about Day 1 and Day 15 (e.g., Day 1 ± 3 days and Day 15 ± 3 days) of each dosing cycle. For example, in some instances, the PD-1 axis binding antagonist (e.g., anti-PD-L1 antagonist antibody (e.g., atezolizumab)) is administered intravenously at a fixed dose of about 840 mg on Day 1 and Day 15 of each 28-day cycle (i.e., at a fixed dose of about 840 mg every two weeks). In some instances, both the anti-TIGIT antagonist antibody (e.g., an anti-TIGIT antagonist antibody as disclosed herein, e.g., tiragolumab) and the PD-1 axis binding antagonist (e.g., anti-PD-L1 antagonist antibody (e.g., atezolizumab)) are administered on about Day 1 and Day 15 (e.g., Day 1 ± 3 days and Day 15 ± 3 days) of each dosing cycle. For example, the anti-TIGIT antagonist antibody (e.g., an anti-TIGIT antagonist antibody as disclosed herein, e.g., tiragolumab) is administered intravenously at a fixed dose of about 420 mg on Day 1 and Day 15 of each 28-day cycle (i.e., at a fixed dose of about 420 mg every two weeks), and the PD-1 axis binding antagonist (e.g., anti-PD-L1 antagonist antibody (e.g., atezolizumab)) is administered intravenously at a fixed dose of about 840 mg on Day 1 and Day 15 of each 28-day cycle (i.e., at a fixed dose of about 840 mg every two weeks).

In some instances, the anti-TIGIT antagonist antibody (e.g., an anti-TIGIT antagonist antibody as disclosed herein, e.g., tiragolumab) is administered on about Day 1 (e.g., Day 1 ± 3 days) of each 28-day dosing cycle. For example, the anti-TIGIT antagonist antibody (e.g., an anti-TIGIT antagonist antibody as disclosed herein, e.g., tiragolumab) is administered intravenously at a fixed dose of about 840 mg on Day 1 of each 28-day cycle (i.e., at a fixed dose of about 420 mg every four weeks). Similarly, in some instances, the PD-1 axis binding antagonist (e.g., anti-PD-L1 antagonist antibody (e.g., atezolizumab)) is administered on about Day 1 (e.g., Day 1 ± 3 days) of each dosing cycle. For example, in some instances, the PD-1 axis binding antagonist (e.g., anti-PD-L1 antagonist antibody (e.g., atezolizumab)) is administered intravenously at a fixed dose of about 1680 mg on Day 1 of each 28-day cycle (i.e., at a fixed dose of about 840 mg every four weeks). In some instances, both the anti-TIGIT antagonist antibody (e.g., an anti-TIGIT antagonist antibody as disclosed herein, e.g., tiragolumab) and the PD-1 axis binding antagonist (e.g., anti-PD-L1 antagonist antibody (e.g., atezolizumab)) are administered on about Day 1 (e.g., Day 1 ± 3 days) of each dosing cycle. For example, the anti-TIGIT antagonist antibody (e.g., an anti-TIGIT antagonist antibody as disclosed herein, e.g., tiragolumab) is administered intravenously at a fixed dose of about 840 mg on Day 1 of each 28-day cycle (i.e., at a fixed dose of about 820 mg every four weeks), and the PD-1 axis binding antagonist (e.g., anti-PD-L1 antagonist antibody (e.g., atezolizumab)) is administered intravenously at a fixed dose of about 1680 mg on Day 1 of each 28-day cycle (i.e., at a fixed dose of about 1680 mg every four weeks).

In some instances, the anti-TIGIT antagonist antibody (e.g., an anti-TIGIT antagonist antibody as disclosed herein, e.g., tiragolumab) is administered to the subject by intravenous infusion over about 60 ± 10 minutes (e.g., about 50 minutes, about 51 minutes, about 52 minutes, about 53 minutes, about 54 minutes, about 55 minutes, about 56 minutes, about 57 minutes, about 58 minutes, about 59 minutes, about 60 minutes, about 61 minutes, about 62 minutes, about 63 minutes, about 64 minutes, about 65 minutes, about 66 minutes, about 67 minutes, about 68 minutes, about 69 minutes, or about 70 minutes). In some instances, the PD-1 axis binding antagonist (e.g., anti-PD-L1 antagonist antibody (e.g., atezolizumab)) is administered to the subject by intravenous infusion over about 60 ± 15 minutes (e.g. about 45 minutes, about 46 minutes, about 47 minutes, about 48 minutes, about 49 minutes, about 50 minutes, about 51 minutes, about 52 minutes, about 53 minutes, about 54 minutes, about 55 minutes, about 56 minutes, about 57 minutes, about 58 minutes, about 59 minutes, about 60 minutes, about 61 minutes, about 62 minutes, about 63 minutes, about 64 minutes, about 65 minutes, about 66 minutes, about 67 minutes, about 68 minutes, about 69 minutes, about 70 minutes, about 71 minutes, about 72 minutes, about 73 minutes, about 74 minutes, or about 75 minutes).

In some instances, the anti-TIGIT antagonist antibody (e.g., an anti-TIGIT antagonist antibody as disclosed herein, e.g., tiragolumab) is administered to the subject before the PD-1 axis binding antagonist (e.g., anti-PD-L1 antagonist antibody (e.g., atezolizumab)). In some instances, for example, following administration of the anti-TIGIT antagonist antibody and before administration of the PD-1 axis binding antagonist, the method includes an intervening first observation period. In some instances, the method further includes a second observation period following administration of the PD-1 axis binding antagonist. In some instances, the method includes both a first observation period following administration of the anti-TIGIT antagonist antibody and second observation period following administration of PD-1 axis binding antagonist. In some instances, the first and second observation periods are each between about 30 minutes to about 60 minutes in length. In instances in which the first and second observation periods are each about 60 minutes in length, the method may include recording the subject's vital signs (e.g., pulse rate, respiratory rate, blood pressure, and temperature) at about 30 ± 10 minutes after administration of the anti-TIGIT antagonist antibody and PD-1 axis binding antagonist during the first and second observation periods, respectively. In instances in which the first and second observation periods are each about 30 minutes in length, the method may include recording the subject's vital signs (e.g., pulse rate, respiratory rate, blood pressure, and temperature) at about 15 ± 10 minutes after administration of the anti-TIGIT antagonist antibody and PD-1 axis binding antagonist during the first and second observation periods, respectively.

In other instances, the PD-1 axis binding antagonist (e.g., anti-PD-L1 antagonist antibody (e.g. atezolizumab)) is administered to the subject before the anti-TIGIT antagonist antibody (e.g., an anti-TIGIT antagonist antibody as disclosed herein, e.g., tiragolumab). In some instances, for example, following administration of the PD-1 axis binding antagonist and before administration of the anti-TIGIT antagonist antibody, the method includes an intervening first observation period. In some instances, the method includes a second observation period following administration of the anti-TIGIT antagonist antibody. In some instances, the method includes both a first observation period following administration of the PD-1 axis binding antagonist and second observation period following administration of the anti-TIGIT antagonist antibody. In some instances, the first and second observation periods are each between about 30 minutes to about 60 minutes in length. In instances in which the first and second observation periods are each about 60 minutes in length, the method may include recording the subject's vital signs (e.g., pulse rate, respiratory rate, blood pressure, and temperature) at about 30 ± 10 minutes after administration of the PD-1 axis binding antagonist and anti-TIGIT antagonist antibody during the first and second observation periods, respectively. In instances in which the first and second observation periods are each about 30 minutes in length, the method may include recording the subject's vital signs (e.g., pulse rate, respiratory rate, blood pressure, and temperature) at about 15 ± 10 minutes after administration of the PD-1 axis binding antagonist and anti-TIGIT antagonist antibody during the first and second observation periods, respectively.

In other instances, the anti-TIGIT antagonist antibody (e.g., an anti-TIGIT antagonist antibody as disclosed herein, e.g., tiragolumab) and the PD-1 axis binding antagonist (e.g., anti-PD-L1 (atezolizumab) antagonist antibody) are administered to the subject simultaneously. In some instances, for example, following administration of the anti-TIGIT antagonist antibody and the PD-1 axis binding antagonist (e.g., anti-PD-L1 antagonist antibody) the method includes an observation period. In some instances, the observation period is between about 30 minutes to about 60 minutes in length. In instances in which the observation period is about 60 minutes in length, the method may include recording the subject's vital signs (e.g., pulse rate, respiratory rate, blood pressure, and temperature) at about 30 ± 10 minutes after administration of the PD-1 axis binding antagonist (e.g., anti-PD-L1 antagonist antibody) and anti-TIGIT antagonist antibody during the observation period. In instances in which the observation period is about 30 minutes in length, the method may include recording the subject's vital signs (e.g., pulse rate, respiratory rate, blood pressure, and temperature) at about 15 ± 10 minutes after administration of the PD-1 axis binding antagonist (e.g., anti-PD-L1 antagonist antibody) and anti-TIGIT antagonist antibody during the observation period.

In another aspect, the invention provides a method of treating a subject having an NSCLC (e.g., squamous or non-squamous NSCLC, e.g., locally advanced unresectable NSCLC (e.g., Stage IIIB NSCLC), or recurrent or metastatic NSCLC (e.g., Stage IV NSCLC)) who has been determined to have a PD-L1-positive tumor cell fraction of greater than, or equal to, 30% (e.g., greater than, or equal to, 50%) by administering to the subject one or more dosing cycles of an anti-TIGIT antagonist antibody at a fixed dose of 600 mg every three weeks and atezolizumab at a fixed dose of 1200 mg every three weeks, wherein the anti-TIGIT antagonist antibody has a VH domain having the amino acid sequence of SEQ ID NO: 17 or 18 and a VL domain having the amino acid sequence of SEQ ID NO: 19, wherein the treatment results in (a) a CR or a PR and/or (b) an increase in PFS as compared to treatment with atezolizumab without the anti-TIGIT antagonist antibody, as described in further detail below. In some instances, the PD-L1-positive tumor cell fraction is greater than, or equal to, 50% (e.g., as determined by positive staining with the anti-PD-L1 antibody SP263 (e.g., using the Ventana assay), as determined by positive staining with the anti-PD-L1 antibody 22C3 (e.g., using the pharmDx assay), or as determined by positive staining with the anti-PD-L1 antibody 28-8). In some embodiments, the PD-L1-positive tumor cell fraction is greater than, or equal to, 30%, as determined by positive staining with the anti-PD-L1 antibody SP142.

In another aspect, the invention provides a method of treating a subject having an NSCLC (e.g., squamous or non-squamous NSCLC, e.g., locally advanced unresectable NSCLC (e.g., Stage IIIB NSCLC), or recurrent or metastatic NSCLC (e.g., Stage IV NSCLC)) who has been determined to have a PD-L1-positive tumor cell fraction of greater than, or equal to, 30% (e.g., greater than, or equal to, 50%) by administering to the subject one or more dosing cycles of tiragolumab at a fixed dose of 600 mg every three weeks and atezolizumab at a fixed dose of 1200 mg every three weeks, wherein the treatment results in (a) a CR or a PR and/or (b) an increase in PFS as compared to treatment with atezolizumab without tiragolumab. In some instances, the PD-L1-positive tumor cell fraction is greater than, or equal to, 50% (e.g., as determined by positive staining with the anti-PD-L1 antibody SP263 (e.g., using the Ventana assay), as determined by positive staining with the anti-PD-L1 antibody 22C3 (e.g., using the pharmDx assay), or as determined by positive staining with the anti-PD-L1 antibody 28-8). In some embodiments, the PD-L1-positive tumor cell fraction is greater than, or equal to, 30%, as determined by positive staining with the anti-PD-L1 antibody SP142.

In another aspect, the invention provides an anti-TIGIT antagonist antibody (e.g., an anti-TIGIT antagonist antibody disclosed herein, e.g., tiragolumab) and PD-1 axis binding antagonist (e.g., anti-PD-L1 antagonist antibody (e.g., atezolizumab)) for use in a method of treating a subject having a cancer (e.g., lung cancer, e.g., non-small cell lung cancer (NSCLC), e.g., squamous or non-squamous NSCLC, e.g., locally advanced unresectable NSCLC (e.g., Stage IIIB NSCLC), or recurrent or metastatic NSCLC (e.g., Stage IV NSCLC)) who has been determined to have a PD-L1-positive tumor cell fraction of greater than, or equal to, 30% (e.g., greater than, or equal to, 50%), wherein the method comprises administering to the subject one or more dosing cycles of an effective amount of an anti-TIGIT antagonist antibody and an effective amount of a PD-1 axis binding antagonist (e.g., anti-PD-L1 antagonist antibody), wherein the treatment results in (a) a CR or a PR and/or (b) an increase in PFS as compared to treatment with the PD-1 axis binding antagonist (e.g., anti-PD-L1 antagonist antibody) without the anti-TIGIT antagonist antibody. In some instances, the PD-L1-positive tumor cell fraction is greater than, or equal to, 50% (e.g., as determined by positive staining with the anti-PD-L1 antibody SP263 (e.g., using the Ventana assay), as determined by positive staining with the anti-PD-L1 antibody 22C3 (e.g., using the pharmDx assay), or as determined by positive staining with the anti-PD-L1 antibody 28-8). In some embodiments, the PD-L1-positive tumor cell fraction is greater than, or equal to, 30%, as determined by positive staining with the anti-PD-L1 antibody SP142.

In some instances, the effective amount of the anti-TIGIT antagonist antibody (e.g., an anti-TIGIT antagonist antibody as disclosed herein, e.g., tiragolumab) is a fixed dose of between about 30 mg to about 1200 mg (e.g., between about 30 mg to about 1100 mg, e.g., between about 60 mg to about 1000 mg, e.g., between about 100 mg to about 900 mg, e.g., between about 200 mg to about 800 mg, e.g., between about 300 mg to about 800 mg, e.g., between about 400 mg to about 800 mg, e.g., between about 400 mg to about 750 mg, e.g., between about 450 mg to about 750 mg, e.g., between about 500 mg to about 700 mg, e.g., between about 550 mg to about 650 mg, e.g., 600 mg ± 10 mg, e.g.,

600 ± 6 mg, e.g., 600 ± 5 mg, e.g., 600 ± 3 mg, e.g., 600 ± 1 mg, e.g., 600 ± 0.5 mg, e.g., 600 mg) every three weeks. In some instances, the effective amount of the anti-TIGIT antagonist antibody (e.g., an anti-TIGIT antagonist antibody as disclosed herein, e.g., tiragolumab) is a fixed dose of between about 30 mg to about 600 mg (e.g., between about 50 mg to between 600 mg, e.g., between about 60 mg to about 600 mg, e.g., between about 100 mg to about 600 mg, e.g., between about 200 mg to about 600 mg, e.g., between about 200 mg to about 550 mg, e.g., between about 250 mg to about 500 mg, e.g., between about 300 mg to about 450 mg, e.g., between about 350 mg to about 400 mg, e.g., about 375 mg) every three weeks. In some instances, the effective amount of the anti-TIGIT antagonist antibody (e.g., an anti-TIGIT antagonist antibody as disclosed herein, e.g., tiragolumab) is a fixed dose of about 600 mg every three weeks. In some instances, effective amount of the anti-TIGIT antagonist antibody (e.g., an anti-TIGIT antagonist antibody as disclosed herein, e.g., tiragolumab) is a fixed dose of 600 mg every three weeks. In some instances, the fixed dose of the anti-TIGIT antagonist antibody (e.g., an anti-TIGIT antagonist antibody as disclosed herein, e.g., tiragolumab) is to be administered in a combination therapy (e.g., a combination treatment with a PD-1 axis binding antagonist (e.g., an anti-PD-L1 antagonist antibody, e.g., atezolizumab)) may be reduced as compared to a standard dose of the anti-TIGIT antagonist antibody is to be administered as a monotherapy.

In some instances, the effective amount of the PD-1 axis binding antagonist (e.g., anti-PD-L1 antagonist antibody (e.g., atezolizumab)) is a fixed dose of between about 80 mg to about 1600 mg (e.g., between about 100 mg to about 1600 mg, e.g., between about 200 mg to about 1600 mg, e.g., between about 300 mg to about 1600 mg, e.g., between about 400 mg to about 1600 mg, e.g., between about 500 mg to about 1600 mg, e.g., between about 600 mg to about 1600 mg, e.g., between about 700 mg to about 1600 mg, e.g., between about 800 mg to about 1600 mg, e.g., between about 900 mg to about 1500 mg, e.g., between about 1000 mg to about 1400 mg, e.g., between about 1050 mg to about 1350 mg, e.g., between about 1100 mg to about 1300 mg, e.g., between about 1150 mg to about 1250 mg, e.g., between about 1175 mg to about 1225 mg, e.g., between about 1190 mg to about 1210 mg, e.g., 1200 mg ± 5 mg, e.g., 1200 ± 2.5 mg, e.g., 1200 ± 1.0 mg, e.g., 1200 ± 0.5 mg, e.g., 1200 ) every three weeks. In some instances, the effective amount of the PD-1 axis binding antagonist (e.g., anti-PD-L1 antagonist antibody (e.g., atezolizumab)) is a fixed dose of about 1200 mg every three weeks. In some instances, the effective amount of the PD-1 axis binding antagonist (e.g., anti-PD-L1 antagonist antibody (e.g., atezolizumab)) is a fixed dose of 1200 mg every three weeks.

In some embodiments, the PD-1 axis binding antagonist is administered at a dose of about 80 mg to about 2000 mg every two weeks, three weeks, or four weeks (e.g., about 840 mg every two weeks, about 1200 mg every three weeks, or about 1680 mg every four weeks). In some embodiments, the PD-1 axis binding antagonist is administered at a dose of about 1680 mg every four weeks. In some embodiments, the anti-TIGIT antagonist antibody is administered at a dose of about 600 mg every three weeks and the PD-1 axis binding antagonist is administered at a dose of about 1680 mg every four weeks. In some embodiments, the PD-1 axis binding antagonist is administered at a dose of about 1200 mg every three weeks. In some embodiments, the anti-TIGIT antagonist antibody is administered at a dose of about 600 mg every three weeks and the PD-1 axis binding antagonist is administered at a dose of about 1200 mg every three weeks. In some embodiments, the PD-1 axis binding antagonist is administered at a dose of about 840 mg every two weeks. In some embodiments, the anti-TIGIT antagonist antibody is administered at a dose of about 600 mg every three weeks and the PD-1 axis binding antagonist is administered at a dose of about 840 mg every two weeks.

In some instances, the effective amount of the PD-1 axis binding antagonist (e.g., anti-PD-L1 antagonist antibody (e.g., atezolizumab)) is a fixed dose of about 840 mg every two weeks. In some instances, the effective amount of the PD-1 axis binding antagonist (e.g., anti-PD-L1 antagonist antibody (e.g., atezolizumab)) is a fixed dose of about 1200 mg every three weeks. In some instances, the effective amount of the PD-1 axis binding antagonist (e.g., anti-PD-L1 antagonist antibody (e.g., atezolizumab)) is a fixed dose of 1680 mg every four weeks.

In some instances, the effective amount of the PD-1 axis binding antagonist (e.g., anti-PD-L1 antagonist antibody (e.g., atezolizumab)) is a fixed dose of between about 20 mg to about 1600 mg (e.g., between about 40 mg to about 1500 mg, e.g., between about 200 mg to about 1400 mg, e.g., between about 300 mg to about 1400 mg, e.g., between about 400 mg to about 1400 mg, e.g., between about 500 mg to about 1300 mg, e.g., between about 600 mg to about 1200 mg, e.g., between about 700 mg to about 1100 mg, e.g., between about 800 mg to about 1000 mg, e.g., between about 800 mg to about 900 mg, e.g., about 800, about 810, about 820, about 830, about 840, about 850, about 860, about 870, about 880, about 890, or about 900 mg) every two weeks (Q2W). In some instances, the effective amount of the PD-1 axis binding antagonist is atezolizumab at a fixed dose of about 840 mg every two weeks (e.g., 840 mg ± 10 mg, e.g., 840 t 6 mg, e.g., 840 ± 5 mg, e.g., 840 ± 3 mg, e.g., 840 ± 1 mg, e.g., 840 ± 0.5 mg, e.g., 840 mg every two weeks). In some embodiments, the effective amount of the PD-1 axis binding antagonist is avelumab at a fixed dose of about 800 mg every two weeks. In some embodiments, the effective amount of the PD-1 axis binding antagonist is nivolumab at a fixed dose of about 240 mg every two weeks.

In some instances, the effective amount of the PD-1 axis binding antagonist (e.g., anti-PD-L1 antagonist antibody (e.g., atezolizumab)) is a fixed dose of between about 500 mg to about 3000 mg (e.g., between about 500 mg to about 2800 mg, e.g., between about 600 mg to about 2700 mg, e.g., between about 650 mg to about 2600 mg, e.g., between about 700 mg to about 2500 mg, e.g., between about 1000 mg to about 2400 mg, e.g., between about 1100 mg to about 2300 mg, e.g., between about 1200 mg to about 2200 mg, e.g., between about 1300 mg to about 2100 mg, e.g., between about 1400 mg to about 2000 mg, e.g., between about 1500 mg to about 1900 mg, e.g., between about 1600 mg to about 1800 mg, e.g., between about 1620 mg to about 1700 mg, e.g., between about 1640 mg to about 1690 mg, e.g., between about 1660 mg to about 1680 mg, about 1680 mg, e.g., about 1600 mg, about 1610 mg, about 1620 mg, about 1630 mg, about 1640 mg, about 1650 mg, about 1660 mg, about 1670 mg, about 1680 mg, about 1690 mg, or about 1700 mg) every four weeks (Q4W). In some instances, the effective amount of the PD-1 axis binding antagonist (e.g., anti-PD-L1 antagonist antibody (e.g., atezolizumab)) is a fixed dose of 1680 mg every four weeks (e.g., 1680 mg ± 10 mg, e.g., 1680 ± 6 mg, e.g., 1680 ± 5 mg, e.g., 1680 ± 3 mg, e.g., 1680 ± 1 mg, e.g., 1680 ± 0.5 mg, e.g., 1680 mg every four weeks). In some embodiments, the effective amount of the PD-1 axis binding antagonist is nivolumab at a fixed dose of about 480 mg every four weeks.

In some instances, the fixed dose of the PD-1 axis binding antagonist (e.g., anti-PD-L1 antagonist antibody (e.g., atezolizumab)) to be administered in a combination therapy (e.g., a combination treatment with an anti-TIGIT antagonist antibody, such as an anti-TIGIT antagonist antibody disclosed herein, e.g., tiragolumab) may be reduced as compared to a standard dose of the PD-1 axis binding antagonist (e.g., anti-PD-L1 antagonist antibody) to be administered as a monotherapy.

In some instances, the effective amount of the PD-1 axis binding antagonist (e.g., anti-PD-L1 antagonist antibody (e.g., atezolizumab)) is a dose of between about 0.01 mg/kg to about 50 mg/kg of the subject's body weight (e.g., between about 0.01 mg/kg to about 45 mg/kg, e.g., between about 0.1 mg/kg to about 40 mg/kg, e.g., between about 1 mg/kg to about 35 mg/kg, e.g., between about 2.5 mg/kg to about 30 mg/kg, e.g., between about 5 mg/kg to about 25 mg/kg, e.g., between about 10 mg/kg to about 20 mg/kg, e.g., between about 12.5 mg/kg to about 15 mg/kg, e.g., about 15 ± 2 mg/kg, about 15 t 1 mg/kg, about 15 ± 0.5 mg/kg, about 15 ± 0.2 mg/kg, or about 15 ± 0.1 mg/kg, e.g., about 15 mg/kg) every three weeks. In some instances, the effective amount of the PD-1 axis binding antagonist (e.g., anti-PD-L1 antagonist antibody (e.g., atezolizumab)) is a dose of between about 0.01 mg/kg to about 15 mg/kg of the subject's body weight (e.g., between about 0.1 mg/kg to about 15 mg/kg, e.g., between about 0.5 mg/kg to about 15 mg/kg, e.g., between about 1 mg/kg to about 15 mg/kg, e.g., between about 2.5 mg/kg to about 15 mg/kg, e.g., between about 5 mg/kg to about 15 mg/kg, e.g., between about 7.5 mg/kg to about 15 mg/kg, e.g., between about 10 mg/kg to about 15 mg/kg, e.g., between about 12.5 mg/kg to about 15 mg/kg, e.g., between about 14 mg/kg to about 15 mg/kg, e.g., about 15 ± 1 mg/kg, e.g., about 15 ± 0.5 mg/kg, e.g., about 15 ± 0.2 mg/kg, e.g., about 15 ± 0.1 mg/kg, e.g., about 15 mg/kg) every three weeks. In some instances, effective amount of PD-1 axis binding antagonist (e.g., anti-PD-L1 antagonist antibody (e.g., atezolizumab)) is a dose of about 15 mg/kg to be administered every three weeks. In some instances, the dose of the PD-1 axis binding antagonist (e.g., anti-PD-L1 antagonist antibody (e.g., atezolizumab)) is administered in a combination therapy (e.g., a combination treatment with an anti-TIGIT antagonist antibody, such as an anti-TIGIT antagonist antibody disclosed herein, e.g., tiragolumab) may be reduced as compared to a standard dose of the PD-1 axis binding antagonist (e.g., anti-PD-L1 antagonist antibody) administered as a monotherapy.

The anti-TIGIT antagonist antibody (e.g., an anti-TIGIT antagonist antibody as disclosed herein, e.g., tiragolumab) and the PD-1 axis binding antagonist (e.g., anti-PD-L1 antagonist antibody (e.g., atezolizumab)) may be administered in one or more dosing cycles (e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 or more dosing cycles). In some instances, the dosing cycles of the anti-TIGIT antagonist antibody (e.g., an anti-TIGIT antagonist antibody as disclosed herein, e.g., tiragolumab) and the PD-1 axis binding antagonist (e.g., anti-PD-L1 antagonist antibody (e.g., atezolizumab)) continue until there is a loss of clinical benefit (e.g., confirmed disease progression, drug resistance, death, or unacceptable toxicity). In some instances, the length of each dosing cycle is about 14 to 28 days (e.g., 14 days, 15 days, 16 days, 17 days, 18 days, 19 days, 20 days, 21 days, 22 days, 23 days, 24 days, 25 days, 26 days, 27 days, or 28 days). In some instances, the length of each dosing cycle is about 21 days. In some instances, the anti-TIGIT antagonist antibody (e.g., an anti-TIGIT antagonist antibody as disclosed herein, e.g., tiragolumab) is to be administered on about Day 1 (e.g., Day 1 ± 3 days) of each dosing cycle. For example, the anti-TIGIT antagonist antibody (e.g., an anti-TIGIT antagonist antibody as disclosed herein, e.g., tiragolumab) is to be administered intravenously at a fixed dose of about 600 mg on Day 1 of each 21-day cycle (i.e., at a fixed dose of about 600 mg every three weeks). Similarly, in some instances, the PD-1 axis binding antagonist (e.g., anti-PD-L1 antagonist antibody (e.g., atezolizumab)) is to be administered on about Day 1 (e.g., Day 1 ± 3 days) of each dosing cycle. For example, the PD-1 axis binding antagonist (e.g., anti-PD-L1 antagonist antibody (e.g., atezolizumab)) is to be administered intravenously at a fixed dose of about 1200 mg on Day 1 of each 21-day cycle (i.e., at a fixed dose of about 1200 mg every three weeks). In some instances, both the anti-TIGIT antagonist antibody (e.g., an anti-TIGIT antagonist antibody as disclosed herein, e.g., tiragolumab) and the PD-1 axis binding antagonist (e.g., anti-PD-L1 antagonist antibody (e.g., atezolizumab)) are to be administered on about Day 1 (e.g., Day 1 ± 3 days) of each dosing cycle. For example, the anti-TIGIT antagonist antibody (e.g., an anti-TIGIT antagonist antibody as disclosed herein, e.g., tiragolumab) is to be administered intravenously at a fixed dose of about 600 mg on Day 1 of each 21-day cycle (i.e., at a fixed dose of about 600 mg every three weeks), and the PD-1 axis binding antagonist (e.g., anti-PD-L1 antagonist antibody (e.g., atezolizumab)) is to be administered intravenously at a fixed dose of about 1200 mg on Day 1 of each 21-day cycle (i.e., at a fixed dose of about 1200 mg every three weeks).

In some instances, the anti-TIGIT antagonist antibody (e.g., an anti-TIGIT antagonist antibody as disclosed herein, e.g., tiragolumab) is to be administered to the subject by intravenous infusion over about 60 ± 10 minutes (e.g., about 50 minutes, about 51 minutes, about 52 minutes, about 53 minutes, about 54 minutes, about 55 minutes, about 56 minutes, about 57 minutes, about 58 minutes, about 59 minutes, about 60 minutes, about 61 minutes, about 62 minutes, about 63 minutes, about 64 minutes, about 65 minutes, about 66 minutes, about 67 minutes, about 68 minutes, about 69 minutes, or about 70 minutes). In some instances, the PD-1 axis binding antagonist (e.g., anti-PD-L1 antagonist antibody (e.g., atezolizumab)) is to be administered to the subject by intravenous infusion over about 60 ± 15 minutes (e.g. about 45 minutes, about 46 minutes, about 47 minutes, about 48 minutes, about 49 minutes, about 50 minutes, about 51 minutes, about 52 minutes, about 53 minutes, about 54 minutes, about 55 minutes, about 56 minutes, about 57 minutes, about 58 minutes, about 59 minutes, about 60 minutes, about 61 minutes, about 62 minutes, about 63 minutes, about 64 minutes, about 65 minutes, about 66 minutes, about 67 minutes, about 68 minutes, about 69 minutes, about 70 minutes, about 71 minutes, about 72 minutes, about 73 minutes, about 74 minutes, or about 75 minutes).

In some instances, the anti-TIGIT antagonist antibody (e.g., an anti-TIGIT antagonist antibody as disclosed herein, e.g., tiragolumab) is to be administered to the subject before the PD-1 axis binding antagonist (e.g., anti-PD-L1 antagonist antibody (e.g., atezolizumab)). In some instances, for example, following administration of the anti-TIGIT antagonist antibody and before administration of the PD-1 axis binding antagonist (e.g., anti-PD-L1 antagonist antibody), the method includes an intervening first observation period. In some instances, the method further includes a second observation period following administration of the PD-1 axis binding antagonist (e.g., anti-PD-L1 antagonist antibody). In some instances, the method includes both a first observation period following administration of the anti-TIGIT antagonist antibody and second observation period following administration of the PD-1 axis binding antagonist (e.g., anti-PD-L1 antagonist antibody). In some instances, the first and second observation periods are each between about 30 minutes to about 60 minutes in length. In instances in which the first and second observation periods are each about 60 minutes in length, the method may include recording the subject's vital signs (e.g., pulse rate, respiratory rate, blood pressure, and temperature) at about 30 ± 10 minutes after administration of the anti-TIGIT antagonist antibody and PD-1 axis binding antagonist (e.g., anti-PD-L1 antagonist antibody) during the first and second observation periods, respectively. In instances in which the first and second observation periods are each about 30 minutes in length, the method may include recording the subject's vital signs (e.g., pulse rate, respiratory rate, blood pressure, and temperature) at about 15 ± 10 minutes after administration of the anti-TIGIT antagonist antibody and anti-PD-L1 antagonist antibody during the first and second observation periods, respectively.

In other instances, the PD-1 axis binding antagonist (e.g., anti-PD-L1 antagonist antibody (e.g. atezolizumab)) is to be administered to the subject before the anti-TIGIT antagonist antibody (e.g., an anti-TIGIT antagonist antibody as disclosed herein, e.g., tiragolumab). In some instances, for example, following administration of the PD-1 axis binding antagonist (e.g., anti-PD-L1 antagonist antibody) and before administration of the anti-TIGIT antagonist antibody, the method includes an intervening first observation period. In some instances, the method includes a second observation period following administration of the anti-TIGIT antagonist antibody. In some instances, the method includes both a first observation period following administration of the PD-1 axis binding antagonist (e.g., anti-PD-L1 antagonist antibody) and second observation period following administration of the anti-TIGIT antagonist antibody. In some instances, the first and second observation periods are each between about 30 minutes to about 60 minutes in length. In instances in which the first and second observation periods are each about 60 minutes in length, the method may include recording the subject's vital signs (e.g., pulse rate, respiratory rate, blood pressure, and temperature) at about 30 ± 10 minutes after administration of the PD-1 axis binding antagonist (e.g., anti-PD-L1 antagonist antibody) and anti-TIGIT antagonist antibody during the first and second observation periods, respectively. In instances in which the first and second observation periods are each about 30 minutes in length, the method may include recording the subject's vital signs (e.g., pulse rate, respiratory rate, blood pressure, and temperature) at about 15 ± 10 minutes after administration of the PD-1 axis binding antagonist (e.g., anti-PD-L1 antagonist antibody) and anti-TIGIT antagonist antibody during the first and second observation periods, respectively.

In other instances, the anti-TIGIT antagonist antibody (e.g., an anti-TIGIT antagonist antibody as disclosed herein, e.g., tiragolumab) and the PD-1 axis binding antagonist (e.g., anti-PD-L1 (atezolizumab) antagonist antibody) is to be administered to the subject simultaneously. In some instances, for example, following administration of the anti-TIGIT antagonist antibody and the PD-1 axis binding antagonist (e.g., anti-PD-L1 antagonist antibody), the method includes an observation period. In some instances, the observation period is between about 30 minutes to about 60 minutes in length. In instances in which the observation period is about 60 minutes in length, the method may include recording the subject's vital signs (e.g., pulse rate, respiratory rate, blood pressure, and temperature) at about 30 ± 10 minutes after administration of the PD-1 axis binding antagonist (e.g., anti-PD-L1 antagonist antibody) and anti-TIGIT antagonist antibody during the observation period. In instances in which the observation period is about 30 minutes in length, the method may include recording the subject's vital signs (e.g., pulse rate, respiratory rate, blood pressure, and temperature) at about 15 ± 10 minutes after administration of the PD-1 axis binding antagonist (e.g., anti-PD-L1 antagonist antibody) and anti-TIGIT antagonist antibody during the observation period.

In another aspect, the invention provides an anti-TIGIT antagonist antibody (e.g., an anti-TIGIT antagonist antibody disclosed herein, e.g., tiragolumab) and PD-1 axis binding antagonist (e.g., anti-PD-L1 antagonist antibody (e.g., atezolizumab)) for use in a method of treating a subject having a cancer (e.g., lung cancer, e.g., non-small cell lung cancer (NSCLC), e.g., squamous or non-squamous NSCLC, e.g., locally advanced unresectable NSCLC (e.g., Stage IIIB NSCLC), or recurrent or metastatic NSCLC (e.g., Stage IV NSCLC)) who has been determined to have a PD-L1-positive tumor cell fraction of greater than, or equal to, 30% (e.g., greater than, or equal to, 50%), wherein the method comprises administering to the subject one or more dosing cycles of an anti-TIGIT antagonist antibody at a fixed dose of 600 mg every three weeks and atezolizumab at a fixed dose of 1200 mg every three weeks, wherein the anti-TIGIT antagonist antibody comprises: a VH domain comprising the amino acid sequence of SEQ ID NO: 17 or 18; and a VL domain comprising the amino acid sequence of SEQ ID NO: 19, and wherein the treatment results in (a) a CR or a PR and/or (b) an increase in PFS as compared to treatment with atezolizumab without the anti-TIGIT antagonist antibody, as described in further detail below. In some instances, the PD-L1-positive tumor cell fraction is greater than, or equal to, 50% (e.g., as determined by positive staining with the anti-PD-L1 antibody SP263 (e.g., using the Ventana assay), as determined by positive staining with the anti-PD-L1 antibody 22C3 (e.g., using the pharmDx assay), or as determined by positive staining with the anti-PD-L1 antibody 28-8). In some embodiments, the PD-L1-positive tumor cell fraction is greater than, or equal to, 30%, as determined by positive staining with the anti-PD-L1 antibody SP142.

In another aspect, the invention provides an anti-TIGIT antagonist antibody (e.g., an anti-TIGIT antagonist antibody disclosed herein, e.g., tiragolumab) and PD-1 axis binding antagonist (e.g., anti-PD-L1 antagonist antibody (e.g., atezolizumab)) for use in a method of treating a subject having a cancer (e.g., lung cancer, e.g., non-small cell lung cancer (NSCLC), e.g., squamous or non-squamous NSCLC, e.g., locally advanced unresectable NSCLC (e.g., Stage IIIB NSCLC), or recurrent or metastatic NSCLC (e.g., Stage IV NSCLC)) who has been determined to have a PD-L1-tumor cell positive fraction of greater than, or equal to, 30% (e.g., greater than, or equal to, 50%), wherein the method comprises administering to the subject one or more dosing cycles of tiragolumab at a fixed dose of 600 mg every three weeks and atezolizumab at a fixed dose of 1200 mg every three weeks, and wherein the treatment results in (a) a CR or a PR and/or (b) an increase in PFS as compared to treatment with atezolizumab without tiragolumab. In some instances, the PD-L1-positive tumor cell fraction is greater than, or equal to, 50% (e.g., as determined by positive staining with the anti-PD-L1 antibody SP263 (e.g., using the Ventana assay), as determined by positive staining with the anti-PD-L1 antibody 22C3 (e.g., using the pharmDx assay), or as determined by positive staining with the anti-PD-L1 antibody 28-8). In some embodiments, the PD-L1-positive tumor cell fraction is greater than, or equal to, 30%, as determined by positive staining with the anti-PD-L1 antibody SP142.

In another aspect, the invention provides uses of an anti-TIGIT antagonist antibody (e.g., an anti-TIGIT antagonist antibody disclosed herein, e.g., tiragolumab) and PD-1 axis binding antagonist (e.g., anti-PD-L1 antagonist antibody (e.g., atezolizumab)) in the manufacture or preparation of a medicament for use in a method of treating a subject having a cancer (e.g., lung cancer, e.g., non-small cell lung cancer (NSCLC), e.g., squamous or non-squamous NSCLC, e.g., locally advanced unresectable NSCLC (e.g., Stage IIIB NSCLC), or recurrent or metastatic NSCLC (e.g., Stage IV NSCLC)) who has been determined to have a PD-L1-positive tumor cell fraction of greater than, or equal to, 30% (e.g., greater than, or equal to, 50%), wherein the method comprises administering to the subject one or more dosing cycles of the medicament, and wherein the medicament is formulated for administration of an effective amount of the anti-TIGIT antagonist antibody and an effective amount of the PD-1 axis binding antagonist (e.g., anti-PD-L1 antagonist antibody), and wherein the treatment results in (a) a CR or a PR and/or (b) an increase in PFS as compared to treatment with the PD-1 axis binding antagonist (e.g., anti-PD-L1 antagonist antibody) without the anti-TIGIT antagonist antibody. In some instances, the PD-L1-positive tumor cell fraction is greater than, or equal to, 50% (e.g., as determined by positive staining with the anti-PD-L1 antibody SP263 (e.g., using the Ventana assay), as determined by positive staining with the anti-PD-L1 antibody 22C3 (e.g., using the pharmDx assay), or as determined by positive staining with the anti-PD-L1 antibody 28-8). In some embodiments, the PD-L1-positive tumor cell fraction is greater than, or equal to, 30%, as determined by positive staining with the anti-PD-L1 antibody SP142.

In another aspect, the invention provides uses of an anti-TIGIT antagonist antibody in the manufacture of a medicament for use in a method of treating a subject having a cancer (e.g., lung cancer, e.g., non-small cell lung cancer (NSCLC), e.g., squamous or non-squamous NSCLC, e.g., locally advanced unresectable NSCLC (e.g., Stage IIIB NSCLC), or recurrent or metastatic NSCLC (e.g., Stage IV NSCLC)) who has been determined to have a PD-L1-positive tumor cell fraction of greater than, or equal to, 30% (e.g., greater than, or equal to, 50%), wherein the method comprises administering to the subject one or more dosing cycles of the medicament and a PD-1 axis binding antagonist (e.g., an anti-PD-L1 antagonist antibody), and wherein the medicament is formulated for administration of an effective amount of the anti-TIGIT antagonist antibody and an effective amount of the PD-1 axis binding antagonist (e.g., anti-PD-L1 antagonist antibody), and wherein the treatment results in (a) a CR or a PR and/or (b) an increase in PFS as compared to treatment with the PD-1 axis binding antagonist (e.g., anti-PD-L1 antagonist antibody) without the anti-TIGIT antagonist antibody. In some instances, the PD-L1-positive tumor cell fraction is greater than, or equal to, 50% (e.g., as determined by positive staining with the anti-PD-L1 antibody SP263 (e.g., using the Ventana assay), as determined by positive staining with the anti-PD-L1 antibody 22C3 (e.g., using the pharmDx assay), or as determined by positive staining with the anti-PD-L1 antibody 28-8). In some embodiments, the PD-L1-positive tumor cell fraction is greater than, or equal to, 30%, as determined by positive staining with the anti-PD-L1 antibody SP142.

In another aspect, the invention provides uses of a PD-1 axis binding antagonist (e.g., an anti-PD-L1 antagonist antibody) in the manufacture of a medicament for use in a method of treating a subject having a cancer (e.g., lung cancer, e.g., non-small cell lung cancer (NSCLC), e.g., squamous or non-squamous NSCLC, e.g., locally advanced unresectable NSCLC (e.g., Stage IIIB NSCLC), or recurrent or metastatic NSCLC (e.g., Stage IV NSCLC)) who has been determined to have a PD-L1-positive tumor cell fraction of greater than, or equal to, 30% (e.g., greater than, or equal to, 50%), wherein the method comprises administering to the subject one or more dosing cycles of the medicament and an anti-TIGIT antagonist antibody, and wherein the medicament is formulated for administration an effective amount of the PD-1 axis binding antagonist (e.g., anti-PD-L1 antagonist antibody) and an effective amount of the anti-TIGIT antagonist antibody is to be administered, and wherein the treatment results in (a) a CR or a PR and/or (b) an increase in PFS as compared to treatment with the PD-1 axis binding antagonist (e.g., anti-PD-L1 antagonist antibody) without the anti-TIGIT antagonist antibody. In some instances, the PD-L1-positive tumor cell fraction is greater than, or equal to, 50% (e.g., as determined by positive staining with the anti-PD-L1 antibody SP263 (e.g., using the Ventana assay), as determined by positive staining with the anti-PD-L1 antibody 22C3 (e.g., using the pharmDx assay), or as determined by positive staining with the anti-PD-L1 antibody 28-8). In some embodiments, the PD-L1-positive tumor cell fraction is greater than, or equal to, 30%, as determined by positive staining with the anti-PD-L1 antibody SP142.

In some embodiments, the anti-TIGIT antagonist antibody is administered at a dose of about 30 mg to about 1200 mg every two weeks, three weeks, or four weeks (e.g., about 30 mg to about 600 mg every two weeks, three weeks, or four weeks (e.g., about 30 mg to about 600 mg every three weeks), e.g., about 600 mg every three weeks). In some embodiments, the anti-TIGIT antagonist antibody is administered at a dose of about 600 mg every three weeks.

In some instances, the effective amount of the anti-TIGIT antagonist antibody (e.g., an anti-TIGIT antagonist antibody as disclosed herein, e.g., tiragolumab) is a fixed dose of between about 30 mg to about 1200 mg (e.g., between about 30 mg to about 1100 mg, e.g., between about 60 mg to about 1000 mg, e.g., between about 100 mg to about 900 mg, e.g., between about 200 mg to about 800 mg, e.g., between about 300 mg to about 800 mg, e.g., between about 400 mg to about 800 mg, e.g., between about 400 mg to about 750 mg, e.g., between about 450 mg to about 750 mg, e.g., between about 500 mg to about 700 mg, e.g., between about 550 mg to about 650 mg, e.g., 600 mg $\pm$ 10 mg, e.g., 600 $\pm$ 6 mg, e.g., 600 $\pm$ 5 mg, e.g., 600 $\pm$ 3 mg, e.g., 600 $\pm$ 1 mg, e.g., 600 $\pm$ 0.5 mg, e.g., 600 mg) every three weeks. In some instances, an effective amount of the anti-TIGIT antagonist antibody (e.g., an anti-TIGIT antagonist antibody as disclosed herein, e.g., tiragolumab) is a fixed dose of between about 30 mg to about 600 mg (e.g., between about 50 mg to between 600 mg, e.g., between about 60 mg to about 600 mg, e.g., between about 100 mg to about 600 mg, e.g., between about 200 mg to about 600 mg, e.g., between about 200 mg to about 550 mg, e.g., between about 250 mg to about 500 mg, e.g., between about 300 mg to about 450 mg, e.g., between about 350 mg to about 400 mg, e.g., about 375 mg) every three weeks. In some instances, the effective amount of the anti-TIGIT antagonist antibody (e.g., an anti-TIGIT antagonist antibody as disclosed herein, e.g., tiragolumab) is a fixed dose of about 600 mg every three weeks. In some instances, effective amount of the anti-TIGIT antagonist antibody (e.g., an anti-TIGIT antagonist antibody as disclosed herein, e.g., tiragolumab) is a fixed dose of 600 mg every three weeks. In some instances, the fixed dose of the anti-TIGIT antagonist antibody (e.g., an anti-TIGIT antagonist antibody as disclosed herein, e.g., tiragolumab) is to be administered in a combination therapy (e.g., a combination treatment with a PD-1 axis binding antagonist (e.g., an anti-PD-L1 antagonist antibody, e.g., atezolizumab)) may be reduced as compared to a standard dose of the anti-TIGIT antagonist antibody is to be administered as a monotherapy.

In some instances, the effective amount of the PD-1 axis binding antagonist (e.g., anti-PD-L1 antagonist antibody (e.g., atezolizumab)) is a fixed dose of between about 80 mg to about 1600 mg (e.g., between about 100 mg to about 1600 mg, e.g., between about 200 mg to about 1600 mg, e.g., between about 300 mg to about 1600 mg, e.g., between about 400 mg to about 1600 mg, e.g., between about 500 mg to about 1600 mg, e.g., between about 600 mg to about 1600 mg, e.g., between about 700 mg to about 1600 mg, e.g., between about 800 mg to about 1600 mg, e.g., between about 900 mg to about 1500 mg, e.g., between about 1000 mg to about 1400 mg, e.g., between about 1050 mg to about 1350 mg, e.g., between about 1100 mg to about 1300 mg, e.g., between about 1150 mg to about 1250 mg, e.g., between about 1175 mg to about 1225 mg, e.g., between about 1190 mg to about 1210 mg, e.g., 1200 mg $\pm$ 5 mg, e.g., 1200 $\pm$ 2.5 mg, e.g., 1200 $\pm$ 1.0 mg, e.g., 1200 $\pm$ 0.5 mg, e.g., 1200 ) every three weeks. In some instances, the effective amount of the PD-1 axis binding antagonist (e.g., anti-PD-L1 antagonist antibody (e.g., atezolizumab)) is a fixed dose of about 1200 mg every three weeks. In some instances, the effective amount of the PD-1 axis binding antagonist (e.g., anti-PD-L1 antagonist antibody (e.g., atezolizumab)) is a fixed dose of 1200 mg every three weeks.

In some embodiments, the PD-1 axis binding antagonist is administered at a dose of about 80 mg to about 2000 mg every two weeks, three weeks, or four weeks (e.g., about 840 mg every two weeks, about 1200 mg every three weeks, or about 1680 mg every four weeks). In some embodiments, the PD-1 axis binding antagonist is administered at a dose of about 1680 mg every four weeks. In some embodiments, the anti-TIGIT antagonist antibody is administered at a dose of about 600 mg every three weeks and the PD-1 axis binding antagonist is administered at a dose of about 1680 mg every four weeks. In some embodiments, the PD-1 axis binding antagonist is administered at a dose of about 1200 mg every three weeks. In some embodiments, the anti-TIGIT antagonist antibody is administered at a dose of about 600 mg every three weeks and the PD-1 axis binding antagonist is administered at a dose of about 1200 mg every three weeks. In some embodiments, the PD-1 axis binding antagonist is administered at a dose of about 840 mg every two weeks. In some embodiments, the anti-TIGIT antagonist antibody is administered at a dose of about 600 mg every three weeks and the PD-1 axis binding antagonist is administered at a dose of about 840 mg every two weeks.

In some instances, the effective amount of the PD-1 axis binding antagonist (e.g., anti-PD-L1 antagonist antibody (e.g., atezolizumab)) is a fixed dose of about 840 mg every two weeks. In some instances, the effective amount of the PD-1 axis binding antagonist (e.g., anti-PD-L1 antagonist antibody (e.g., atezolizumab)) is a fixed dose of about 1200 mg every three weeks. In some instances, the effective amount of the PD-1 axis binding antagonist (e.g., anti-PD-L1 antagonist antibody (e.g., atezolizumab)) is a fixed dose of 1680 mg every four weeks.

In some instances, the fixed dose of the PD-1 axis binding antagonist (e.g., anti-PD-L1 antagonist antibody (e.g., atezolizumab)) to be administered in a combination therapy (e.g., a combination treatment with an anti-TIGIT antagonist antibody, such as an anti-TIGIT antagonist antibody disclosed herein, e.g., tiragolumab) may be reduced as compared to a standard dose of the PD-1 axis binding antagonist (e.g., anti-PD-L1 antagonist antibody) to be administered as a monotherapy.

In some instances, the effective amount of the PD-1 axis binding antagonist (e.g., anti-PD-L1 antagonist antibody (e.g., atezolizumab)) is a dose of between about 0.01 mg/kg to about 50 mg/kg of the subject's body weight (e.g., between about 0.01 mg/kg to about 45 mg/kg, e.g., between about 0.1 mg/kg to about 40 mg/kg, e.g., between about 1 mg/kg to about 35 mg/kg, e.g., between about 2.5 mg/kg to about 30 mg/kg, e.g., between about 5 mg/kg to about 25 mg/kg, e.g., between about 10 mg/kg to about 20 mg/kg, e.g., between about 12.5 mg/kg to about 15 mg/kg, e.g., about 15 ± 2 mg/kg, about 15 t 1 mg/kg, about 15 ± 0.5 mg/kg, about 15 ± 0.2 mg/kg, or about 15 ± 0.1 mg/kg, e.g., about 15 mg/kg) every three weeks. In some instances, the effective amount of the PD-1 axis binding antagonist (e.g., anti-PD-L1 antagonist antibody (e.g., atezolizumab)) is a dose of between about 0.01 mg/kg to about 15 mg/kg of the subject's body weight (e.g., between about 0.1 mg/kg to about 15 mg/kg, e.g., between about 0.5 mg/kg to about 15 mg/kg, e.g., between about 1 mg/kg to about 15 mg/kg, e.g., between about 2.5 mg/kg to about 15 mg/kg, e.g., between about 5 mg/kg to about 15 mg/kg, e.g., between about 7.5 mg/kg to about 15 mg/kg, e.g., between about 10 mg/kg to about 15 mg/kg, e.g., between about 12.5 mg/kg to about 15 mg/kg, e.g., between about 14 mg/kg to about 15 mg/kg, e.g., about 15 ± 1 mg/kg, e.g., about 15 ± 0.5 mg/kg, e.g., about 15 ± 0.2 mg/kg, e.g., about 15 ± 0.1 mg/kg, e.g., about 15 mg/kg) every three weeks. In some instances, the effective amount of PD-1 axis binding antagonist (e.g., anti-PD-L1 antagonist antibody (e.g., atezolizumab)) is a dose of about 15 mg/kg to be administered every three weeks. In some instances, the dose of the PD-1 axis binding antagonist (e.g., anti-PD-L1 antagonist antibody (e.g., atezolizumab)) administered in a combination therapy (e.g., a combination treatment with an anti-TIGIT antagonist antibody, such as an anti-TIGIT antagonist antibody disclosed herein, e.g., tiragolumab) may be reduced as compared to a standard dose of the PD-1 axis binding antagonist (e.g., anti-PD-L1 antagonist antibody) administered as a monotherapy.

In any of the uses of the invention, the medicament comprising the anti-TIGIT antagonist antibody (e.g., an anti-TIGIT antagonist antibody as disclosed herein, e.g., tiragolumab) and the PD-1 axis binding antagonist (e.g., anti-PD-L1 antagonist antibody (e.g., atezolizumab)) may be administered in one or more dosing cycles (e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 or more dosing cycles). In some instances, the dosing cycles of the medicament comprising anti-TIGIT antagonist antibody (e.g., an anti-TIGIT antagonist antibody as disclosed herein, e.g., tiragolumab) and the PD-1 axis binding antagonist (e.g., anti-PD-L1 antagonist antibody (e.g., atezolizumab)) continue until there is a loss of clinical benefit (e.g., confirmed disease progression, drug resistance, death, or unacceptable toxicity). In some instances, the length of each dosing cycle is about 14 to 28 days (e.g., 14 days, 15 days, 16 days, 17 days, 18 days, 19 days, 20 days, 21 days, 22 days, 23 days, 24 days, 25 days, 26 days, 27 days, or 28 days).

In some instances, the length of each dosing cycle is about 21 days. In some instances, the medicament comprising the anti-TIGIT antagonist antibody (e.g., an anti-TIGIT antagonist antibody as disclosed herein, e.g., tiragolumab) is to be administered on about Day 1 (e.g., Day 1 ± 3 days) of each dosing cycle. For example, the medicament comprising the anti-TIGIT antagonist antibody (e.g., an anti-TIGIT antagonist antibody as disclosed herein, e.g., tiragolumab) is to be administered intravenously at a fixed dose of about 600 mg on Day 1 of each 21-day cycle (i.e., at a fixed dose of about 600 mg every three weeks). Similarly, in some instances, the medicament comprising the PD-1 axis binding antagonist (e.g., anti-PD-L1 antagonist antibody (e.g., atezolizumab)) is to be administered on about Day 1 (e.g., Day 1 ± 3 days) of each dosing cycle. For example, the medicament comprising the PD-1 axis binding antagonist (e.g., anti-PD-L1 antagonist antibody (e.g., atezolizumab)) is to be administered intravenously at a fixed dose of about 1200 mg on Day 1 of each 21-day cycle (i.e., at a fixed dose of about 1200 mg every three weeks). In some instances, the medicament comprising both the anti-TIGIT antagonist antibody (e.g., an anti-TIGIT antagonist antibody as disclosed herein, e.g., tiragolumab) and the PD-1 axis binding antagonist (e.g., anti-PD-L1 antagonist antibody (e.g., atezolizumab)) are to be administered on about Day 1 (e.g., Day 1 ± 3 days) of each dosing cycle. For example, the medicament comprising the anti-TIGIT antagonist antibody (e.g., an anti-TIGIT antagonist antibody as disclosed herein, e.g., tiragolumab) is to be administered intravenously at a fixed dose of about 600 mg on Day 1 of each 21-day cycle (i.e., at a fixed dose of about 600 mg every three weeks), and the medicament comprising the PD-1 axis binding antagonist (e.g., anti-PD-L1 antagonist antibody (e.g., atezolizumab)) is to be administered intravenously at a fixed dose of about 1200 mg on Day 1 of each 21-day cycle (i.e., at a fixed dose of about 1200 mg every three weeks).

In some instances, the length of each dosing cycle is about 28 days. In some instances, the medicament comprising the anti-TIGIT antagonist antibody (e.g., an anti-TIGIT antagonist antibody as disclosed herein, e.g., tiragolumab) is administered on about Day 1 (e.g., Day 1 ± 3 days) of each dosing cycle. For example, the medicament comprising the anti-TIGIT antagonist antibody (e.g., an anti-TIGIT antagonist antibody as disclosed herein, e.g., tiragolumab) is administered intravenously at a fixed dose of about 420 mg on Day 1 and Day 15 of each 28-day cycle (i.e., at a fixed dose of about 420 mg every two weeks). Similarly, in some instances, the medicament comprising the PD-1 axis binding antagonist (e.g., anti-PD-L1 antagonist antibody (e.g., atezolizumab)) is administered on about Day 1 and Day 15 (e.g., Day 1 ± 3 days and Day 15 ± 3 days) of each dosing cycle. For example, in some instances, the medicament comprising the PD-1 axis binding antagonist (e.g., anti-PD-L1 antagonist antibody (e.g., atezolizumab)) is administered intravenously at a fixed dose of about 840 mg on Day 1 and Day 15 of each 28-day cycle (i.e., at a fixed dose of about 840 mg every two weeks). In some instances, the medicament comprising both the anti-TIGIT antagonist antibody (e.g., an anti-TIGIT antagonist antibody as disclosed herein, e.g., tiragolumab) and the PD-1 axis binding antagonist (e.g., anti-PD-L1 antagonist antibody (e.g., atezolizumab)) are administered on about Day 1 and Day 15 (e.g., Day 1 ± 3 days and Day 15 ± 3 days) of each dosing cycle. For example, the medicament comprising the anti-TIGIT antagonist antibody (e.g., an anti-TIGIT antagonist antibody as disclosed herein, e.g., tiragolumab) is administered intravenously at a fixed dose of about 420 mg on Day 1 and Day 15 of each 28-day cycle (i.e., at a fixed dose of about 420 mg every two weeks), and the medicament comprising the PD-1 axis binding antagonist (e.g., anti-PD-L1 antagonist antibody (e.g., atezolizumab)) is administered intravenously at a fixed dose of about 840 mg on Day 1 and Day 15 of each 28-day cycle (i.e., at a fixed dose of about 840 mg every two weeks).

In some instances, the medicament comprising the anti-TIGIT antagonist antibody (e.g., an anti-TIGIT antagonist antibody as disclosed herein, e.g., tiragolumab) is administered on about Day 1 (e.g., Day 1 ± 3 days) of each 28-day dosing cycle. For example, the medicament comprising the anti-TIGIT antagonist antibody (e.g., an anti-TIGIT antagonist antibody as disclosed herein, e.g., tiragolumab) is administered intravenously at a fixed dose of about 840 mg on Day 1 of each 28-day cycle (i.e., at a fixed dose of about 420 mg every four weeks). Similarly, in some instances, the medicament comprising the PD-1 axis binding antagonist (e.g., anti-PD-L1 antagonist antibody (e.g., atezolizumab)) is administered on about Day 1 (e.g., Day 1 ± 3 days) of each dosing cycle. For example, in some instances, the medicament comprising the PD-1 axis binding antagonist (e.g., anti-PD-L1 antagonist antibody (e.g., atezolizumab)) is administered intravenously at a fixed dose of about 1680 mg on Day 1 of each 28-day cycle (i.e., at a fixed dose of about 840 mg every four weeks). In some instances, the medicament comprising both the anti-TIGIT antagonist antibody (e.g., an anti-TIGIT antagonist antibody as disclosed herein, e.g., tiragolumab) and the PD-1 axis binding antagonist (e.g., anti-PD-L1 antagonist antibody (e.g., atezolizumab)) are administered on about Day 1 (e.g., Day 1 ± 3 days) of each dosing cycle. For example, the medicament comprising the anti-TIGIT antagonist antibody (e.g., an anti-TIGIT antagonist antibody as disclosed herein, e.g., tiragolumab) is administered intravenously at a fixed dose of about 840 mg on Day 1 of each 28-day cycle (i.e., at a fixed dose of about 820 mg every four weeks), and the medicament comprising the PD-1 axis binding antagonist (e.g., anti-PD-L1 antagonist antibody (e.g., atezolizumab)) is administered intravenously at a fixed dose of about 1680 mg on Day 1 of each 28-day cycle (i.e., at a fixed dose of about 1680 mg every fourweeks).In some instances, the medicament comprising the anti-TIGIT antagonist antibody (e.g., an anti-TIGIT antagonist antibody as disclosed herein, e.g., tiragolumab) is administered to the subject by intravenous infusion over about 60 t 10 minutes (e.g., about 50 minutes, about 51 minutes, about 52 minutes, about 53 minutes, about 54 minutes, about 55 minutes, about 56 minutes, about 57 minutes, about 58 minutes, about 59 minutes, about 60 minutes, about 61 minutes, about 62 minutes, about 63 minutes, about 64 minutes, about 65 minutes, about 66 minutes, about 67 minutes, about 68 minutes, about 69 minutes, or about 70 minutes). In some instances, the medicament comprising the PD-1 axis binding antagonist (e.g., anti-PD-L1 antagonist antibody (e.g., atezolizumab)) is to be administered to the subject by intravenous infusion over about 60 ± 15 minutes (e.g. about 45 minutes, about 46 minutes, about 47 minutes, about 48 minutes, about 49 minutes, about 50 minutes, about 51 minutes, about 52 minutes, about 53 minutes, about 54 minutes, about 55 minutes, about 56 minutes, about 57 minutes, about 58 minutes, about 59 minutes, about 60 minutes, about 61 minutes, about 62 minutes, about 63 minutes, about 64 minutes, about 65 minutes, about 66 minutes, about 67 minutes, about 68 minutes, about 69 minutes, about 70 minutes, about 71 minutes, about 72 minutes, about 73 minutes, about 74 minutes, or about 75 minutes).

In some instances, the medicament comprising the anti-TIGIT antagonist antibody (e.g., an anti-TIGIT antagonist antibody as disclosed herein, e.g., tiragolumab) is to be administered to the subject before the medicament comprising the PD-1 axis binding antagonist (e.g., anti-PD-L1 antagonist antibody (e.g., atezolizumab)). In some instances, for example, following administration of the medicament comprising the anti-TIGIT antagonist antibody and before administration of the medicament comprising the PD-1 axis binding antagonist (e.g., anti-PD-L1 antagonist antibody), the method includes an intervening first observation period. In some instances, the method further includes a second observation period following administration of the PD-1 axis binding antagonist (e.g., anti-PD-L1 antagonist antibody). In some instances, the method includes both a first observation period following administration of the medicament comprising the anti-TIGIT antagonist antibody and second observation period following administration of the medicament comprising the PD-1 axis binding antagonist (e.g., anti-PD-L1 antagonist antibody). In some instances, the first and second observation periods are each between about 30 minutes to about 60 minutes in length. In instances in which the first and second observation periods are each about 60 minutes in length, the method may include recording the subject's vital signs (e.g., pulse rate, respiratory rate, blood pressure, and temperature) at about 30 $\pm$ 10 minutes after administration of the medicament comprising the anti-TIGIT antagonist antibody and the medicament comprising the PD-1 axis binding antagonist (e.g., anti-PD-L1 antagonist antibody) during the first and second observation periods, respectively. In instances in which the first and second observation periods are each about 30 minutes in length, the method may include recording the subject's vital signs (e.g., pulse rate, respiratory rate, blood pressure, and temperature) at about 15 $\pm$ 10 minutes after administration of the medicament comprising the anti-TIGIT antagonist antibody and the medicament comprising the PD-1 axis binding antagonist (e.g., anti-PD-L1 antagonist antibody) during the first and second observation periods, respectively.

In other instances, the medicament comprising the PD-1 axis binding antagonist (e.g., anti-PD-L1 antagonist antibody (e.g. atezolizumab)) is to be administered to the subject before the anti-TIGIT antagonist antibody (e.g., an anti-TIGIT antagonist antibody as disclosed herein, e.g., tiragolumab). In some instances, for example, following administration of the medicament comprising the PD-1 axis binding antagonist (e.g., anti-PD-L1 antagonist antibody) and before administration of the medicament comprising the anti-TIGIT antagonist antibody, the method includes an intervening first observation period. In some instances, the method includes a second observation period following administration of the medicament comprising the anti-TIGIT antagonist antibody. In some instances, the method includes both a first observation period following administration of the medicament comprising the PD-1 axis binding antagonist (e.g., anti-PD-L1 antagonist antibody) and second observation period following administration of the medicament comprising the anti-TIGIT antagonist antibody. In some instances, the first and second observation periods are each between about 30 minutes to about 60 minutes in length. In instances in which the first and second observation periods are each about 60 minutes in length, the method may include recording the subject's vital signs (e.g., pulse rate, respiratory rate, blood pressure, and temperature) at about 30 $\pm$ 10 minutes after administration of the medicament comprising the PD-1 axis binding antagonist (e.g., anti-PD-L1 antagonist antibody) and the medicament comprising the anti-TIGIT antagonist antibody during the first and second observation periods, respectively. In instances in which the first and second observation periods are each about 30 minutes in length, the method may include recording the subject's vital signs (e.g., pulse rate, respiratory rate, blood pressure, and temperature) at about 15 $\pm$ 10 minutes after administration of the medicament comprising the PD-1 axis binding antagonist (e.g., anti-PD-L1 antagonist antibody) and the medicament comprising the anti-TIGIT antagonist antibody during the first and second observation periods, respectively.

In other instances, the medicament comprising the anti-TIGIT antagonist antibody (e.g., an anti-TIGIT antagonist antibody as disclosed herein, e.g., tiragolumab) and the medicament comprising the PD-1 axis binding antagonist (e.g., anti-PD-L1 (atezolizumab) antagonist antibody) is to be administered to the subject simultaneously. In some instances, for example, following administration of the medicament comprising the anti-TIGIT antagonist antibody and the medicament comprising the PD-1 axis binding antagonist (e.g., anti-PD-L1 antagonist antibody) the method includes an observation period. In some instances, the observation period is between about 30 minutes to about 60 minutes in length. In instances in which the observation period is about 60 minutes in length, the method may include recording the subject's vital signs (e.g., pulse rate, respiratory rate, blood pressure, and temperature) at about 30 $\pm$ 10 minutes after administration of the medicament comprising the PD-1 axis binding antagonist (e.g., anti-PD-L1 antagonist antibody) and the medicament comprising the anti-TIGIT antagonist antibody during the observation period. In instances in which the observation period is about 30 minutes in length, the method may include recording the subject's vital signs (e.g., pulse rate, respiratory rate, blood pressure, and temperature) at about 15 $\pm$ 10 minutes after administration of the medicament comprising the PD-1 axis binding antagonist (e.g., anti-PD-L1 antagonist antibody) and the medicament comprising the anti-TIGIT antagonist antibody during the observation period.

In another aspect, the invention provides uses of an anti-TIGIT antagonist antibody (e.g., an anti-TIGIT antagonist antibody disclosed herein, e.g., tiragolumab) and PD-1 axis binding antagonist (e.g., anti-PD-L1 antagonist antibody (e.g., atezolizumab)) in the manufacture or preparation of a medicament for use in a method of treating a subject having a cancer (e.g., lung cancer, e.g., non-small cell lung cancer (NSCLC), e.g., squamous or non-squamous NSCLC, e.g., locally advanced unresectable NSCLC (e.g., Stage IIIB NSCLC), or recurrent or metastatic NSCLC (e.g., Stage IV NSCLC)) who has been determined to have a PD-L1-positive tumor cell fraction of greater than, or equal to, 30% (e.g.,

greater than, or equal to, 50%), wherein the method comprises administering to the subject one or more dosing cycles of the medicament, wherein the medicament is formulated for administration of the anti-TIGIT antagonist antibody at a fixed dose of between about 30 mg to about 1200 mg every three weeks and the PD-1 axis binding antagonist (e.g., anti-PD-L1 antagonist antibody) at a fixed dose of between about 80 mg to about 1600 mg every three weeks, and wherein the treatment results in (a) a CR or a PR and/or (b) an increase in PFS as compared to treatment with the PD-1 axis binding antagonist (e.g., anti-PD-L1 antagonist antibody) without the anti-TIGIT antagonist antibody.

In another aspect, the invention provides uses of a PD-1 axis binding antagonist (e.g., an anti-PD-L1 antagonist antibody) in the manufacture of a medicament for use in a method of treating a subject having a cancer (e.g., lung cancer, e.g., non-small cell lung cancer (NSCLC), e.g., squamous or non-squamous NSCLC, e.g., locally advanced unresectable NSCLC (e.g., Stage IIIB NSCLC), or recurrent or metastatic NSCLC (e.g., Stage IV NSCLC)) who has been determined to have a PD-L1- positive tumor cell fraction of greater than, or equal to, 30% (e.g., greater than, or equal to, 50%, wherein the method comprises administering to the subject one or more dosing cycles of the medicament and an anti-TIGIT antagonist antibody, wherein the medicament is formulated for administration of the PD-1 axis binding antagonist (e.g., anti-PD-L1 antagonist antibody) at a fixed dose of between about 80 mg to about 1600 mg every three weeks and the anti-TIGIT antagonist antibody is to be administered at a fixed dose of between about 30 mg to about 1200 mg every three weeks, and wherein the treatment results in (a) a CR or a PR and/or (b) an increase in PFS as compared to treatment with the PD-1 axis binding antagonist (e.g., anti-PD-L1 antagonist antibody) without the anti-TIGIT antagonist antibody. In some instances, the PD-L1-positive tumor cell fraction is greater than, or equal to, 50% (e.g., as determined by positive staining with the anti-PD-L1 antibody SP263 (e.g., using the Ventana assay), as determined by positive staining with the anti-PD-L1 antibody 22C3 (e.g., using the pharmDx assay), or as determined by positive staining with the anti-PD-L1 antibody 28-8). In some embodiments, the PD-L1-positive tumor cell fraction is greater than, or equal to, 30%, as determined by positive staining with the anti-PD-L1 antibody SP142.

In another aspect, the invention provides uses of an anti-TIGIT antagonist antibody in the manufacture of a medicament for use in a method of treating a subject having a cancer (e.g., lung cancer, e.g., non-small cell lung cancer (NSCLC), e.g., squamous or non-squamous NSCLC, e.g., locally advanced unresectable NSCLC (e.g., Stage IIIB NSCLC), or recurrent or metastatic NSCLC (e.g., Stage IV NSCLC)) who has been determined to have a PD-L1-positive tumor cell fraction of greater than, or equal to, 30% (e.g., greater than, or equal to, 50%, wherein the method comprises administering to the subject one or more dosing cycles of the medicament and a PD-1 axis binding antagonist (e.g., anti-PD-L1 antagonist antibody), wherein the medicament is formulated for administration of the anti-TIGIT antagonist antibody at a fixed dose of between about 30 mg to about 1200 mg every three weeks and the PD-1 axis binding antagonist (e.g., anti-PD-L1 antagonist antibody) at a fixed dose of between about 80 mg to about 1600 mg every three weeks, and wherein the treatment results in (a) a CR or a PR and/or (b) an increase in PFS as compared to treatment with the PD-1 axis binding antagonist (e.g., anti-PD-L1 antagonist antibody) without the anti-TIGIT antagonist antibody. In some instances, the PD-L1-positive tumor cell fraction is greater than, or equal to, 50% (e.g., as determined by positive staining with the anti-PD-L1 antibody SP263 (e.g., using the Ventana assay), as determined by positive staining with the anti-PD-L1 antibody 22C3 (e.g., using the pharmDx assay), or as determined by positive staining with the anti-PD-L1 antibody 28-8). In some embodiments, the PD-L1-positive tumor cell fraction is greater than, or equal to, 30%, as determined by positive staining with the anti-PD-L1 antibody SP142.

In another aspect, the invention provides uses of an anti-TIGIT antagonist antibody and atezolizumab in the manufacture of a medicament for use in a method of treating a subject having a cancer (e.g., lung cancer, e.g., non-small cell lung cancer (NSCLC), e.g., squamous or non-squamous NSCLC, e.g., locally advanced unresectable NSCLC (e.g., Stage IIIB NSCLC), or recurrent or metastatic NSCLC (e.g., Stage IV NSCLC)) who has been determined to have a PD-L1-positive tumor cell fraction of greater than, or equal to, 30% (e.g., greater than, or equal to, 50%, wherein the method comprises administering to the subject one or more dosing cycles of the medicament, wherein the medicament is formulated for administration of the anti-TIGIT antagonist antibody at a fixed dose of 600 mg every three weeks and atezolizumab at a fixed dose of 1200 mg every three weeks, wherein the anti-TIGIT antagonist antibody comprises: a VH domain comprising the amino acid sequence of SEQ ID NO: 17 or 18; and a VL domain comprising the amino acid sequence of SEQ ID NO: 19, and wherein the treatment results in (a) a CR or a PR and/or (b) an increase in PFS as compared to treatment with atezolizumab without the anti-TIGIT antagonist antibody, as described in further detail below. In some instances, the PD-L1-positive tumor cell fraction is greater than, or equal to, 50% (e.g., as determined by positive staining with the anti-PD-L1 antibody SP263 (e.g., using the Ventana assay), as determined by positive staining with the anti-PD-L1 antibody 22C3 (e.g., using the pharmDx assay), or as determined by positive staining with the anti-PD-L1 antibody 28-8). In some embodiments, the PD-L1-positive tumor cell fraction is greater than, or equal to, 30%, as determined by positive staining with the anti-PD-L1 antibody SP142.

In another aspect, the invention provides uses of an anti-TIGIT antagonist antibody in the manufacture of a medicament for use in a method of treating a subject having a cancer (e.g., lung cancer, e.g., non-small cell lung cancer (NSCLC), e.g., squamous or non-squamous NSCLC, e.g., locally advanced unresectable NSCLC (e.g., Stage IIIB NSCLC), or recurrent or metastatic NSCLC (e.g., Stage IV NSCLC)) who has been determined to have a PD-L1-positive tumor cell fraction of greater than, or equal to, 30% (e.g., greater than, or equal to, 50%, wherein the method comprises administering to the subject one or more dosing cycles of the medicament and atezolizumab, wherein the medicament is formulated for administration of the anti-TIGIT antagonist antibody at a fixed dose of 600 mg every three weeks and atezolizumab is to be administered at a fixed dose of 1200 mg every three weeks, wherein the anti-TIGIT antagonist antibody comprises: a VH domain comprising the amino acid sequence of SEQ ID NO: 17 or 18; and a VL domain comprising the amino acid sequence of SEQ ID NO: 19, as described in further detail below, and wherein the treatment results in (a) a CR or a PR and/or (b) an increase in PFS as compared to treatment with atezolizumab without the anti-TIGIT antagonist antibody. In some instances, the PD-L1-positive tumor cell fraction is greater than, or equal to, 50% (e.g., as determined by positive staining with the anti-PD-L1 antibody SP263 (e.g., using the Ventana assay), as determined by positive staining with the anti-PD-L1 antibody 22C3 (e.g., using the pharmDx assay), or as determined by positive staining with the anti-PD-L1 antibody 28-8). In some embodiments, the PD-L1-positive tumor cell fraction is greater than, or equal to, 30%, as determined by positive staining with the anti-PD-L1 antibody SP142.

In another aspect, the invention provides uses of atezolizumab in the manufacture of a medicament for use in a method of treating a subject having a cancer (e.g., lung cancer, e.g., non-small cell lung cancer (NSCLC), e.g., squamous or non-squamous NSCLC, e.g., locally advanced unresectable NSCLC (e.g., Stage IIIB NSCLC), or recurrent or metastatic NSCLC (e.g., Stage IV NSCLC)) who has been determined to have a PD-L1-positive tumor cell fraction of greater than, or equal to, 30% (e.g., greater than, or equal to, 50%, wherein the method comprises administering to the subject one or more dosing cycles of the medicament and an anti-TIGIT antibody, wherein the medicament is formulated for administration of atezolizumab at a fixed dose of 1200 mg every three weeks and the anti-TIGIT antagonist antibody is to be administered at a fixed dose of 600 mg every three weeks, wherein the anti-TIGIT antagonist antibody comprises: a VH domain comprising the amino acid sequence of SEQ ID NO: 17 or 18; and a VL domain comprising the amino acid sequence of SEQ ID NO: 19, as described in further detail below, and wherein the treatment results in (a) a CR or a PR and/or (b) an increase in PFS as compared to treatment with atezolizumab without the anti-TIGIT antagonist antibody. In some instances, the PD-L1-positive tumor cell fraction is greater than, or equal to, 50% (e.g., as determined by positive staining with the anti-PD-L1 antibody SP263 (e.g., using the Ventana assay), as determined by positive staining with the anti-PD-L1 antibody 22C3 (e.g., using the pharmDx assay), or as determined by positive staining with the anti-PD-L1 antibody 28-8). In some embodiments, the PD-L1-positive tumor cell fraction is greater than, or equal to, 30%, as determined by positive staining with the anti-PD-L1 antibody SP142.

In another aspect, the invention provides uses of tiragolumab and atezolizumab in the manufacture of a medicament for use in a method of treating a subject having a cancer (e.g., lung cancer, e.g., non-small cell lung cancer (NSCLC), e.g., squamous or non-squamous NSCLC, e.g., locally advanced unresectable NSCLC (e.g., Stage IIIB NSCLC), or recurrent or metastatic NSCLC (e.g., Stage IV NSCLC)) who has been determined to have a PD-L1-positive tumor cell fraction of greater than, or equal to, 30% (e.g., greater than, or equal to, 50%, wherein the method comprises administering to the subject one or more dosing cycles of the medicament, wherein the medicament is formulated for administration of tiragolumab at a fixed dose of 600 mg every three weeks and atezolizumab at a fixed dose of 1200 mg every three weeks, and wherein the treatment results in (a) a CR or a PR and/or (b) an increase in PFS as compared to treatment with atezolizumab without tiragolumab. In some instances, the PD-L1-positive tumor cell fraction is greater than, or equal to, 50% (e.g., as determined by positive staining with the anti-PD-L1 antibody SP263 (e.g., using the Ventana assay), as determined by positive staining with the anti-PD-L1 antibody 22C3 (e.g., using the pharmDx assay), or as determined by positive staining with the anti-PD-L1 antibody 28-8). In some embodiments, the PD-L1-positive tumor cell fraction is greater than, or equal to, 30%, as determined by positive staining with the anti-PD-L1 antibody SP142.

In another aspect, the invention provides uses of tiragolumab in the manufacture of a medicament for use in a method of treating a subject having a cancer (e.g., lung cancer, e.g., non-small cell lung cancer (NSCLC), e.g., squamous or non-squamous NSCLC, e.g., locally advanced unresectable NSCLC (e.g., Stage IIIB NSCLC), or recurrent or metastatic NSCLC (e.g., Stage IV NSCLC)) who has been determined to have a PD-L1-positive tumor cell fraction of greater than, or equal to , 30% (e.g., greater than, or equal to, 50%, wherein the method comprises administering to the subject one or more dosing cycles of the medicament and atezolizumab, wherein the medicament is formulated for administration of tiragolumab at a fixed dose of 600 mg every three weeks and atezolizumab is to be administered at a fixed dose of 1200 mg every three weeks, and wherein the treatment results in (a) a CR or a PR and/or (b) an increase in PFS as compared to treatment with atezolizumab without tiragolumab. In some instances, the PD-L1-positive tumor cell fraction is greater than, or equal to, 50% (e.g., as determined by positive staining with the anti-PD-L1 antibody SP263 (e.g., using the Ventana assay), as determined by positive staining with the anti-PD-L1 antibody 22C3 (e.g., using the pharmDx assay), or as determined by positive staining with the anti-PD-L1 antibody 28-8). In some embodiments, the PD-L1-positive tumor cell fraction is greater than, or equal to, 30%, as determined by positive staining with the anti-PD-L1 antibody SP142.

In another aspect, the invention provides uses of atezolizumab in the manufacture of a medicament for use in a method of treating a subject having a cancer (e.g., lung cancer, e.g., non-small cell lung cancer (NSCLC), e.g., squamous or non-squamous NSCLC, e.g., locally advanced unresectable NSCLC (e.g., Stage IIIB NSCLC), or recurrent or metastatic NSCLC (e.g., Stage IV NSCLC)) who has been determined to have a PD-L1-positive tumor cell fraction of greater than, or equal to, 30% (e.g., greater than, or equal to, 50%, wherein the method comprises administering to the subject one or more dosing cycles of the medicament and tiragolumab, wherein the medicament is formulated for administration of atezolizumab at a fixed dose of 1200 mg every three weeks and tiragolumab is to be administered at a fixed dose of 600 mg every three weeks, and wherein the treatment results in (a) a CR or a PR and/or (b) an increase in PFS as compared to treatment with atezolizumab without tiragolumab. In some instances, the PD-L1-positive tumor cell fraction is greater than, or equal to, 50% (e.g., as determined by positive staining with the anti-PD-L1 antibody SP263 (e.g., using the Ventana assay), as determined by positive staining with the anti-PD-L1 antibody 22C3 (e.g., using the pharmDx assay), or as determined by positive staining with the anti-PD-L1 antibody 28-8). In some embodiments, the PD-L1-positive tumor cell fraction is greater than, or equal to, 30%, as determined by positive staining with the anti-PD-L1 antibody SP142.

In any of the methods, uses, or compositions for use described herein, the anti-TIGIT antagonist antibody (e.g., an anti-TIGIT antagonist antibody as disclosed herein, e.g., tiragolumab) and PD-1 axis binding antagonist (e.g., anti-PD-L1 antibody (e.g., atezolizumab)), or a medicament thereof, may be administered in conjunction with (either separately or together), one or more additional anti-cancer therapeutic agent(s) (e.g., an immunomodulatory agent (e.g., an agent that decreases or inhibits one or more immune co-inhibitory receptors (e.g., one or more immune co-inhibitory receptors selected from TIGIT, PD-L1, PD-1, CTLA-4, LAG3, TIM3, BTLA, and/or VISTA), such as a CTLA-4 antagonist, e.g., an anti-CTLA-4 antagonist antibody (e.g., ipilimumab (YERVOY®)), or an agent that increases or activates one or more immune co-stimulatory receptors (e.g., one or more immune co-stimulatory receptors selected from CD226, OX-40, CD28, CD27, CD137, HVEM, and/or GITR), such as an OX-40 agonist, e.g., an OX-40 agonist antibody), a chemotherapeutic agent, a cytotoxic agent, a growth inhibitory agent, a radiotherapy/radiation therapy, and/or an anti-hormonal agent, such as those recited herein above).

In any of the methods, uses, or compositions for use described herein, the anti-TIGIT antagonist antibody (e.g., an anti-TIGIT antagonist antibody as disclosed herein, e.g., tiragolumab) and PD-1 axis binding antagonist (e.g., anti-PD-L1 antibody (e.g., atezolizumab)), or a medicament thereof, is for treating a subject having a lung cancer. In some instances, the lung cancer is a NSCLC. The cancer may be at an early or late stage. In some instances, the NSCLC is a squamous NSCLC. In some instances, the NSCLC is a non-squamous NSCLC. In some instances, the NSCLC is a locally advanced unresectable NSCLC. In some instances, the NSCLC is a Stage IIIB NSCLC. In some instances, the NSCLC is a recurrent or metastatic NSCLC. In some instances, the NSCLC is a Stage IV NSCLC. In some instances, the subject has not been previously treated for Stage IV NSCLC.

In some instances, in any of the methods, uses, or compositions for use described herein, the subject has no EGFR or ALK genomic tumor aberrations. In some instances, in any of the methods, uses, or compositions for use described herein, the subject does not have a sensitizing epidermal growth factor receptor (*EGFR*) gene mutation or anaplastic lymphoma kinase (*ALK*) gene rearrangement. In some instances, the subject has an Eastern Cooperative Oncology Group (ECOG) Performance Status (PS) of 0 or 1.

Methods for detecting the mutational status *EGFR* and *ALK* are well known in the art, and include, but are not limited to, sequencing DNA from clinical samples (e.g., tumor biopsies or blood samples (e.g., circulating tumor DNA in blood)) using a next-generation sequencing method, such as the targeted gene pulldown and sequencing method described in Frampton et al. (Nature Biotechnology. 31(11): 1023-1033, 2013), which is incorporated by reference herein in its entirety. Such a next-generation sequencing method can be used with any of the methods disclosed herein to detect various mutations (e.g., insertions, deletions, base substitutions, focal gene amplifications, and/or homozygous gene deletions), while enabling the use of small samples (e.g., from small-core needle biopsies, fine-needle aspirations, and/or cell blocks) or fixed samples (e.g., formalin-fixed and paraffin-embedded (FFPE) samples). Other methods for the detection of the mutational status of *EGFR* and *ALK* include fluorescence in situ hybridization (FISH) and immunohistochemical (IHC) methods. Exemplary methods for the detection of the mutational status of *ALK* are disclosed in U.S. Patent No: 9,651,555, which is herein incorporated by reference in its entirety. In some instances, the VENTANA® anti-ALK (D5F3) IHC assay is used to determine the mutational status of the *ALK* gene.

In some instances of any of the methods described herein, the mutation is a sensitizing *EGFR* mutation. Sensitizing *EGFR* mutations are well known in the art and include those described in U.S. Publication No: US 2018/0235968 and in Juan et al. (Therapeutic Advances in Medical Oncology. 9(3): 201-216, 2017), which are incorporated by reference herein in their entireties. In some instances, the sensitizing *EGFR* mutation is a mutation in any one of exons 18-21 (e.g., a mutation in exon 18, exon 19, exon 20, and/or exon 21). In some instances, the sensitizing *EGFR* mutation is a deletion of exon 19 (del19). In other instances, sensitizing *EGFR* mutation is a L858R point mutation in exon 21. In some instances, the sensitizing *EGFR* mutation is a G719X point mutation in exon 18, wherein "X" is most commonly C, A, or S. In some instances, the sensitizing *EGFR* mutation is a G719S point mutation in exon 18. In some instances, the sensitizing *EGFR* mutation is a G719A point mutation in exon 18. In some instances, the sensitizing *EGFR* mutation is a S720F point mutation in exon 18. In some instances, the sensitizing *EGFR* mutation is a L861Q point mutation in exon 21. In some instances, the sensitizing *EGFR* mutation is a L861R point mutation in exon 21. In other instances, the sensitizing *EGFR* mutation is a T790M point mutation. In some instances, the sensitizing *EGFR* mutation is an E709X point mutation, where "X" is most commonly K, A, or H. In some instances, the sensitizing *EGFR* mutation is a S768I point mutation.

In some instances of any of the methods described herein, the mutation is an *ALK* gene rearrangement. *ALK* gene rearrangements are well known in the art and include those described in U.S. Patent No: 9,651,555 and in Du et al. (Thoracic Cancer. 9: 423-430, 2018), which are incorporated herein by reference in their entireties. In some instances, the *ALK* gene rearrangement results in the creation of an oncogenic ALK tyrosine kinase that activates downstream signaling pathways resulting in increased cell proliferation and survival. In some instances, the *ALK* gene rearrangement is an *ALK* rearrangement with a gene selected from the group consisting of *EML4, KIF5B, KLC1, TFG, TPR, HIP1, STRN, DCTN1, SQSTM1, NPM1, BCL11A, BIRC6, RANBP2, ATIC, CLTC, TMP4,* and MSN resulting in the formation of a fusion oncogene. In some instances, the *ALK* gene rearrangement is an *EML4* rearrangement with *ALK* resulting in the formation of the fusion oncogene *EML4-ALK.*

In some instances, in any of the methods, uses, or compositions for use described herein, the subject does not have a pulmonary lymphoepithelioma-like carcinoma subtype of NSCLC. Methods for detecting the subtype of NSCLC are well known in the art, and include, but are not limited to, methods of determination by histopathological criteria, or by molecular features (e.g., a subtype characterized by expression of one or a combination of biomarkers (e.g., particular genes or proteins encoded by said genes)). In some instances, the sample is selected from the group consisting of a tissue sample, a whole blood sample, a serum sample, and a plasma sample. In some instances, the tissue sample is a tumor sample.

In some instances, in any of the methods, uses, or compositions for use described herein, the subject does not have an active Epstein-Barr virus (EBV) infection or a known or suspected chronic active EBV infection. Indicators of active or chronic active EBV infections for use in the methods described herein can include, but are not limited to, EBV IgM, EBV IgG, Epstein-Barr nuclear antigen (EBNA), and Epstein-Barr viral particles detected in a sample from the subject (e.g., a blood or serum sample). Methods for detecting the presence of one or more indicators of active or chronic active EBV infection, including EBV IgM, EBV IgG, Epstein-Barr nuclear antigen (EBNA), and Epstein-Barr viral particles in a sample from a subject are well known in the art, and include, but are not limited to, methods involving serological diagnosis (e.g., the detection of EBV DNA (e.g., by PCR analysis of a blood sample for the detection of EBV viral particles) or EBV antigens or anti-EBV antibodies (e.g., detection of EBNA, EBV IgM, or EBV IgG using heterophilic antibodies). In some instances, the sample is selected from the group consisting of a whole blood sample, a serum sample, and a plasma sample. In some instances, the subject is negative for EBV IgM and/or negative by EBV PCR. In some instances, the subject is negative for EBV IgM and/or negative by EBV PCR and is positive for EBV IgG and/or positive for Epstein-Barr nuclear antigen (EBNA). In other instances, the subject is negative for EBV IgG and/or negative for EBNA.

In some instances, in any of the methods, uses, or compositions for use described herein, the subject has a PD-L1 selected tumor (e.g., a tumor having high PD-L1 expression, e.g., a tumor PD-L1 expression with a minimum PD-L1-positive tumor cell fraction or TPS $\geq$ 30% (e.g., $\geq$ 50%) as determined by an IHC with the SP263 or 22C3 antibody). In some instances, the PD-L1 selected tumor is a tumor that has been determined to have a PD-L1-positive tumor cell fraction or PD-L1 TPS of greater than, or equal to, 30% (e.g., greater than, or equal to, 50%) by an immunohistochemical (IHC) assay. In some instances, the IHC assay uses the anti-PD-L1 antibody SP263, 22C3, SP142, or 28-8. In some instances, the IHC assay uses anti-PD-L1 antibody SP263. In some instances, the IHC assay uses anti-PD-L1 antibody 22C3. In some instances, the tumor sample has been determined to have a TPS of greater than, or equal to, 50%. In some instances, the PD-L1-positive tumor cell fraction is greater than, or equal to, 50% (e.g., as determined by positive staining with the anti-PD-L1 antibody SP263 (e.g., using the Ventana assay), as determined by positive staining with the anti-PD-L1 antibody 22C3 (e.g., using the pharmDx assay), or as determined by positive staining with the anti-PD-L1 antibody 28-8). In some embodiments, the PD-L1-positive tumor cell fraction is greater than, or equal to, 30%, as determined by positive staining with the anti-PD-L1 antibody SP142.

In some instances, in any of the methods, uses, or compositions for use described herein, a tumor sample obtained from the individual has a detectable protein expression level of PD-L1. In some instances, the detectable protein expression level of PD-L1 has been determined by an IHC assay. In some instances, the IHC assay uses anti-PD-L1 antibody SP142. In some instances, the tumor sample has been determined to have a detectable expression level of PD-L1 in greater than, or equal to, 1% of the tumor cells in the tumor sample. In some instances, the tumor sample has been determined to have a detectable expression level of PD-L1 in greater than, or equal to, 1% and less than 5% of the tumor cells in the tumor sample. In some instances, the tumor sample has been determined to have a detectable expression level of PD-L1 in greater than, or equal to, 5% and less than 50% of the tumor cells in the tumor sample. In some instances, the tumor sample has been determined to have a detectable expression level of PD-L1 in greater than, or equal to, 50% of the tumor cells in the tumor sample. In some instances, the tumor sample has been determined to have a detectable expression level of PD-L1 in tumor-infiltrating immune cells that comprise greater than, or equal to, 1% of the tumor sample. In some instances, the tumor sample has been determined to have a detectable expression level of PD-L1 in tumor-infiltrating immune cells that comprise greater than, or equal to, 1% and less than 5% of the tumor sample. In some instances, the tumor sample has been determined to have a detectable expression level of PD-L1 in tumor-infiltrating immune cells that comprise greater than, or equal to, 5% and less than 10% of the tumor sample. In some instances, the tumor sample has been determined to have a detectable expression level of PD-L1 in tumor-infiltrating immune cells that comprise greater than, or equal to, 10% of the tumor sample.

In some instances, a PD-L1-positive tumor cell fraction of the subject is determined. In some embodiments, the PD-L1-positive tumor cell fraction is determined by positive staining with an anti-PD-L1 antibody, wherein the anti-PD-L1 antibody is SP263, 22C3, SP142, or 28-8 (e.g., as part of an IHC assay). In some embodiments, the PD-L1-positive tumor cell fraction is greater than or equal to 1% tumor cell (TC), as determined by positive staining with an anti-PD-L1 antibody SP263 (e.g., as calculated using the Ventana SP263 IHC assay) or 22C3 (e.g., as calculated using the pharmDx 22C3 IHC assay). In some embodiments, the PD-L1-positive tumor cell fraction is less than 1% TC (e.g., from 0% to 1% TC, e.g., PD-L1-negative), as determined by positive staining with an anti-PD-L1 antibody SP263 (e.g., as calculated using the Ventana SP263 IHC assay) or 22C3 (e.g., as calculated using the pharmDx 22C3 IHC assay). In some instances, in any of the methods, uses, or compositions for use described herein, a tumor sample obtained from the individual has a detectable nucleic acid expression level of PD-L1. In some instances, the detectable nucleic acid expression level of PD-L1 has been determined by RNA-seq, RT-qPCR, qPCR, multiplex qPCR or RT-qPCR, microarray analysis, SAGE, MassARRAYtechnique, ISH, or a combination thereof. In some instances, the sample is selected from the group consisting of a tissue sample, a whole blood sample, a serum sample, and a plasma sample. In some instances, the tissue sample is a tumor sample. In some instances, the tumor sample comprises tumor-infiltrating immune cells, tumor cells, stromal cells, and any combinations thereof.

In some instances, the methods, uses, and/or compositions for use described herein involve treating a subject having a lung cancer (e.g., NSCLC, e.g., squamous NSCLC or non-squamous NSCLC, locally advanced unresectable NSCLC (e.g., a Stage III NSCLC (e.g., a Stage IIIA NSCLC, Stage IIIB NSCLC, and/or Stage IIIC NSCLC))) who has previously received cCRT for lung cancer and has not progressed after the cCRT (e.g., as determined by radiographic disease progression after the cCRT). In some instances, the methods, uses, or compositions for use described herein involve treating a subject having an NSCLC, e.g., squamous NSCLC or non-squamous NSCLC, locally advanced unresectable NSCLC (e.g., a Stage III NSCLC (e.g., a Stage IIIA NSCLC, Stage IIIB NSCLC, and/or Stage IIIC NSCLC)) who has previously received cCRT for NSCLC, and has not progressed after the cCRT (e.g., as determined by radiographic disease progression after the cCRT). In some instances, the methods, uses, or compositions for use described herein involve treating a subject having a locally advanced unresectable NSCLC (e.g., a Stage III NSCLC (e.g., a Stage IIIA NSCLC, Stage IIIB NSCLC, and/or Stage IIIC NSCLC)) who has previously received cCRT for NSCLC (e.g., a locally advanced unresectable NSCLC (e.g., a Stage III NSCLC (e.g., a Stage IIIA NSCLC, Stage IIIA NSCLC, and/or Stage IIlc NSCLC))) and has not progressed after the cCRT (e.g., as determined by radiographic disease progression after the cCRT). In some instances, the NSCLC is not a Stage IV NSCLC.

Disease progression can be determined by RESIST v1.1. In some embodiments, the subject previously received at least two cycles of the cCRT (e.g., at least three cycles of the cCRT, at least four cycles of the cCRT, at least five cycles of the cCRT, at least six cycles of the cCRT, or more).

In some embodiments, the cCRT administered to the subject includes a platinum-based chemotherapy (e.g., the cCRT is a concurrent platinum-based CRT, e.g., a concurrent CRT comprising administration of cisplatin (e.g., cisplatin-etoposide or cisplatin-vinorelbine) or a concurrent CRT comprising administration of carboplatin (e.g., carboplatin-paclitaxel)). In some embodiments, the cCRT comprises a thoracic radiotherapy. In some embodiments, the radiotherapy was administered to the subject with a dose no less than the biological equivalent of 60 Gy in 2.0 Gy fractions (e.g., at a dose of 60-66 Gy in 30-33 fractions). In some instances, the radiotherapy is administered over the course of six-to-seven weeks. In some instances, the cCRT was administered with curative intent. In some embodiments, the cCRT was administered as a consolidation therapy.

In some instances, the subject has good performance status, e.g., Grade 0 or 1 on the Eastern Cooperative Oncology Group Performance Status Scale. In some instances, the subject is characterized as fully active and/or able to carry on all pre-disease performance without restriction (e.g., Grade 0 on the Eastern Cooperative Oncology Group Performance Status Scale). In some instances, the subject is characterized as restricted in physically strenuous activity but ambulatory and able to carry out work of a light or sedentary nature (e.g., light housework or office work) (e.g., Grade 1 on the Eastern Cooperative Oncology Group Performance Status Scale).

In some instances, the progression-free survival (PFS) of the subject is increased as compared to a reference PFS time. In some instances, wherein the reference PFS time is the median PFS time of a population of subjects who have received a treatment comprising a PD-1 axis binding antagonist (e.g., an anti-PD-L1 antagonist antibody) without an anti-TIGIT antagonist antibody.

In some embodiments, the PFS of the individual is measured according to RECIST v1.1 criteria, as described in Eisenhauer et al., Eur. J. Cancer. 2009, 45:228-47. In some embodiments, PFS is measured as the period of time from the start of treatment to the first occurrence of disease progression as determined by RECIST v1.1 criteria. In some embodiments, PFS is measured as the time from the start of treatment to the time of death.

In some embodiments, the treatment extends the PFS of the subject by at least about 3.1 months (e.g., by 3.1-120 months, by 3.5-100 months, by 4.0-60 months, by 5.0-48 months, by 6.0-36 months, by 8.0-24 months, or by 10-12 months, e.g., by at least about 2.4 months, 2.5 months, 2.6 months, 2.7 months, 2.8 months, 2.9 months, 3.0 months, 3.1 months, 3.2 months, 3.3 months, 3.4 months, 3.5 months, 3.6 months, 3.7 months, 3.8 months, 3.9 months, 4.0 months, 4.1 months, 4.2 months, 4.3 months, 4.4 months, 4.5 months, 4.6 months, 4.7 months, 4.8 months, 4.9 months, 5.0 months, 5.5 months, 6.0 months, 6.5 months, 7.0 months, 7.5 months, 8.0 months, 8.5 months, 9.0 months, 9.5 months, 10 months, 10.5 months, 11 months, 11.5 months, 12 months, 13 months, 14 months, 15 months, 16 months, 17 months, 18 months, 19 months, 20 months, 21 months, 22 months, 23 months, 24 months, 25 months, 26 months, 27 months, 28 months, 29 months, 30 months, 31 months, 32 months, 33 months, 34 months, 35 months, or 36 months) as compared to treatment with a PD-1 axis binding antagonist (e.g., atezolizumab or durvalumab) without the anti-TIGIT antagonist antibody. In some embodiments, the treatment extends the PFS of the subject by at least about 4.9 months (e.g., by 4.9-120 months, by 5-100 months, by 6-80 months, by 7-60 months, by 8-48 months, by 9-36 months, or by 10-24 months, e.g., by at least about 4.9 months, 5.0 months, 5.5 months, 6.0 months, 6.5 months, 7.0 months, 7.5 months, 8.0 months, 8.5 months, 9.0 months, 9.5 months, 10 months, 10.5 months, 11 months, 11.5 months, 12 months, 13 months, 14 months, 15 months, 16 months, 17 months, 18 months, 19 months, 20 months, 21 months, 22 months, 23 months, 24 months, 25 months, 26 months, 27 months, 28 months, 29 months, 30 months, 31 months, 32 months, 33 months, 34 months, 35 months, or 36 months) as compared to treatment with the PD-1 axis binding antagonist without the anti-TIGIT antagonist antibody. In some embodiments, the treatment extends the PFS of the subject by at least about 2 months (e.g., by 2-120 months, by 3-100 months, by 4-80 months, by 6-60 months, by 8-48 months, by 9-36 months, or by 10-24 months, e.g., by at least about 2.0 months, 2.1 months, 2.2 months, 2.3 months, 2.4 months, 2.5 months, 2.6 months, 2.7 months, 2.8 months, 2.9 months, 3.0 months, 3.1 months, 3.2 months, 3.3 months, 3.4 months, 3.5 months, 3.6 months, 3.7 months, 3.8 months, 3.9 months, 4.0 months, 4.1 months, 4.2 months, 4.3 months, 4.4 months, 4.5 months, 4.6 months, 4.7 months, 4.8 months, 4.9 months, 5.0 months, 5.5 months, 6.0 months, 6.5 months, 7.0 months, 7.5 months, 8.0 months, 8.5 months, 9.0 months, 9.5 months, 10 months, 10.5 months, 11 months, 11.5 months, 12 months, 13 months, 14 months, 15 months, 16 months, 17 months, 18 months, 19 months, 20 months, 21 months, 22 months, 23 months, 24 months, 25 months, 26 months, 27 months, 28 months, 29 months, 30 months, 31 months, 32 months, 33 months, 34 months, 35 months, or 36 months) as compared to treatment with a PD-1 axis binding antagonist (e.g., atezolizumab or durvalumab) without the anti-TIGIT antagonist antibody.

In some embodiments, OS is measured as the period of time from the start of treatment to death. In some instances, the treatment extends the OS of the subject by at least about 2 months (e.g., by 2-120 months, by 3-110 months, by 4-100 months, by 5-80 months, by 6-60 months, by 7-48 months, by 8-36 months, or by 10-24 months, e.g., by at least about 2 months, 2.1 months, 2.2 months, 2.3 months, 2.4 months, 2.5 months, 2.6 months, 2.7 months, 2.8 months, 2.9 months, 3.0 months, 3.1 months, 3.2 months, 3.3 months, 3.4 months, 3.5 months, 3.6 months, 3.7 months, 3.8 months, 3.9 months, 4.0 months, 4.1 months, 4.2 months, 4.3 months, 4.4 months, 4.5 months, 4.6 months, 4.7 months, 4.8 months, 4.9 months, 5.0 months, 5.5 months, 6.0 months, 6.5 months, 7.0 months, 7.5 months, 8.0 months, 8.5 months, 9.0 months, 9.5 months, 10 months, 10.5 months, 11 months, 11.5 months, 12 months, 13 months, 14 months, 15 months, 16 months, 17 months, 18 months, 19 months, 20 months, 21 months, 22 months, 23 months, 24 months, 25 months, 26 months, 27 months, 28 months, 29 months, 30 months, 31 months, 32 months, 33 months, 34 months, 35 months, or 36 months) as compared to treatment with a PD-1 axis binding antagonist (e.g., atezolizumab or durvalumab) without the anti-TIGIT antagonist antibody. In some instances, the treatment extends the OS of the subject by at least about 5.7 months (e.g., by 5.7-120 months, by 6-100 months, by 7-80 months, by 8-60 months, by 9-48 months, by 10-36 months, or by 11-24 months, e.g., by at least about 5.7 months, 6.0 months, 6.5 months, 7.0 months, 7.5 months, 8.0 months, 8.5 months, 9.0 months, 9.5 months, 10 months, 10.5 months, 11 months, 11.5 months, 12 months, 13 months, 14 months, 15 months, 16 months, 17 months, 18 months, 19 months, 20 months, 21 months, 22 months, 23 months, 24 months, 25 months, 26 months, 27 months, 28 months, 29 months, 30 months, 31 months, 32 months, 33 months, 34 months, 35 months, or 36 months) as compared to treatment with the PD-1 axis binding antagonist without the anti-TIGIT antagonist antibody. In some instances, the treatment extends the OS of the subject by at least about 9 months (e.g., by 9-120, by 10-60 months, by 11-48 months, or by 12-36 months, e.g., by at least about 9.0 months, 9.5 months, 10 months, 10.5 months, 11 months, 11.5 months, 12 months, 13 months, 14 months, 15 months, 16 months, 17 months, 18 months, 19 months, 20 months, 21 months, 22 months, 23 months, 24 months, 25 months, 26 months, 27 months, 28 months, 29 months, 30 months, 31 months, 32 months, 33 months, 34 months, 35 months, or 36 months) as compared to treatment with a PD-1 axis binding antagonist (e.g., atezolizumab or durvalumab) without the anti-TIGIT antagonist antibody.

In some embodiments, administration of the anti-TIGIT antagonist antibody (e.g., tiragolumab) and the PD-1 axis binding antagonist (e.g., atezolizumab) to a plurality of subjects results in a median OS of at least about 12 months (e.g., about 12.5 months, about 13 months, about 13.5 months, about 14 months, about 14.5 months, about 15 months, about 15.5 months, about 16 months, about 16.5 months, about 17 months, about 17.5 months, about 18 months, about 18.5 months, about 19 months, about 19.5 months, about 20 months, about 20.5 months, about 21 months, about 21.5 months, about 22 months, about 22.5 months, about 23 months, about 23.5 months, about 24 months, about 24.5 months, about 25 months, about 25.5. months, about 26 months, about 26.5 months, about 27 months, about 27.5 months, about 28 months, about 28.5 months, about 29 months, about 29.5 months, about 30 months, about 30.5 months, about 31 months, about 31.5 months, about 32 months, about 32.5 months, about 33 months, about 33.5 months, about 34 months, about 34.5 months, about 35 months, about 35.5 months, about 36 months, about 36.5 months, about 37 months, about 37.5 months, about 38 months, about 38.5 months, about 39 months, about 39.5 months, about 40 months, or more) after the start of treatment with the anti-TIGIT antagonist antibody (e.g., tiragolumab) and the PD-1 axis binding antagonist (e.g., atezolizumab). In some embodiments, administration of the anti-TIGIT antagonist antibody (e.g., tiragolumab) and the PD-1 axis binding antagonist (e.g., atezolizumab) to a plurality of subjects results in a median OS between 12 months and 60 months (e.g., between 14 and 60 months, between 16 and 60 months, between 18 and 60 months, between 20 and 60 months, between 24 and 60 months, between 28 and 60 months, between 30 and 60 months, between 32 and 60 months, between 33 and 60 months, between 34 and 60 months, between 35 and 60 months, between 36 and 60 months, between 37 and 60 months, between 38 and 60 months, between 39 and 60 months, between 40 and 60 months, between 41 and 60 months, between 42 and 60 months, between 43 and 60 months, between 44 and 60 months, between 45 and 60 months, between 46 and 60 months, between 47 and 60 months, between 48 and 60 months, between 49 and 60 months, between 50 and 60 months, between 51 and 60 months, between 52 and 60 months, between 53 and 60 months, between 54 and 60 months, between 55 and 60 months, between 56 and 60 months, between 57 and 60 months, between 58 and 60 months, or between 59 and 60 months) after the start of treatment with the anti-TIGIT antagonist antibody (e.g., tiragolumab) and the PD-1 axis binding antagonist (e.g., atezolizumab).

In some instances, the treatment results in an increase in duration of objective response (DOR) in the subject as compared to treatment with a PD-1 axis binding antagonist (e.g., atezolizumab or durvalumab) without the anti-TIGIT antagonist antibody or as compared to treatment with the anti-TIGIT antagonist antibody without the PD-1 axis binding antagonist. In some instances, the treatment results in an increase in DOR in the subject as compared to treatment without the anti-TIGIT antagonist antibody and without the PD-1 axis binding antagonist. In some embodiments, the treatment results in an increase in DOR in the subject as compared to treatment without the anti-TIGIT antagonist antibody and without the PD-1 axis binding antagonist. In some embodiments, the increase in DOR is about 4 months, about 5 months, about 6 months, about 7 months, about 8 months, about 9 months, about 10 months, about 11 months, about 12 months, about 12 months, about 13 months, about 14 months, about 15 months, about 16 months, about 17 months, about 18 months, about 19 months, about 20 months, about 21 months, about 22 months, about 23 months, about 24 months, or more. In some embodiments, administration of the anti-TIGIT antagonist antibody (e.g., tiragolumab) and the PD-1 axis binding antagonist (e.g., atezolizumab) to a plurality of subjects results in a median DOR of at least about 4 months or more (e.g., about 5 months, about 6 months, about 7 months, about 8 months, about 9 months, about 10 months, about 11 months, about 12 months, about 13 months, about 14 months, about 15 months, about 16 months, about 17 months, about 18 months, about 19 months, about 20 months, about 21 months, about 22 months, about 23 months, about 24 months or more) after the start of treatment with the anti-TIGIT antagonist antibody (e.g., tiragolumab) and the PD-1 axis binding antagonist (e.g., atezolizumab).

## IV. EXEMPLARY ANTIBODIES FOR USE IN THE METHODS AND USES OF THE INVENTION

Exemplary anti-TIGIT antagonist antibodies and PD-1 axis binding antagonists (e.g., anti-PD-L1 antagonist antibodies) useful for treating a subject (e.g., a human, e.g., an adult patient) having cancer (e.g., lung cancer, e.g., non-small cell lung cancer (NSCLC), e.g., squamous or non-squamous NSCLC, e.g., locally advanced unresectable NSCLC (e.g., Stage IIIB NSCLC), or recurrent or metastatic NSCLC (e.g., Stage IV NSCLC)) in accordance with the methods, uses, and compositions for use of the invention are described herein.

### A. *Exemplary Anti-TIGIT Antagonist Antibodies*

The invention provides anti-TIGIT antagonist antibodies useful for treating cancer (e.g., lung cancer, e.g., non-small cell lung cancer (NSCLC), e.g., squamous or non-squamous NSCLC, e.g., locally advanced unresectable NSCLC (e.g., Stage IIIB NSCLC), or recurrent or metastatic NSCLC (e.g., Stage IV NSCLC)) in a subject (e.g., a human, e.g., an adult patient).

In some instances, the anti-TIGIT antagonist antibody is tiragolumab (CAS Registry Number: 1918185-84-8). Tiragolumab (Genentech) is also known as MTIG7192A.

In certain instances, the anti-TIGIT antagonist antibodies includes at least one, two, three, four, five, or six HVRs selected from: (a) an HVR-H1 comprising the amino acid sequence of SNSAAWN (SEQ ID NO: 1); (b) an HVR-H2 comprising the amino acid sequence of KTYYRFKWYSDYAVSVKG (SEQ ID NO: 2); (c) an HVR-H3 comprising the amino acid sequence of ESTTYDLLAGPFDY (SEQ ID NO: 3); (d) an HVR-L1 comprising the amino acid sequence of KSSQTVLYSSNNKKYLA (SEQ ID NO: 4), (e) an HVR-L2 comprising the amino acid sequence of WASTRES (SEQ ID NO: 5); and/or (f) an HVR-L3 comprising the amino acid sequence of QQYYSTPFT (SEQ ID NO: 6), or a combination of one or more of the above HVRs and one or more variants thereof having at least about 90% sequence identity (e.g., 90%, 91%, 92%, 93%, 94%, 95%, 96%, 97%, 98%, or 99% identity) to any one of SEQ ID NOs: 1-6.

In some instances, any of the above anti-TIGIT antagonist antibodies includes (a) an HVR-H1 comprising the amino acid sequence of SNSAAWN (SEQ ID NO: 1); (b) an HVR-H2 comprising the amino acid sequence of KTYYRFKWYSDYAVSVKG (SEQ ID NO: 2); (c) an HVR-H3 comprising the amino acid sequence of ESTTYDLLAGPFDY (SEQ ID NO: 3); (d) an HVR-L1 comprising the amino acid sequence of KSSQTVLYSSNNKKYLA (SEQ ID NO: 4); (e) an HVR-L2 comprising the amino acid sequence of WASTRES (SEQ ID NO: 5); and (f) an HVR-L3 comprising the amino acid sequence of QQYYSTPFT (SEQ ID NO: 6). In some instances, the anti-TIGIT antagonist antibody has a VH domain comprising an amino acid sequence having at least 90% sequence identity (e.g., at least 91%, 92%, 93%, 94%, 95%, 96%, 97%, 98%, or 99% sequence identity) to, or the sequence of, EVQLQQSGPGLVKPSQTLSLTCAISGDSVSSNSAAWNWIRQSPSRGLEWLGKTYYRFKWYSDYAVSVK GRITINPDTSKNQFSLQLNSVTPEDTAVFYCTRESTTYDLLAGPFDYWGQGTLVTVSS (SEQ ID NO: 17) or an amino acid sequence having at least 90% sequence identity (e.g., at least 91%, 92%, 93%, 94%, 95%, 96%, 97%, 98%, or 99% sequence identity) to, or the sequence of, QVQLQQSGPGLVKPSQTLSLTCAISGDSVSSNSAAWNWIRQSPSR-GLEWLGKTYYRFKWYSDYAVSVK GRITINPDTSKNQFSLQLNSVTPEDTAVFYCTRESTTYDLLAGPFDY-WGQGTLVTVSS (SEQ ID NO: 18); and/or a VL domain comprising an amino acid sequence having at least 90% sequence identity (e.g., at least 91%, 92%, 93%, 94%, 95%, 96%, 97%, 98%, or 99% sequence identity) to, or the sequence of, DIVMTQSPDSLAVSLGERATINCKSSQTVLYSSNNKKYLAWYQQKPGQPPNLLIYWASTRESGVPDRFS GSGSGTDFTLTISSLQAEDVAVYYCQQYYSTPFTFGPGTKVEIK (SEQ ID NO: 19). In some instances, the anti-TIGIT antagonist antibody has a VH domain comprising an amino acid sequence having at least at least 90% sequence identity (e.g., at least 91%, 92%, 93%, 94%, 95%, 96%, 97%, 98%, or 99% sequence identity) to, or the sequence of, SEQ ID NO: 17 or 18 and/or a VL domain comprising an amino acid sequence having at least 90% sequence identity (e.g., at least 91%, 92%, 93%, 94%, 95%, 96%, 97%, 98%, or 99% sequence identity) to, or the sequence of, SEQ ID NO: 19. In some instances, the anti-TIGIT antagonist antibody has a VH domain comprising an amino acid sequence having at least at least 90% sequence identity (e.g., at least 91%, 92%, 93%, 94%, 95%, 96%, 97%, 98%, or 99% sequence identity) to, or the sequence of, SEQ ID NO: 17 and/or a VL domain comprising an amino acid sequence having at least 90% sequence identity (e.g., at least 91%, 92%, 93%, 94%, 95%, 96%, 97%, 98%, or 99% sequence identity) to, or the sequence of, SEQ ID NO: 19. In some instances, the anti-TIGIT antagonist antibody has a VH domain comprising an amino acid sequence having at least at least 90% sequence identity (e.g., at least 91%, 92%, 93%, 94%, 95%, 96%, 97%, 98%, or 99% sequence identity) to, or the sequence of, SEQ ID NO: 18 and/or a VL domain comprising an amino acid sequence having at least 90% sequence identity (e.g., at least 91%, 92%, 93%, 94%, 95%, 96%, 97%, 98%, or 99% sequence identity) to, or the sequence of, SEQ ID NO: 19.

In some instances, the anti-TIGIT antagonist antibody includes a heavy chain and a light chain sequence, wherein: (a) the heavy chain comprises the amino acid sequence:
EVQLQQSGPGLVKPSQTLSLTCAISGDSVSSNSAAWNWIRQSPSRGLEWLGKTYYRFKWYSDYAVSVK
GRITINPDTSKNQFSLQLNSVTPEDTAVFYCTRESTTYDLLAGPFDYWGQGTLVTVSSASTKGPSVFPLA
PSSKSTSGGTAALGCLVKDYFPEPVTVSWNSGALTSGVHTFPAVLQSSGLYSLSSVVTVPSSSLGTQTYI
CNVNHKPSNTKVDKKVEPKSCDKTHTCPPCPAPELLGGPSVFLFPPKPKDTLMISRTPEVTCVVVDVSHE
DPEVKFNWYVDGVEVHNAKTKPREEQYNSTYRVVSVLTVLHQDWLNGKEYKCKVSNKALPAPIEKTISK
AKGQPREPQVYTLPPSREEMTKNQVSLTCLVKGFYPSDIAVEWESNGQPENNYKTTPPVLDSDGSFFLY
SKLTVDKSRWQQGNVFSCSVMHEALHNHYTQKSLSLSPGK (SEQ ID NO: 33); and (b) the light chain comprises the amino acid sequence:

DIVMTQSPDSLAVSLGERATINCKSSQTVLYSSNNKKYLAWYQQKPGQPPNLLIYWASTRESGVPDRFS
GSGSGTDFTLTISSLQAEDVAVYYCQQYYSTPFTFGPGTKVEIKRTVAAPSVFIFPPSDEQLKSGTASVVC
LLNNFYPREAKVQWKVDNALQSGNSQESVTEQDSKDSTYSLSSTLTLSKADYEKHKVYACEVTHQGLSS
PVTKSFNRGEC (SEQ ID NO: 34).

In some instances, the anti-TIGIT antagonist antibody further comprises at least one, two, three, or four of the following light chain variable region framework regions (FRs): an FR-L1 comprising the amino acid sequence of DIVMTQSPDSLAVSLGERATINC (SEQ ID NO: 7); an FR-L2 comprising the amino acid sequence of WYQQKPGQPPNLLIY (SEQ ID NO: 8); an FR-L3 comprising the amino acid sequence of GVPDRFSGSGSGTDFTLTISSLQAEDVAVYYC (SEQ ID NO: 9); and/or an FR-L4 comprising the amino acid sequence of FGPGTKVEIK (SEQ ID NO: 10), or a combination of one or more of the above FRs and one or more variants thereof having at least about 90% sequence identity (e.g., 90%, 91%, 92%, 93%, 94%, 95%, 96%, 97%, 98%, or 99% identity) to any one of SEQ ID NOs: 7-10. In some instances, for example, the antibody further comprises an FR-L1 comprising the amino acid sequence of DIVMTQSPDSLAVSLGERATINC (SEQ ID NO: 7); an FR-L2 comprising the amino acid sequence of WYQQKPGQPPNLLIY (SEQ ID NO: 8); an FR-L3 comprising the amino acid sequence of GVPDRFSGSGSGTDFTLTISSLQAEDVAVYYC (SEQ ID NO: 9); and an FR-L4 comprising the amino acid sequence of FGPGTKVEIK (SEQ ID NO: 10).

In some instances, the anti-TIGIT antagonist antibody further comprises at least one, two, three, or four of the following heavy chain variable region FRs: an FR-H1 comprising the amino acid sequence of $X_1$VQLQQSGPGLVKPSQTLSLTCAISGDSVS (SEQ ID NO: 11), wherein $X_1$ is Q or E; an FR-H2 comprising the amino acid sequence of WIRQSPSRGLEWLG (SEQ ID NO: 12); an FR-H3 comprising the amino acid sequence of RITINPDTSKNQFSLQLNSVTPEDTAVFYCTR (SEQ ID NO: 13); and/or an FR-H4 comprising the amino acid sequence of WGQGTLVTVSS (SEQ ID NO: 14), or a combination of one or more of the above FRs and one or more variants thereof having at least about 90% sequence identity (e.g., 90%, 91%, 92%, 93%, 94%, 95%, 96%, 97%, 98%, or 99% identity) to any one of SEQ ID NOs: 11-14. The anti-TIGIT antagonist antibody may further include, for example, at least one, two, three, or four of the following heavy chain variable region FRs: an FR-H1 comprising the amino acid sequence of EVQLQQSGPGLVKPSQTLSLTCAISGDSVS (SEQ ID NO: 15); an FR-H2 comprising the amino acid sequence of WIRQSPSRGLEWLG (SEQ ID NO: 12); an FR-H3 comprising the amino acid sequence of RITINPDTSKNQFSLQLNSVTPEDTAVFYCTR (SEQ ID NO: 13); and/or an FR-H4 comprising the amino acid sequence of WGQGTLVTVSS (SEQ ID NO: 14), or a combination of one or more of the above FRs and one or more variants thereof having at least about 90% sequence identity (e.g., 90%, 91%, 92%, 93%, 94%, 95%, 96%, 97%, 98%, or 99% identity) to any one of SEQ ID NOs: 12-15. In some instances, the anti-TIGIT antagonist antibody includes an FR-H1 comprising the amino acid sequence of EVQLQQSGPGLVKPSQTLSLTCAISGDSVS (SEQ ID NO: 15); an FR-H2 comprising the amino acid sequence of WIRQSPSRGLEWLG (SEQ ID NO: 12); an FR-H3 comprising the amino acid sequence of RITINPDTSKNQFSLQLNSVTPEDTAVFYCTR (SEQ ID NO: 13); and an FR-H4 comprising the amino acid sequence of WGQGTLVTVSS (SEQ ID NO: 14. In another instance, for example, the anti-TIGIT antagonist antibody may further include at least one, two, three, or four of the following heavy chain variable region FRs: an FR-H1 comprising the amino acid sequence of QVQLQQSGPGLVKPSQTLSLTCAISGDSVS (SEQ ID NO: 16); an FR-H2 comprising the amino acid sequence of WIRQSPSRGLEWLG (SEQ ID NO: 12); an FR-H3 comprising the amino acid sequence of RITINPDTSKNQFSLQLNSVTPEDTAVFYCTR (SEQ ID NO: 13); and/or an FR-H4 comprising the amino acid sequence of WGQGTLVTVSS (SEQ ID NO: 14), or a combination of one or more of the above FRs and one or more variants thereof having at least about 90% sequence identity (e.g., 90%, 91%, 92%, 93%, 94%, 95%, 96%, 97%, 98%, or 99% identity) to any one of SEQ ID NOs: 12-14 and 16. In some instances, the anti-TIGIT antagonist antibody includes an FR-H1 comprising the amino acid sequence of QVQLQQSGPGLVKPSQTLSLTCAISGDSVS (SEQ ID NO: 16); an FR-H2 comprising the amino acid sequence of WIRQSPSRGLEWLG (SEQ ID NO: 12); an FR-H3 comprising the amino acid sequence of RITINPDTSKNQFSLQLNSVTPEDTAVFYCTR (SEQ ID NO: 13); and an FR-H4 comprising the amino acid sequence of WGQGTLVTVSS (SEQ ID NO: 14).

In another aspect, an anti-TIGIT antagonist antibody is provided, wherein the antibody comprises a VH as in any of the instances provided above, and a VL as in any of the instances provided above, wherein one or both of the variable domain sequences include post-translational modifications.

In some instances, any one of the anti-TIGIT antagonist antibodies described above is capable of binding to rabbit TIGIT, in addition to human TIGIT. In some instances, any one of the anti-TIGIT antagonist antibodies described above is capable of binding to both human TIGIT and cynomolgus monkey (cyno) TIGIT. In some instances, any one of the anti-TIGIT antagonist antibodies described above is capable of binding to human TIGIT, cyno TIGIT, and rabbit TIGIT. In some instances, any one of the anti-TIGIT antagonist antibodies described above is capable of binding to human TIGIT, cyno TIGIT, and rabbit TIGIT, but not murine TIGIT.

In some instances, the anti-TIGIT antagonist antibody binds human TIGIT with a $K_D$ of about 10 nM or lower and cyno TIGIT with a $K_D$ of about 10 nM or lower (e.g., binds human TIGIT with a $K_D$ of about 0.1 nM to about 1 nM and cyno TIGIT with a $K_D$ of about 0.5 nM to about 1 nM, e.g., binds human TIGIT with a $K_D$ of about 0.1 nM or lower and cyno TIGIT with a $K_D$ of about 0.5 nM or lower).

In some instances, the anti-TIGIT antagonist antibody specifically binds TIGIT and inhibit or block TIGIT interaction with poliovirus receptor (PVR) (e.g., the antagonist antibody inhibits intracellular signaling mediated by TIGIT binding to PVR). In some instances, the antagonist antibody inhibits or blocks binding of human TIGIT to human PVR with an IC50 value of 10 nM or lower (e.g., 1 nM to about 10 nM). In some instances, the antagonist antibody inhibits or blocks binding of cyno TIGIT to cyno PVR with an IC50 value of 50 nM or lower (e.g., 1 nM to about 50 nM, e.g., 1 nM to about 5 nM).

In some instances, the methods or uses described herein may include using or administering an isolated anti-TIGIT antagonist antibody that competes for binding to TIGIT with any of the anti-TIGIT antagonist antibodies described above. For example, the method may include administering an isolated anti-TIGIT antagonist antibody that competes for binding to TIGIT with an anti-TIGIT antagonist antibody having the following six HVRs: (a) an HVR-H1 comprising the amino acid sequence of SNSAAWN (SEQ ID NO: 1); (b) an HVR-H2 comprising the amino acid sequence of KTYYRFKWYSDYAVSVKG (SEQ ID NO: 2); (c) an HVR-H3 comprising the amino acid sequence of ESTTYDLLAGPFDY (SEQ ID NO: 3); (d) an HVR-L1 comprising the amino acid sequence of KSSQTVLYSSNNKKYLA (SEQ ID NO: 4), (e) an HVR-L2 comprising the amino acid sequence of WASTRES (SEQ ID NO: 5); and (f) an HVR-L3 comprising the amino acid sequence of QQYYSTPFT (SEQ ID NO: 6). The methods described herein may also include administering an isolated anti-TIGIT antagonist antibody that binds to the same epitope as an anti-TIGIT antagonist antibody described above.

An anti-TIGIT antagonist antibody according to any of the above instances may be a monoclonal antibody, comprising a chimeric, humanized, or human antibody. In some instances, the anti-TIGIT antagonist antibody is tiragolumab. In one instance, an anti-TIGIT antagonist antibody is an antibody fragment, for example, a Fv, Fab, Fab', scFv, diabody, or $F(ab')_2$ fragment. In another instance, the antibody is a full-length antibody, e.g., an intact IgG antibody (e.g., an intact IgG1 antibody) or other antibody class or isotype as defined herein.

In a further aspect, an anti-TIGIT antagonist antibody according to any of the above instances may incorporate any of the features, singly or in combination, as described in Sections 1-6 below.

*B. Exemplary PD-1 Axis Binding Antagonists*

Provided herein are methods for treating cancer (e.g., lung cancer, e.g., non-small cell lung cancer (NSCLC), e.g., squamous or non-squamous NSCLC, e.g., locally advanced unresectable NSCLC (e.g., Stage IIIB NSCLC), or recurrent or metastatic NSCLC (e.g., Stage IV NSCLC)) in a subject (e.g., a human) in a subject comprising administering to the subject an effective amount of a PD-1 axis binding antagonist. PD-1 axis binding antagonists include PD-L1 binding antagonists (e.g., PD-L1 antagonist antibodies), PD-1 binding antagonists (e.g., PD-1 antagonist antibodies), and PD-2 binding antagonists (e.g., PD-L2 antagonist antibodies).

In some instances, the PD-1 axis binding antagonist is an anti-PD-L1 antagonist antibody that inhibits the binding of PD-L1 to its binding partners. In a specific aspect, PD-L1 binding partners are PD-1 and/or B7-1. In some instances, the anti-PD-L1 antagonist antibody is capable of inhibiting binding between PD-L1 and PD-1 and/or between PD-L1 and B7-1.

In some instances, the PD-1 axis binding antagonist is an anti-PD-L1 antibody.

In some instances, the anti-PD-L1 antibody is atezolizumab (CAS Registry Number: 1422185-06-5). Atezolizumab (Genentech) is also known as MPDL3280A.

In some instances, the anti-PD-L1 antibody (e.g., atezolizumab) includes at least one, two, three, four, five, or six HVRs selected from: (a) an HVR-H1 sequence is GFTFSDSWIH (SEQ ID NO: 20); (b) an HVR-H2 sequence is AWISPYGGSTYYADSVKG (SEQ ID NO: 21); (c) an HVR-H3 sequence is RHWPGGFDY (SEQ ID NO: 22), (d) an HVR-L1 sequence is RASQDVSTAVA (SEQ ID NO: 23); (e) an HVR-L2 sequence is SASFLYS (SEQ ID NO: 24); and (f) an HVR-L3 sequence is QQYLYHPAT (SEQ ID NO: 25).

In some instances, the anti-PD-L1 antibody (e.g., atezolizumab) comprises a heavy chain and a light chain sequence, wherein: (a) the heavy chain variable (VH) region sequence comprises the amino acid sequence: EVQLVESGGGLVQPGGSLRLSCAASGFTFSDSWIHWVRQAPGKGLEWVAWISPYGGSTYYADSVKGRF TISADTSKNTAYLQMNSLRAEDTAVYYCARRHWPGGFDYWGQGTLVTVSS (SEQ ID NO: 26); and (b) the light chain variable (VL) region sequence comprises the amino acid sequence:

DIQMTQSPSSLSASVGDRVTITCRASQDVSTAVAWYQQKPGKAPKLLIYSASFLYSGVPSRFSGSGSGTD FTLTISSLQPEDFATYYCQQYLYHPATFGQGTKVEIKR (SEQ ID NO: 27).

In some instances, the anti-PD-L1 antibody (e.g., atezolizumab) comprises a heavy chain and a light chain sequence, wherein: (a) the heavy chain comprises the amino acid sequence: EVQLVESGGGLVQPGGSLRLSCAASGFTFSDSWIHWVRQAPGKGLEWVAWISPYGGSTYYADSVKGRF TISADTSKNTAYLQMNSLRAEDTAVYYCARRHWPGGFDYWGQGTLVTVSSASTKGPSVFPLAPSSKSTS GGTAALGCLVKDYFPEPVTVSWNSGALTSGVHTFPAVLQSSGLYSLSSWTVPSSSLGTQTYICNVNHKP SNTKVDKKVEPKSCDKTHTCPPCPAPELLGGPSVFLFPPKPKDTLMISRTPEVTCVVVDVSHEDPEVKFN WYVDGVEVHNAKTKPREEQYASTYRVVSVLTVLHQDWLNGKEYKCKVSNKALPAPIEKTISKAKGQPRE PQVYTLPPSREEMTKNQVSLTCLVKGFYPSDIAVEWESNGQPENNYKTTPPVLDSDGSFFLYSKLTVDKS RWQQGNVFSCSVMHEALHNHYTQKSLSLSPG (SEQ ID NO: 28); and (b) the light chain comprises the amino acid sequence:

DIQMTQSPSSLSASVGDRVTITCRASQDVSTAVAWYQQKPGKAPKLLIYSASFLYSGVPSRFSGSGSGTD
FTLTISSLQPEDFATYYCQQYLYHPATFGQGTKVEIKRTVAAPSVFIFPPSDEQLKSGTASVVCLLNNFYP
REAKVQWKVDNALQSGNSQESVTEQDSKDSTYSLSSTLTLSKADYEKHKVYACEVTHQGLSSPVTKSFN
RGEC (SEQ ID NO: 29).

In some instances, the anti-PD-L1 antibody comprises (a) a VH domain comprising an amino acid sequence comprising having at least 95% sequence identity (e.g., at least 95%, 96%, 97%, 98%, or 99% sequence identity) to, or the sequence of (SEQ ID NO: 26); (b) a VL domain comprising an amino acid sequence comprising having at least 95% sequence identity (e.g., at least 95%, 96%, 97%, 98%, or 99% sequence identity) to, or the sequence of (SEQ ID NO: 27); or (c) a VH domain as in (a) and a VL domain as in (b). In other instances, the anti-PD-L1 antagonist antibody is selected from YW243.55.S70, MDX-1105, and MEDI4736 (durvalumab), and MSB0010718C (avelumab). Antibody YW243.55.S70 is an anti-PD-L1 described in PCT Pub. No. WO 2010/077634. MDX-1105, also known as BMS-936559, is an anti-PD-L1 antibody described in PCT Pub. No. WO 2007/005874. MEDI4736 (durvalumab) is an anti-PD-L1 monoclonal antibody described in PCT Pub. No. WO 2011/066389 and U.S. Pub. No. 2013/034559. Examples of anti-PD-L1 antibodies useful for the methods of this invention, and methods for making thereof are described in PCT Pub. Nos. WO 2010/077634, WO 2007/005874, and WO 2011/066389, and also in U.S. Pat. No. 8,217,149, and U.S. Pub. No. 2013/034559, which are incorporated herein by reference. The anti-PD-L1 antagonist antibodies (e.g., atezolizumab) useful in this invention, including compositions containing such antibodies, may be used in combination with an anti-TIGIT antagonist antibody to treat cancer (e.g., lung cancer, e.g., non-small cell lung cancer (NSCLC), e.g., squamous or non-squamous NSCLC, e.g., locally advanced unresectable NSCLC (e.g., Stage IIIB NSCLC), or recurrent or metastatic NSCLC (e.g., Stage IV NSCLC)).

In some instances, the anti-PD-L1 antagonist antibody is a monoclonal antibody. In some instances, the anti-PD-L1 antagonist antibody is an antibody fragment selected from the group consisting of Fab, Fab'-SH, Fv, scFv, and $(Fab')_2$ fragments. In some instances, the anti-PD-L1 antagonist antibody is a humanized antibody. In some instances, the anti-PD-L1 antagonist antibody is a human antibody. In some instances, the anti-PD-L1 antagonist antibody described herein binds to human PD-L1.

In some instances, the PD-1 axis binding antagonist is an anti-PD-1 antagonist antibody that inhibits the binding of PD-1 to its binding partner (e.g., PD-L1). In some instances, the anti-PD-1 antagonist antibody is capable of inhibiting binding between PD-L1 and PD-1.

In some instances, the PD-1 axis binding antagonist is an anti-PD-1 antibody.

In some instances, the PD-1 axis binding antagonist is AMP-224.

In some instances, the anti-PD-1 antibody is nivolumab (MDX-1106) or pembrolizumab (formerly lambrolizumab (MK-3475)).

In a further aspect, a PD-1 axis binding antagonist is a PD-1 axis binding antagonist antibody according to any of the above instances may incorporate any of the features, singly or in combination, as described in Sections 1-6 below.

*1. Antibody Affinity*

In certain instances, an anti-TIGIT antagonist antibody and/or PD-1 axis binding antagonist antibody (e.g., anti-PD-L1 antagonist antibody) provided herein has a dissociation constant ($K_D$) of $\leq 1\mu M$, $\leq$ 100 nM, $\leq$ 10 nM, $\leq$ 1 nM, $\leq$ 0.1 nM, $\leq$ 0.01 nM, or $\leq$ 0.001 nM (e.g., $10^{-8}$ M or less, e.g., from $10^{-8}$ M to $10^{-13}$ M, e.g., from $10^{-9}$ M to $10^{-13}$ M).

In one instance, $K_D$ is measured by a radiolabeled antigen binding assay (RIA). In one instance, an RIA is performed with the Fab version of an antibody of interest and its antigen. For example, solution binding affinity of Fabs for antigen is measured by equilibrating Fab with a minimal concentration of ($^{125}$I)-labeled antigen in the presence of a titration series of unlabeled antigen, then capturing bound antigen with an anti-Fab antibody-coated plate (see, e.g., Chen et al., J. Mol. Biol. 293:865-881 (1999)). To establish conditions for the assay, MICROTITER® multi-well plates (Thermo Scientific) are coated overnight with 5 $\mu$g/ml of a capturing anti-Fab antibody (Cappel Labs) in 50 mM sodium carbonate (pH 9.6), and subsequently blocked with 2% (w/v) bovine serum albumin in PBS for two to five hours at room temperature (approximately 23°C). In a non-adsorbent plate (Nunc #269620), 100 pM or 26 pM [$^{125}$I]-antigen are mixed with serial dilutions of a Fab of interest (e.g., consistent with assessment of the anti-VEGF antibody, Fab-12, in Presta et al., Cancer Res. 57:4593-4599 (1997)). The Fab of interest is then incubated overnight; however, the incubation may continue for a longer period (e.g., about 65 hours) to ensure that equilibrium is reached. Thereafter, the mixtures are transferred to the capture plate for incubation at room temperature (e.g., for one hour). The solution is then removed and the plate washed eight times with 0.1% polysorbate 20 (TWEEN-20®) in PBS. When the plates have dried, 150 $\mu$l/well of scintillant (MICROSCINT-20 ™; Packard) is added, and the plates are counted on a TOPCOUNT™ gamma counter (Packard) for ten minutes. Concentrations of each Fab that give less than or equal to 20% of maximal binding are chosen for use in competitive binding assays.

According to another instance, $K_D$ is measured using a BIACORE® surface plasmon resonance assay. For example, an assay using a BIACORE®-2000 or a BIACORE ®-3000 (BIAcore, Inc., Piscataway, NJ) is performed at 25°C with immobilized antigen CM5 chips at ~10 response units (RU). In one instance, carboxymethylated dextran biosensor chips (CM5, BIACORE, Inc.) are activated with N-ethyl-N'- (3-dimethylaminopropyl)-carbodiimide hydrochloride (EDC) and N-hydroxysuccinimide (NHS) according to the supplier's instructions. Antigen is diluted with 10 mM sodium acetate, pH 4.8, to 5 μg/ml (~0.2 μM) before injection at a flow rate of 5 μl/minute to achieve approximately 10 response units (RU) of coupled protein. Following the injection of antigen, 1 M ethanolamine is injected to block unreacted groups. For kinetics measurements, two-fold serial dilutions of Fab (0.78 nM to 500 nM) are injected in PBS with 0.05% polysorbate 20 (TWEEN-20™) surfactant (PBST) at 25°C at a flow rate of approximately 25 μl/min. Association rates ($k_{on}$) and dissociation rates ($k_{off}$) are calculated using a simple one-to-one Langmuir binding model (BIACORE® Evaluation Software version 3.2) by simultaneously fitting the association and dissociation sensorgrams. The equilibrium dissociation constant ($K_D$) is calculated as the ratio $k_{off}/k_{on}$. See, for example, Chen et al., J. Mol. Biol. 293:865-881 (1999). If the on-rate exceeds $10^6 M^{-1}s^{-1}$ by the surface plasmon resonance assay above, then the on-rate can be determined by using a fluorescent quenching technique that measures the increase or decrease in fluorescence emission intensity (excitation = 295 nm; emission = 340 nm, 16 nm band-pass) at 25°C of a 20 nM anti-antigen antibody (Fab form) in PBS, pH 7.2, in the presence of increasing concentrations of antigen as measured in a spectrometer, such as a stop-flow equipped spectrophometer (Aviv Instruments) or a 8000-series SLM-AMINCO ™ spectrophotometer (ThermoSpectronic) with a stirred cuvette.

*2. Antibody Fragments*

In certain instances, an anti-TIGIT antagonist antibody and/or PD-1 axis binding antagonist antibody (e.g., anti-PD-L1 antagonist antibody) provided herein is an antibody fragment. Antibody fragments include, but are not limited to, Fab, Fab', Fab'-SH, $F(ab')_2$, Fv, and scFv fragments, and other fragments described below. For a review of certain antibody fragments, see Hudson et al. Nat. Med. 9:129-134 (2003). For a review of scFv fragments, see, e.g., Pluckthün, in The Pharmacology of Monoclonal Antibodies, vol. 113, Rosenburg and Moore eds., (Springer-Verlag, New York), pp. 269-315 (1994); see also WO 93/16185; and U.S. Patent Nos. 5,571,894 and 5,587,458. For discussion of Fab and $F(ab')_2$ fragments comprising salvage receptor binding epitope residues and having increased *in vivo* half-life, see U.S. Patent No. 5,869,046.

Diabodies are antibody fragments with two antigen-binding sites that may be bivalent or bispecific. See, for example, EP 404,097; WO 1993/01161; Hudson et al. Nat. Med. 9:129-134 (2003); and Hollinger et al. Proc. Natl. Acad. Sci. USA 90: 6444-6448 (1993). Triabodies and tetrabodies are also described in Hudson et al. Nat. Med. 9:129-134 (2003).

Single-domain antibodies are antibody fragments comprising all or a portion of the heavy chain variable domain or all or a portion of the light chain variable domain of an antibody. In certain instances, a single-domain antibody is a human single-domain antibody (Domantis, Inc., Waltham, MA; see, e.g., U.S. Patent No. 6,248,516 B1).

Antibody fragments can be made by various techniques, including but not limited to proteolytic digestion of an intact antibody as well as production by recombinant host cells (e.g. *E. coli* or phage), as described herein.

*3. Chimeric and Humanized Antibodies*

In certain instances, an anti-TIGIT antagonist antibody and/or PD-1 axis binding antagonist antibody (e.g., anti-PD-L1 antagonist antibody) provided herein is a chimeric antibody. Certain chimeric antibodies are described, e.g., in U.S. Patent No. 4,816,567; and Morrison et al. Proc. Natl. Acad. Sci. USA, 81:6851-6855 (1984)). In one example, a chimeric antibody comprises a non-human variable region (e.g., a variable region derived from a mouse, rat, hamster, rabbit, or non-human primate, such as a monkey) and a human constant region. In a further example, a chimeric antibody is a "class switched" antibody in which the class or subclass has been changed from that of the parent antibody. Chimeric antibodies include antigen-binding fragments thereof.

In certain instances, a chimeric antibody is a humanized antibody. Typically, a non-human antibody is humanized to reduce immunogenicity to humans, while retaining the specificity and affinity of the parental non-human antibody. Generally, a humanized antibody comprises one or more variable domains in which HVRs, e.g., CDRs, (or portions thereof) are derived from a non-human antibody, and FRs (or portions thereof) are derived from human antibody sequences. A humanized antibody optionally will also comprise at least a portion of a human constant region. In some instances, some FR residues in a humanized antibody are substituted with corresponding residues from a non-human antibody (e.g., the antibody from which the HVR residues are derived), e.g., to restore or improve antibody specificity or affinity.

Humanized antibodies and methods of making them are reviewed, e.g., in Almagro and Fransson, Front. Biosci. 13:1619-1633 (2008), and are further described, e.g., in Riechmann et al., Nature 332:323-329 (1988); Queen et al., Proc. Natl Acad. Sci. USA 86:10029-10033 (1989); US Patent Nos. 5, 821,337, 7,527,791, 6,982,321, and 7,087,409; Kashmiri et al., Methods 36:25-34 (2005) (describing specificity determining region (SDR) grafting); Padlan, Mol. Immunol. 28:489-498 (1991) (describing "resurfacing"); Dall'Acqua et al., Methods 36:43-60 (2005) (describing "FR shuffling"); and Osbourn et al., Methods 36:61-68 (2005) and Klimka et al., Br. J. Cancer, 83:252-260 (2000) (describing the "guided selection" approach to FR shuffling).

Human framework regions that may be used for humanization include but are not limited to: framework regions selected using the "best-fit" method (see, e.g., Sims et al. J. Immunol. 151:2296 (1993)); framework regions derived from the consensus sequence of human antibodies of a particular subgroup of light or heavy chain variable regions (see, e.g., Carter et al. Proc. Natl. Acad. Sci. USA, 89:4285 (1992); and Presta et al. J. Immunol., 151:2623 (1993)); human mature (somatically mutated) framework regions or human germline framework regions (see, e.g., Almagro and Fransson, Front. Biosci. 13:1619-1633 (2008)); and framework regions derived from screening FR libraries (see, e.g., Baca et al., J. Biol. Chem. 272:10678-10684 (1997) and Rosok et al., J. Biol. Chem. 271:22611-22618 (1996)).

*4. Human Antibodies*

In certain instances, an anti-TIGIT antagonist antibody and/or PD-1 axis binding antagonist antibody (e.g., anti-PD-L1 antagonist antibody) provided herein is a human antibody. Human antibodies can be produced using various techniques known in the art. Human antibodies are described generally in van Dijk and van de Winkel, Curr. Opin. Pharmacol. 5: 368-74 (2001) and Lonberg, Curr. Opin. Immunol. 20:450-459 (2008).

Human antibodies may be prepared by administering an immunogen to a transgenic animal that has been modified to produce intact human antibodies or intact antibodies with human variable regions in response to antigenic challenge. Such animals typically contain all or a portion of the human immunoglobulin loci, which replace the endogenous immunoglobulin loci, or which are present extrachromosomally or integrated randomly into the animal's chromosomes. In such transgenic mice, the endogenous immunoglobulin loci have generally been inactivated. For review of methods for obtaining human antibodies from transgenic animals, see Lonberg, Nat. Biotech. 23:1117-1125 (2005). See also, e.g., U.S. Patent Nos. 6,075,181 and 6,150,584 describing XENOMOUSE™ technology; U.S. Patent No. 5,770,429 describing HUMAB® technology; U.S. Patent No. 7,041,870 describing K-M MOUSE® technology, and U.S. Patent Application Publication No. US 2007/0061900, describing VELOCIMOUSE® technology). Human variable regions from intact antibodies generated by such animals may be further modified, e.g., by combining with a different human constant region.

Human antibodies can also be made by hybridoma-based methods. Human myeloma and mouse-human heteromyeloma cell lines for the production of human monoclonal antibodies have been described. (See, e.g., Kozbor J. Immunol., 133: 3001 (1984); Brodeur et al., Monoclonal Antibody Production Techniques and Applications, pp. 51-63 (Marcel Dekker, Inc., New York, 1987); and Boerner et al., J. Immunol., 147: 86 (1991).) Human antibodies generated via human B-cell hybridoma technology are also described in Li et al., Proc. Natl. Acad. Sci. USA, 103:3557-3562 (2006). Additional methods include those described, for example, in U.S. Patent No. 7,189,826 (describing production of monoclonal human IgM antibodies from hybridoma cell lines) and Ni, Xiandai Mianyixue, 26(4):265-268 (2006) (describing human-human hybridomas). Human hybridoma technology (Trioma technology) is also described in Vollmers and Brandlein, Histology and Histopathology, 20(3):927-937 (2005) and Vollmers and Brandlein, Methods and Findings in Experimental and Clinical Pharmacology, 27(3):185-91 (2005).

Human antibodies may also be generated by isolating Fv clone variable domain sequences selected from human-derived phage display libraries. Such variable domain sequences may then be combined with a desired human constant domain. Techniques for selecting human antibodies from antibody libraries are described below.

*5. Library-Derived Antibodies*

Anti-TIGIT antagonist antibody and/or PD-1 axis binding antagonist antibodies (e.g., anti-PD-L1 antagonist antibodies) of the invention may be isolated by screening combinatorial libraries for antibodies with the desired activity or activities. For example, a variety of methods are known in the art for generating phage display libraries and screening such libraries for antibodies possessing the desired binding characteristics. Such methods are reviewed, e.g., in Hoogenboom et al. in Methods in Molecular Biology 178:1-37 (O'Brien et al., ed., Human Press, Totowa, NJ, 2001) and further described, e.g., in the McCafferty et al., Nature 348:552-554; Clackson et al., Nature 352: 624-628 (1991); Marks et al., J. Mol. Biol. 222: 581-597 (1992); Marks and Bradbury, in Methods in Molecular Biology 248:161-175 (Lo, ed., Human Press, Totowa, NJ, 2003); Sidhu et al., J. Mol. Biol. 338(2): 299-310 (2004); Lee et al., J. Mol. Biol. 340(5): 1073-1093 (2004); Fellouse, Proc. Natl. Acad. Sci. USA 101(34): 12467-12472 (2004); and Lee et al., J. Immunol. Methods 284(1-2): 119-132(2004).

In certain phage display methods, repertoires of VH and VL genes are separately cloned by polymerase chain reaction (PCR) and recombined randomly in phage libraries, which can then be screened for antigen-binding phage as described in Winter et al., Ann. Rev. Immunol., 12: 433-455 (1994). Phage typically display antibody fragments, either as single-chain Fv (scFv) fragments or as Fab fragments. Libraries from immunized sources provide high-affinity antibodies to the immunogen without the requirement of constructing hybridomas. Alternatively, the naive repertoire can be cloned (e.g., from human) to provide a single source of antibodies to a wide range of non-self and also self antigens without any immunization as described by Griffiths et al., EMBO J, 12: 725-734 (1993). Finally, naive libraries can also be made synthetically by cloning unrearranged V-gene segments from stem cells, and using PCR primers containing random sequence to encode the highly variable CDR3 regions and to accomplish rearrangement *in vitro,* as described by Hoogenboom and Winter, J. Mol. Biol., 227: 381-388 (1992). Patent publications describing human antibody phage libraries include, for example: US Patent No. 5,750,373, and US Patent Publication Nos. 2005/0079574, 2005/0119455, 2005/0266000, 2007/0117126, 2007/0160598, 2007/0237764, 2007/0292936, and 2009/0002360.

Anti-TIGIT antagonist antibody and/or PD-1 axis binding antagonist antibodies (e.g., anti-PD-L1 antagonist antibodies) or antibody fragments isolated from human antibody libraries are considered human antibodies or human antibody fragments herein.

*6. Antibody Variants*

In certain instances, amino acid sequence variants of the anti-TIGIT antagonist antibodies and/or PD-1 axis binding antagonist antibodies (e.g., anti-PD-L1 antagonist antibodies) of the invention are contemplated. As described in detail herein, anti-TIGIT antagonist antibodies and PD-1 axis binding antagonist antibodies (e.g., anti-PD-L1 antagonist antibodies) may be optimized based on desired structural and functional properties. For example, it may be desirable to improve the binding affinity and/or other biological properties of the antibody. Amino acid sequence variants of an antibody may be prepared by introducing appropriate modifications into the nucleotide sequence encoding the antibody, or by peptide synthesis. Such modifications include, for example, deletions from, and/or insertions into and/or substitutions of residues within the amino acid sequences of the antibody. Any combination of deletion, insertion, and substitution can be made to arrive at the final construct, provided that the final construct possesses the desired characteristics, for example, antigen-binding.

*I. Substitution, Insertion, and Deletion Variants*

In certain instances, anti-TIGIT antagonist antibody and/or PD-1 axis binding antagonist antibody (e.g., anti-PD-L1 antagonist antibody) variants having one or more amino acid substitutions are provided. Sites of interest for substitutional mutagenesis include the HVRs and FRs. Conservative substitutions are shown in Table 1 under the heading of "preferred substitutions." More substantial changes are provided in Table 1 under the heading of "exemplary substitutions," and as further described below in reference to amino acid side chain classes. Amino acid substitutions may be introduced into an antibody of interest and the products screened for a desired activity, for example, retained/improved antigen binding, decreased immunogenicity, or improved ADCC or CDC.

**Table 1. Exemplary and Preferred Amino Acid Substitutions**

| Original Residue | Exemplary Substitutions | Preferred Substitutions |
|---|---|---|
| Ala (A) | Val; Leu; Ile | Val |
| Arg (R) | Lys; Gln; Asn | Lys |
| Asn (N) | Gln; His; Asp, Lys; Arg | Gln |
| Asp (D) | Glu; Asn | Glu |
| Cys (C) | Ser; Ala | Ser |
| Gln (Q) | Asn; Glu | Asn |
| Glu (E) | Asp; Gln | Asp |
| Gly (G) | Ala | Ala |
| His (H) | Asn; Gln; Lys; Arg | Arg |
| Ile (I) | Leu; Val; Met; Ala; Phe; Norleucine | Leu |
| Leu (L) | Norleucine; Ile; Val; Met; Ala; Phe | Ile |

(continued)

| Original Residue | Exemplary Substitutions | Preferred Substitutions |
|---|---|---|
| Lys (K) | Arg; Gln; Asn | Arg |
| Met (M) | Leu; Phe; Ile | Leu |
| Phe (F) | Trp; Leu; Val; Ile; Ala; Tyr | Tyr |
| Pro (P) | Ala | Ala |
| Ser (S) | Thr | Thr |
| Thr (T) | Val; Ser | Ser |
| Trp (W) | Tyr; Phe | Tyr |
| Tyr (Y) | Trp; Phe; Thr; Ser | Phe |
| Val (V) | Ile; Leu; Met; Phe; Ala; Norleucine | Leu |

Amino acids may be grouped according to common side-chain properties:

(1) hydrophobic: Norleucine, Met, Ala, Val, Leu, Ile;
(2) neutral hydrophilic: Cys, Ser, Thr, Asn, Gln;
(3) acidic: Asp, Glu;
(4) basic: His, Lys, Arg;
(5) residues that influence chain orientation: Gly, Pro;
(6) aromatic: Trp, Tyr, Phe.

Non-conservative substitutions will entail exchanging a member of one of these classes for another class.

One type of substitutional variant involves substituting one or more hypervariable region residues of a parent antibody (e.g. a humanized or human antibody). Generally, the resulting variant(s) selected for further study will have modifications (e.g., improvements) in certain biological properties (e.g., increased affinity, reduced immunogenicity) relative to the parent antibody and/or will have substantially retained certain biological properties of the parent antibody. An exemplary substitutional variant is an affinity matured antibody, which may be conveniently generated, e.g., using phage display-based affinity maturation techniques such as those described herein. Briefly, one or more HVR residues are mutated and the variant antibodies displayed on phage and screened for a particular biological activity (e.g. binding affinity).

Alterations (e.g., substitutions) may be made in HVRs, e.g., to improve antibody affinity. Such alterations may be made in HVR "hotspots," i.e., residues encoded by codons that undergo mutation at high frequency during the somatic maturation process (see, e.g., Chowdhury, Methods Mol. Biol. 207:179-196 (2008)), and/or residues that contact antigen, with the resulting variant VH or VL being tested for binding affinity. Affinity maturation by constructing and reselecting from secondary libraries has been described, e.g., in Hoogenboom et al. in Methods in Molecular Biology 178:1-37 (O'Brien et al., ed., Human Press, Totowa, NJ, (2001).) In some instances of affinity maturation, diversity is introduced into the variable genes chosen for maturation by any of a variety of methods (e.g., error-prone PCR, chain shuffling, or oligonucleotide-directed mutagenesis). A secondary library is then created. The library is then screened to identify any antibody variants with the desired affinity. Another method to introduce diversity involves HVR-directed approaches, in which several HVR residues (e.g., 4-6 residues at a time) are randomized. HVR residues involved in antigen binding may be specifically identified, e.g., using alanine scanning mutagenesis or modeling. CDR-H3 and CDR-L3 in particular are often targeted.

In certain instances, substitutions, insertions, or deletions may occur within one or more HVRs so long as such alterations do not substantially reduce the ability of the antibody to bind antigen. For example, conservative alterations (e.g., conservative substitutions as provided herein) that do not substantially reduce binding affinity may be made in HVRs. Such alterations may, for example, be outside of antigen contacting residues in the HVRs. In certain instances of the variant VH and VL sequences provided above, each HVR either is unaltered, or includes no more than one, two, or three amino acid substitutions.

A useful method for identification of residues or regions of an antibody that may be targeted for mutagenesis is called "alanine scanning mutagenesis" as described by Cunningham and Wells (1989) Science, 244:1081-1085. In this method, a residue or group of target residues (e.g., charged residues such as Arg, Asp, His, Lys, and Glu) are identified and replaced by a neutral or negatively charged amino acid (e.g., alanine or polyalanine) to determine whether the interaction of the antibody with antigen is affected. Further substitutions may be introduced at the amino acid locations demonstrating functional sensitivity to the initial substitutions. Alternatively, or additionally, a crystal structure of an antigen-antibody complex to identify contact points between the antibody and antigen. Such contact residues and neighboring residues may be targeted or eliminated as candidates for substitution. Variants may be screened to determine whether they contain the desired properties.

Amino acid sequence insertions include amino- and/or carboxyl-terminal fusions ranging in length from one residue to polypeptides containing a hundred or more residues, as well as intrasequence insertions of single or multiple amino acid residues. Examples of terminal insertions include an antibody with an N-terminal methionyl residue. Other insertional variants of the antibody molecule include the fusion to the N- or C-terminus of the antibody to an enzyme (e.g. for ADEPT) or a polypeptide which increases the serum half-life of the antibody.

*II. Glycosylation variants*

In certain instances, anti-TIGIT antagonist antibodies and/or PD-1 axis binding antagonist antibodies (e.g., anti-PD-L1 antagonist antibodies) of the invention can be altered to increase or decrease the extent to which the antibody is glycosylated. Addition or deletion of glycosylation sites to anti-TIGIT antagonist antibody and/or PD-1 axis binding antagonist antibody (e.g., anti-PD-L1 antagonist antibody) of the invention may be conveniently accomplished by altering the amino acid sequence such that one or more glycosylation sites is created or removed.

Where the antibody comprises an Fc region, the carbohydrate attached thereto may be altered. Native antibodies produced by mammalian cells typically comprise a branched, biantennary oligosaccharide that is generally attached by an N-linkage to Asn297 of the CH2 domain of the Fc region. See, e.g., Wright et al. TIBTECH 15:26-32 (1997). The oligosaccharide may include various carbohydrates, e.g., mannose, N-acetyl glucosamine (GlcNAc), galactose, and sialic acid, as well as a fucose attached to a GlcNAc in the "stem" of the biantennary oligosaccharide structure. In some instances, modifications of the oligosaccharide in an antibody of the invention are made in order to create antibody variants with certain improved properties.

In one instance, anti-TIGIT antagonist antibody and/or PD-1 axis binding antagonist antibody (e.g., anti-PD-L1 antagonist antibody) variants are provided having a carbohydrate structure that lacks fucose attached (directly or indirectly) to an Fc region. For example, the amount of fucose in such antibody may be from 1% to 80%, from 1% to 65%, from 5% to 65% or from 20% to 40%. The amount of fucose is determined by calculating the average amount of fucose within the sugar chain at Asn297, relative to the sum of all glycostructures attached to Asn 297 (e.g., complex, hybrid and high mannose structures) as measured by MALDI-TOF mass spectrometry, as described in WO 2008/077546, for example. Asn297 refers to the asparagine residue located at about position 297 in the Fc region (EU numbering of Fc region residues); however, Asn297 may also be located about ± 3 amino acids upstream or downstream of position 297, i.e., between positions 294 and 300, due to minor sequence variations in antibodies. Such fucosylation variants may have improved ADCC function. See, e.g., US Patent Publication Nos. US 2003/0157108 (Presta, L.); US 2004/0093621 (Kyowa Hakko Kogyo Co., Ltd). Examples of publications related to "defucosylated" or "fucose-deficient" antibody variants include: US 2003/0157108; WO 2000/61739; WO 2001/29246; US 2003/0115614; US 2002/0164328; US 2004/0093621; US 2004/0132140; US 2004/0110704; US 2004/0110282; US 2004/0109865; WO 2003/085119; WO 2003/084570; WO 2005/035586; WO 2005/035778; WO2005/053742; WO2002/031140; Okazaki et al. J. Mol. Biol. 336:1239-1249 (2004); Yamane-Ohnuki et al. Biotech. Bioeng. 87: 614 (2004). Examples of cell lines capable of producing defucosylated antibodies include Led 3 CHO cells deficient in protein fucosylation (Ripka et al. Arch. Biochem. Biophys. 249:533-545 (1986); US Pat Appl No US 2003/0157108 A1, Presta, L; and WO 2004/056312 A1, Adams et al., especially at Example 11), and knockout cell lines, such as alpha-1,6-fucosyltransferase gene, *FUT8,* knockout CHO cells (see, e.g., Yamane-Ohnuki et al. Biotech. Bioeng. 87: 614 (2004); Kanda, Y. et al., Biotechnol. Bioeng., 94(4):680-688 (2006); and WO2003/085107).

In view of the above, in some instances, the methods of the invention involve administering to the subject in the context of a fractionated, dose-escalation dosing regimen an anti-TIGIT antagonist antibody (e.g., an anti-TIGIT antagonist antibody disclosed herein, e.g., tiragolumab) and/or PD-1 axis binding antagonist antibody (e.g., anti-PD-L1 antagonist antibody (e.g., atezolizumab)) variant that comprises an aglycosylation site mutation. In some instances, the aglycosylation site mutation reduces effector function of the antibody. In some instances, the aglycosylation site mutation is a substitution mutation. In some instances, the antibody comprises a substitution mutation in the Fc region that reduces effector function. In some instances, the substitution mutation is at amino acid residue N297, L234, L235, and/or D265 (EU numbering). In some instances, the substitution mutation is selected from the group consisting of N297G, N297A, L234A, L235A, D265A, and P329G. In some instances, the substitution mutation is at amino acid residue N297. In a preferred instance, the substitution mutation is N297A.

Anti-TIGIT antagonist antibody and/or PD-1 axis binding antagonist antibody (e.g., anti-PD-L1 antagonist antibody) variants are further provided with bisected oligosaccharides, for example, in which a biantennary oligosaccharide attached to the Fc region of the antibody is bisected by GlcNAc. Such antibody variants may have reduced fucosylation and/or improved ADCC function. Examples of such antibody variants are described, e.g., in WO 2003/011878 (Jean-Mairet et al.); US Patent No. 6,602,684 (Umana et al.); and US 2005/0123546 (Umana et al.). Antibody variants with at least one galactose residue in the oligosaccharide attached to the Fc region are also provided. Such antibody variants may have improved CDC function. Such antibody variants are described, e.g., in WO 1997/30087 (Patel et al.); WO 1998/58964 (Raju, S.); and WO 1999/22764 (Raju, S.).

*III. Fc region variants*

In certain instances, one or more amino acid modifications are introduced into the Fc region of an anti-TIGIT antagonist (e.g., an anti-TIGIT antagonist antibody disclosed herein, e.g., tiragolumab) antibody and/or PD-1 axis binding antagonist antibody (e.g.,anti-PD-L1 antagonist antibody (e.g., atezolizumab)) of the invention, thereby generating an Fc region variant (see e.g., US 2012/0251531). The Fc region variant may comprise a human Fc region sequence (e.g., a human IgG1, IgG2, IgG3 or IgG4 Fc region) comprising an amino acid modification (e.g., a substitution) at one or more amino acid positions.

In certain instances, the invention contemplates an anti-TIGIT antagonist antibody and/or PD-1 axis binding antagonist antibody (e.g., anti-PD-L1 antagonist antibody variant that possesses some but not all effector functions, which make it a desirable candidate for applications in which the half-life of the antibody *in vivo* is important yet certain effector functions (such as complement and ADCC) are unnecessary or deleterious. *In vitro* and/or *in vivo* cytotoxicity assays can be conducted to confirm the reduction/depletion of CDC and/or ADCC activities. For example, Fc receptor (FcR) binding assays can be conducted to ensure that the antibody lacks FcγR binding (hence likely lacking ADCC activity), but retains FcRn binding ability. The primary cells for mediating ADCC, NK cells, express Fc(RIII only, whereas monocytes express Fc(RI, Fc(RII, and Fc(RIII. FcR expression on hematopoietic cells is summarized in Table 3 on page 464 of Ravetch and Kinet, Annu. Rev. Immunol. 9:457-492 (1991). Nonlimiting examples of *in vitro* assays to assess ADCC activity of a molecule of interest is described in U.S. Patent No. 5,500,362 (see, e.g. Hellstrom, I. et al. Proc. Nat'l Acad. Sci. USA 83:7059-7063 (1986)) and Hellstrom, I et al., Proc. Nat'l Acad. Sci. USA 82:1499-1502 (1985); 5,821,337 (see Bruggemann, M. et al., J. Exp. Med. 166:1351-1361 (1987)). Alternatively, non-radioactive assays methods may be employed (see, for example, ACTI™ non-radioactive cytotoxicity assay for flow cytometry (CellTechnology, Inc. Mountain View, CA; and CytoTox 96® non-radioactive cytotoxicity assay (Promega, Madison, WI). Useful effector cells for such assays include peripheral blood mononuclear cells (PBMC) and Natural Killer (NK) cells. Alternatively, or additionally, ADCC activity of the molecule of interest may be assessed *in vivo, e.g.,* in an animal model such as that disclosed in Clynes et al. Proc. Nat'l Acad. Sci. USA 95:652-656 (1998). C1q binding assays may also be carried out to confirm that the antibody is unable to bind C1q and hence lacks CDC activity. See, e.g., C1q and C3c binding ELISA in WO 2006/029879 and WO 2005/100402. To assess complement activation, a CDC assay may be performed (see, for example, Gazzano-Santoro et al. J. Immunol. Methods 202:163 (1996); Cragg, M.S. et al. Blood. 101:1045-1052 (2003); and Cragg, M.S. and M.J. Glennie Blood. 103:2738-2743 (2004)). FcRn binding and *in vivo* clearance/half-life determinations can also be performed using methods known in the art (see, e.g., Petkova, S.B. et al. Int'l. Immunol. 18(12):1759-1769 (2006)).

Antibodies with reduced effector function include those with substitution of one or more of Fc region residues 238, 265, 269, 270, 297, 327 and 329 (U.S. Patent Nos. 6,737,056 and 8,219,149). Such Fc mutants include Fc mutants with substitutions at two or more of amino acid positions 265, 269, 270, 297 and 327, including the so-called "DANA" Fc mutant with substitution of residues 265 and 297 to alanine (US Patent No. 7,332,581 and 8,219,149).

In certain instances, the proline at position 329 of a wild-type human Fc region in the antibody is substituted with glycine or arginine or an amino acid residue large enough to destroy the proline sandwich within the Fc/Fc.gamma receptor interface that is formed between the proline 329 of the Fc and tryptophan residues Trp 87 and Trp 110 of FcgRIII (Sondermann et al.: Nature 406, 267-273 (20 Jul. 2000)). In certain instances, the antibody comprises at least one further amino acid substitution. In one instance, the further amino acid substitution is S228P, E233P, L234A, L235A, L235E, N297A, N297D, or P331S, and still in another instance the at least one further amino acid substitution is L234A and L235A of the human IgG1 Fc region or S228P and L235E of the human IgG4 Fc region (see e.g., US 2012/0251531), and still in another instance the at least one further amino acid substitution is L234A and L235A and P329G of the human IgG1 Fc region.

Certain antibody variants with improved or diminished binding to FcRs are described. (See, e.g., U.S. Patent No. 6,737,056; WO 2004/056312, and Shields et al., J. Biol. Chem. 9(2): 6591-6604 (2001).)

In certain instance, an antibody variant comprises an Fc region with one or more amino acid substitutions which improve ADCC, e.g., substitutions at positions 298, 333, and/or 334 of the Fc region (EU numbering of residues).

In some instances, alterations are made in the Fc region that result in altered (i.e., either improved or diminished) C1q binding and/or Complement Dependent Cytotoxicity (CDC), e.g., as described in US Patent No. 6,194,551, WO 99/51642, and Idusogie et al. J. Immunol. 164: 4178-4184 (2000).

Antibodies with increased half-lives and improved binding to the neonatal Fc receptor (FcRn), which is responsible for the transfer of maternal IgGs to the fetus (Guyer et al., J. Immunol. 117:587 (1976) and Kim et al., J. Immunol. 24:249 (1994)), are described in US2005/0014934A1 (Hinton et al.). Those antibodies comprise an Fc region with one or more substitutions therein which improve binding of the Fc region to FcRn. Such Fc variants include those with substitutions at one or more of Fc region residues: 238, 256, 265, 272, 286, 303, 305, 307, 311, 312, 317, 340, 356, 360, 362, 376, 378, 380, 382, 413, 424, or 434, e.g., substitution of Fc region residue 434 (US Patent No. 7,371,826).

See also Duncan & Winter, Nature 322:738-40 (1988); U.S. Patent No. 5,648,260; U.S. Patent No. 5,624,821; and WO 94/29351 concerning other examples of Fc region variants.

In some aspects, the anti-TIGIT antagonist antibody (e.g., an anti-TIGIT antagonist antibody disclosed herein, e.g., tiragolumab) and/or anti-PD-L1 antagonist antibody (e.g., atezolizumab) comprises an Fc region comprising an N297G mutation.

In some instances, the anti-TIGIT antagonist antibody (e.g., an anti-TIGIT antagonist antibody disclosed herein, e.g., tiragolumab) and/or PD-1 axis binding antagonist antibody (e.g., anti-PD-L1 antagonist antibody (e.g., atezolizumab)) comprises one or more heavy chain constant domains, wherein the one or more heavy chain constant domains are selected from a first CH1 ($CH1_1$) domain, a first CH2 ($CH2_1$) domain, a first CH3 ($CH3_1$) domain, a second CH1 ($CH1_2$) domain, second CH2 ($CH2_2$) domain, and a second CH3 ($CH3_2$) domain. In some instances, at least one of the one or more heavy chain constant domains is paired with another heavy chain constant domain. In some instances, the $CH3_1$ and $CH3_2$ domains each comprise a protuberance or cavity, and wherein the protuberance or cavity in the $CH3_1$ domain is positionable in the cavity or protuberance, respectively, in the $CH3_2$ domain. In some instances, the $CH3_1$ and $CH3_2$ domains meet at an interface between said protuberance and cavity. In some instances, the $CH2_1$ and $CH2_2$ domains each comprise a protuberance or cavity, and wherein the protuberance or cavity in the $CH2_1$ domain is positionable in the cavity or protuberance, respectively, in the $CH2_2$ domain. In other instances, the $CH2_1$ and $CH2_2$ domains meet at an interface between said protuberance and cavity. In some instances, the anti-TIGIT antagonist antibody (e.g., an anti-TIGIT antagonist antibody disclosed herein, e.g., tiragolumab) and/or anti-PD-L1 antagonist antibody (e.g., atezolizumab) is an IgG1 antibody.

*IV. Cysteine engineered antibody variants*

In certain instances, it is desirable to create cysteine engineered anti-TIGIT antagonist antibodies and/or PD-1 axis binding antagonist antibodies (e.g., anti-PD-L1 antagonist antibodies), e.g., "thioMAbs," in which one or more residues of an antibody are substituted with cysteine residues. In particular instances, the substituted residues occur at accessible sites of the antibody. By substituting those residues with cysteine, reactive thiol groups are thereby positioned at accessible sites of the antibody and may be used to conjugate the antibody to other moieties, such as drug moieties or linker-drug moieties, to create an immunoconjugate, as described further herein. In certain instances, any one or more of the following residues are substituted with cysteine: V205 (Kabat numbering) of the light chain; A118 (EU numbering) of the heavy chain; and S400 (EU numbering) of the heavy chain Fc region. Cysteine engineered antibodies may be generated as described, for example, in U.S. Patent No. 7,521,541.

*V. Antibody derivatives*

In certain instances, an anti-TIGIT antagonist antibody of the invention (e.g., an anti-TIGIT antagonist antibody (e.g., tiragolumab) or a variant thereof) and/or PD-1 axis binding antagonist antibody (e.g., anti-PD-L1 antagonist antibody of the invention (e.g., atezolizumab or a variant thereof)) provided herein are further modified to contain additional nonproteinaceous moieties that are known in the art and readily available. The moieties suitable for derivatization of the antibody include but are not limited to water soluble polymers. Non-limiting examples of water soluble polymers include, but are not limited to, polyethylene glycol (PEG), copolymers of ethylene glycol/propylene glycol, carboxymethylcellulose, dextran, polyvinyl alcohol, polyvinyl pyrrolidone, poly-1, 3-dioxolane, poly-1,3,6-trioxane, ethylene/maleic anhydride copolymer, polyaminoacids (either homopolymers or random copolymers), and dextran or poly(n-vinyl pyrrolidone)polyethylene glycol, propropylene glycol homopolymers, prolypropylene oxide/ethylene oxide co-polymers, polyoxyethylated polyols (e.g., glycerol), polyvinyl alcohol, and mixtures thereof. Polyethylene glycol propionaldehyde may have advantages in manufacturing due to its stability in water. The polymer may be of any molecular weight, and may be branched or unbranched. The number of polymers attached to the antibody may vary, and if more than one polymer are attached, they can be the same or different molecules. In general, the number and/or type of polymers used for derivatization can be determined based on considerations including, but not limited to, the particular properties or functions of the antibody to be improved, whether the antibody derivative will be used in a therapy under defined conditions, etc.

In another instance, conjugates of an antibody and nonproteinaceous moiety that may be selectively heated by exposure to radiation are provided. In one instance, the nonproteinaceous moiety is a carbon nanotube (Kam et al., Proc. Natl. Acad. Sci. USA 102: 11600-11605 (2005)). The radiation may be of any wavelength, and includes, but is not limited to, wavelengths that do not harm ordinary cells, but which heat the nonproteinaceous moiety to a temperature at which cells proximal to the antibody-nonproteinaceous moiety are killed.

*Recombinant Production Methods*

Anti-TIGIT antagonist antibodies (e.g., an anti-TIGIT antagonist antibody disclosed herein, e.g., tiragolumab) and/or PD-1 axis binding antagonist antibodies (e.g.,anti-PD-L1 antagonist antibodies (e.g., atezolizumab)) of the invention may be produced using recombinant methods and compositions, for example, as described in U.S. Patent No. 4,816,567, which is incorporated herein by reference in its entirety.

For recombinant production of an anti-TIGIT antagonist antibody and/or PD-1 axis binding antagonist antibody (e.g., anti-PD-L1 antagonist antibody), nucleic acid encoding an antibody, is isolated and inserted into one or more vectors for further cloning and/or expression in a host cell. Such nucleic acid may be readily isolated and sequenced using conventional procedures (e.g., by using oligonucleotide probes that are capable of binding specifically to genes encoding the heavy and light chains of the antibody).

Suitable host cells for cloning or expression of antibody-encoding vectors include prokaryotic or eukaryotic cells described herein. For example, antibodies may be produced in bacteria, in particular when glycosylation and Fc effector function are not needed. For expression of antibody fragments and polypeptides in bacteria, see, e.g., U.S. Patent Nos. 5,648,237, 5,789,199, and 5,840,523. (See also Charlton, Methods in Molecular Biology, Vol. 248 (B.K.C. Lo, ed., Humana Press, Totowa, NJ, 2003), pp. 245-254, describing expression of antibody fragments in *E. coli*.) After expression, the antibody may be isolated from the bacterial cell paste in a soluble fraction and can be further purified.

In addition to prokaryotes, eukaryotic microbes such as filamentous fungi or yeast are suitable cloning or expression hosts for antibody-encoding vectors, including fungi and yeast strains whose glycosylation pathways have been "humanized," resulting in the production of an antibody with a partially or fully human glycosylation pattern. See Gerngross, Nat. Biotech. 22:1409-1414 (2004), and Li et al., Nat. Biotech. 24:210-215 (2006).

Suitable host cells for the expression of glycosylated antibody are also derived from multicellular organisms (invertebrates and vertebrates). Examples of invertebrate cells include plant and insect cells. Numerous baculoviral strains have been identified which may be used in conjunction with insect cells, particularly for transfection of *Spodoptera frugiperda* cells.

Plant cell cultures can also be utilized as hosts. See, e.g., US Patent Nos. 5,959,177, 6,040,498, 6,420,548, 7,125,978, and 6,417,429 (describing PLANTIBODIES™ technology for producing antibodies in transgenic plants).

Vertebrate cells may also be used as hosts. For example, mammalian cell lines that are adapted to grow in suspension may be useful. Other examples of useful mammalian host cell lines are monkey kidney CV1 line transformed by SV40 (COS-7); human embryonic kidney line (293 or 293 cells as described, e.g., in Graham et al., J. Gen Virol. 36:59 (1977)); baby hamster kidney cells (BHK); mouse sertoli cells (TM4 cells as described, e.g., in Mather, Biol. Reprod. 23:243-251 (1980)); monkey kidney cells (CV1); African green monkey kidney cells (VERO-76); human cervical carcinoma cells (HELA); canine kidney cells (MDCK; buffalo rat liver cells (BRL 3A); human lung cells (W138); human liver cells (Hep G2); mouse mammary tumor (MMT 060562); TRI cells, as described, e.g., in Mather et al., Annals N.Y. Acad. Sci. 383:44-68 (1982); MRC 5 cells; and FS4 cells. Other useful mammalian host cell lines include Chinese hamster ovary (CHO) cells, including DHFR$^-$ CHO cells (Urlaub et al., Proc. Natl. Acad. Sci. USA 77:4216 (1980)); and myeloma cell lines such as Y0, NS0 and Sp2/0. For a review of certain mammalian host cell lines suitable for antibody production, see, e.g., Yazaki and Wu, Methods in Molecular Biology, Vol. 248 (B.K.C. Lo, ed., Humana Press, Totowa, NJ), pp. 255-268 (2003).

*Immunoconjugates*

The invention also provides immunoconjugates comprising an anti-TIGIT antagonist (e.g., an anti-TIGIT antagonist antibody as disclosed herein, e.g., tiragolumab) and/or PD-1 axis binding antagonist (e.g., anti-PD-L1 antagonist antibody (e.g., atezolizumab)) of the invention conjugated to one or more cytotoxic agents, such as chemotherapeutic agents or drugs, growth inhibitory agents, toxins (e.g., protein toxins, enzymatically active toxins of bacterial, fungal, plant, or animal origin, or fragments thereof), or radioactive isotopes.

In some instances, an immunoconjugate is an antibody-drug conjugate (ADC) in which an antibody is conjugated to one or more drugs, including but not limited to a maytansinoid (see U.S. Patent Nos. 5,208,020, 5,416,064 and European Patent EP 0 425 235 B1); an auristatin such as monomethylauristatin drug moieties DE and DF (MMAE and MMAF) (see U.S. Patent Nos. 5,635,483 and 5,780,588, and 7,498,298); a dolastatin; a calicheamicin or derivative thereof (see U.S. Patent Nos. 5,712,374, 5,714,586, 5,739,116, 5,767,285, 5,770,701, 5,770,710, 5,773,001, and 5,877,296; Hinman et al., Cancer Res. 53:3336-3342 (1993); and Lode et al., Cancer Res. 58:2925-2928 (1998)); an anthracycline such as daunomycin or doxorubicin (see Kratz et al., Current Med. Chem. 13:477-523 (2006); Jeffrey et al., Bioorganic & Med. Chem. Letters 16:358-362 (2006); Torgov et al., Bioconj. Chem. 16:717-721 (2005); Nagy et al., Proc. Natl. Acad. Sci. USA 97:829-834 (2000); Dubowchik et al., Bioorg. & Med. Chem. Letters 12:1529-1532 (2002);

King et al., J. Med. Chem. 45:4336-4343 (2002); and U.S. Patent No. 6,630,579); methotrexate; vindesine; a taxane such as docetaxel, paclitaxel, larotaxel, tesetaxel, and ortataxel; a trichothecene; and CC1065.

In another instance, an immunoconjugate comprises an anti-TIGIT antagonist antibody as described herein (e.g., tiragolumab) or a PD-1 axis binding antagonist (e.g., an anti-PD-L1 antagonist antibody (e.g., atezolizumab)) conjugated to an enzymatically active toxin or fragment thereof, including but not limited to diphtheria A chain, nonbinding active fragments of diphtheria toxin, exotoxin A chain (from Pseudomonas aeruginosa), ricin A chain, abrin A chain, modeccin A chain, alpha-sarcin, Aleurites fordii proteins, dianthin proteins, Phytolaca americana proteins (PAPI, PAPII, and PAP-S), momordica charantia inhibitor, curcin, crotin, sapaonaria officinalis inhibitor, gelonin, mitogellin, restrictocin, phenomycin, enomycin, and the tricothecenes.

In another instance, an immunoconjugate comprises an anti-TIGIT antagonist antibody as described herein (e.g., tiragolumab) and/or a PD-1 axis binding antagonist (e.g., an anti-PD-L1 antagonist antibody) as described herein (e.g., atezolizumab) conjugated to a radioactive atom to form a radioconjugate. A variety of radioactive isotopes are available for the production of radioconjugates. Examples include $At^{211}$, $I^{131}$, $I^{125}$, $Y^{90}$, $Re^{186}$, $Re^{188}$, $Sm^{153}$, $Bi^{212}$, $P^{32}$, $Pb^{212}$ and radioactive isotopes of Lu. When the radioconjugate is used for detection, it may comprise a radioactive atom for scintigraphic studies, for example tc99m or 1123, or a spin label for nuclear magnetic resonance (NMR) imaging (also known as magnetic resonance imaging, mri), such as iodine-123 again, iodine-131, indium-111, fluorine-19, carbon-13, nitrogen-15, oxygen-17, gadolinium, manganese or iron.

Conjugates of an antibody and cytotoxic agent may be made using a variety of bifunctional protein coupling agents such as N-succinimidyl-3-(2-pyridyldithio) propionate (SPDP), succinimidyl-4-(N-maleimidomethyl) cyclohexane-1-carboxylate (SMCC), iminothiolane (IT), bifunctional derivatives of imidoesters (such as dimethyl adipimidate HCl), active esters (such as disuccinimidyl suberate), aldehydes (such as glutaraldehyde), bis-azido compounds (such as bis (p-azidobenzoyl) hexanediamine), bis-diazonium derivatives (such as bis-(p-diazoniumbenzoyl)-ethylenediamine), diisocyanates (such as toluene 2,6-diisocyanate), and bis-active fluorine compounds (such as 1,5-difluoro-2,4-dinitrobenzene). For example, a ricin immunotoxin can be prepared as described in Vitetta et al., Science 238:1098 (1987). Carbon-14-labeled 1-isothiocyanatobenzyl-3-methyldiethylene triaminepentaacetic acid (MX-DTPA) is an exemplary chelating agent for conjugation of radionucleotide to the antibody. See WO94/11026. The linker may be a "cleavable linker" facilitating release of a cytotoxic drug in the cell. For example, an acid-labile linker, peptidase-sensitive linker, photolabile linker, dimethyl linker, or disulfide-containing linker (Chari et al., Cancer Res. 52:127-131 (1992); U.S. Patent No. 5,208,020) may be used.

The immunuoconjugates or ADCs herein expressly contemplate, but are not limited to such conjugates prepared with cross-linker reagents including, but not limited to, BMPS, EMCS, GMBS, HBVS, LC-SMCC, MBS, MPBH, SBAP, SIA, SIAB, SMCC, SMPB, SMPH, sulfo-EMCS, sulfo-GMBS, sulfo-KMUS, sulfo-MBS, sulfo-SIAB, sulfo-SMCC, and sulfo-SMPB, and SVSB (succinimidyl-(4-vinylsulfone)benzoate) which are commercially available (e.g., from Pierce Biotechnology, Inc., Rockford, IL., U.S.A).

## V. PHARMACEUTICAL COMPOSITIONS AND FORMULATIONS

Any of the anti-TIGIT antagonist antibodies and PD-1 axis binding antagonists (e.g., anti-PD-L1 antagonist antibodies) described herein can be used in pharmaceutical compositions and formulations. Pharmaceutical compositions and formulations of an anti-TIGIT antagonist antibody and a PD-1 axis binding antagonist (e.g., an anti-PD-L1 antagonist antibody) can be prepared by mixing such antibodies having the desired degree of purity with one or more optional pharmaceutically acceptable carriers (Remington's Pharmaceutical Sciences 16th edition, Osol, A. Ed. (1980)), in the form of lyophilized formulations or aqueous solutions. Pharmaceutically acceptable carriers are generally nontoxic to recipients at the dosages and concentrations employed, and include, but are not limited to: buffers such as phosphate, citrate, and other organic acids; antioxidants including ascorbic acid and methionine; preservatives (such as octadecyldimethylbenzyl ammonium chloride; hexamethonium chloride; benzalkonium chloride; benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g. Zn-protein complexes); and/or non-ionic surfactants such as polyethylene glycol (PEG). Exemplary pharmaceutically acceptable carriers herein further include insterstitial drug dispersion agents such as soluble neutral-active hyaluronidase glycoproteins (sHASEGP), for example, human soluble PH-20 hyaluronidase glycoproteins, such as rHuPH20 (HYLENEX®, Baxter International, Inc.). Certain exemplary sHASEGPs and methods of use, including rHuPH20, are described in US Patent Publication Nos. 2005/0260186 and 2006/0104968. In one aspect, a sHASEGP is combined with one or more additional glycosaminoglycanases such as chondroitinases.

Exemplary lyophilized antibody formulations are described in US Patent No. 6,267,958. Aqueous antibody formulations include those described in US Patent No. 6,171,586 and WO2006/044908, the latter formulations including a histidine-acetate buffer.

The formulation herein may also contain more than one active ingredients as necessary for the particular indication being treated, preferably those with complementary activities that do not adversely affect each other. For example, it may be desirable to further provide an additional therapeutic agent (e.g., a chemotherapeutic agent, a cytotoxic agent, a growth inhibitory agent, and/or an anti-hormonal agent, such as those recited herein above). Such active ingredients are suitably present in combination in amounts that are effective for the purpose intended.

Active ingredients may be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nano-particles and nanocapsules) or in macroemulsions. Such techniques are disclosed in Remington's Pharmaceutical Sciences 16th edition, Osol, A. Ed. (1980).

Sustained-release preparations may be prepared. Suitable examples of sustained-release preparations include semipermeable matrices of solid hydrophobic polymers containing the antibody, which matrices are in the form of shaped articles, for example, films, or microcapsules. The formulations to be used for *in vivo* administration are generally sterile. Sterility may be readily accomplished, e.g., by filtration through sterile filtration membranes.

## VI. EXAMPLES

The following are examples of the methods of the invention. It is understood that various other embodiments may be practiced, given the general description provided above.

### Example 1. Efficacy of an anti-TIGIT antagonist antibody in combination with an anti-PD-L1 antagonist antibody in patients with lung cancer

To evaluate the efficacy and safety of treatment with an anti-TIGIT antagonist antibody (e.g., an anti-TIGIT antibody disclosed herein, e.g., tiragolumab) in combination with an anti-PD-L1 antagonist antibody (atezolizumab) compared with placebo in combination with atezolizumab in patients with lung cancer (e.g., non-small cell lung cancer (NSCLC), e.g., squamous or non-squamous NSCLC, e.g., locally advanced unresectable NSCLC (e.g., Stage IIIB NSCLC), or recurrent or metastatic NSCLC (e.g., Stage IV NSCLC)), patients were enrolled in a phase II, global, multicenter, randomized, blinded, placebo-controlled study. To be eligible, patients must (i) have not been previously treated for locally advanced unresectable or metastatic NSCLC, (ii) have had an Eastern Cooperative Oncology Group (ECOG) Performance Status (PS) of 0 or 1, (iii) have had a PD-L1 selected tumor (e.g., a tumor having high PD-L1 expression, e.g., a tumor PD-L1 expression with a tumor proportion score (TPS) ≥1% as determined by the PD-L1 IHC 22C3 pharmDx assay), (iv) not have had an epidermal growth factor receptor (*EGFR*) or anaplastic lymphoma kinase (*ALK*) gene mutation, (v) not have had a pulmonary lymphoepithelioma-like carcinoma subtype of NSCLC, and (vi) not have had an active Epstein-Barr virus (EBV) infection or a known or suspected chronic active EBV infection.

If a patient had positive serology for EBV IgG and/or was positive for Epstein-Barr nuclear antigen (EBNA), then EBV IgM testing and/or EBV PCR was required for consideration of eligibility. If the patient had positive serology for EBV IgG and/or is positive for EBNA, they must have been negative for EBV IgM and/or negative by EBV PCR. Additional EBV serology tests were performed for patients who subsequently experience an acute inflammatory event, e.g., systemic inflammatory response syndrome, while receiving study treatment.

The clinical trial consisted of a single phase, as described in detail below and diagrammed in Fig. 1.

*Randomization*

In this study, 135 patients were enrolled and randomized to one of two treatment arms in a 1:1 ratio (experimental arm to control arm). In the experimental arm, patients received an anti-TIGIT antagonist antibody (e.g., an anti-TIGIT antibody disclosed herein, e.g., tiragolumab) in combination with atezolizumab. In the control arm, patients received a placebo in combination with atezolizumab. The randomization was stratified on the basis of PD-L1 IHC 22C3 pharmDx assay results (e.g., a TPS of between 1-49% versus a TPS of ≥ 50%), histology of NSCLC (e.g., non-squamous versus squamous), and the patient's history of tobacco use (e.g., yes or no). These stratification factors were identified as critical prognostic factors for patients with NSCLC. Prospective stratification by these factors minimized differences in the two treatment arms due to sources other than the anti-TIGIT antagonist antibody.

*Study Treatment Dosage and Administration*

During treatment, atezolizumab was administered by intravenous infusion at a dose of 1200 mg every 3 weeks (21 ± 3 days). The atezolizumab dose was fixed and was not dependent on body weight. Atezolizumab was administered on Day 1 of each 21-day dosing cycle. In the experimental arm, patients received a fixed dose of 600 mg of an anti-TIGIT antagonist antibody (e.g., an anti-TIGIT antibody disclosed herein, e.g., tiragolumab) or placebo administered by intravenous infusion every 3 weeks (q3w) (21 ± 3 days). The anti-TIGIT antagonist antibody (e.g., an anti-TIGIT antibody disclosed herein, e.g., tiragolumab) or placebo is administered on Day 1 of each 21-day dosing cycle.

In one experiment, on the days of administration, atezolizumab was administered prior to the anti-TIGIT antagonist antibody (e.g., an anti-TIGIT antibody disclosed herein, e.g., tiragolumab) or placebo, with an intervening observation period. Prior to the first infusion of atezolizumab, the patient's vital signs (e.g., pulse rate, respiratory rate, blood pressure, and temperature) were recorded within 60 minutes before starting the infusion. The first infusion of atezolizumab was administered over 60 (±15) minutes. During this time, the patient's vital signs (pulse rate, respiratory rate, blood pressure, and temperature) were recorded at 15-minute intervals. Following infusion, the patient was observed for 60 minutes, during which time, the vital signs were monitored as described above. The first infusion of the anti-TIGIT antibody (e.g., an anti-TIGIT antibody disclosed herein, e.g., tiragolumab) or placebo was administered over 60 (±10) minutes. During this time, the patient's vital signs were recorded at 15-minute intervals. Following infusion, the patient was observed for 60 minutes, during which time the vital signs were monitored as described above. If no infusion-associated adverse events were experienced during the first infusions of atezolizumab, placebo, or the anti-TIGIT antagonist antibody (e.g., an anti-TIGIT antibody disclosed herein, e.g., tiragolumab), subsequent infusions could be administered over 30 (± 10) minutes. Additionally, the post-infusion observation periods could be reduced to 30 minutes. Pre-infusion recordation of vital signs continued to be recorded within 30 minutes prior to the start of infusion of atezolizumab.

In another experiment, on the days of administration, the anti-TIGIT antagonist antibody (e.g., an anti-TIGIT antibody disclosed herein, e.g., tiragolumab) or placebo was administered prior to atezolizumab, with an intervening observation period. Prior to the first infusion of the anti-TIGIT antibody or placebo, the patient's vital signs (e.g., pulse rate, respiratory rate, blood pressure, and temperature) were recorded within 60 minutes before starting the infusion. The first infusion of the anti-TIGIT antibody (e.g., an anti-TIGIT antibody disclosed herein, e.g., tiragolumab) or placebo was administered over 60 (±10) minutes. During this time, the patient's vital signs (pulse rate, respiratory rate, blood pressure, and temperature) were recorded at 15-minute intervals. Following infusion, the patient was observed for 60 minutes, during which time, the vital signs were monitored as described above. The first infusion of atezolizumab was administered over 60 (±15) minutes. During this time, the patient's vital signs were recorded at 15-minute intervals. Following infusion, the patient was observed for 60 minutes, during which time the vital signs were monitored as described above. If no infusion-associated adverse events were experienced during the first infusions of the anti-TIGIT antagonist antibody (e.g., an anti-TIGIT antibody disclosed herein, e.g., tiragolumab), placebo, or atezolizumab, subsequent infusions could be administered over 30 (± 10) minutes. Additionally, the post-infusion observation periods could be reduced to 30 minutes. Pre-infusion recordation of vital signs continued to be recorded within 60 minutes prior to the start of infusion of the anti-TIGIT antagonist antibody (e.g., an anti-TIGIT antibody disclosed herein, e.g., tiragolumab) or placebo.

Treatment continued until lack of clinical benefit, worsening of symptoms attributed to disease progression following an integrated assessment of radiographic data, biopsy results, and clinical status, decline in performance status, intolerable toxicity related to the study treatment, or tumor progression at a critical site that could not be managed with protocol-accepted therapy. Treatment was allowed to continue beyond progression upon consent of the patient and approval by the investigator.

*Concomitant Therapy*

Certain concomitant therapies were permitted. Concomitant therapies included any medication (e.g., prescription drugs, over the counter drugs, vaccines, herbal or homeopathic remedies, nutritional supplements) used by a patient in addition to protocol-mandated study treatment from seven days prior to initiation of study treatment to the treatment discontinuation visit. Patients were permitted to use the following concomitant therapies during the study.

Systemic corticosteroids and other immune-modulating medications may, in theory, attenuate the potential beneficial immunologic effects of treatment with the anti-TIGIT antagonist antibody and/or atezolizumab, but were administered at the discretion of the treating physician in line with the management guidelines. No premedication was allowed for the first infusion of atezolizumab, the anti-TIGIT antagonist antibody (e.g., an anti-TIGIT antibody disclosed herein, e.g., tiragolumab), or placebo. If the patient experienced an infusion-related reaction (IRR) during any previous infusion of atezolizumab, the anti-TIGIT antagonist antibody (e.g., an anti-TIGIT antibody disclosed herein, e.g., tiragolumab), or placebo, premedication with an antihistamine and/or antipyretic could be administered for Cycles ≥ 2 at the discretion of the treating physician after consultation with the medical monitor. The use of inhaled corticosteroids and mineralocorticoids (e.g., fludrocortisone) for patients with orthostatic hypotension or adrenocortical insufficiency was also allowed. Physiologic doses of corticosteroids for adrenal insufficiency were allowed.

Patients with abnormal renal function were evaluated and treated for other more common etiologies (e.g., prerenal and postrenal causes and concomitant medications including NSAIDs). In some cases, renal biopsies were performed to determine a definitive diagnosis and appropriate treatment. Patients presenting with signs and symptoms of nephritis, in the absence of an identified alternate etiology, were evaluated and treated according to the severity of the event. If the patient presented with a grade 1 renal event, study treatment continued while kidney functions (e.g., creatinine levels) were monitored and resolved to within normal limits and/or baseline values. Patients who experienced a grade 2 event had the study treatment withheld for up to twelve weeks and were treated with corticosteroids until the resolution of symptoms. Patients could resume the study treatment following a tapering period over at least one month of corticosteroids to an equivalent dose of ≤ 10 mg/day oral prednisone. Patients who experienced a grade 3 or grade 4 renal event permanently discontinued treatment with the anti-TIGIT antibody (e.g., tiragolumab)/placebo and atezolizumab and were treated with corticosteroids and/or immunosuppressive agents.

Megestrol administered as an appetite stimulant was acceptable while the patient was enrolled in the study. Patients who used oral contraceptives, hormone-replacement therapy, prophylactic or therapeutic anticoagulation therapy (such as low molecular weight heparin or warfarin at a stable dose level), or other maintenance therapy for non-malignant indications continued their use. Cannabinoids were permitted only if obtained in accordance with local regulations, and only if an established part of patient management prior to study enrolment.

Certain forms of radiotherapy were considered for pain palliation if patients were deriving benefit (e.g., treatment of known bony metastases) and provided they did not compromise assessments of tumor target lesions. In addition, the anti-TIGIT antagonist antibody (e.g., an anti-TIGIT antibody disclosed herein, e.g., tiragolumab) or placebo and atezolizumab treatment could continue during palliative radiotherapy. Patients who experienced a mixed response requiring local therapy (e.g., surgery, stereotactic radiosurgery, radiotherapy, radiofrequency ablation) for control of three or fewer lesions were still eligible to continue study treatment, at the discretion of the investigator, and after discussion with the medical monitor. Subsequent tumor assessments needed to take the local treatment into account in determining overall response per the response evaluation criteria in solid tumors (RECIST) v1.1 or per the immune-modified RECIST (imRECIST) criteria (see, e.g., Hodi et al. J. Clin. Oncol. e-pub, January 17, 2018, which is hereby incorporated by reference in its entirety), as appropriate.

Patients receiving denosumab prior to enrollment were maintained on bisphosphonate therapy instead (if willing and eligible) during screening and while actively treated with study drug. Initiation of bisphosphonates was discouraged during the treatment phase of the study due to potential immunomodulatory properties, however, initiation of such treatment did not result in discontinuation of study treatment.

In some instances, premedication with antihistamines, antipyretics, and/or analgesics were administered for the second and subsequent anti-TIGIT antagonist antibody (e.g., an anti-TIGIT antibody disclosed herein, e.g., tiragolumab) or placebo and atezolizumab infusions only, at the discretion of the investigator. In general, investigators managed a patient's care with supportive therapies as clinically indicated, per local standard practice. Patients who experienced infusion associated symptoms were eligible to receive treatment symptomatically with acetaminophen, ibuprofen, diphenhydramine, and/or H2 receptor antagonists (e.g., famotidine, cimetidine), or equivalent medications per local standard practice. Serious infusion-associated events manifested by dyspnea, hypotension, wheezing, bronchospasm, tachycardia, reduced oxygen saturation, or respiratory distress were managed with supportive therapies as clinically indicated (e.g., supplemental oxygen and $\beta_2$ adrenergic agonists).

*Efficacy Endpoints*

Co-primary and secondary efficacy analyses among all randomized patients were conducted when approximately 80 total PFS events occur.

To evaluate the efficacy of the anti-TIGIT antagonist antibody (e.g., an anti-TIGIT antibody disclosed herein, e.g., tiragolumab) in combination with atezolizumab compared with placebo in combination with atezolizumab, the objective response rate (ORR), with ORR defined as the percentage of patients who experienced a complete response (CR) or a partial response (PR) on two consecutive occasions ≥4 weeks apart (as determined by the investigator according to RECIST v1.1), was measured as a primary endpoint. The difference in ORR between the two study arms was estimated, along with PFS hazard ratios (HRs) with 90% confidence interval (CI). The ORRs between the two treatment arms were compared at the two-sided significance level of 5% using the Mantel-Haenszel Test, stratified by the study's stratification factors (i.e., PD-L1 IHC SP263 (Ventana) assay or PD-L1 IHC 22C3 pharmDx assay results (e.g., a TPS of between 1-49% versus a TPS of ≥ 50%), histology of NSCLC (e.g., non-squamous versus squamous), and the patient's history of tobacco use (e.g., yes or no)). An additional primary efficacy endpoint further included PFS, defined as the time from randomization to the date of first documented disease progression or death, whichever occurred first. A stratified Cox proportional-hazards model was used to estimate the HR and its 90% CI. PFS between treatment arms was compared using the two-sided stratified log-rank test. Kaplan-Meier methodology was used to estimate a PFS curve and median PFS for each treatment arm.

Secondary efficacy endpoints included duration of objective response (DOR), defined as the time from the first occurrence of a documented objective response to disease progression (as determined by the investigator according to RECIST v1.1), or death from any cause, whichever occurred first, and overall survival (OS) (i.e., the time from randomization to death from any cause). A stratified Cox proportional-hazards model was used to estimate the HR and its 90% CI. OS between treatment arms was compared using the two-sided stratified log-rank test. Kaplan-Meier methodology was used to estimate an OS curve and median OS for each treatment arm.

Additional exploratory efficacy endpoints further include evaluating ORR, DOR, and PFS according to immune-modified RECIST (imRECIST) criteria (see, e.g., Hodi et al. J. Clin. Oncol. e-pub, January 17, 2018, which is hereby incorporated by reference in its entirety), which are based on key principles from immune-related response criteria that were originally designed to account for tumor change patterns observed in melanoma patients treated with the CTLA-4 inhibitor ipilimumab (see, e.g., Wolchok et al. Clin. Can. Res. 15(23): 7412-20, 2009, which is hereby incorporated by reference in its entirety).

To evaluate the safety and tolerability of the anti-TIGIT antagonist antibody (e.g., an anti-TIGIT antibody disclosed herein, e.g., tiragolumab) in combination atezolizumab compared with the placebo in combination atezolizumab, the incidence, nature, and severity of adverse events (AEs) (e.g., AEs graded according to the National Cancer Institute Common Terminology Criteria for Adverse Events version 4.0 (NCI CTCAE v4.0)) were measured. Additionally, clinically significant changes in vital signs, physical findings, and clinical laboratory results from baseline during and following administration of the anti-TIGIT antagonist antibody (e.g., an anti-TIGIT antibody disclosed herein, e.g., tiragolumab) in combination with atezolizumab compared with placebo in combination with atezolizumab were also measured as an endpoint. Yet further efficacy endpoints included changes in health-related quality of life (HRQoL) as assessed by symptoms in lung cancer (SILC) scale (e.g., time to deterioration (TTD) in cough dyspenea and chest pain), the European organization for research and treatment of Cancer (EORTC) quality of life questionnaire C30 (QLC-C-30) (e.g., mean change from baseline in HRQoL and day-to-day function as measured by the global health status, physical function, and role function scales), and the EuroQol 5-Dimension, 5-Level Questionnaire (EQ-5D-5L) questionnaire (e.g., capture utility values) for health economic modeling, and/or tolerability of the anti-TIGIT antagonist antibody (e.g., an anti-TIGIT antibody disclosed herein, e.g., tiragolumab) in combination with atezolizumab or the placebo in combination with atezolizumab.

*Biomarkers*

Patient samples, including archival tumor tissues, as well as serum, plasma, whole blood, and stool are collected for exploratory biomarker assessments for all patients in the randomized study. In addition to assessing PD-L1 status, biomarkers related to resistance, disease progression, and clinical benefit of the anti-TIGIT antagonist antibody (e.g., an anti-TIGIT antibody disclosed herein, e.g., tiragolumab) and/or atezolizumab are analyzed. For example, potential predictive and prognostic biomarkers related to the clinical benefit and safety of the anti-TIGIT antagonist antibody (e.g., an anti-TIGIT antibody disclosed herein, e.g., tiragolumab) and/or atezolizumab are analyzed.

Tumor tissue and blood samples collected at baseline (and, if deemed clinically feasible by the investigator, tumor tissue collected at the time of disease progression) enables whole-exome sequencing (WES) and/or next-generation sequencing (NGS) to identify somatic mutations that are predictive of response to study treatment, are associated with progression to a more severe disease state, are associated with acquired resistance to study treatment, are associated with susceptibility to developing adverse events, or can increase the knowledge and understanding of disease biology.

Biomarkers include, but are not limited to, PD-L1 and TIGIT expression on tumor tissues and germline and somatic mutations from tumor tissue and/or from circulating tumor DNA in blood (including, but not limited to, mutation load, MSI, and MMR defects), identified through WGS and/or NGS, and plasma derived cytokines.

To assess the effect of the PD-L1/PD-1 pathway on ORR, PFS, DOR, and/or OS in the primary patient population, the relationship between protein, RNA, DNA, tumor mutational burden, and other exploratory biomarkers in tumor tissue and/or blood to efficacy, safety, PK, immunogenicity, and patient-reported outcomes (PROs) may be evaluated. Additionally, to assess the effect of the TIGIT pathway on ORR, PFS, DOR, and/or OS following in the primary population, ORR, DOR, PFS, and OS may be evaluated in a patient population whose tumors have TIGIT expression, as defined by protein and/or RNA expression.

Exploratory biomarker analyses may be performed in an effort to understand the association of these markers (e.g., TIGIT IHC status) with study treatment efficacy. The efficacy outcomes may be explored in a population of patients whose tumors have high TIGIT expression, as determined by IHC and/or RNA analysis. Exploratory analysis of WGS data may be conducted in the context of this study and explored in aggregate with data from other studies to increase researcher’s understanding of disease pathobiology and guide the development of new therapeutic approaches.

*Immunogenicity Analyses*

To evaluate the immune response to the anti-TIGIT antagonist antibody (e.g., an anti-TIGIT antibody disclosed herein, e.g., tiragolumab) and atezolizumab, the incidence of treatment-emergent anti-drug antibodies (ADAs) and their potential impact on safety, efficacy, and pharmacokinetics (PK) will be assessed (with assessments grouped according to treatment received).

*Pharmacokinetic Analyses*

To characterize the pharmacokinetics of the anti-TIGIT antagonist antibody (e.g., an anti-TIGIT antibody disclosed herein, e.g., tiragolumab) when given in combination with atezolizumab, serum concentrations of the anti-TIGIT antagonist antibody are determined from subjects at different time points. Further, to characterize the pharmacokinetics of atezolizumab when atezolizumab is administered in combination with the anti-TIGIT antagonist antibody (e.g., tiragolumab) or in combination with the placebo, plasma concentration of atezolizumab is obtained from subjects at different time points during the study. PK analyses are reported and summarized using descriptive statistics.

*Results*

*Enrollment and Demographics*

Patients were enrolled in study centers across Europe, East Asian, and the United States and randomized into the treatment arms (Table 2).

**Table 2. Patient Enrollment**

| **Region** | **Country** | **Placebo + Atezolizumab** ($n$ = 68) | **Tiragolumab + Atezolizumab** ($n$ = 67) |
|---|---|---|---|
| Europe | | 29 (43%) | 28 (42%) |
| | Spain | 20 (29%) | 15 (22%) |
| | France | 4 (6%) | 8 (12%) |
| | Serbia | 5 (7%) | 5 (7%) |
| East Asia | | 24 (35%) | 18 (27%) |
| | South Korea | 19 (28%) | 11 (16%) |
| | Taiwan | 5 (7%) | 7 (10%) |
| North America | | 15 (22%) | 21 (31%) |
| | USA | 15 (22%) | 21 (31%) |

Tables 3 and 4, below, summarize the demographic distribution of the patients that were enrolled and evaluated in this study.

**Table 3. Patient Demographics**

| | | **Placebo + Atezolizumab** ($n$ = 68) | **Tiragolumab + Atezolizumab** ($n$ = 67) |
|---|---|---|---|
| Age (years) | | | |
| | Mean (SD) | 67 (9.9) | 65.8 (10.4) |
| | Median | 68 | 68 |
| | Range | 40 - 83 | 42 - 84 |
| Age group (years) | | | |
| | < 65 | 28 (41.2%) | 28 (41.8%) |
| | ≥ 65 | 40 (58.8%) | 39 (58.2%) |

(continued)

| | | | |
|---|---|---|---|
| Sex | | | |
| | Male | **48 (70.6%)** | **39 (58.2%)** |
| | Female | 20 (29.4%) | 28 (41.8%) |
| Race | | | |
| | Asian | **23 (33.8%)** | **18 (26.9%)** |
| | White | 40 (58.8%) | 42 (62.7%) |
| | Multiple | 1 (1.5%) | |
| | Unknown | 4 (5.9%) | 7 (10.4%) |
| BMI (kg/$m^2$) | | | |
| | Mean (SD) | 25.3 (5.7) | 24.6 (4.7) |
| | Median | 24.5 | 24.1 |
| | Range | 15.2 - 43 | 15.7 - 36.5 |
| ECOG | | | |
| | 0 | 19 (27.9%) | 20 (29.9%) |
| | 1 | 48 (70.6%) | 47 (70.1%) |
| | 2* | 1 (1.5%) | - |
| PD-L1 IHC 22C3 TPS | | | |
| | 1 - 49% | 39 (57.4%) | 38 (56.7%) |
| | ≥ 50% | 29 (42.6%) | 29 (43.3%) |
| Tobacco history | | | |
| | Never | 7 (10.3%) | 7 (10.4%) |
| | Former | 44 (64.7%) | 41 (61.2%) |
| | Current | 17 (25%) | 19 (28.4%) |
| Tumor histology | | | |
| | Non-squamous | 40 (58.8%) | 40 (59.7%) |
| | Squamous | 28 (41.2%) | 27 (40.3%) |
| CRP (Covance) | | | |
| | < 3 mg/L | 11 (16.2%) | 10 (14.9%) |
| | >= 3 mg/L | 54 (79.4%) | 52 (77.6%) |
| | Missing | 3 (4.4%) | 5 (7.5%) |

**Table 4. Number and percent of patients in each stratum by PD-L1 TPS and tumor histology**

| **PD-L1 IHC 22C3 pharmDx assay result** | **Tumor histology** | **History of tobacco use** | **All patients *n* (%)** | **Placebo + Atezolizumab *n* (%)** | **Tiragolumab + Atezolizumab *n* (%)** |
|---|---|---|---|---|---|
| TPS 1-49% | Non-squamous | No | 7 (5%) | 4 (6%) | 3 (4%) |
| | | Yes | 32 (24%) | 16 (24%) | 16 (24%) |
| | Squamous | No | 2 (1%) | 1 (1%) | 1 (1%) |
| | | Yes | 36 (27%) | 18 (26%) | 18 (27%) |

(continued)

| **PD-L1 IHC 22C3 pharmDx assay result** | **Tumor histology** | **History of tobacco use** | **All patients *n* (%)** | **Placebo + Atezolizumab *n* (%)** | **Tiragolumab + Atezolizumab *n* (%)** |
|---|---|---|---|---|---|
| TPS ≥ 50% | Non-squamous | No | 5 (4%) | 2 (3%) | 3 (4%) |
| | | Yes | 36 (27%) | 18 (26%) | 18 (27%) |
| | Squamous | No | - | - | - |
| | | Yes | 17 (13%) | 9 (13%) | 8 (12%) |

*Patient Disposition*

Tables 5-7, below, summarize the quantity of patients that received either the monotherapy or the combination therapy during this study, as well as the status of the patients following their participation in the study. Briefly, all 67 patients allocated to the tiragolumab and atezolizumab combination therapy arm received the study treatment. Of these patients, 35 discontinued the treatment and 17 discontinued the study. All of the 68 patients allocated to the atezolizumab and placebo arm received the study treatment. Of these patients, 49 patients discontinued the treatment and 23 discontinued the study. Data from all patients from both treatment arms were analyzed.

**Table 5. Subject Disposition**

| | | Placebo + Atezolizumab (*n* = 68) | Tiragolumab + Atezolizumab (*n* = 67) |
|---|---|---|---|
| Subject in treatment study period | | 19 (28%) | 32 (48%) |
| Subject completed or discontinued from treatment study period | | **34 (50%)** | **22 (33%)** |
| | RADIOGRAPHIC PROGRESSIVE DISEASE | **18 (26%)** | **12 (18%)** |
| | WITHDRAWAL BY SUBJECT | 6 (9%) | 3 (4%) |
| | ADVERSE EVENT | 4 (6%) | 3 (4%) |
| | DEATH | 5 (7%) | 2 (3%) |
| | PHYSICIAN DECISION | 1 (1%) | 1 (1%) |
| | SYMPTOMATIC DETERIORATION | - | 1 (1%) |
| Subject completed or discontinued from long-term follow-up study period | | 15 (22%) | 13 (19%) |
| | DEATH | 15 (22%) | 12 (18%) |
| | WITHDRAWAL BY SUBJECT | - | 1 (1%) |
| 8 placebo + atezolizumab patients and 4 tiragolumab + atezolizumab patients completed the treatment study period but did not enter long-term follow-up. | | | |

**Table 6. Atezolizumab Exposure**

| | | **Placebo + Atezolizumab** (*n* = 68) | **Tiragolumab + Atezolizumab** (*n* = 67) |
|---|---|---|---|
| Treatment duration (months) | | | |
| | Mean (SD) | 3.24 (2.53) | 4.08 (2.75) |
| | Median | 2.81 | 4.27 |
| | Range | 0 - 9.2 | 0 - 10.1 |

(continued)

| | | | |
|---|---|---|---|
| Number of doses | | | |
| | Mean (SD) | 5.5 (3.6) | 6.6 (3.9) |
| | Median | 5 | 7 |
| | Range | 1 - 14 | 1 - 15 |
| Dose intensity based on total dose (%) | | | |
| | Mean (SD) | 103.97 (19.28) | 102.15 (18.95) |
| | Median | 100 | 100 |
| | Range | 75 - 200 | 66.7 - 200 |
| Dose intensity based on number of doses (%) | | | |
| | Mean (SD) | 104.22 (19.16) | 102.34 (18.86) |
| | Median | 100 | 100 |
| | Range | 75 - 200 | 66.7 - 200 |
| Dose modified at least once? | | | |
| | No | 68 (100%) | 67 (100%) |
| Infusion modified at least once? | | | |
| | Yes | 1 (1.5%) | 5 (7.5%) |
| Reason for infusion modification | | | |
| | Adverse event | 1 (1.5%) | 5 (7.5%) |
| | Other | - | 1 (1.5%) |

**Table 7. Tiragolumab Exposure**

| | | **Placebo + Atezolizumab** (*n* = 68) | **Tiragolumab + Atezolizumab** (*n* = 67) |
|---|---|---|---|
| Treatment duration (months) | | | |
| | Mean (SD) | 3.24 (2.53) | 4.07 (2.76) |
| | Median | 2.81 | 4.27 |
| | Range | 0 - 9.2 | 0 - 10.1 |
| Number of doses | | | |
| | Mean (SD) | 5.5 (3.6) | 6.6 (3.9) |
| | Median | 5 | 7 |
| | Range | 1 - 14 | 1 - 15 |
| Dose intensity based on total dose (%) | | | |
| | Mean (SD) | 104.18 (19.18) | 100.79 (20.64) |
| | Median | 100 | 100 |
| | Range | 75 - 200 | 50 - 200 |
| Dose intensity based on number of doses (%) | | | |
| | Mean (SD) | 104.22 (19.16) | 101.43 (20.16) |
| | Median | 100 | 100 |

(continued)

| | | | |
|---|---|---|---|
| Dose intensity based on number of doses (%) | | | |
| | Range | 75 - 200 | 50 - 200 |
| Dose modified at least once? | | | |
| | No | 68 (100%) | 67 (100%) |
| Infusion modified at least once? | | | |
| | Yes | - | 2 (3%) |
| Reason for infusion modification | | | |
| | Adverse event | - | 2 (3%) |

*Interim Analysis*

*Patient Demographics and Dispositions*

As shown in Fig. 2, minor imbalances were observed in sex, race, and ECOG in baseline demographics divided by TPS. More males were observed in the monotherapy arm, but more having an ECOG of 0 were observed in the combination therapy arm for patients in the TPS ≥ 50% population. In addition, more white patients and those having an ECOG of 0 were observed in the combination therapy arm in the PD-L1 TPS of between 1-49% population. Overall, less squamous cell cancer patients were present in the PD-L1 TPS ≥ 50% versus PD-L1 TPS of between 1-49% populations. Imbalances in treatment and study discontinuations was greater in the PD-L1 TPS ≥ 50% (Fig. 3). More patients discontinued treatment in the monotherapy arm in both populations. Discontinuations due to radiographic progressive disease were more prevalent in the monotherapy arm in both populations. Additionally, more patients discontinued the study and more deaths were observed in the monotherapy arm for patients within the PD-L1 TPS ≥ 50% population. Overall, more patients discontinued treatment in the local PD-L1 TPS of between 1-49% population than the PD-L1 TPS ≥ 50% population.

*Efficacy*

As of the interim cutoff date of April 21, 2019, of the 27 patients having a PD-L1 TPS ≥ 50% receiving tiragolumab in combination with atezolizumab, 14 (51.9%) achieved a best overall response of unconfirmed partial response (uPR) + confirmed partial response (cPR) as compared to only 10 out of 27 (18.5%) patients having a PD-L1 TPS ≥ 50% receiving the placebo in combination with atezolizumab alone (Fig. 4), amounting to a 33% difference in best overall response rate for patients having a PD-L1 TPS ≥ 50% receiving the combination therapy as opposed to the monotherapy. No differences in response were observed between the monotherapy or combination therapy treatment arms for patients having a PD-L1 TPS of between 1-49%. Further analysis of the subgroups revealed that squamous cell cancer patients in the ITT population responded better to the combination therapy of tiragolumab and atezolizumab compared to the atezolizumab monotherapy. In addition, in the PD-L1 TPS ≥ 50% population, whites and males responded better to the combination therapy (Fig. 5).

*Safety*

Of the adverse events observed in the monotherapy and combination therapy treatment arms, most were Grade 1 or Grade 2. Although a higher rate of fatigue, rash, IRR, pruritis and arthralgia was observed in the combination therapy arm, as shown in Fig. 6, similar safety profiles were observed for both treatment arms with no new safety signals identified. Serious and high grade treatment-related adverse events were well balanced between the two arms. Treatment-related and immune-related adverse events were imbalanced due to rash and IRR (Fig. 7). These results demonstrate that tiragolumab in combination with atezolizumab is safe relative to atezolizumab alone.

*Primary Endpoint Analysis*

*Efficacy*

The ORR for all patients receiving the tiragolumab in combination with atezolizumab versus the placebo in combination with atezolizumab (atezolizumab monotherapy) was evaluated across several categories including demographic information, tumor histology, and baseline risk factors (Fig. 8). The ORR for patients receiving tiragolumab in combination with atezolizumab was 31.3% compared to only 16.2% for patients receiving atezolizumab and the placebo, representing a 15.17% difference in ORR between the two groups (Fig. 9A). Patients having a PD-L1 TPS ≥ 50% appeared to derive an increased benefit from the combination therapy of tiragolumab and atezolizumab as these patients achieved an ORR or 55.2%, compared to patients receiving the same combination therapy having a PD-L1 TPS between 1 and 49%, who exhibited an ORR or 13.2% (Fig. 9B). Patients having a PD-L1 TPS ≥ 50% receiving the combination therapy exhibited an improvement in ORR of 37.93% over patients having a PD-L1 TPS ≥ 50% receiving the atezolizumab monotherapy (55.2% vs 17.2%). No differences in response were observed between the monotherapy or combination therapy treatment arms for patients having a PD-L1 TPS of between 1-49%.

PFS and OS were similarly evaluated for patients enrolled in the combination therapy and monotherapy arms (Figs. 10 and 12). Patients receiving the combination therapy experienced a median PFS of 5.42 months as compared to 3.58 months for those receiving the monotherapy (Fig.11A). Patients receiving the combination therapy exhibited an increase in OS compared to those receiving the monotherapy (Fig.13A). These differences in PFS and OS were observable within the subgroup of patients having a PD-L1 TPS ≥ 50% (Figs.11B and 13B), but not for patients having a PD-L1 TPS of between 1-49% (Figs.11C and 13C). The DOR was immature as of the primary analysis cutoff of June 30, 2019.

As shown in Figs. 14 and 15, patients having a PD-L1 TPS ≥ 50% treated with tiragolumab in combination with atezolizumab responded earlier with deeper, more durable responses than those having a PD-L1 TPS of between 1-49%. Additionally, patients having a PD-L1 TPS ≥ 50% treated with tiragolumab in combination with atezolizumab responded earlier with deeper, more durable responses than those having a PD-L1 TPS of ≥1% treated with the atezolizumab monotherapy. Overall, these results demonstrate that patients with high PD-L1 expression (TPS ≥ 50%) received the most benefit from the combination therapy.

*Safety*

The combination treatment of tiragolumab with atezolizumab was generally well tolerated with manageable toxicities. As shown in Table 8, the overall safety profile of the combination therapy was comparable to that of the atezolizumab monotherapy.

**Table 8. Overall adverse event (AE) profile**

| **Category** | **Placebo + Atezolizumab** (*n* = 68) | **Tiragolumab + Atezolizumab** (*n* = 67) | **All patients** (*n* = 135) |
|---|---|---|---|
| Total number of patients with at least one AE | 65 (95.6%) | 66 (98.5%) | 131 (97%) |
| Total number of AEs | 477 | 504 | 981 |
| Total number of deaths | 20 (29.4%) | 15 (22.4%) | 35 (25.9%) |
| Total number of patients withdrawn from study due to an AE | 6 (8.8%) | 4 (6%) | 10 (7.4%) |
| ≥ 1 AE with fatal outcome | 5 (7.4%) | 2 (3%) | 7 (5.2%) |
| ≥ 1 Serious AE | 24 (35.3%) | 23 (34.3%) | 47 (34.8%) |
| ≥ 1 Serious AE leading to withdrawal from tiragolumab /placebo | 5 (7.4%) | 4 (6%) | 9 (6.7%) |
| ≥ 1 Serious AE leading to withdrawal from atezolizumab | 5 (7.4%) | 4 (6%) | 9 (6.7%) |
| ≥ 1 Serious AE leading to dose modification/ interruption of tiragolumab/placebo | 10 (14.7%) | 12 (17.9%) | 22 (16.3%) |

(continued)

| Category | Placebo + Atezolizumab (*n* = 68) | Tiragolumab + Atezolizumab (*n* = 67) | All patients (*n* = 135) |
|---|---|---|---|
| ≥ 1 Serious AE leading to dose modification/ interruption of atezolizumab | 10 (14.7%) | 11 (16.4%) | 21 (15.6%) |
| ≥ 1 AE leading to withdrawal from tiragolumab /placebo | 7 (10.3%) | 5 (7.5%) | 12 (8.9%) |
| ≥ 1 AE leading to withdrawal from atezolizumab | 7 (10.3%) | 5 (7.5%) | 12 (8.9%) |
| ≥ 1 AE leading to dose modification/interruption of tiragolumab /placebo | 18 (26.5%) | 23 (34.3%) | 41 (30.4%) |
| ≥ 1 AE leading to dose modification/interruption of atezolizumab | 18 (26.5%) | 23 (34.3%) | 41 (30.4%) |
| ≥ 1 Grade 3-5 AE | 30 (44.1%) | 28 (41.8%) | 58 (43%) |
| ≥ 1 serious AE related to tiragolumab /placebo | 10 (14.7%) | 7 (10.4%) | 17 (12.6%) |
| ≥ 1 serious AE related to atezolizumab | 10 (14.7%) | 7 (10.4%) | 17 (12.6%) |
| ≥ 1 AE related to tiragolumab /placebo | 46 (67.6%) | 49 (73.1%) | 95 (70.4%) |
| ≥ 1 AE related to atezolizumab | 47 (69.1%) | 52 (77.6%) | 99 (73.3%) |
| ≥ 1 AE related to tiragolumab /placebo leading to withdrawal from tiragolumab /placebo | 7 (10.3%) | 4 (6%) | 11 (8.1%) |
| ≥ 1 AE related to atezolizumab that leading to withdrawal from atezolizumab | 7 (10.3%) | 4 (6%) | 11 (8.1%) |
| ≥ 1 AE related to tiragolumab /placebo leading to dose modification/interruption of tiragolumab /placebo | 6 (8.8%) | 10 (14.9%) | 16 (11.9%) |
| ≥ 1 AE related to atezolizumab leading to dose modification/interruption of atezolizumab | 6 (8.8%) | 11 (16.4%) | 17 (12.6%) |

Although the incidence of most adverse events occurred with similar frequency across the two treatment arms, the incidence of several adverse events, including, for example, infusion-related reactions, fatigue, rash, and arthralgia occurred with greater frequency in one arm versus the other. Adverse events with a difference of at least 5% in the frequency between treatment arms are shown in Table 9, and adverse events with a difference of at least 10% in frequency between the treatment arms are shown in Table 10.

**Table 9. Adverse events with a difference of ≥ 5%**

| Adverse Event | Placebo + Atezolizumab (*n* = 68) | Tiragolumab + Atezolizumab (*n* = 67) |
|---|---|---|
| INFUSION RELATED REACTION | 7 (10.3%) | 19 (28.4%) |
| FATIGUE | 9 (13.2%) | 15 (22.4%) |
| DYSPNOEA | 14 (20.6%) | 9 (13.4%) |
| PRURITUS | 8 (11.8%) | 13 (19.4%) |
| RASH | 6 (8.8%) | 13 (19.4%) |
| ARTHRALGIA | 6 (8.8%) | 11 (16.4%) |
| ALANINE AMINOTRANSFERASE INCREASED | 7 (10.3%) | 3 (4.5%) |
| OEDEMA PERIPHERAL | 3 (4.4%) | 7 (10.4%) |

(continued)

| Adverse Event | **Placebo + Atezolizumab** (*n* = 68) | **Tiragolumab + Atezolizumab** (*n* = 67) |
|---|---|---|
| PRODUCTIVE COUGH | 7 (10.3%) | 3 (4.5%) |
| BACK PAIN | 2 (2.9%) | 6 (9%) |
| PLEURAL EFFUSION | 2 (2.9%) | 6 (9%) |
| RASH MACULO-PAPULAR | 2 (2.9%) | 6 (9%) |
| RESPIRATORY TRACT INFECTION | 6 (8.8%) | 2 (3%) |
| HYPERCALCAEMIA | 4 (5.9%) | - |

**Table 10. Adverse events with a difference of ≥ 10%**

| Adverse Event | **Placebo + Atezolizumab** (*n* = 68) | **Tiragolumab + Atezolizumab** (*n* = 67) | **All patients** (*n* = 135) |
|---|---|---|---|
| ASTHENIA | 18 (26.5%) | 17 (25.4%) | 35 (25.9%) |
| INFUSION RELATED REACTION | 7 (10.3%) | 19 (28.4%) | 26 (19.3%) |
| DECREASED APPETITE | 13 (19.1%) | 11 (16.4%) | 24 (17.8%) |
| FATIGUE | 9 (13.2%) | 15 (22.4%) | 24 (17.8%) |
| DYSPNOEA | 14 (20.6%) | 9 (13.4%) | 23 (17%) |
| PRURITUS | 8 (11.8%) | 13 (19.4%) | 21 (15.6%) |
| DIARRHOEA | 10 (14.7%) | 9 (13.4%) | 19 (14.1%) |
| RASH | 6 (8.8%) | 13 (19.4%) | 19 (14.1%) |
| CONSTIPATION | 9 (13.2%) | 9 (13.4%) | 18 (13.3%) |
| PYREXIA | 9 (13.2%) | 9 (13.4%) | 18 (13.3%) |
| ARTHRALGIA | 6 (8.8%) | 11 (16.4%) | 17 (12.6%) |

Immune-related adverse events were more frequent in the tiragolumab + atezolizumab arm, but driven by Grade 1-2 IRR and rash (Tables 11 and 12).

**Table 11. Frequency of Immune-Mediated Adverse Events by Grade**

| Highest grade | **Placebo + Atezolizumab** (*n* = 68) | **Tiragolumab + Atezolizumab** (*n* = 67) | **All patients** (*n* = 135) |
|---|---|---|---|
| All | 32 (47.1%) | 44 (65.7%) | 76 (56.3%) |
| 1 | 13 (19.1%) | 16 (23.9%) | 29 (21.5%) |
| 2 | 11 (16.2%) | 19 (28.4%) | 30 (22.2%) |
| 3 | 5 (7.4%) | 6 (9%) | 11 (8.1%) |
| 4 | 3 (4.4%) | 3 (4.5%) | 6 (4.4%) |

**Table 12. Frequency of Immune-Mediated Adverse Events by Type**

| Adverse Event | **Placebo + Atezolizumab** (*n* = 68) | **Tiragolumab + Atezolizumab** (*n* = 67) | **All patients** (*n* = 135) |
|---|---|---|---|
| Immune-Mediated Rash | 10 (14.7%) | 26 (38.8%) | 36 (26.7%) |

(continued)

| **Adverse Event** | **Placebo + Atezolizumab** (*n* = 68) | **Tiragolumab + Atezolizumab** (*n* = 67) | **All patients** (*n* = 135) |
|---|---|---|---|
| Infusion-Related Reactions | 7 (10.3%) | 19 (28.4%) | 26 (19.3%) |
| Immune-Mediated Hepatitis (Diagnosis and Lab Abnormalities) | 11 (16.2%) | 7 (10.4%) | 18 (13.3%) |
| Immune-Mediated Hepatitis (Lab Abnormalities) | 10 (14.7%) | 6 (9%) | 16 (11.9%) |
| Immune-Mediated Hypothyroidism | 4 (5.9%) | 5 (7.5%) | 9 (6.7%) |
| Immune-Mediated Pancreatitis | 2 (2.9%) | 7 (10.4%) | 9 (6.7%) |
| Immune-Mediated Hyperthyroidism | 3 (4.4%) | 3 (4.5%) | 6 (4.4%) |
| Immune-Mediated Colitis | 1 (1.5%) | 2 (3%) | 3 (2.2%) |
| Immune-Mediated Hepatitis (Diagnosis) | 1 (1.5%) | 2 (3%) | 3 (2.2%) |
| Immune-Mediated Ocular Inflammatory Toxicity | 1 (1.5%) | 2 (3%) | 3 (2.2%) |
| Immune-Mediated Diabetes Mellitus | 1 (1.5%) | 1 (1.5%) | 2 (1.5%) |
| Immune-Mediated Pneumonitis | 1 (1.5%) | 1 (1.5%) | 2 (1.5%) |
| Immune-Mediated Myocarditis | 1 (1.5%) | - | 1 (0.7%) |
| Immune-Mediated Nephritis | - | 1 (1.5%) | 1 (0.7%) |
| Immune-Mediated Vasculitis | 1 (1.5%) | - | 1 (0.7%) |
| **Categories with Δ ≥10%** | | | |

Incidences of overall adverse events, grade ≥3 adverse events and serious adverse events was slightly increased in the tiragolumab and atezolizumab combination therapy compared with atezolizumab monotherapy (Tables 13 and 14).

**Table 13. Highest Grade Adverse Events**

| **Highest grade** | **Placebo + Atezolizumab** (*n* = 68) | **Tiragolumab + Atezolizumab** (*n* = 67) | **All patients** (*n* = 135) |
|---|---|---|---|
| 1 | 12 (17.6%) | 6 (9%) | 18 (13.3%) |
| 2 | 23 (33.8%) | 32 (47.8%) | 55 (40.7%) |
| 3 | 21 (30.9%) | 20 (29.9%) | 41 (30.4%) |
| 4 | 4 (5.9%) | 6 (9%) | 10 (7.4%) |
| 5 | 5 (7.4%) | 2 (3%) | 7 (5.2%) |

**Table 14. Grade ≥3 AEs in ≥2 patients**

| **Adverse Event** | **Placebo + Atezolizumab** (*n* = 68) | **Tiragolumab + Atezolizumab** (*n* = 67) | **All patients** (*n* = 135) |
|---|---|---|---|
| PNEUMONIA | 3 (4.4%) | 6 (9%) | 9 (6.7%) |
| LIPASE INCREASED | 2 (2.9%) | 4 (6%) | 6 (4.4%) |
| PLEURAL EFFUSION | 1 (1.5%) | 4 (6%) | 5 (3.7%) |
| ANAEMIA | 1 (1.5%) | 2 (3%) | 3 (2.2%) |
| HYPONATRAEMIA | 2 (2.9%) | 1 (1.5%) | 3 (2.2%) |

(continued)

| **Adverse Event** | **Placebo + Atezolizumab** (*n* = 68) | **Tiragolumab + Atezolizumab** (*n* = 67) | **All patients** (*n* = 135) |
|---|---|---|---|
| ABDOMINAL PAIN | 1 (1.5%) | 1 (1.5%) | 2 (1.5%) |
| ACUTE KIDNEY INJURY | 1 (1.5%) | 1 (1.5%) | 2 (1.5%) |
| ALANINE AMINOTRANSFERASE INCREASED | 1 (1.5%) | 1 (1.5%) | 2 (1.5%) |
| AMYLASE INCREASED | 2 (2.9%) | - | 2 (1.5%) |
| ASPARTATE AMINOTRANSFERASE INCREASED | 1 (1.5%) | 1 (1.5%) | 2 (1.5%) |
| ASTHENIA | 2 (2.9%) | - | 2 (1.5%) |
| BLOOD CREATININE INCREASED | 1 (1.5%) | 1 (1.5%) | 2 (1.5%) |
| DIABETES MELLITUS | 1 (1.5%) | 1 (1.5%) | 2 (1.5%) |
| HYPERTENSION | 1 (1.5%) | 1 (1.5%) | 2 (1.5%) |
| HYPOKALAEMIA | | 2 (3%) | 2 (1.5%) |
| MUSCULOSKELETAL PAIN | 1 (1.5%) | 1 (1.5%) | 2 (1.5%) |
| PULMONARY EMBOLISM | 2 (2.9%) | - | 2 (1.5%) |

Serious adverse events, including those that resulted in death, were less frequently observed and were comparable between the two treatment arms (Tables 15 and 16).

**Table 15. Serious Adverse Events in ≥ 2 Patients**

| **Adverse Event** | **Placebo + Atezolizumab** (*n* = 68) | **Tiragolumab + Atezolizumab** (*n* = 67) | **All patients** (*n* = 135) |
|---|---|---|---|
| PNEUMONIA | 4 (5.9%) | 5 (7.5%) | 9 (6.7%) |
| PLEURAL EFFUSION | - | 4 (6%) | 4 (3%) |
| DYSPNOEA | 1 (1.5%) | 1 (1.5%) | 2 (1.5%) |
| INFLUENZA | - | 2 (3%) | 2 (1.5%) |
| PULMONARY EMBOLISM | 2 (2.9%) | - | 2 (1.5%) |
| PYREXIA | 1 (1.5%) | 1 (1.5%) | 2 (1.5%) |

**Table 16. Serious Adverse Events Resulting in Death**

| **Adverse Event** | **Placebo + Atezolizumab** (*n* = 68) | **Tiragolumab + Atezolizumab** (*n* = 67) | **All patients** (*n* = 135) |
|---|---|---|---|
| Total number of patients with at least one adverse event | 5 (7.4%) | 2 (3%) | 7 (5.2%) |
| | | | |
| CARDIO-RESPIRATORY ARREST | 1 (1.5%) | - | 1 (0.7%) |
| CEREBROVASCULAR ACCIDENT | 1 (1.5%) | - | 1 (0.7%) |
| *EPSTEIN-BARR VIRUS INFECTION* | - | 1 (1.5%) | 1 (0.7%) |

(continued)

| Adverse Event | **Placebo + Atezolizumab** (*n* = 68) | **Tiragolumab + Atezolizumab** (*n* = 67) | **All patients** (*n* = 135) |
|---|---|---|---|
| MULTIPLE ORGAN DYSFUNCTION SYNDROME | 1 (1.5%) | - | 1 (0.7%) |
| PNEUMONIA | 1 (1.5%) | - | 1 (0.7%) |
| PULMONARY EMBOLISM | 1 (1.5%) | - | 1 (0.7%) |
| *PYREXIA* | - | 1 (1.5%) | 1 (0.7%) |
| *Investigator assessed as related to tiragolumab/placebo and atezolizumab* | | | |

Overall, the tolerability and toxicities of the tiragolumab and atezolizumab combination therapy were within acceptable ranges and comparable to that of the atezolizumab monotherapy.

**Example 2. A Phase III, randomized, double bind, placebo-controlled study of tiragolumab, and anti-TIGIT antibody, in combination with atezolizumab compared with placebo in combination with atezolizumab in patients with previously untreated locally advanced unresectable or metastatic PD-L1-selected non-small cell lung cancer.**

In this study, patients are selected on the basis of the PD-L1 status of their tumor, with a TPS ≥ 50%, as assessed by central testing using the PD-L1 IHC 22C3 pharmDx assay required for enrollment. All patients in this study receive the PD-L1 inhibitor atezolizumab. Efficacy and safety of tiragolumab, an anti-TIGIT antibody, plus atezolizumab is evaluated, compared with placebo plus atezolizumab, in patients with previously untreated locally advanced, unresectable or metastatic PD-L1-selected NSCLC, with no *EGFR* mutation or *ALK* translocation. The primary population is defined as all randomized patients who are selected on the basis of a minimum level of PD-L1 expression (TPS ≥ 50%) by central testing using the PD-L1 IHC 22C3 pharmDx assay.

**Study Design**

The study design is shown in FIG. 16. Previously untreated male and female patients age ≥ 18 years with ECOG Performance Status of 0 or 1 who have locally advanced, unresectable, or metastatic PD-L1-selected NSCLC are eligible. After providing informed consent, patients undergo screening procedures, during which tumor specimens from each potentially eligible patient are prospectively tested for PD-L1 expression by a central laboratory using the PD-L1 IHC 22C3 pharmDx assay. Only patients who are PD-L1 positive with a TPS > 50% assessed centrally are enrolled.

Patients whose tumors have a known *EGFR* mutation or ALK rearrangement are excluded from this study. Patients with tumors of non-squamous histology with unknown *EGFR* or ALK mutational status are required to be tested prior to enrollment. Patients with tumors of squamous histology who have an unknown *EGFR* or ALK mutational status are not required to be tested. Eligible patients are in a randomized 1:1 ratio to receive either tiragolumab plus atezolizumab or placebo plus atezolizumab. Eligible patients are stratified by ECOG Performance Status (0 vs. 1), tumor histology (non-squamous vs. squamous), and geographic region of the enrolling site (Asia vs. non-Asia).

In the experimental arm, patients receive atezolizumab at a fixed dose of 1200 mg administered by IV infusion Q3W on Day 1 of each 21-day cycle, followed by tiragolumab at a fixed dose of 600 mg administered to patients by IV infusion Q3W on Day 1 of each 21-day cycle.

In the control arm, patients receive atezolizumab at a fixed dose of 1200 mg administered by IV infusion Q3W on Day 1 of each 21-day cycle, followed by placebo administered by IV infusion Q3W on Day 1 of each 21-day cycle.

Treatment is continued as long as patients are experiencing clinical benefit, as assessed by the investigator, in the absence of unacceptable toxicity or symptomatic deterioration attributed to disease progression after an integrated assessment of radiographic data, biopsy results (if available), and clinical status. Patients who meet the criteria for disease progression per RECIST v1.1 are permitted to continue treatment (tiragolumab plus atezolizumab or placebo plus atezolizumab) if they exhibit evidence of clinical benefit, as assessed by the investigator; absence of symptoms and signs (including worsening of laboratory values (e.g., new or worsening hypercalcemia) indicating unequivocal progression of disease; no decline in ECOG Performance Status that can be attributed to disease progression; and absence of tumor progression at critical anatomical sites (e.g., leptomeningeal disease) that cannot be managed by protocol-allowed medical interventions.

Patients undergo tumor assessments at baseline and every 6 weeks (± 7 days) for 48 weeks following Day 1 of Cycle 1. After completion of the Week 48 tumor assessment, tumor assessment occurs every 9 weeks (± 7 days) until radiographic disease progression per RECIST v1.1, withdrawal of consent, death, or study termination, whichever occurs first. Patients who are treated beyond disease progression per RECIST v1.1 undergo tumor assessments at the frequency described above until study treatment is discontinued. Patients who discontinue treatment for reasons other than radiographic disease progression per RECIST v1.1 (e.g., toxicity, symptomatic deterioration) continue scheduled tumor assessments at the frequency described above until radiographic disease progression per RECIST v1.1, withdrawal of consent, death, or study termination, whichever occurs first. In the absence of radiographic disease progression per RECIST v1.1, tumor assessments can continue (e.g., regardless of whether a patient starts a new anti-cancer therapy).

Response is assessed according to RECIST v1.1 and modified RECIST v1.1 for immune-based therapeutics (iRECIST). Objective response at a single timepoint is determined by the investigator according to RECIST v1.1. Objective response per iRECIST is calculated programmatically on the basis of investigator assessments of individual lesions at each specified timepoint.

In order not to confound the OS endpoint, crossover from the control arm (placebo plus atezolizumab) to the experimental arm (tiragolumab plus atezolizumab) does not occur.

During the study, serum samples are collected to monitor tiragolumab and atezolizumab pharmacokinetics and to detect the presence of antibodies to tiragolumab and atezolizumab. Patient samples, including archival and fresh tumor tissue, serum, plasma, and blood samples, are collected for further assessment (e.g., safety assessment, e.g., incidence, nature, and severity of adverse events, protocol-mandated vital signs, and laboratory abnormalities).

During the study, patients are asked to complete a patient reported outcome (PRO) survey at the beginning of the study when visits for tumor assessments are scheduled, at treatment discontinuation, and survival follow-up at 3 and 6 months.

## Dosing

Atezolizumab is administered to all patients at a fixed dose of 1200 mg IV Q3W on Day 1 of each 21-day cycle, which is an approved dosage for atezolizumab,.

Tiragolumab is administered to all patients in the experimental arm at a fixed dose of 600 mg IV Q3W on Day 1 of each 21-day cycle. In the Phase II study described in Example 1, 67 patients (in the tiragolumab plus atezolizumab arm) received 600 mg tiragolumab. At this dose, tiragolumab was well tolerated and the combination of tiragolumab plus atezolizumab resulted in a clinically meaningful improvement in PFS and a higher ORR compared to placebo plus atezolizumab. Given the favorable benefit-risk ratio observed at 600 mg, this same dose of tiragolumab is used for this study.

Atezolizumab and tiragolumab/placebo are administered per the instructions outlined in Table 17, below.

**Table 17: Administration of First and Subsequent Infusions of Atezolizumab and Tiragolumab/Placebo**

| | First infusion | Subsequent infusion |
|---|---|---|
| **Atezolizumab infusion** | • No premedication is administered for the first infusion of atezolizumab.<br>•Vital signs (pulse rate, respiratory rate, blood pressure, and temperature) are recorded within 60 minutes prior to starting the infusion of atezolizumab.<br>• Atezolizumab is infused over 60 (± 15) minutes.<br>• Vital signs (pulse rate, respiratory rate, blood pressure, and temperature) are recorded during the infusion of atezolizumab at 15, 30, 45, and 60 minutes (± 5 minute windows are allowed for all timepoints). | If the patient experienced an IRR during any previous infusion of atezolizumab, premedication with an antihistamine and/or antipyretic may be administered for Cycle > 2 and beyond at the discretion of the treating physician.<br>• Vital signs (pulse rate, respiratory rate, blood pressure, and temperature) are recorded within 30 minutes before starting the infusion of atezolizumab.<br>• If the patient tolerated the first infusion of atezolizumab well without infusion-associated adverse events, the next infusion of atezolizumab may be infused over 30 (± 10) minutes. |

(continued)

| | First infusion | Subsequent infusion |
|---|---|---|
| | | • If no reaction occurs, subsequent infusions of atezolizumab continue over 30 (± 10) minutes.<br>• Vital signs continue to be recorded within 30 minutes before starting the infusion of atezolizumab.<br>• Vital signs are recorded during the infusion of atezolizumab if clinically indicated. |
| **Observation period after infusion of atezolizumab** | • After the infusion of atezolizumab, the patient begins a 60-minute observation period.<br>• Vital signs (pulse rate, respiratory rate, blood pressure, and temperature) are recorded at 30 (± 10) minutes after the infusion of atezolizumab. | • If the patient tolerated the first or a subsequent infusion of atezolizumab (without premedication) well without infusion-associated adverse events, the observation period after the next and following infusions may be reduced to 30 minutes.<br>• If the patient experienced infusion-associated adverse events in the previous infusion, the observation period is 60 minutes.<br>• If clinically indicated, vital signs (pulse rate, respiratory rate, blood pressure, and temperature) are recorded at 15 (± 10) minutes after the infusion of atezolizumab. |
| **Tiragolumab/ placebo infusion** | • No premedication is administsered for the first infusion of tiragolumab/placebo.<br>• Vital signs (pulse rate, respiratory rate, blood pressure, and temperature) are recorded within 60 minutes before starting the infusion of tiragolumab/placebo.<br>• Tiragolumab/placebo is infused over 60 (± 10) minutes.<br>• Vital signs (pulse rate, respiratory rate, blood pressure, and temperature) are recorded during the infusion of tiragolumab/ placebo at 15, 30, 45, and 60 minutes (±5-minute windows are allowed for all timepoints). | • If the patient experienced an IRR during any previous infusion of tiragolumab/placebo, premedication with an antihistamine and/or antipyretic may be administered for Cycles ≥ 2 and beyond at the discretion of the treating physician.<br>• Vital signs (pulse rate, respiratory rate, blood pressure, and temperature) are recorded within 60 minutes before starting the infusion of tiragolumab/placebo.<br>• If the patient tolerated the first or a subsequent infusion of tiragolumab/placebo (without premedication) well without infusion-associated adverse events, the next infusion of tiragolumab/placebo is infused over 30 (± 10) minutes.<br>• If no reaction occurs, subsequent infusions of tiragolumab/placebo continue over 30 (± 10) minutes.<br>• Vital signs continue to be recorded within 60 minutes before starting infusion of tiragolumab/ placebo.<br>• Vital signs are recorded during and after the infusion if clinically indicated. |

(continued)

| | First infusion | Subsequent infusion |
|---|---|---|
| **Observation period after infusion of tiragolumab/ placebo** | • After the infusion of tiragolumab/placebo, the patient begins a 60-minute observation period.<br>• Vital signs (pulse rate, respiratory rate, blood pressure, and temperature) are recorded at 30 (± 10) minutes after the infusion of tiragolumab/placebo.<br>• Patients are informed about the possibility of delayed post-infusion symptoms and will be instructed to contact their study physician if they develop such symptoms. | If the patient tolerated the previous infusion of tiragolumab/placebo well without infusion-associated adverse events, the observation period is reduced to 30 minutes.<br>• If clinically indicated, vital signs (pulse rate, respiratory rate, blood pressure, and temperature) are recorded at 15 (± 10) minutes after the infusion of tiragolumab/placebo.<br>• Patients are informed about the possibility of delayed post-infusion symptoms and will be instructed to contact their study physician if they develop such symptoms. |

Treatment cycles begin with dosing of atezolizumab and tiragolumab/placebo on Day 1 of each 21-day cycle. If either study drug is delayed for a related toxicity, the other study drug is also delayed; however, a cycle may begin with the administration of the other study drug if considered appropriate at the discretion of the investigator.

In case of delays in dosing of one study drug for drug-related toxicity while the other study drug is given as planned, the study drug being delayed is administered at the next scheduled infusion (i.e., at the next scheduled 21-day cycle).

## Assessment

Tumor and Response Evaluations

Screening and subsequent tumor assessments include CT scans (with oral or IV contrast unless contraindicated). A CT scan of the pelvis is required at screening and as clinically indicated or as per local standard of care at subsequent response evaluations. Magnetic resonance imaging (MRI) scans with contrast of the chest, abdomen, and pelvis with a non-contrast CT scan of the chest may be used in patients for whom CT scans with contrast are contraindicated (i.e., patients with contrast allergy or impaired renal clearance).

Further investigations such as bone scans and CT scans of the neck are also performed if clinically indicated. At the investigator's discretion, other methods of assessment of measurable disease according to RECIST v1.1 are used.

Tumor assessments performed as standard of care prior to obtaining informed consent and within 28 days of Day 1 of Cycle 1 may be used rather than repeating tests. Known sites of disease, including measurable and/or non-measurable disease, are documented at screening and re-assessed at each subsequent tumor evaluation. At subsequent (post-screening) tumor assessments, patients with a history of irradiated brain metastases at screening are not required to undergo brain scans unless clinically indicated (e.g., in patients with neurologic symptoms). The same radiographic procedure used to assess disease sites at screening should be used throughout the study (e.g., the same contrast protocol for CT scans).

Patients undergo tumor assessments at baseline and at every 6 weeks (± 7 days) for 48 weeks following Day 1 of Cycle 1, regardless of treatment delays. After the completion of the Week 48 tumor assessment, tumor assessments are conducted every 9 weeks (± 7 days) regardless of treatment delays, until radiographic disease progression per RECIST v1.1 (or loss of clinical benefit for patients who continue study treatment after disease progression per RECIST v1.1), withdrawal of consent, death, or study termination by the Sponsor, whichever occurs first. At the investigator's discretion, scans are performed at any time if progressive disease or loss of clinical benefit is suspected.

Response is assessed by the investigator on the imaging modalities detailed above, using RECIST v1.1. The investigator's assessment of overall tumor response at all timepoints is based on RECIST v1.1. Results are reviewed by the investigator before dosing at the next cycle.

Study treatment is continued as long as patients are experiencing clinical benefit as assessed by the investigator in the absence of unacceptable toxicity or symptomatic deterioration attributed to disease progression after an integrated assessment of radiographic data, biopsy results (if available), and clinical status. Patients who meet criteria for disease progression per RECIST v1.1 can be permitted to continue treatment (tiragolumab plus atezolizumab or placebo plus atezolizumab).

Patients who discontinue treatment for reasons other than radiographic disease progression per RECIST v1.1 (e.g., toxicity, symptomatic deterioration) continue scheduled tumor assessments until radiographic disease progression per RECIST v1.1, withdrawal of consent, death, or study termination by Sponsor, whichever occurs first. Patients who start a new anti-cancer therapy in the absence of radiographic disease progression per RECIST v1.1 continue tumor assessments until radiographic disease progression per RECIST v1.1, withdrawal of consent, death, or study termination by the Sponsor, whichever occurs first.

Investigator assessment of overall tumor response at all timepoints is based on RECIST v1.1. The overall tumor assessment is derived per iRECIST based on entries for all target lesions, non-target lesions, and new lesions. To facilitate evaluation of response per iRECIST, tumor assessments are continued after disease progression per RECIST v1.1 for patients who receive study treatment beyond progression. This includes continued measurement of target lesions, evaluation of non-target lesions (including monitoring for further worsening of any non-target lesions that have shown unequivocal progression), and evaluation of any newly identified lesions (including measurements, if lesions are measurable) at all subsequent assessments.

Archival or fresh tissue tumor samples can be analyzed for expression of PD-L1. Archival tumor tissue samples obtained outside of this study for central assessment of PD-L1 results are collected from all patients (paraffin blocks are preferred; or at least 15-20 unstained serial slides are acceptable). The availability of archival tumor tissue is confirmed prior to study entry. Patients who do not have tissue specimens meeting eligibility requirements may undergo a biopsy during the screening period. Acceptable samples include core-needle biopsies for deep tumor tissue (minimum three cores) or excisional, incisional, punch, or forceps biopsies for cutaneous, subcutaneous, or mucosal lesions. Patients having additional tissue samples from procedures performed at different times during this study are requested (but not required) to also submit these samples for central testing.

For patients with non-squamous NSCLC, if *EGFR* and/or ALK status is unknown, these are assessed locally or at a central laboratory. If samples are submitted for central testing, an additional five unstained slides must be provided (see additional details in the laboratory manual). For patients with a confirmed, prolonged CR and/or PR (e.g., of approximately 1 year in duration) who have an accessible residual mass, a biopsy of that residual mass is can be conducted to assess for viable TCs (vs. fibrotic or necrotic tissue). Optional biopsies consist of core-needle biopsies for deep tumor tissue or organs, or excisional, incisional, punch, or forceps biopsies for cutaneous, subcutaneous, or mucosal lesions. Optional biopsy samples of enrolled patients are evaluated for biomarkers using characterized assays for analysis of proteins, RNA, and DNA.

<u>Laboratory, Biomarker, and Other Biological Samples</u>

Samples for the following laboratory tests are sent to the study site's local laboratory for analysis:

- Hematology: WBC count, RBC count, hemoglobin, hematocrit, platelet count, and differential count (neutrophils, eosinophils, basophils, monocytes, and lymphocytes).
- Chemistry panel (serum or plasma): bicarbonate or total carbon dioxide (if considered standard of care for the region), sodium, potassium, magnesium, chloride, glucose, BUN or urea, creatinine, total protein, albumin, phosphate, calcium, total bilirubin, ALP, ALT, AST, and lactate dehydrogenase.
- Coagulation: INR or aPTT.
- Thyroid function testing: thyroid-stimulating hormone, free triiodothyronine (T3) (or total T3 at sites free T3 is not performed), and free thyroxine (also known as T4).
- EBV serology (EBV IgM, EBV IgG, and/or Epstein-Barr nuclear antigen) and/or EBV PCR. If a patient has positive serology for EBV IgG and/or Epstein-Barr nuclear antigen, then EBV IgM testing and/or EBV PCR is conducted prior to randomization for consideration of eligibility.
- HIV serology.
- HBV serology: HBsAg, total HBcAb, and (if HBsAg test is negative and total HBcAb test is positive) HBV DNA. If a patient has a negative HBsAg test and a positive total HBcAb test at screening, an HBV DNA test is also performed to determine if the patient has an HBV infection.
- HCV serology: HCV antibody and (if HCV antibody test is positive) HCV RNA. If a patient has a positive HCV antibody test at screening, an HCV RNA test is also performed to determine if the patient has an HCV infection.
- Pregnancy test. All women of childbearing potential have a serum pregnancy test at screening. During the study, urine pregnancy tests are performed on Day 1 of every cycle. If a urine pregnancy test is positive, it must be confirmed by a serum pregnancy test. A woman is considered to be of childbearing potential if she is post-menarcheal, has not reached a postmenopausal state (≥ 12 continuous months of amenorrhea with no identified cause other than menopause), and is not permanently infertile due to surgery (i.e., removal of ovaries, fallopian tubes, and/or uterus) or another cause (e.g., Mullerian agenesis).
- Urinalysis: pH, specific gravity, glucose, protein, ketones, and blood.

One or more of the following assessments can be performed on the blood samples:

- PK assays. Serum samples are obtained for measurement of tiragolumab or atezolizumab concentrations using validated immunoassays.
- ADA assays. Serum samples are obtained for measurement of ADAs to tiragolumab or to atezolizumab using validated assays.
- Exploratory biomarker assays. Plasma and serum samples are obtained for biomarker evaluation from all eligible patients.
- Auto-antibody assays. Serum samples collected for the assessment of PK, ADAs, or biomarkers at baseline on Day 1 of Cycle 1 prior to the first dose of study treatment, may be used for auto-antibody testing if an immune-mediated adverse event develops in a patient that would warrant such an assessment.
- WGS. A single blood sample is collected for WGS and may be sent to one or more laboratories for analysis. WGS data can be analyzed in the context of this study and explored in aggregate with other studies to better understand disease pathobiology and adverse events and guide the development of new therapeutic approaches.

Patient Reported Outcomes

Patent Reported Outcomes (PRO) data are collected to document the treatment benefit and more fully characterize the clinical profile of tiragolumab + atezolizumab. PRO data are collected using the following instruments: EORTC QLQ-C30, single-item EORTC IL46, select items from the PRO-CTCAE, and the EQ-5D-5L.

The EORTC QLQ-C30 is a validated, reliable self-reported measure (Aaronson et al., J. Natl. Cancer Inst. 1993, 85:365-76; Fitzsimmons et al., Eur. J. Cancer 1999, 35:939-41). It consists of 30 questions that assess five aspects of patient functioning (physical, emotional, role, cognitive, and social), three symptom scales (fatigue, nausea and vomiting, and pain), GHS and QoL, and six single items (dyspnea, insomnia, appetite loss, constipation, diarrhea, and financial difficulties) with a recall period of the previous week. Scale scores can be obtained for the multi-item scales. The functioning and symptoms items are scored on a 4-point scale that ranges from "not at all" to "very much," and the GHS and QoL items are scored on a 7-point scale that ranges from "very poor" to "excellent." The EORTC QLQ-C30 module takes approximately 10 minutes to complete.

The PRO-CTCAE is a validated item bank that is used to characterize the presence, frequency of occurrence, severity, and/or degree of interference with daily function of 78 patient-reportable symptomatic treatment toxicities (Basch et al., J. Natl Cancer Inst. 2014, 106:1-11; Dueck et al., JAMA Oncol. 2015, 1:1051-52). The PRO-CTCAE contains 124 questions that are rated either dichotomously (for determination of presence vs. absence) or on a 5-point Likert scale (for determination of frequency of occurrence, severity, and interference with daily function).

Treatment toxicities can occur with observable signs (e.g., vomiting) or non-observable symptoms (e.g., nausea). The standard PRO-CTCAE recall period is the previous 7 days. A subset of three symptoms that were deemed most applicable to the current treatments were selected for this study. The PRO-CTCAE takes approximately 10 minutes to complete.

The EQ-5D-5L is a validated self-reported health status questionnaire that is used to calculate a health status utility score for use in health economic analyses. There are two components to the EQ-5D-5L: a five-item health state profile that assesses mobility, self-care, usual activities, pain/discomfort, and anxiety/depression, as well as visual analog scale that measures health state. The EQ-5D-5L is designed to capture the patient's current health status. Published weighting systems allow for creation of a single composite score of the patient's health status. The EQ-5D-5L takes approximately 3 minutes to complete. It will be used in this study for informing pharmacoeconomic evaluations.

**Endpoints and Analysis**

The analysis population for the efficacy analyses will consist of all randomized patients, with patients grouped according to their assigned treatment. The co-primary efficacy endpoints are PFS, as assessed by the investigator according to RECIST v1.1, and OS.

PFS and OS are compared between treatment arms with use of the stratified log-rank test. The HR for PFS and OS are estimated using a stratified Cox proportional hazards model. The 95% CI for the HR is provided. The stratification factors are the same as the randomization stratification factors: ECOG Performance Status (0 vs. 1), tumor histology (non-squamous vs. squamous), and geographic region of the enrolling site (Asia vs. non-Asia). Stratification factor(s) are removed from the stratified analyses if there is risk of overstratification. Analyses based on stratification factors recorded on the eCRF is also provided if considerable discrepancy is observed between IxRS records and eCRFs.

Kaplan-Meier methodology is used to estimate the median PFS and median OS for each treatment arm, and Kaplan-Meier curve is constructed to provide a visual description of the difference between treatment arms. The Brookmeyer-Crowley methodology is used to construct the 95% CI for the median PFS and median OS for each treatment arm.

Progression-free survival

The primary analysis of the co-primary endpoint of PFS occurs when approximately 337 PFS events (67% of 500 patients) have been observed in the ITT population. This provides a 92% power to detect a target HR of 0.59 for PFS at a two-sided significance level of 0.001 based on the following assumptions: (i) PFS curve follows the exponential distributions; (ii) Median PFS of 7.1 months in the placebo plus atezolizumab arm and 12 months in the tiragolumab plus atezolizumab arm (corresponding to a target HR of 0.59); (iii) the dropout rate is 5% over 12 months for PFS; and (iv) no interim analysis for PFS. An observed HR of 0.699 or better for PFS results in a statistically significant difference between the treatment arms. That is, an HR of 0.699 is the minimally detectable difference for this analysis; this corresponds to an improvement of 3.1 months in median PFS from 7.1 months in the placebo plus atezolizumab arm to 10.2 months in the tiragolumab plus atezolizumab arm. The primary analysis of PFS occurs at approximately 30 months after the first patient is randomized with the additional assumptions on accrual over a period of 22 months.

Overall Survival

The final analysis of the co-primary endpoint of OS occurs when approximately 293 deaths (58% of 500) have been observed in the ITT population. This provides an 85% power to detect a target HR of 0.70 for OS at a two-sided significance level of 0.049 based on the following assumptions: (i) OS curve follows the exponential distributions; (ii) median OS of 21 months in the placebo plus atezolizumab arm and 30 months in the tiragolumab plus atezolizumab arm (corresponding to a target HR of 0.70); (iii) the dropout rate is 5% over 24 months for OS; (iv) two planned interim analyses for OS at approximately 63% and 84% of the information fraction, with the interim boundary for statistical significance determined on the basis of the Lan-DeMets approximation of the O'Brien-Fleming function. An observed HR of 0.786 or better for OS results in a statistically significant difference between the treatment arms. That is, an HR of 0.786 is the minimally detectable difference for the analysis; this corresponds to an improvement of 5.7 months in median OS from 21 months in the placebo plus atezolizumab arm to 26.7 months in the tiragolumab plus atezolizumab arm). The timing of the two interim analyses and the final analysis for OS are summarized in the Table 18 with the additional assumption on accrual.

**Table 18: Analysis timing for overall survival**

| | Analysis Timing | | ITT Population | |
|---|---|---|---|---|
| Type of Analysis | Months from FPI | Percentage Information | No. of Events (Event Patient Ratio) | Power, %[a] |
| OS first IA | 30 | 63% | 184 (37%) | 42 |
| OS second IA | 38 | 84% | 245 (49%) | 72 |
| OS final analysis | 47 | 100% | 292 (58%) | 85 |

FPI = first patient in; IA = interim analysis; ITT = intent-to-treat; OS = overall survival
[a] Power is calculated using two-sided $\alpha$ of 0.049

The first OS interim analysis is conducted at the time of the final PFS analysis. It is expected that there will be approximately 184 deaths in the ITT population at this timepoint, in which case the interim OS analysis will be conducted with the stopping boundaries for the OS interim and final analyses computed using the Lan-DeMets approximation to the O'Brien-Fleming function based on the actual observed events. If there are significantly fewer than 184 deaths at the time of PFS final analysis, a nominal $\alpha$ of 0.001% (negligible impact on the overall type I error rate) is spent on the OS analysis at the time of the PFS final analysis. The next interim analysis will be conducted after approximately 184 deaths have been observed, with the stopping boundaries for the OS interim and final analyses computed the same way as above.

A total of three analyses (two interim analyses and one final analysis) are conducted for the co-primary endpoint of OS. The timing of these two interim analyses and the final analysis for OS for which the prespecified boundaries for OS of all different scenarios are presented below in Table 19. The boundary for statistical significance at each interim analysis and the final analysis will be determined on the basis of the Lan-DeMets approximation of the O'Brien-Fleming function.

**Table 19: Stopping Boundaries for Overall Survival**

| Analysis | Stopping Boundary: HR (p-value [a]) | | |
|---|---|---|---|
| | Number of Events | If $\alpha$ = 0.049 | If $\alpha$ = 0.05 |
| OS first IA | 184 | HR ≤ 0.680 (p ≤ 0.0091) | HR ≤ 0.681 (p ≤ 0.0094) |
| OS second IA | 245 | HR ≤ 0.752 (p ≤ 0.0255) | HR ≤ 0.752 (p ≤ 0.0261) |
| OS final analysis | 292 | HR ≤ 0.786 (p ≤ 0.0401) | HR ≤ 0.787 (p ≤ 0.0409) |
| HR = hazard ratio; IA = interim analysis; OS = overall survival<br>[a] The p values are two-sided | | | |

Overall Response Rate

An objective response is either a confirmed CR or PR, as determined by the investigator according to RECIST v1.1. Patients not meeting these criteria, including patients without any post-baseline tumor assessment, are considered non-responders.

ORR is the proportion of patients who achieve an objective response. ORR is analyzed in the randomized patients with measurable disease at baseline. An estimate of ORR and its 95% CI is calculated using the Clopper-Pearson method for each treatment arm. CIs for the difference in ORRs between the two treatment arms are determined using the normal approximation to the binomial distribution. The ORR is compared between the two treatment arms using the stratified Mantel-Haenszel test.

Duration of Response

DOR is assessed in patients who achieved an objective response, as determined by the investigator according to RECIST v1.1. DOR is the time interval from the date of the first occurrence of a confirmed CR or PR (whichever status is recorded first) until the first date that progressive disease or death is documented, whichever occurs first. Patients who have not progressed and who have not died at the time of analysis will be censored at the time of last tumor assessment date. If no tumor assessments are performed after the date of the first occurrence of a CR or PR, DOR is censored at the date of the first occurrence of a CR or PR. DOR is based on a non-randomized subset of patients (specifically, patients who achieve an objective response).

**Example 3: A Phase III, open-label, randomized study of atezolizumab and tiragolumab compared with durvalumab in patients with locally advanced, unresectable stage III non-small cell lung cancer who have not progressed after concurrent platinum-based chemoradiation**

This Phase III, open-label study evaluates the efficacy and safety of atezolizumab in combination with tiragolumab compared with durvalumab administered in patients with locally advanced, unresectable Stage III NSCLC who have not progressed following concurrent platinum-based CRT as consolidation therapy. The study design is shown in FIG. 17.

**Objectives and Endpoints**

This study evaluates the efficacy and safety of consolidation maintenance treatment consisting of atezolizumab and tiragolumab compared with durvalumab in patients with locally advanced, unresectable Stage III NSCLC who have received two cycles of concurrent platinum-based CRT and have not had radiographic disease progression.

The primary populations are defined as all randomized patients (the ITT population and patients whose tumor is PD-L1 positive [≥1% TC]).

In this example, study treatment refers to the combination of treatments assigned to patients as part of this study.

The primary efficacy objective for this study is to evaluate the efficacy of atezolizumab in combination with tiragolumab compared with durvalumab in the ITT and the PD-L1-positive populations on the basis of the following endpoint: IRF-assessed PFS after randomization, defined as the time from randomization to the first occurrence of disease progression, as determined by the IRF according to RECIST v1.1, or death from any cause, whichever occurs first.

The secondary efficacy objective for this study is to evaluate the efficacy of atezolizumab plus tiragolumab compared with durvalumab in the ITT and the PD-L1-positive populations on the basis of the following endpoints:

- OS after randomization, defined as the time from randomization to death from any cause
- Investigator-assessed PFS after randomization, defined as the time from randomization to the first occurrence of disease progression as determined by the investigator according to RECIST v1.1 or death from any cause, whichever occurs first.
- Confirmed ORR, defined as the proportion of patients with a confirmed objective response (i.e., CR or a PR on two consecutive occasions > 4 weeks apart), as determined by the IRF and investigator according to RECIST v1.1
- DOR in patients with confirmed ORR, defined as the time from the first occurrence of a documented objective response to disease progression as determined by the IRF and investigator according to RECIST v1.1 or death from any cause, whichever occurs first.
- PFS rate at 12 months, 18 months, and 24 months, defined as the proportion of patients who have not experienced disease progression as determined by the IRF and investigator according to RECIST v1.1 or death from any cause at 12 months, 18 months and 24 months, respectively.
- OS rate at 12 months, 24 months, 36 months, and 48 months, defined as the proportion of patients who have not died from any cause at 12 months, 24 months, 36 months, and 48 months, respectively.
- Time to death or distant metastasis (TTDM), defined as the time from the date of randomization until the first date of distant metastasis or death in the absence of distant metastasis.
- Time to confirmed deterioration (TTCD) in patient-reported physical functioning and global health status (GHS), as measured by the European Organisation for Research and Treatment of Cancer (EORTC) Quality of Life Questionnaire Core 30 (QLQ-C30), and in patient-reported lung cancer symptoms for cough, dyspnea (a multi-item subscale), and chest pain, as measured through the use of the EORTC Quality-of-Life Questionnaire Lung Cancer Module (QLQ-LC13)

The exploratory efficacy objective for this study is to evaluate the efficacy of tiragolumab plus atezolizumab compared with durvalumab on the basis of the following endpoints:

- Time to second disease progression (PFS2), defined as the time between the date of randomization to second documented disease progression following the first documented disease progression as assessed by the investigator according to RECIST v1.1, or death from any cause, whichever occurs first.

- Change from baseline in patient-reported outcomes (PROs) of health-related quality-of-life (HRQoL), lung-cancer related symptoms, and their impact on functioning, as assessed through the use of the EORTC QLQ-C30 and QLQ-LC13

**Study Design**

Male and female patients age >18 years old with ECOG Performance Status of 0 or 1 and known PD-L1 status with locally advanced, unresectable Stage III NSCLC who do not have disease progression following concurrent platinum-based CRT are eligible. After providing informed consent, patients will undergo screening procedures. Patients must meet all eligibility criteria for participation. Patients who do not meet the criteria for participation in this study (screen failure) cannot be re-screened. The investigator records reasons for screen failure in the screening log.
During pre-screening or screening, tumor specimens collected prior to the first dose of cCRT from each potentially eligible patient are tested for PD-L1 expression by a central laboratory using the investigational Ventana PD-L1 (SP263) CDx Assay. Patients whose tumors have a known *EGFR* mutation or ALK rearrangement are excluded from enrollment in this study. Patients with tumors of non-squamous histology with unknown *EGFR* or ALK mutational status are required to be tested prior to enrollment. Patients with tumors of squamous histology who have an unknown *EGFR* or ALK mutational status are not required to be tested.
Patients must have histologically or cytologically documented NSCLC who present with locally advanced, unresectable (Stage III) disease (according to 8th revised edition of the AJCC (Amin et al., AJCC cancer staging manual. 8th revised edition. New York: Springer, 2017)/UICC NSCLC staging system).
Randomization must be completed within 1 to 42 days after the final dose of RT. Eligible patients are randomized in a 1:1 ratio to receive either atezolizumab plus tiragolumab or durvalumab.
Eligible patients are stratified by ECOG Performance Status (0 vs. 1), PD-L1 expression, determined using the investigational Ventana PD-L1 (SP263) CDx Assay (TC < 1% vs.TC ≥ 1%), tumor histology (non-squamous vs. squamous), and staging (Stage IIIA vs. Stage IIIB or IIIC).
In the experimental arm, atezolizumab is administered to patients by IV infusion at a fixed dose of 1680 mg, followed by tiragolumab at a fixed dose of 840 mg administered by IV infusion on Day 1 of each 28-day cycle for a maximum of 13 cycles. FIG. 18.
In the comparator arm, patients will receive the approved durvalumab dose, 10 mg/kg Q2W, administered by IV infusion on Days 1 and 15 of each 28-day cycle for a maximum of 13 cycles (not to exceed 26 doses). FIG. 18.

Treatment may be continued for 13 cycles, as long as patients are experiencing clinical benefit, as assessed by the investigator, in the absence of unacceptable toxicity or symptomatic deterioration attributed to disease progression after an integrated assessment of radiographic data, biopsy results (if available), and clinical status. Patients will undergo tumor assessments at screening and every 8 weeks (+/- 7 days) for 48 weeks following Day 1 of Cycle 1 regardless of treatment delays. After completion of the Week 48 tumor assessment, tumor assessment is required every 12 weeks ( +/- 7 days) regardless of treatment delays until confirmed, investigator-assessed radiographic disease progression (as defined by growth of existing lesions, new lesions, or recurrence of previously resolved lesions) per RECIST v1.1, withdrawal of consent, or study termination, whichever occurs first. Patients who are treated beyond disease progression per RECIST v1.1 will undergo tumor assessments at the frequency described above. Patients who discontinue treatment for reasons other than radiographic disease progression per RECIST v1.1 (e.g., toxicity, symptomatic deterioration, completion of study treatment) will continue scheduled tumor assessments at the frequency described above until confirmed radiographic disease progression per RECIST v1.1, withdrawal of consent, death, or study termination, whichever occurs first. In the absence of radiographic disease progression confirmed by scan per RECIST v1.1, tumor assessments should continue regardless of whether a patient starts a new anti-cancer therapy.

If a tumor assessment shows radiographic disease progression (as defined by growth of existing lesions, new lesions, or recurrence of previously resolved lesions) per RECIST v1.1, a confirmatory scan should be performed no later than the next scheduled assessment, or earlier if clinically indicated. Administration of study treatment will continue between the initial assessment of progression and confirmation of radiographic disease progression.

If a tumor assessment shows disease progression, it is confirmed pathologically and/or by unequivocal radiographic evidence from the scan. If the scan shows equivocal findings (e.g., mediastinal nodes measure < 1.5 cm in the short axis, lung parenchymal lesions or visceral lesions measuring < 1 cm in the longest diameter), a biopsy is performed. If a biopsy is not feasible or safe, then confirmatory scans are performed no later than the next scheduled assessment, or earlier if clinically indicated. If a biopsy for disease progression confirmation is performed, any leftover biopsy tissue is strongly encouraged to be submitted for exploratory biomarker research (optional consent required for exploratory research). The biopsy is performed prior to starting the next anti-cancer therapy. If the biopsy does not show evidence of disease progression (e.g., non-malignant infiltrates), then the patient continues with scheduled study treatment, assessments, and/or follow-up. After patients who are assessed with confirmed radiographic disease progression per RECIST v1.1 and have discontinued or completed study treatment, they continue to undergo tumor assessments according to local standard of care.

Response is assessed according to RECIST v1.1 and modified RECIST v1.1 for immune-based therapeutics (iRECIST). Objective response at a single timepoint is determined by the investigator according to RECIST v1.1. Objective response per iRECIST is calculated programmatically on the basis of investigator assessments of individual lesions at each specified timepoint.

In order not to confound the OS endpoint, crossover is not allowed.

During the study, serum samples are collected to monitor tiragolumab and atezolizumab pharmacokinetics and to detect the presence of antibodies to tiragolumab and atezolizumab. Patient samples, including archival and fresh tumor tissue, serum, plasma, and blood samples, will also be collected for exploratory biomarker assessments.

Safety assessments include the incidence, nature, and severity of adverse events, protocol-mandated vital signs, laboratory abnormalities, and other protocol-specified tests that are deemed critical to the safety evaluation of the study. After initiation of study treatment, all adverse events are reported until 30 days after the final dose of study treatment or until initiation of another anti-cancer therapy, whichever occurs first, and serious adverse events will continue to be reported until 90 days after the final dose of study treatment or until initiation of a new systemic anticancer therapy, whichever occurs first. In addition, adverse events of special interest are reported until 90 days after the final dose of study treatment, regardless of initiation of new anti-cancer therapy. After this period, investigators report any deaths, serious adverse events, or adverse events of special interest that are believed to be related to prior treatment with study drug(s). The investigator follows each adverse event until the event has resolved to baseline grade or better, the event is assessed as stable by the investigator, the patient is lost to follow-up, or the patient withdraws consent. Every effort should be made to follow all serious adverse events considered to be related to study treatment or protocol-related procedures until a final outcome can be reported.

*Treatment after Disease Progression*

During the study, patients who meet criteria for disease progression per RECIST v1.1 and show evidence of clinical benefit continue study treatment for up to 13 cycles of treatment, at the investigator's discretion, provided that the patient meets all of the following criteria: evidence of clinical benefit, as assessed by the investigator; absence of symptoms and signs (including worsening of laboratory values (e.g., new or worsening hypercalcemia) indicating unequivocal progression of disease; no decline in ECOG Performance Status that can be attributed to disease progression;

absence of tumor progression at critical anatomical sites (e.g., leptomeningeal disease) that cannot be managed by protocol-allowed medical interventions

For patients who receive study treatment beyond radiographic disease progression (as defined by growth of existing lesions, new lesions, or recurrence of previously resolved lesions), new lesions are assessed according to iRECIST and applicable measurements entered on the electronic Case Report Form (eCRF). Investigator assessment of overall tumor response at all timepoints are based only on RECIST v1.1. Objective response per iRECIST is calculated programmatically on the basis of investigator assessments of individual lesions at each specified timepoint.

## Dosage and Administration

On Day 1 of each 28-day cycle, all eligible patients will receive study treatment by IV infusion in the following order:

- Experimental arm: atezolizumab 1680 mg IV→ tiragolumab 840 mg IV
- Comparator arm: durvalumab 10 mg/kg IV

On Day 15 of each 28-day cycle, patients in the comparator arm receive durvalumab 10 mg/kg IV.

On Day 1 of Cycle 1, premedication administered for atezolizumab and/or tiragolumab is not permitted. Administration of all study treatment (atezolizumab, tiragolumab, and durvalumab) is performed in a monitored setting where there is immediate access to trained personnel and adequate equipment and medicine to manage potentially serious reactions.

Tiragolumab (840 mg) and atezolizumab (1680 mg) are administered by IV infusion.

Durvalumab is administered at a dose of 10 mg/kg by IV infusion.

## Atezolizumab

Atezolizumab is supplied as a sterile liquid in a single-use, 14-mL glass vial. The vial contains approximately 14 mL (840 mg) of atezolizumab solution.

Patients in the experimental arm will receive 1680 mg atezolizumab at a fixed dose administered by IV infusion on Day 1 of each 28-day cycle. The atezolizumab dose is fixed and is not dependent on body weight.

Atezolizumab infusions are administered per the instructions outlined in Table 20. No dose modification for atezolizumab is allowed.

**Table 20.** Administration of First and Subsequent Infusions of Atezolizumab

| | Day 1, Cycle 1 Infusion | Day 1 Infusion of Subsequent Cycles |
|---|---|---|
| Atezolizumab infusion | No premedication is permitted prior to the atezolizumab infusion. Vital signs (pulse rate, respiratory rate, blood pressure, and temperature) should be recorded within 60 minutes prior to the infusion. | If the patient experienced an IRR with any previous infusion of atezolizumab, premedication with an antihistamine and/or antipyretic medication may be administered for subsequent doses at the discretion of the investigator. |
| | Atezolizumab should be infused over 60 (+/- 15) minutes. | infusion was tolerated without an IRR or 60 (+/- 15) minutes if the patient experienced an IRR with the previous infusion. |
| | If clinically indicated, vital signs should be recorded every 15 (+/- 5) minutes during the infusion. | If the patient experienced an IRR with the previous infusion, or if clinically indicated, vital signs should be recorded during the infusion. |

(continued)

| | Day 1, Cycle 1 Infusion | Day 1 Infusion of Subsequent Cycles |
|---|---|---|
| Observation period after atezolizumab infusion | After the infusion of atezolizumab, the patient begins a 60-minute observation period.<br>Vital signs should be recorded at 30 (+/- 10) minutes after the infusion of atezolizumab.<br><br>Patients are informed about the possibility of delayed postinfusion symptoms and instructed to contact their study physician if they develop such symptoms. | If the patient tolerated the previous atezolizumab infusion well without infusion-associated adverse events, the observation period after the next and following infusions may be reduced to 30 minutes.<br>If the patient experienced infusion associated adverse events in the previous infusion, the observation period should be 60 minutes.<br>If clinically indicated, vital signs should be recorded at 30 (+/- 10) minutes after the infusion of atezolizumab. |

**Tiragolumab**

Tiragolumab is supplied as a sterile liquid in a single-use, 15-mL glass vial. The vial contains approximately 10 mL (600 mg) of tiragolumab solution.

Following the administration of atezolizumab and an observation period (see Table 20), patients in the experimental arm will receive 840 mg tiragolumab at a fixed dose administered by IV infusion on Day 1 of each 28-day cycle. The tiragolumab dose is fixed and is not dependent on body weight.

Tiragolumab infusions are administered per the instructions outlined in Table 21.

**Table 21.** Administration of First and Subsequent Infusions of Tiragolumab

| | Day 1, Cycle 1 Infusion | Day 1 Infusion of Subsequent Cycles |
|---|---|---|
| Tiragolumab infusion | No premedication is permitted prior to the tiragolumab infusion. Vital signs (pulse rate, respiratory rate, blood pressure, and temperature) should be recorded within 60 minutes prior to the infusion. Tiragolumab should be infused over 60 (+/- 15) minutes.<br>If clinically indicated, vital signs should be recorded every 15 (+/- 5) minutes during the infusion. | If the patient experienced an IRR with any previous infusion of tiragolumab, premedication with an antihistamine and/or antipyretic medication may be administered for subsequent doses at the discretion of the investigator.<br>Vital signs should be recorded within 60 minutes prior to the infusion.<br><br>Tiragolumab should be infused over 30 (+/- 10) minutes if the previous infusion was tolerated without an IRR or 60 (+/- 15) minutes if the patient experienced an IRR with the previous infusion.<br>If the patient experienced an IRR with the previous infusion, or if clinically indicated, vital signs should be recorded during the infusion. |

(continued)

| | Day 1, Cycle 1 Infusion | Day 1 Infusion of Subsequent Cycles |
|---|---|---|
| Observation period after tiragolumab infusion | After the infusion of tiragolumab, the patient begins a 60-minute observation period.<br>Vital signs should be recorded at 30 (+/- 10) minutes after the infusion of tiragolumab.<br><br>Patients are informed about the possibility of delayed postinfusion symptoms and instructed to contact their study physician if they develop such symptoms. | If the patient tolerated the previous tiragolumab infusion well without infusion-associated adverse events, the observation period after the next and following infusions may be reduced to 30 minutes.<br>If the patient experienced infusion associated adverse events in the previous infusion, the observation period should be 60 minutes.<br>If clinically indicated, vital signs should be recorded at 30 (+/- 10) minutes after the infusion of tiragolumab.<br>Patients are informed about the possibility of delayed postinfusion symptoms and instructed to contact their study physician if they develop such symptoms. |

**Atezolizumab and Tiragolumab**

The following rules apply as long as neither atezolizumab nor tiragolumab has been permanently discontinued: Treatment cycles begin with dosing of atezolizumab followed by tiragolumab on Day 1 of each 28-day cycle in the experimental arm. If either study drug is delayed for a related toxicity, it is recommended that the other study drug is also delayed since the safety profiles for atezolizumab and tiragolumab are similar; however, a cycle may begin with the administration of the other study drug if considered appropriate at the discretion of the investigator.

In case of delays in dosing of one study drug for study drug-related toxicity while the other study drug is given as planned, it is recommended that the study drug being delayed is administered at the next scheduled infusion (i.e., at the next scheduled 28-day cycle, a maximum of 13 cycles can be given in total).

**Durvalumab**

Durvalumab is supplied to the sites in its commercially available formulation.

Patients in the comparator arm will receive 10 mg/kg durvalumab administered by IV infusion on Days 1 and 15 of each 28-day cycle. The dose of durvalumab is 10 mg/kg and is dependent on a patient’s body weight at baseline or on the respective dosing day. If a patient’s weight changes by ≥ 10% of the baseline weight, the dose must be re-calculated based on the weight change. No dose modification for durvalumab is allowed except for adjustment for body weight on dosing days. For further details on dose preparation, storage, administration, and treatment interruption or discontinuation instructions for durvalumab, refer to the pharmacy manual and/or the durvalumab prescribing information.

For every durvalumab infusion, vital signs (pulse rate, respiratory rate, blood pressure, and temperature) should be recorded within 60 minutes prior to the infusion. If clinically indicated, vital signs should be recorded every 15 (+/- 5) minutes during the infusion and at 30 (+/- 10) minutes after the infusion. Please refer to the durvalumab prescribing information for premedications for durvalumab infusion and management guidelines of IRRs associated with durvalumab.

***Inclusion Criteria***

Patients must meet the following criteria for study entry:

- Signed Informed Consent Form
- Age ≥ 18 years at time of signing Informed Consent Form
- Ability to comply with the study protocol, including willingness to remain in the post-treatment period
- ECOG Performance Status of 0 or 1
- Histologically or cytologically documented NSCLC with locally advanced, unresectable Stage III NSCLC of either squamous or non-squamous histology Staging should be based on the 8th revised edition of the AJCC (Amin et al., AJCC cancer staging manual. 8th revised edition. New York: Springer, 2017)/UICC NSCLC staging system).

  - Patients with tumors of mixed NSCLC histology must be classified as being non-squamous or squamous on the basis of the major histologic component.
- Patients with T4 primary NSCLC with a separate nodule in a different ipsilateral lobe are not eligible.
- Patients with tumors of mixed histology containing both NSCLC and small-cell lung cancer are not eligible for the study.

- Whole-body positron emission tomography (PET)-CT scan (from the base of skull to mid-thighs) for the purposes of staging, performed prior and within 42 days of the first dose of concurrent CRT
- At least two prior cycles of platinum-based chemotherapy concurrent with RT (cCRT), which must be completed within 1 to 42 days prior to randomization in the study. To ensure the best patient outcomes, sites are strongly encouraged to complete screening procedures within the first 14 days after the final dose of cCRT. The platinum-based chemotherapy regimen must contain one of the following agents: etoposide, a taxane (paclitaxel), pemetrexed, or vinorelbine. Concurrent chemotherapy must be given per the NCCN® (2019) and/or the European Society of Medical Oncology guidelines (Postmus et al., Ann. Oncol. 2017, 28(Suppl. 4):iv1-iv21). The final cycle of chemotherapy must end prior to or concurrently with the final dose of RT. Consolidation chemotherapy is not permitted, but administration of chemotherapy prior to concurrent CRT is acceptable (but not to exceed more than one cycle).
- The RT component in the cCRT must have been at a total dose of radiation of 60 ($\pm$ 10%) Gy (54 Gy to 66 Gy) administered by IMRT (preferred) or 3D-conforming technique. Sites are encouraged to adhere to mean organ radiation dosing as follows:

    - The mean dose of radiation in the lung must be < 20 Gy and/or V20 must be < 35%.
    - The mean dose of radiation in the esophagus must be < 34 Gy.
    - The mean dose of radiation in the heart must be V45 < 35% or V30 < 30%.

- No progression during or following concurrent platinum-based CRT
- Tumor PD-L1 expression, as determined by the investigational Ventana PD-L1 (SP263) CDx Assay and documented by means of central testing of a representative tumor tissue, in either a previously obtained archival tumor tissue or fresh tissue obtained from a biopsy prior to the first dose of cCRT Confirmed availability of representative formalin-fixed, paraffin-embedded (FFPE) tumor specimens in blocks (preferred) or at least 15-20 unstained serial slides, along with an associated pathology report. If central testing for EGFR mutations and/or ALK translocations are required, an additional 5 unstained slides must be provided. Tumor tissue should be of good quality based on total and viable tumor content (i.e., preserved cellular context and tissue architecture). Acceptable samples include core-needle biopsies for deep tumor tissue (with a minimum of three cores for freshly collected biopsies); excisional, incisional, punch, or forceps biopsies for cutaneous, subcutaneous, or mucosal lesions; or endobronchial ultrasound (EBUS) core-needle biopsy. Endobronchial ultrasound: transbronchial needle aspiration (EBUS-TBNA), which is sometimes referred to as a fine-needle aspiration, is acceptable (particularly if a larger-gauge needle is used) provided tissue is of good quality as described above (i.e., preserved cellular context and tissue architecture). For needle aspirations, an 18-gauge or larger needle is recommended. Fine-needle aspirations, brushings, cell pellets from pleural effusions, and lavage samples are not acceptable.
- Life expectancy $\geq$ 12 weeks
- Adequate hematologic and end-organ function, defined by the following laboratory test results, obtained within 14 days prior to initiation of study treatment (Day 1 of Cycle 1):

    - ANC > 1.5 $\times$ 109/L ($\geq$ 1500/$\mu$L) without granulocyte colony-stimulating factor support
    - Lymphocyte count > 0.5 $\times$ 109/L ($\geq$ 500/$\mu$L)
    - Platelet count > 100 $\times$ 109/L ($\geq$ 100,000/$\mu$L) without transfusion
    - Hemoglobin $\geq$ 90 g/L ($\geq$ 9 g/dL). Patients may be transfused or receive erythropoietic treatment as per local standard of care to meet this criterion.
    - AST, ALT, and ALP $\leq$ 2.5 $\times$ upper limit of normal (ULN), with the following exceptions: Patients with documented liver metastases: AST and ALT < 5 $\times$ ULN; and patients with documented liver or bone metastases: ALP < 5 $\times$ ULN
    - Bilirubin < 1.5 $\times$ ULN with the following exception:
      Patients with known Gilbert disease: bilirubin level < 3 $\times$ ULN
    - Creatinine < 1.5 $\times$ ULN. Creatinine clearance (CrCl) $\geq$ 50 mL/min, calculated using the Cockcroft-Gault formula (Cockcroft and Gault, Nephron 1976, 16:31-41) or by 24-hour urine collection for determination of CrCl:

      For males:

$$\text{CrCl (mL/min)} = \frac{\text{Weight (kg)} \times (140 - \text{Age})}{72 \times \text{serum creatinine (mg/dL)}}$$

For females:

$$\text{CrCl (mL/min)} = \frac{\text{Weight (kg)} \times (140 - \text{Age}) \times 85}{72 \times \text{serum creatinine (mg/dL)}}$$

$$\text{Albumin} \geq 25\ \text{g/L}\ (\geq 2.5\ \text{g/dL})$$

- For patients not receiving therapeutic anticoagulation: INR and aPTT < 1.5 × ULN
- For patients receiving therapeutic anticoagulation: stable anticoagulant regimen

- Negative HIV test at screening
- Negative hepatitis B surface antigen (HBsAg) test at screening
- Positive hepatitis B surface antibody (HBsAb) test at screening, or negative HBsAb at screening accompanied by either of the following:
  - Negative total hepatitis B core antibody (HBcAb)
  - Positive total HBcAb test followed by a negative (per local laboratory definition) hepatitis B virus (HBV) DNA test. The HBV DNA test is performed only for patients who have a negative HBsAg test, a negative HBsAb test, and a positive total HBcAb test.
- Negative hepatitis C virus (HCV) antibody test at screening, or positive HCV antibody test followed by a negative HCV RNA test at screening. The HCV RNA test is performed only for patients who have a positive HCV antibody test.
- For women of childbearing potential: agreement to remain abstinent (refrain from heterosexual intercourse) or use contraception, as defined herein: Women must remain abstinent or use contraceptive methods with a failure rate of < 1% per year during the treatment period, and for 90 days after the final dose of tiragolumab and 5 months after the final dose of atezolizumab, or for 3 months after the final dose of durvalumab. A woman is considered to be of childbearing potential if she is postmenarcheal, has not reached a postmenopausal state (≥ 12 continuous months of amenorrhea with no identified cause other than menopause), and is not permanently infertile due to surgery (i.e., removal of ovaries, fallopian tubes, and/or uterus) or another cause as determined by the investigator (e.g., Mullerian agenesis). The definition of childbearing potential may be adapted for alignment with local guidelines or regulations. Examples of contraceptive methods with a failure rate of < 1% per year include bilateral tubal ligation, male sterilization, hormonal contraceptives that inhibit ovulation, hormone-releasing intrauterine devices, and copper intrauterine devices. The reliability of sexual abstinence should be evaluated in relation to the duration of the clinical trial and the preferred and usual lifestyle of the patient. Periodic abstinence (e.g., calendar, ovulation, symptothermal, or postovulation methods) and withdrawal are not adequate methods of contraception. If required per local guidelines or regulations, locally recognized adequate methods of contraception and information about the reliability of abstinence is described in the local Informed Consent Form.
- For men: agreement to remain abstinent (refrain from heterosexual intercourse) or use a condom, and agreement to refrain from donating sperm, as defined herein: With a female partner of childbearing potential or pregnant female partner, men must remain abstinent or use a condom during the treatment period and for 90 days after the final dose of tiragolumab to avoid exposing the embryo. Men must refrain from donating sperm during this same period. The reliability of sexual abstinence should be evaluated in relation to the duration of the clinical trial and the preferred and usual lifestyle of the patient. Periodic abstinence (e.g., calendar, ovulation, symptothermal, or postovulation methods) and withdrawal are not adequate methods of preventing drug exposure. If required per local guidelines or regulations, information about the reliability of abstinence is described in the local Informed Consent Form.

***Safety***

Based on results from nonclinical and/or clinical studies with tiragolumab and atezolizumab each as a single agent, clinical data with tiragolumab plus atezolizumab, and data from molecules with similar mechanisms of action, there is a potential for overlapping toxicity in patients treated with tiragolumab plus atezolizumab. Because the expected pharmacologic activity of these two molecules is to increase adaptive TC immune responses, there is the possibility of heightened immune responses.

Based on the mechanism of action of tiragolumab and atezolizumab, immune-mediated adverse events are potential overlapping toxicities associated with combination use of tiragolumab plus atezolizumab.

Based on clinical experience to date, it is anticipated that immune-mediated adverse events following treatment with tiragolumab and atezolizumab are amenable to monitoring and manageable in the setting of this combination study. The extensive experience with immune CPIs to date has been incorporated into the design and safety management plan in order to reduce the potential risks to participating patients. Patients with a history of autoimmune disease are excluded from this study. Patients previously treated with approved or experimental cancer immunotherapy are excluded from participation in this study. Owing to the risks of active viral infection and viral reactivation, patients with active infection (including, but not limited to, HIV, HBV, HCV, EBV, known and/or suspected chronic active EBV infection, or tuberculosis) and/or patients with recent severe infections are excluded from this study.

*Infustion-Related Reactions:*

Because tiragolumab is a therapeutic mAb and targets ICs, IRRs associated with hypersensitivity reactions, target-mediated cytokine release, and/or emergent ADAs may occur. Clinical signs and symptoms of such reactions may include rigors, chills, wheezing, pruritus, flushing, rash, hypotension, hypoxemia, and fever. IRRs have been reported in patients treated with tiragolumab, with or without atezolizumab. The majority of events were mild to moderate and manageable.

To minimize the risk and sequelae of IRRs, the initial dose of tiragolumab is administered over 60 minutes followed by a 60-minute observation period. Subsequent infusions and observation times may be shortened if the preceding infusion was well tolerated. All infusions are administered in an appropriate medical setting.

*Immune-Mediated Adverse Events*

Nonclinical models have suggested a role of TIGIT signaling interruption in autoimmunity. In a knockout model (TIGIT-/-), loss of TIGIT signaling resulted in hyperproliferative T-cell responses and exacerbation of experimental autoimmune encephalitis (EAE). TIGIT-/- and wild-type B6 mice were immunized with myelin oligodendrocyte glycoprotein peptide in an EAE using suboptimal doses. In contrast to the wild-type B6 mice, the majority of the TIGIT-/- mice developed severe EAE (Joller et al., J. Immunol. 2011, 186:1338-42).

Clinical experience with therapeutic agents intended to enhance anti-tumor T-cell responses has demonstrated that development of autoimmune inflammatory conditions is a general risk and may therefore be considered a potential risk of tiragolumab. Such immune-mediated adverse events have been described for virtually all organ systems and include, but are not limited to, colitis, hepatitis, pneumonitis, endocrinopathies, ocular toxicity, pancreatic toxicity, neurologic toxicity, myocarditis, and rash. Rash and hypothyroidism have been reported in patients treated with tiragolumab, with or without atezolizumab.

Patients with a history of autoimmune disease are excluded from this study.

*Treatment Interruption for Toxicities*

Study treatment may be temporarily suspended as appropriate for management of toxicity. On the basis of the available characterization of mechanism of action, tiragolumab may cause adverse events similar to but independent of atezolizumab, may exacerbate the frequency or severity of atezolizumab-related adverse events, or may have non-overlapping toxicities with atezolizumab. Because these scenarios may not be distinguished from one another in the clinical setting, immune-mediated adverse events should generally be attributed to atezolizumab and tiragolumab, and dose interruptions or treatment discontinuation in response to immune-mediated adverse events should be applied to atezolizumab and tiragolumab.

Atezolizumab and tiragolumab may be held for a maximum of approximately 12 weeks (or approximately four cycles). If tiragolumab is interrupted for more than approximately 12 weeks for any reason, the patient will have to permanently discontinue tiragolumab treatment but may continue atezolizumab if there is no contraindication and after discussion with the Medical Monitor to determine whether the toxicity is considered related to tiragolumab and/or to the combination study treatment. Continued dosing with single-agent atezolizumab administered to patients Q4W will require that all other study eligibility criteria continue to be met.

An exception can be made if in the judgment of the investigator, the patient is likely to derive clinical benefit from resuming tiragolumab after a hold of > 12 weeks. In this case, tiragolumab may be restarted with the approval of the Medical Monitor.

If atezolizumab is interrupted for approximately > 12 weeks (or approximately four cycles), the patient will have to permanently discontinue atezolizumab. However, if, in the judgment of the investigator, the patient is likely to derive clinical benefit from atezolizumab after a hold of approximately > 12 weeks, atezolizumab may be restarted.

If a patient must be tapered off steroids used to treat adverse events, atezolizumab may be withheld for additional time beyond approximately 12 weeks from the final dose, and tiragolumab may be withheld for an additional time beyond approximately 12 weeks from the final dose until steroids are discontinued, or until steroids are reduced to < 10 mg/day dose of prednisone (or dose equivalent). The acceptable length of interruption will depend on an agreement between the investigator and the Medical Monitor. Atezolizumab and/or tiragolumab treatment may be suspended for reasons other than toxicity (e.g., surgical procedures) with Medical Monitor approval.

**Tumor and Response Evaluations**

Screening and subsequent tumor assessments must include CT scans (with oral or IV contrast unless contraindicated). A CT scan of the pelvis is required at screening and as clinically indicated or as per local standard of care at subsequent response evaluations. Magnetic resonance imaging (MRI) scans with contrast of the chest, abdomen, and pelvis with a non-contrast CT scan of the chest may be used in patients for whom CT scans with contrast are contraindicated (i.e., patients with contrast allergy or impaired renal clearance).

A CT scan with contrast or MRI scan with contrast (if CT contrast is contraindicated) of the head must be performed at screening to evaluate CNS metastasis in all patients. If a CT scan with contrast is performed and the presence of brain metastases is considered equivocal, an MRI scan of the head is required to confirm or refute the diagnosis of CNS metastases at baseline. Patients with CNS metastases are not eligible for the study.

If a CT scan for tumor assessment is performed in a positron emission tomography (PET)/CT scanner, the CT acquisition must be consistent with the standards for a full contrast diagnostic CT scan.

Further investigations, such as bone scans and CT scans of the neck, should also be performed if clinically indicated. At the investigator's discretion, other methods of assessment of measurable disease according to RECIST v1.1 may be used. Tumor assessments performed as standard of care prior to obtaining informed consent, after final dose of concurrent CRT, and within 28 days of randomization, may be used rather than repeating tests. All known sites of disease, including measurable and/or non-measurable disease, must be documented at screening and re-assessed at each subsequent tumor evaluation. The same radiographic procedure used to assess disease sites at screening should be used throughout the study (e.g., the same contrast protocol for CT scans).

Patients undergo tumor assessments at screening and every 8 weeks (± 7 days) for 48 weeks following Day 1 of Cycle 1 regardless of treatment delays. After the completion of the Week 48 tumor assessment, tumor assessment is required every 12 weeks (± 7 days) regardless of treatment delays until confirmed radiographic disease progression (as defined by growth of existing lesions, new lesions, or recurrence of previously resolved lesions) per RECIST v1.1, withdrawal of consent, or study termination, whichever occurs first. Patients who are treated beyond disease progression per RECIST v1.1 will undergo tumor assessments at the frequency described above until study treatment is discontinued. At the investigator's discretion, scans may be performed at any time if progressive disease or loss of clinical benefit is suspected.

Patients who discontinue treatment for reasons other than radiographic disease progression per RECIST v1.1 (e.g., toxicity, symptomatic deterioration, completion of study treatment) will continue scheduled tumor assessments at the frequency described above until confirmed radiographic disease progression per RECIST v1.1, withdrawal of consent, death, or study termination, whichever occurs first. Patients who start a new anti-cancer therapy, in the absence of confirmed radiographic disease progression per RECIST v1.1, will also continue tumor assessments at the frequency described above until radiographic disease progression per RECIST v1.1, withdrawal of consent, death, or study termination, whichever occurs first.

If a tumor assessment shows radiographic disease progression per RECIST v1.1, a confirmatory scan should be performed no later than the next scheduled assessment, or earlier if clinically indicated. Administration of study treatment will continue between the initial assessment of progression and confirmation of radiographic disease progression. If the confirmatory scan shows unequivocal radiographic disease progression per RECIST v1.1, the date of disease progression is the date of the first assessment of progression.

Response is assessed by the investigator on the imaging modalities detailed above, using RECIST v1.1. The investigator's assessment of overall tumor response at all timepoints should only be based on RECIST v1.1. Assessments are performed by the same evaluator if possible to ensure internal consistency across visits. Results are reviewed by the investigator before dosing at the next cycle. If a tumor assessment shows disease progression, it should be confirmed pathologically and/or by unequivocal radiographic evidence from the scan. If the scan shows equivocal findings (e.g., mediastinal nodes measure < 1.5 cm in the short axis, lung parenchymal lesions or visceral lesions measuring < 1 cm in the longest diameter), a biopsy should be performed. If a biopsy is not feasible or safe, then confirmatory scans should be performed no later than the next scheduled assessment, or earlier if clinically indicated.

If a biopsy for disease progression confirmation is performed, any leftover biopsy tissue is strongly encouraged to be submitted for exploratory biomarker research (optional consent required for exploratory research; see Section 4.5.7 for details). The biopsy is performed prior to starting the next anti-cancer therapy. If the biopsy does not show evidence of disease progression (e.g., non-malignant infiltrates), then the patient may continue with scheduled study treatment, assessments, and/or follow-up.

Study treatment may be continued for 13 cycles of treatment as long as patients are experiencing clinical benefit, as assessed by the investigator, and in the absence of unacceptable toxicity or symptomatic deterioration attributed to disease progression after an integrated assessment of radiographic data, biopsy results (if available), and clinical status. Patients who meet criteria for disease progression per RECIST v1.1 are permitted to continue treatment (atezolizumab plus tiragolumab or durvalumab) if they meet all of the criteria specified.

After radiographic disease progression per RESIST v1.1 and discontinuation of study treatment, patients will undergo tumor assessments per local standard of care, as assessed by the investigator per RECIST v1.1, regardless of whether a patient starts a new anti-cancer therapy.

Investigator assessment of overall tumor response at all timepoints are based on RECIST v1.1 only. The overall tumor assessment is derived per iRECIST based on entries for all target lesions, non-target lesions, and new lesions. To facilitate evaluation of response per iRECIST, tumor assessments must be continued after disease progression per RECIST v1.1 for patients who receive study treatment beyond progression. This includes continued measurement of target lesions, evaluation of non-target lesions (including monitoring for further worsening of any non-target lesions that have shown unequivocal progression), and evaluation of any newly identified lesions or recurrence of previously resolved lesions (including measurements, if lesions are measurable) at all subsequent assessments.

**Assessments Performed on Tumor Samples**

The following assessments are performed on tumor samples:

- Archival or fresh tissue tumor samples are analyzed for expression of PD-L1 and for exploratory research on other biomarkers and biomarker development.
- Biomarker assays in archival tumor tissue samples are analyzed for determination of eligibility.

Tumor tissue should be of good quality based on total and viable tumor content (i.e., preserved cellular context and tissue architecture). Acceptable samples include samples from resections, core-needle biopsies for deep tumor tissue (with a minimum of three cores for freshly collected biopsies); excisional, incisional, punch, or forceps biopsies for cutaneous, subcutaneous, or mucosal lesions; or EBUS core-needle biopsy. EBUS-TBNA, which is sometimes referred to as a fine-needle aspiration, is acceptable (particularly if a larger gauge needle is used) provided tissue is of good quality as described above (i.e., preserved cellular context and tissue architecture). For needle aspirations, an 18-gauge or larger needle is recommended.

Sites are informed if the quality of the submitted specimen is inadequate to determine PD-L1 status. Fine-needle aspiration, brushing, cell pellets from pleural effusion, and lavage samples are not acceptable. For core-needle biopsy specimens, at least three cores should be submitted for evaluation.

Archival tumor tissue samples obtained outside of this study for central assessment of PD-L1 results and other biomarker analyses are collected from all patients (paraffin blocks are preferred or at least 15-20 unstained serial slides are acceptable). Fine-needle aspirates, cell pellets from effusions or ascites, lavage samples, and bone biopsies do not satisfy the requirement for archival tissue.

If adequate tissue from distinct timepoints (such as time of initial diagnosis and at the time of disease recurrence) and/or multiple metastatic tumors is available, priority should be given to the tissue most recently collected prior to starting cCRT.

Patients having additional tissue samples from procedures performed at different times during this study are requested (but not required) to also submit these samples for central testing. Tissue samples are obtained at multiple times for individual patients will greatly contribute to understanding an improved understanding of the mechanism of action of the treatment and disease biology.

**Efficacy Analysis**

The analysis population for the efficacy analyses consist of all randomized patients, with patients grouped according to their assigned treatment.

**Primary Efficacy Endpoints**

The primary efficacy endpoint is IRF-assessed PFS after randomization, defined as the time between the date of randomization and the date of first documented disease progression as assessed by the IRF according to RECIST v1.1, or death, whichever occurs first. Patients who have not experienced disease progression or died at the time of analysis are censored at the time of the last tumor assessment. Patients with no post-baseline tumor assessment are censored at the date of randomization.

The primary efficacy analysis is performed for the PD-L1-positive population and the ITT population. The null and alternative hypotheses for the IRF-assessed PFS analysis can be phrased in terms of the survival functions SA(t) and SB(t) in the tiragolumab plus atezolizumab arm and durvalumab arm, respectively:

$$H_0: S_A(t) = S_B(t) \text{ vs. } H_1: S_A(t) \neq S_B(t)$$

IRF-assessed PFS is compared between treatment arms with use of the stratified log-rank test. The HR for IRF-assessed PFS is estimated using a stratified Cox proportional hazards model. The 95% CI for the HR is provided. The stratification factors are the same as the randomization stratification factors: ECOG Performance Status (0 vs. 1), PD-L1 status, as determined by the investigational Ventana PD-L1 (SP263) CDx Assay ( ≥1% TC positive vs. <1% TC positive), histology (squamous vs. non-squamous), and disease staging (Stage IIIA vs. Stage IIIB or Stage IIIC). Stratification factor(s) may be removed from the stratified analyses if there is risk of overstratification. Analyses based on stratification factors recorded on the eCRF will also be provided if considerable discrepancy is observed between IxRS records and eCRFs. Results from an unstratified analysis will also be provided. Kaplan-Meier methodology is used to estimate the median PFS for each treatment arm, and Kaplan-Meier curve is constructed to provide a visual description of the difference between treatment arms. The Brookmeyer-Crowley methodology is used to construct the 95% CI for the median PFS for each treatment arm (Brookmeyer and Crowley, Biometrics 1982, 38:29-41).

A group sequential design is used for testing IRF-assessed PFS to account for the interim analysis, which is expected to occur approximately 40 months after the first patient is enrolled in the study.

**Secondary Efficacy Endpoints**

The secondary efficacy endpoints ware analyzed in the PD-L1-positive population and/or the ITT population, and the statistical testing of the hypotheses depends on the results of the primary endpoint analyses.

*Overall Survival*

OS is defined as the time from randomization to death from any cause. Data for patients who are not reported as having died at the time of the analysis are censored at the date when they were last known to be alive. Patients who do not have post-baseline information are censored at the date of randomization. OS is analyzed through use of the same methods described for the IRF-assessed PFS analysis. If the primary endpoint of IRF-assessed PFS shows statistically significant in the ITT population, OS in the PD-L1-positive population and in the ITT population are formally tested in a fixed order to control the overall type I error rate at the same level for the IRF-assessed PFS for the ITT population (i.e., 0.03 or 0.05).

*Investigator-assessed PFS*

Investigator-assessed PFS is defined as the time between the date of randomization and the date of first documented disease progression, as assessed by the investigator according to RECIST v1.1, or death, whichever occurs first. Patients who have not experienced disease progression nor died at the time of analysis are censored at the time of the last tumor assessment. Patients with no post-baseline tumor assessment are censored at the date of randomization.

*Overall Response Rate*

A confirmed objective response is defined as either a CR or PR on two consecutive occasions ≥ 4 weeks apart, as determined by the IRF according to RECIST v1.1. Patients who do not meet these criteria, including patients without any post-baseline tumor assessment, are considered non-responders.

Confirmed ORR is defined as the proportion of patients who achieve a confirmed objective response. Confirmed ORR is analyzed in the randomized patients with measurable disease at baseline. An estimate of confirmed ORR and its 95% CI is calculated using the Clopper-Pearson method for each treatment arm. CIs for the difference in confirmed ORRs between the two treatment arms are determined using the normal approximation to the binomial distribution. The confirmed ORR is compared between the two treatment arms using the stratified Mantel-Haenszel test. Confirmed ORR as determined by the investigator according to RECIST v1.1 will also be analyzed.

*Duration of Response*

DOR is assessed in patients who achieved a confirmed objective response, as determined by the IRF according to RECIST v1.1. DOR is defined as the time interval from the date of the first occurrence of a confirmed objective response until the first date of progressive disease as determined by the IRF according to RECIST v1.1 or death from any cause, whichever occurs first. Patients who have not progressed and who have not died at the time of analysis are censored at the time of last tumor assessment date. DOR is based on a non-randomized subset of patients (specifically, patients who achieve a confirmed objective response); therefore, formal hypothesis testing will not be performed for this endpoint. Comparisons between treatment arms are made for descriptive purposes. DOR for patients with confirmed objective response, as determined by the investigator according to RECIST v1.1, will also be analyzed.

**VII. OTHER EMBODIMENTS**

Some embodiments of the technology described herein can be defined according to any of the following numbered embodiments:

1. A method for treating a subject having a lung cancer, the method comprising administering to the subject one or more dosing cycles of an anti-TIGIT antagonist antibody (e.g., at a fixed dose of between about 30 mg to about 1200 mg every three weeks) and a PD-1 axis binding antagonist (e.g., at a fixed dose of between about 80 mg to about 1600 mg every three weeks), wherein the subject has been determined to have a high PD-L1 expression (e.g., PD-L1-positive tumor cell fraction of greater than, or equal to, 30%, and/or a PD-L1-positive tumor proportion score (TPS) of greater than, or equal to, 50%), and the treatment results in (a) a complete response (CR) or a partial response (PR) and/or (b) an increase in progression-free survival (PFS) as compared to treatment with the PD-1 axis binding antagonist without the anti-TIGIT antagonist antibody.
2. The method of embodiment 1, wherein the method comprises administering to the subject an anti-TIGIT antagonist antibody at a fixed dose of between about 30 mg to about 600 mg every three weeks.
3. The method of embodiment 2, wherein the method comprises administering to the subject an anti-TIGIT antagonist antibody at a fixed dose of about 600 mg every three weeks.
4. The method of any one of embodiments 1-3, wherein the anti-TIGIT antagonist antibody comprises the following hypervariable regions (HVRs):

an HVR-H1 sequence comprising the amino acid sequence of SNSAAWN (SEQ ID NO: 1);
an HVR-H2 sequence comprising the amino acid sequence of KTYYRFKWYSDYAVSVKG (SEQ ID NO: 2);
an HVR-H3 sequence comprising the amino acid sequence of ESTTYDLLAGPFDY (SEQ ID NO: 3);
an HVR-L1 sequence comprising the amino acid sequence of KSSQTVLYSSNNKKYLA (SEQ ID NO: 4);
an HVR-L2 sequence comprising the amino acid sequence of WASTRES (SEQ ID NO: 5); and
an HVR-L3 sequence comprising the amino acid sequence of QQYYSTPFT (SEQ ID NO: 6).

5. The method of embodiment 4, wherein the anti-TIGIT antagonist antibody further comprises the following light chain variable region framework regions (FRs):

an FR-L1 comprising the amino acid sequence of DIVMTQSPDSLAVSLGERATINC (SEQ ID NO: 7);
an FR-L2 comprising the amino acid sequence of WYQQKPGQPPNLLIY (SEQ ID NO: 8);
an FR-L3 comprising the amino acid sequence of GVPDRFSGSGSGTDFTLTISSLQAEDVAVYYC (SEQ ID NO: 9); and
an FR-L4 comprising the amino acid sequence of FGPGTKVEIK (SEQ ID NO: 10).

6. The method of embodiment 4, wherein the anti-TIGIT antagonist antibody further comprises the following heavy chain variable region FRs:

an FR-H1 comprising the amino acid sequence of $X_1$VQLQQSGPGLVKPSQTLSLTCAISGDSVS (SEQ ID NO: 11), wherein $X_1$ is Q or E;

an FR-H2 comprising the amino acid sequence of WIRQSPSRGLEWLG (SEQ ID NO: 12);
an FR-H3 comprising the amino acid sequence of RITINPDTSKNQFSLQLNSVTPEDTAVFYCTR (SEQ ID NO: 13); and
an FR-H4 comprising the amino acid sequence of WGQGTLVTVSS (SEQ ID NO: 14).

7. The method of embodiment 6, wherein $X_1$ is Q.
8. The method of embodiment 6, wherein $X_1$ is E.
9. The method of any one of embodiments 4-8, wherein the anti-TIGIT antagonist antibody comprises:

(a) a heavy chain variable (VH) domain comprising an amino acid sequence having at least 95% sequence identity to the amino acid sequence of SEQ ID NO: 17 or 18;
(b) a light chain variable (VL) domain comprising an amino acid sequence having at least 95% sequence identity to the amino acid sequence of SEQ ID NO: 19; or
(c) a VH domain as in (a) and a VL domain as in (b).

10. The method of any one of embodiments 1-9, wherein the anti-TIGIT antagonist antibody comprises:

a VH domain comprising the amino acid sequence of SEQ ID NO: 17 or 18; and
a VL domain comprising the amino acid sequence of SEQ ID NO: 19.

11. The method of any one of embodiments 1-10, wherein the anti-TIGIT antagonist antibody is a monoclonal antibody.
12. The method of embodiment 11, wherein the anti-TIGIT antagonist antibody is a human antibody.
13. The method of any one of embodiments 1-12, wherein the anti-TIGIT antagonist antibody is a full-length antibody.
14. The method of any one of embodiments 1-6 and 8-13, wherein the anti-TIGIT antagonist antibody is tiragolumab.
15. The method of any one of embodiments 1-12, wherein the anti-TIGIT antagonist antibody is an antibody fragment that binds TIGIT selected from the group consisting of Fab, Fab', Fab'-SH, Fv, single chain variable fragment (scFv), and $(Fab')_2$ fragments.
16. The method of any one of embodiments 1-15, wherein the anti-TIGIT antagonist antibody is an IgG class antibody.
17. The method of embodiment 16, wherein the IgG class antibody is an IgG1 subclass antibody.
18. The method of any one of embodiments 1-17, wherein the method comprises administering to the subject an anti-PD-L1 antibody at a fixed dose of about 1200 mg every three weeks.
19. The method of any one of embodiments 1-18, wherein the PD-1 axis binding antagonist is a PD-L1 binding antagonist or a PD-1 binding antagonist.
20. The method of embodiment 19, wherein the PD-L1 binding antagonist is an anti-PD-L1 antagonist antibody.
21. The method of embodiment 20, wherein the anti-PD-L1 antagonist antibody is atezolizumab (MPDL3280A), MSB0010718C, MDX-1105, or MEDI4736.
22. The method of embodiment 21, wherein the anti-PD-L1 antagonist antibody is atezolizumab.
23. The method of embodiment 19, wherein the PD-1 binding antagonist is an anti-PD-1 antagonist antibody.
24. The method of embodiment 23, wherein the anti-PD-1 antagonist antibody is nivolumab (MDX-1106), pembrolizumab (MK-3475), MED1-0680, spartalizumab (PDR001), cemiplimab (REGN2810), BGB-108, prolgolimab, camrelizumab, sintilimab, tislelizumab, or toripalimab.
25. The method of embodiment 20, wherein the anti-PD-L1 antagonist antibody comprises the following HVRs:

an HVR-H1 sequence comprising the amino acid sequence of GFTFSDSWIH (SEQ ID NO: 20);
an HVR-H2 sequence comprising the amino acid sequence of AWISPYGGSTYYADSVKG (SEQ ID NO: 21);
an HVR-H3 sequence comprising the amino acid sequence of RHWPGGFDY (SEQ ID NO: 22);
an HVR-L1 sequence comprising the amino acid sequence of RASQDVSTAVA (SEQ ID NO: 23);
an HVR-L2 sequence comprising the amino acid sequence of SASFLYS (SEQ ID NO: 24); and
an HVR-L3 sequence comprising the amino acid sequence of QQYLYHPAT (SEQ ID NO: 25).

26. The method of embodiment 25, wherein the anti-PD-L1 antagonist antibody comprises:

(a) a heavy chain variable (VH) domain comprising an amino acid sequence having at least 95% sequence identity to the amino acid sequence of SEQ ID NO: 26;
(b) a light chain variable (VL) domain comprising an amino acid sequence having at least 95% sequence identity to the amino acid sequence of SEQ ID NO: 27; or
(c) a VH domain as in (a) and a VL domain as in (b).

27. The method of embodiment 26, wherein the anti-PD-L1 antagonist antibody comprises:

a VH domain comprising the amino acid sequence of SEQ ID NO: 26; and
a VL domain comprising the amino acid sequence of SEQ ID NO: 27.

28. The method of any one of embodiments 1-27, wherein the PD-1 axis binding antagonist is a monoclonal antibody.
29. The method of any one of embodiments 1-28, wherein the PD-1 axis binding antagonist is a humanized antibody.
30. The method of any one of embodiments 1-29, wherein the PD-1 axis binding antagonist is a full-length antibody.
31. The method of any one of embodiments 1-29, wherein the PD-1 axis binding antagonist is an antibody fragment that binds PD-L1 selected from the group consisting of Fab, Fab', Fab'-SH, Fv, single chain variable fragment (scFv), and $(Fab')_2$ fragments.
32. The method of any one of embodiments 1-30, wherein the PD-1 axis binding antagonist is an IgG class antibody.
33. The method of embodiment 32, wherein the IgG class antibody is an IgG1 subclass antibody.
34. The method of any one of embodiments 1-33, wherein the method comprises administering to the subject the anti-TIGIT antagonist antibody at a fixed dose of about 600 mg every three weeks and the PD-1 axis binding antagonist at a fixed dose of about 1200 mg every three weeks.
35. The method of any one of embodiments 1-34, wherein the length of each of the one or more dosing cycles is 21 days.
36. The method of any one of embodiments 1-35, wherein the method comprises administering to the subject the anti-TIGIT antagonist antibody and the PD-1 axis binding antagonist on about Day 1 of each of the one or more dosing cycles.
37. The method of any one of embodiments 1-36, wherein the method comprises administering to the subject the PD-1 axis binding antagonist before the anti-TIGIT antagonist antibody.
38. The method of embodiment 37, wherein the method comprises a first observation period following administration of the PD-1 axis binding antagonist and second observation period following administration of the anti-TIGIT antagonist antibody.
39. The method of embodiment 38, wherein the first observation period and the second observation period are each between about 30 minutes to about 60 minutes in length.
40. The method of any one of embodiments 1-36, wherein the method comprises administering to the subject the anti-TIGIT antagonist antibody before the PD-1 axis binding antagonist.
41. The method of embodiment 40, wherein the method comprises a first observation period following administration of the anti-TIGIT antagonist antibody and second observation period following administration of the PD-1 axis binding antagonist.
42. The method of embodiment 41, wherein the first observation period and the second observation period are each between about 30 minutes to about 60 minutes in length.
43. The method of any one of embodiments 1-36, wherein the method comprises administering to the subject the anti-TIGIT antagonist antibody and the PD-1 axis binding antagonist simultaneously.
44. The method of any one of embodiments 1-43, wherein the method comprises administering to the subject the anti-TIGIT antagonist antibody and PD-1 axis binding antagonist intravenously.
45. The method of embodiment 44, wherein the method comprises administering to the subject the anti-TIGIT antagonist antibody by intravenous infusion over 60 ± 10 minutes.
46. The method of embodiment 44 or 45, wherein the method comprises administering to the subject the PD-1 axis binding antagonist by intravenous infusion over 60 ± 15 minutes.
47. The method of any one of embodiments 1-43, wherein the method comprises administering to the subject the anti-TIGIT antagonist antibody and PD-1 axis binding antagonist subcutaneously.
48. The method of any one of embodiments 1-47, wherein the PD-L1-positive tumor cell fraction has been determined by an immunohistochemical (IHC) assay.
49. The method of any one of embodiments 1-48, wherein the PD-L1-positive tumor cell fraction is determined by positive staining with an anti-PD-L1 antibody, wherein the anti-PD-L1 antibody is SP263, 22C3, SP142, or 28-8.
50. The method of embodiment 49, wherein the PD-L1-positive tumor cell fraction is greater than, or equal to, 50%, as determined by positive staining with the anti-PD-L1 antibody SP263.
51. The method of embodiment 50, wherein the PD-L1-positive tumor cell fraction is calculated using the Ventana SP263 IHC assay.
52. The method of embodiment 49, wherein the PD-L1-positive tumor cell fraction is greater than, or equal to, 50%, as determined by positive staining with the anti-PD-L1 antibody 22C3.
53. The method of embodiment 52, wherein the PD-L1-positive tumor cell fraction is calculated using the pharmDx 22C3 IHC assay.
54. The method of embodiment 49, wherein the PD-L1-positive tumor cell fraction is greater than, or equal to, 30%, as determined by positive staining with the anti-PD-L1 antibody SP142.

55. The method of embodiment 49, wherein the PD-L1-positive tumor cell fraction is greater than, or equal to, 50%, as determined by positive staining with the anti-PD-L1 antibody 28-8.

56. The method of any one of embodiments 1-55, wherein a tumor sample obtained from the subject has been determined to have a detectable nucleic acid expression level of PD-L1.

57. The method of embodiment 56, wherein the detectable nucleic acid expression level of PD-L1 has been determined by RNA-seq, RT-qPCR, qPCR, multiplex qPCR or RT-qPCR, microarray analysis, SAGE, MassARRAY technique, ISH, or a combination thereof.

58. The method of any one of embodiments 1-57, wherein the lung cancer is a non-small cell lung cancer (NSCLC).

59. The method of embodiment 58, wherein the NSCLC is a squamous NSCLC.

60. The method of embodiment 59, wherein the NSCLC is a non-squamous NSCLC.

61. The method of any one of embodiments 58-60, wherein the NSCLC is a locally advanced unresectable NSCLC.

62. The method of embodiment 61, wherein the NSCLC is a Stage IIIB NSCLC.

63. The method of any one of embodiments 58-60, wherein the NSCLC is a recurrent or metastatic NSCLC.

64. The method of embodiment 63, wherein the NSCLC is a Stage IV NSCLC.

65. The method of embodiment 63 or 64, wherein the subject has not been previously treated for Stage IV NSCLC.

66. The method of any one of embodiments 1-65, wherein the subject does not have a sensitizing epidermal growth factor receptor *(EGFR)* gene mutation or anaplastic lymphoma kinase *(ALK)* gene rearrangement.

67. The method of any one of embodiments 1-66, wherein the subject does not have a pulmonary lymphoepithelioma-like carcinoma subtype of NSCLC.

68. The method of any one of embodiments 1-67, wherein the subject does not have an active Epstein-Barr virus (EBV) infection or a known or suspected chronic active EBV infection.

69. The method of any one of embodiments 1-68, wherein the subject is negative for EBV IgM or negative by EBV PCR.

70. The method of embodiment 69, wherein the subject is negative for EBV IgM and negative by EBV PCR.

71. The method of embodiment 69 or 70, wherein the subject is positive for EBV IgG or positive for Epstein-Barr nuclear antigen (EBNA).

72. The method of embodiment 71, wherein the subject is positive for EBV IgG and positive for EBNA.

73. The method of any one of embodiments 1-72, wherein the subject is negative for EBV IgG or negative for EBNA.

74. The method of embodiment 73, wherein the subject is negative for EBV IgG and negative for EBNA.

75. The method of any one of embodiments 1-74, wherein the PFS is increased as compared to a reference PFS time.

76. The method of embodiment 75, wherein the reference PFS time is the median PFS time of a population of subjects who have received a treatment comprising a PD-1 axis binding antagonist without an anti-TIGIT antagonist antibody.

77. A method for treating a subject having a NSCLC, the method comprising administering to the subject one or more dosing cycles of an anti-TIGIT antagonist antibody (e.g., at a fixed dose of 600 mg every three weeks) and atezolizumab (e.g., at a fixed dose of 1200 mg every three weeks), wherein the anti-TIGIT antagonist antibody comprises:

a VH domain comprising the amino acid sequence of SEQ ID NO: 17 or 18; and
a VL domain comprising the amino acid sequence of SEQ ID NO: 19, and wherein the subject has been determined to have a high PD-L1 expression (e.g., a PD-L1-positive tumor cell fraction of greater than, or equal to, 30%, or a PD-L1 TPS of greater than, or equal to, 50), and the treatment results in (a) a CR or a PR and/or (b) an increase in PFS as compared to treatment with atezolizumab without the anti-TIGIT antagonist antibody.

78. A method of treating a subject having a NSCLC, the method comprising:

(a) obtaining a tumor sample from the subject;
(b) detecting the protein expression level of PD-L1 in the tumor sample by staining tumor cells from the tumor sample with anti-PD-L1 antibody SP263 and determining a percentage of PD-L1-positive tumor cells therefrom, wherein 50% or more of the tumor cells stained with the anti-PD-L1 antibody SP263 are PD-L1-positive tumor cells; and
(c) administering to the subject a therapy comprising one or more dosing cycles of an anti-TIGIT antagonist antibody (e.g., at a fixed dose of 600 mg every three weeks) and atezolizumab (e.g., at a fixed dose of 1200 mg every three weeks), wherein the anti-TIGIT antagonist antibody comprises:

a VH domain comprising the amino acid sequence of SEQ ID NO: 17 or 18; and
a VL domain comprising the amino acid sequence of SEQ ID NO: 19, and
wherein the treatment results in (a) a CR or a PR and/or (b) an increase in PFS as compared to treatment with atezolizumab without the anti-TIGIT antagonist antibody.

79. A method for treating a subject having a NSCLC, the method comprising administering to the subject one or more dosing cycles of tiragolumab (e.g., at a fixed dose of 600 mg every three weeks) and atezolizumab (e.g., at a fixed dose of 1200 mg every three weeks), wherein the subject has been determined to have a high PD-L1 expression (e.g., a PD-L1-positive tumor cell fraction of greater than, or equal to, 30%, or a PD-L1 TPS of greater than, or equal to, 50), and the treatment results in (a) a CR or a PR and/or (b) an increase in PFS as compared to treatment with atezolizumab without tiragolumab.
80. A method of treating a subject having a NSCLC, the method comprising:

(a) obtaining a tumor sample from the subject;
(b) detecting the protein expression level of PD-L1 in the tumor sample by an IHC assay using anti-PD-L1 antibody SP263 and determining a PD-L1-positive tumor cell fraction therefrom, wherein the PD-L1-positive tumor cell fraction is determined to be greater than, or equal to, 50%; and
(c) administering to the subject a therapy comprising one or more dosing cycles of an anti-TIGIT antagonist antibody (e.g., at a fixed dose of 600 mg every three weeks) and atezolizumab (e.g., at a fixed dose of 1200 mg every three weeks), wherein the anti-TIGIT antagonist antibody comprises:

a VH domain comprising the amino acid sequence of SEQ ID NO: 17 or 18; and
a VL domain comprising the amino acid sequence of SEQ ID NO: 19, and
wherein the treatment results in (a) a CR or a PR and/or (b) an increase in PFS as compared to treatment with atezolizumab without the anti-TIGIT antagonist antibody.

81. An anti-TIGIT antagonist antibody and a PD-1 axis binding antagonist for use in a method of treating a subject having a lung cancer, wherein the method comprises administering to the subject one or more dosing cycles of the anti-TIGIT antagonist antibody (e.g., at a fixed dose of between about 30 mg to about 1200 mg every three weeks) and the PD-1 axis binding antagonist (e.g., at a fixed dose of between about 80 mg to about 1600 mg every three weeks), wherein the subject has been determined to have a high PD-L1 expression (e.g., a PD-L1-positive tumor cell fraction of greater than, or equal to, 30%, or a PD-L1 TPS of greater than, or equal to, 50), and the treatment results in (a) a CR or a PR and/or (b) an increase in PFS as compared to treatment with the PD-1 axis binding antagonist without the anti-TIGIT antagonist antibody.
82. The anti-TIGIT antagonist antibody and PD-1 axis binding antagonist for use of embodiment 81, wherein the anti-TIGIT antagonist antibody is to be administered to the subject at a fixed dose of between about 30 mg to about 600 mg every three weeks.
83. The anti-TIGIT antagonist antibody and PD-1 axis binding antagonist for use of embodiment 82, wherein the anti-TIGIT antagonist antibody is to be administered to the subject at a fixed dose of about 600 mg every three weeks.
84. The anti-TIGIT antagonist antibody and PD-1 axis binding antagonist for use of any one of embodiments 81-83, wherein the anti-TIGIT antagonist antibody comprises the following HVRs:

an HVR-H1 sequence comprising the amino acid sequence of SNSAAWN (SEQ ID NO: 1);
an HVR-H2 sequence comprising the amino acid sequence of KTYYRFKWYSDYAVSVKG (SEQ ID NO: 2);
an HVR-H3 sequence comprising the amino acid sequence of ESTTYDLLAGPFDY (SEQ ID NO: 3);
an HVR-L1 sequence comprising the amino acid sequence of KSSQTVLYSSNNKKYLA (SEQ ID NO: 4);
an HVR-L2 sequence comprising the amino acid sequence of WASTRES (SEQ ID NO: 5); and
an HVR-L3 sequence comprising the amino acid sequence of QQYYSTPFT (SEQ ID NO: 6).

85. The anti-TIGIT antagonist antibody and PD-1 axis binding antagonist for use of embodiment 84, wherein the anti-TIGIT antagonist antibody further comprises the following light chain variable region FRs:

an FR-L1 comprising the amino acid sequence of DIVMTQSPDSLAVSLGERATINC (SEQ ID NO: 7);
an FR-L2 comprising the amino acid sequence of WYQQKPGQPPNLLIY (SEQ ID NO: 8);
an FR-L3 comprising the amino acid sequence of
GVPDRFSGSGSGTDFTLTISSLQAEDVAVYYC (SEQ ID NO: 9); and
an FR-L4 comprising the amino acid sequence of FGPGTKVEIK (SEQ ID NO: 10).

86. The anti-TIGIT antagonist antibody and PD-1 axis binding antagonist for use of embodiment 84, wherein the anti-TIGIT antagonist antibody further comprises the following heavy chain variable region FRs:

an FR-H1 comprising the amino acid sequence of $X_1$VQLQQSGPGLVKPSQTLSLTCAISGDSVS (SEQ ID NO: 11), wherein $X_1$ is Q or E;
an FR-H2 comprising the amino acid sequence of WIRQSPSRGLEWLG (SEQ ID NO: 12);
an FR-H3 comprising the amino acid sequence of RITINPDTSKNQFSLQLNSVTPEDTAVFYCTR (SEQ ID NO: 13); and
an FR-H4 comprising the amino acid sequence of WGQGTLVTVSS (SEQ ID NO: 14).

87. The anti-TIGIT antagonist antibody and PD-1 axis binding antagonist for use of embodiment 86, wherein $X_1$ is Q.
88. The anti-TIGIT antagonist antibody and PD-1 axis binding antagonist of embodiment 86, wherein $X_1$ is E.
89. The anti-TIGIT antagonist antibody and PD-1 axis binding antagonist for use of any one of embodiments 84-88, wherein the anti-TIGIT antagonist antibody comprises:

(a) a heavy chain variable (VH) domain comprising an amino acid sequence having at least 95% sequence identity to the amino acid sequence of SEQ ID NO: 17 or 18;
(b) a light chain variable (VL) domain comprising an amino acid sequence having at least 95% sequence identity to the amino acid sequence of SEQ ID NO: 19; or
(c) a VH domain as in (a) and a VL domain as in (b).

90. The anti-TIGIT antagonist antibody and PD-1 axis binding antagonist for use of any one of embodiments 81-89, wherein the anti-TIGIT antagonist antibody comprises:

a VH domain comprising the amino acid sequence of SEQ ID NO: 17 or 18; and
a VL domain comprising the amino acid sequence of SEQ ID NO: 19.

91. The anti-TIGIT antagonist antibody and PD-1 axis binding antagonist for use of any one of embodiments 81-90, wherein the anti-TIGIT antagonist antibody is a monoclonal antibody.
92. The anti-TIGIT antagonist antibody and PD-1 axis binding antagonist for use of embodiment 91, wherein the anti-TIGIT antagonist antibody is a human antibody.
93. The anti-TIGIT antagonist antibody and PD-1 axis binding antagonist for use of any one of embodiments 81-92, wherein the anti-TIGIT antagonist antibody is a full-length antibody.
94. The anti-TIGIT antagonist antibody and PD-1 axis binding antagonist for use of any one of embodiments 81-86 and 88-93, wherein the anti-TIGIT antagonist antibody is tiragolumab.
95. The anti-TIGIT antagonist antibody and PD-1 axis binding antagonist for use of any one of embodiments 81-92, wherein the anti-TIGIT antagonist antibody is an antibody fragment that binds TIGIT selected from the group consisting of Fab, Fab', Fab'-SH, Fv, single chain variable fragment (scFv), and $(Fab')_2$ fragments.
96. The anti-TIGIT antagonist antibody and PD-1 axis binding antagonist for use of any one of embodiments 81-95, wherein the anti-TIGIT antagonist antibody is an IgG class antibody.
97. The anti-TIGIT antagonist antibody and PD-1 axis binding antagonist for use of embodiment 96, wherein the IgG class antibody is an IgG1 subclass antibody.
98. The anti-TIGIT antagonist antibody and PD-1 axis binding antagonist for use of any one of embodiments 81-97, wherein the PD-1 axis binding antagonist is to be administered to the subject at a fixed dose of about 1200 mg every three weeks.
99. The anti-TIGIT antagonist antibody and PD-1 axis binding antagonist for use of any one of embodiments 81-98, wherein the PD-1 axis binding antagonist is a PD-L1 binding antagonist or a PD-1 binding antagonist.
100. The anti-TIGIT antagonist antibody and PD-1 axis binding antagonist of embodiment 99, wherein the PD-L1 binding antagonist is an anti-PD-L1 antagonist antibody.
101. The anti-TIGIT antagonist antibody and PD-1 axis binding antagonist of embodiment 100, wherein the anti-PD-L1 antagonist antibody is atezolizumab (MPDL3280A), MSB0010718C, MDX-1105, or MEDI4736.
102. The anti-TIGIT antagonist antibody and anti-PD-L1 antagonist antibody for use of embodiment 101, wherein the anti-PD-L1 antagonist antibody is atezolizumab.
103. The anti-TIGIT antagonist antibody and PD-1 axis binding antagonist of embodiment 99, wherein the PD-1 binding antagonist is an anti-PD-1 antagonist antibody.
104. The anti-TIGIT antagonist antibody and PD-1 axis binding antagonist of embodiment 103, wherein the anti-PD-1 antagonist antibody is nivolumab (MDX-1106), pembrolizumab (MK-3475), MED1-0680, spartalizumab (PDR001), cemiplimab (REGN2810), BGB-108, prolgolimab, camrelizumab, sintilimab, tislelizumab, or toripalimab.
105. The anti-TIGIT antagonist antibody and PD-1 axis binding antagonist for use of embodiment 100, wherein the anti-PD-L1 antagonist antibody comprises the following HVRs:

an HVR-H1 sequence comprising the amino acid sequence of GFTFSDSWIH (SEQ ID NO: 20);
an HVR-H2 sequence comprising the amino acid sequence of AWISPYGGSTYYADSVKG (SEQ ID NO: 21);
an HVR-H3 sequence comprising the amino acid sequence of RHWPGGFDY (SEQ ID NO: 22);
an HVR-L1 sequence comprising the amino acid sequence of RASQDVSTAVA (SEQ ID NO: 23);
an HVR-L2 sequence comprising the amino acid sequence of SASFLYS (SEQ ID NO: 24); and
an HVR-L3 sequence comprising the amino acid sequence of QQYLYHPAT (SEQ ID NO: 25).

106. The anti-TIGIT antagonist antibody and PD-1 axis binding antagonist for use of embodiment 105, wherein the anti-PD-L1 antagonist antibody comprises:

(a) a heavy chain variable (VH) domain comprising an amino acid sequence having at least 95% sequence identity to the amino acid sequence of SEQ ID NO: 26;
(b) a light chain variable (VL) domain comprising an amino acid sequence having at least 95% sequence identity to the amino acid sequence of SEQ ID NO: 27; or
(c) a VH domain as in (a) and a VL domain as in (b).

107. The anti-TIGIT antagonist antibody and PD-1 axis binding antagonist for use of embodiment 106, wherein the anti-PD-L1 antagonist antibody comprises:

a VH domain comprising the amino acid sequence of SEQ ID NO: 26; and
a VL domain comprising the amino acid sequence of SEQ ID NO: 27.

108. The anti-TIGIT antagonist antibody and PD-1 axis binding antagonist for use of any one of embodiments 81-107, wherein the PD-1 axis binding antagonist is a monoclonal antibody.
109. The anti-TIGIT antagonist antibody and PD-1 axis binding antagonist for use of embodiment 108, wherein the PD-1 axis binding antagonist is a humanized antibody.
110. The anti-TIGIT antagonist antibody and PD-1 axis binding antagonist for use of embodiment 108 or 109, wherein the PD-1 axis binding antagonist is a full-length antibody.
111. The anti-TIGIT antagonist antibody and PD-1 axis binding antagonist for use of any one of embodiments 81-109, wherein the PD-1 axis binding antagonist is an antibody fragment that binds PD-L1 selected from the group consisting of Fab, Fab', Fab'-SH, Fv, single chain variable fragment (scFv), and $(Fab')_2$ fragments.
112. The anti-TIGIT antagonist antibody and PD-1 axis binding antagonist for use of any one of embodiments 81-110, wherein the PD-1 axis binding antagonist is an IgG class antibody.
113. The anti-TIGIT antagonist antibody and PD-1 axis binding antagonist for use of embodiment 112, wherein the IgG class antibody is an IgG1 subclass antibody.
114. The anti-TIGIT antagonist antibody and PD-1 axis binding antagonist for use of any one of embodiments 81-113, wherein the anti-TIGIT antagonist antibody is to be administered to the subject at a fixed dose of about 600 mg every three weeks and the PD-1 axis binding antagonist is to be administered to the subject at a fixed dose of about 1200 mg every three weeks.
115. The anti-TIGIT antagonist antibody and PD-1 axis binding antagonist for use of any one of embodiments 81-114, wherein the length of each of the one or more dosing cycles is 21 days.
116. The anti-TIGIT antagonist antibody and PD-1 axis binding antagonist for use of any one of embodiments 81-115, wherein the anti-TIGIT antagonist antibody and PD-1 axis binding antagonist are to be administered to the subject on about Day 1 of each of the one or more dosing cycles.
117. The anti-TIGIT antagonist antibody and PD-1 axis binding antagonist for use of any one of embodiments 81-116, wherein the PD-1 axis binding antagonist is to be administered to the subject before the anti-TIGIT antagonist antibody.
118. The anti-TIGIT antagonist antibody and PD-1 axis binding antagonist for use of embodiment 117, wherein a first observation period is to follow administration of the PD-1 axis binding antagonist and second observation period is to follow administration of the anti-TIGIT antagonist antibody.
119. The anti-TIGIT antagonist antibody and PD-1 axis binding antagonist for use of embodiment 118, wherein the first observation period and the second observation period are each between about 30 minutes to about 60 minutes in length.
120. The anti-TIGIT antagonist antibody and PD-1 axis binding antagonist for use of any one of embodiments 81-116, wherein the anti-TIGIT antagonist antibody is to be administered to the subject before the PD-1 axis binding antagonist.
121. The anti-TIGIT antagonist antibody and PD-1 axis binding antagonist for use of embodiment 120, wherein a first observation period is to follow administration of the anti-TIGIT antagonist antibody and second observation period is to follow administration of the PD-1 axis binding antagonist.

122. The anti-TIGIT antagonist antibody and PD-1 axis binding antagonist for use of embodiment 121, wherein the first observation period and the second observation period are each between about 30 minutes to about 60 minutes in length.

123. The anti-TIGIT antagonist antibody and PD-1 axis binding antagonist for use of any one of embodiments 81-116, wherein the anti-TIGIT antagonist antibody is to be administered to the subject simultaneously with the PD-1 axis binding antagonist.

124. The anti-TIGIT antagonist antibody and PD-1 axis binding antagonist for use of any one of embodiments 81-123, wherein the anti-TIGIT antagonist antibody and PD-1 axis binding antagonist are to be administered to the subject intravenously.

125. The anti-TIGIT antagonist antibody and PD-1 axis binding antagonist for use of embodiment 124, wherein the anti-TIGIT antagonist antibody is to be administered to the subject by intravenous infusion over 60 $\pm$ 10 minutes.

126. The anti-TIGIT antagonist antibody and PD-1 axis binding antagonist for use of embodiment 124 or 125, wherein the PD-1 axis binding antagonist is to be administered to the subject by intravenous infusion over 60 $\pm$ 15 minutes.

127. The anti-TIGIT antagonist antibody and PD-1 axis binding antagonist for use of any one of embodiments 81-123, wherein the anti-TIGIT antagonist antibody and PD-1 axis binding antagonist are to be administered to the subject subcutaneously.

128. The anti-TIGIT antagonist antibody and PD-1 axis binding antagonist for use of any one of embodiments 81-127, wherein the PD-L1-positive tumor cell fraction is determined by positive staining with an anti-PD-L1 antibody, wherein the anti-PD-L1 antibody is SP263, 22C3, SP142, or 28-8.

129. The anti-TIGIT antagonist antibody and PD-1 axis binding antagonist for use of embodiment 128, wherein the staining is part of an immunohistochemical (IHC) assay.

130. The anti-TIGIT antagonist antibody and PD-1 axis binding antagonist for use of embodiment 128 or 129, wherein the PD-L1-positive tumor cell fraction is greater than, or equal to, 50%, as determined by positive staining with the anti-PD-L1 antibody SP263, 22C3, or 28-8.

131. The anti-TIGIT antagonist antibody and PD-1 axis binding antagonist for use of embodiment 130, wherein the PD-L1-positive tumor cell fraction is determined by positive staining with the anti-PD-L1 antibody SP263 and using the Ventana SP263 IHC assay. -.

132. The anti-TIGIT antagonist antibody and PD-1 axis binding antagonist for use of embodiment 130, wherein the PD-L1-positive tumor cell fraction is determined by positive staining with the anti-PD-L1 antibody 22C3 and using the pharmDx 22C3 IHC assay.

133. The anti-TIGIT antagonist antibody and PD-1 axis binding antagonist for use of any one of embodiments 81-132, wherein a tumor sample obtained from the subject has been determined to have a detectable nucleic acid expression level of PD-L1.

134. The anti-TIGIT antagonist antibody and PD-1 axis binding antagonist for use of embodiment 133, wherein the detectable nucleic acid expression level of PD-L1 has been determined by RNA-seq, RT-qPCR, qPCR, multiplex qPCR or RT-qPCR, microarray analysis, SAGE, MassARRAY technique, ISH, or a combination thereof.

135. The anti-TIGIT antagonist antibody and PD-1 axis binding antagonist for use of any one of embodiments 81-134, wherein the subject has been determined to have a PD-L1-positive tumor cell fraction of greater than, or equal to, 50%, as determined by positive staining with the anti-PD-L1 antibody SP263, 22C3, or 28-8.

136. The anti-TIGIT antagonist antibody and PD-1 axis binding antagonist for use of any one of embodiments 81-135, wherein the lung cancer is a non-small cell lung cancer (NSCLC).

137. The anti-TIGIT antagonist antibody and PD-1 axis binding antagonist for use of embodiment 136, wherein the NSCLC is a squamous NSCLC.

138. The anti-TIGIT antagonist antibody and PD-1 axis binding antagonist for use of embodiment 137, wherein the NSCLC is a non-squamous NSCLC.

139. The anti-TIGIT antagonist antibody and PD-1 axis binding antagonist for use of any one of embodiments 136-138, wherein the NSCLC is a locally advanced unresectable NSCLC.

140. The anti-TIGIT antagonist antibody and PD-1 axis binding antagonist for use of embodiment 139, wherein the NSCLC is a Stage IIIB NSCLC.

141. The anti-TIGIT antagonist antibody and PD-1 axis binding antagonist for use of any one of embodiments 136-138, wherein the NSCLC is a recurrent or metastatic NSCLC.

142. The anti-TIGIT antagonist antibody and PD-1 axis binding antagonist for use of embodiment 141, wherein the NSCLC is a Stage IV NSCLC.

143. The anti-TIGIT antagonist antibody and PD-1 axis binding antagonist for use of embodiment 141 or 142, wherein the subject has not been previously treated for Stage IV NSCLC.

144. The anti-TIGIT antagonist antibody and PD-1 axis binding antagonist for use of any one of embodiments 81-143, wherein the subject does not have a sensitizing epidermal growth factor receptor *(EGFR)* gene mutation or anaplastic lymphoma kinase *(ALK)* gene rearrangement.

145. The anti-TIGIT antagonist antibody and PD-1 axis binding antagonist for use of any one of embodiments 81-144, wherein the subject does not have a pulmonary lymphoepithelioma-like carcinoma subtype of NSCLC.

146. The anti-TIGIT antagonist antibody and PD-1 axis binding antagonist for use of any one of embodiments 81-145, wherein the subject does not have an active EBV infection or a known or suspected chronic active EBV infection.

147. The anti-TIGIT antagonist antibody and PD-1 axis binding antagonist for use of any one of embodiments 81-146, wherein the subject is negative for EBV IgM or negative by EBV PCR.

148. The anti-TIGIT antagonist antibody and PD-1 axis binding antagonist for use of embodiment 147, wherein the subject is negative for EBV IgM and negative by EBV PCR.

149. The anti-TIGIT antagonist antibody and PD-1 axis binding antagonist for use of embodiment 147 or 148, wherein the subject is positive for EBV IgG or positive for EBNA.

150. The anti-TIGIT antagonist antibody and PD-1 axis binding antagonist for use of embodiment 149, wherein the subject is positive for EBV IgG and positive for EBNA.

151. The anti-TIGIT antagonist antibody and PD-1 axis binding antagonist for use of any one of embodiments 81-148, wherein the subject is negative for EBV IgG or negative for EBNA.

152. The anti-TIGIT antagonist antibody and PD-1 axis binding antagonist for use of embodiment 151, wherein the subject is negative for EBV IgG and negative for EBNA.

153. The anti-TIGIT antagonist antibody and PD-1 axis binding antagonist for use of any one of embodiments 81-152, wherein the PFS is increased as compared to a reference PFS time.

154. The anti-TIGIT antagonist antibody and PD-1 axis binding antagonist for use of embodiment 153, wherein the reference PFS time is the median PFS time of a population of subjects who have received a treatment comprising a PD-1 axis binding antagonist without an anti-TIGIT antagonist antibody.

155. An anti-TIGIT antagonist antibody and atezolizumab for use in a method of treating a subject having a NSCLC, wherein the method comprises administering to the subject one or more dosing cycles of an anti-TIGIT antagonist antibody (e.g., at a fixed dose of 600 mg every three weeks) and atezolizumab (e.g., at a fixed dose of 1200 mg every three weeks), wherein the anti-TIGIT antagonist antibody comprises:

a VH domain comprising the amino acid sequence of SEQ ID NO: 17 or 18; and

a VL domain comprising the amino acid sequence of SEQ ID NO: 19, and wherein the subject has been determined to have a high PD-L1 expression (e.g., a PD-L1-positive tumor cell fraction of greater than, or equal to, 30%, or a PD-L1 TPS of greater than, or equal to, 50), and the treatment results in (a) a CR or a PR and/or (b) an increase in PFS as compared to treatment with atezolizumab without the anti-TIGIT antagonist antibody.

156. Tiragolumab and atezolizumab for use in a method of treating a subject having a NSCLC, wherein the method comprises administering to the subject one or more dosing cycles of tiragolumab (e.g., at a fixed dose of 600 mg every three weeks) and atezolizumab (e.g., at a fixed dose of 1200 mg every three weeks), and wherein the subject has been determined to have a high PD-L1 expression (e.g., a PD-L1-positive tumor cell fraction of greater than, or equal to, 30%, or a PD-L1 TPS of greater than, or equal to, 50), and the treatment results in (a) a CR or a PR and/or (b) an increase in PFS as compared to treatment with atezolizumab without tiragolumab.

157. Use of an anti-TIGIT antagonist antibody and a PD-1 axis binding antagonist in the manufacture of a medicament for use in a method of treating a subject having a lung cancer, wherein the method comprises administering to the subject one or more dosing cycles of the medicament, and wherein the medicament is formulated for administration of the anti-TIGIT antagonist antibody (e.g., at a fixed dose of between about 30 mg to about 1200 mg every three weeks) and the PD-1 axis binding antagonist (e.g., at a fixed dose of between about 80 mg to about 1600 mg every three weeks), wherein the subject has been determined to have a high PD-L1 expression (e.g., a PD-L1-positive tumor cell fraction of greater than, or equal to, 30%, or a PD-L1 TPS of greater than, or equal to, 50), and the treatment results in (a) a CR or a PR and/or (b) an increase in PFS as compared to treatment with the PD-1 axis binding antagonist without the anti-TIGIT antagonist antibody.

158. Use of an anti-TIGIT antagonist antibody in the manufacture of a medicament for use in a method of treating a subject having a lung cancer, wherein the method comprises administering to the subject one or more dosing cycles of the medicament and a PD-1 axis binding antagonist, and wherein the medicament is formulated for administration of the anti-TIGIT antagonist antibody (e.g., at a fixed dose of between about 30 mg to about 1200 mg every three weeks) and the PD-1 axis binding antagonist (e.g., at a fixed dose of between about 80 mg to about 1600 mg every three weeks), wherein the subject has been determined to have a high PD-L1 expression (e.g., a PD-L1-positive tumor cell fraction of greater than, or equal to, 30%, or a PD-L1 TPS of greater than, or equal to, 50), and the treatment results in (a) a CR or a PR and/or (b) an increase in PFS as compared to treatment with the PD-1 axis binding antagonist without the anti-TIGIT antagonist antibody.

159. Use of a PD-1 axis binding antagonist in the manufacture of a medicament for use in a method of treating a subject having a lung cancer, wherein the method comprises administering to the subject one or more dosing cycles of the medicament and an anti-TIGIT antagonist antibody, and wherein the medicament is formulated for administration of the PD-1 axis binding antagonist (e.g., at a fixed dose of between about 80 mg to about 1600 mg every three weeks) and the anti-TIGIT antagonist antibody (e.g., at a fixed dose of between about 30 mg to about 1200 mg every three weeks), wherein the subject has been determined to have a high PD-L1 expression (e.g., a PD-L1-positive tumor cell fraction of greater than, or equal to, 30%, or a PD-L1 TPS of greater than, or equal to, 50), and the treatment results in (a) a CR or a PR and/or (b) an increase in PFS as compared to treatment with the PD-1 axis binding antagonist without the anti-TIGIT antagonist antibody.

160. The use of any one of embodiments 157-159, wherein the anti-TIGIT antagonist antibody is to be administered to the subject at a fixed dose of between about 30 mg to about 600 mg every three weeks.

161. The use of embodiment 160, wherein the anti-TIGIT antagonist antibody is to be administered to the subject at a fixed dose of about 600 mg every three weeks.

162. The use of any one of embodiments 157-161, wherein the anti-TIGIT antagonist antibody comprises the following hypervariable regions (HVRs):

an HVR-H1 sequence comprising the amino acid sequence of SNSAAWN (SEQ ID NO: 1);
an HVR-H2 sequence comprising the amino acid sequence of KTYYRFKWYSDYAVSVKG (SEQ ID NO: 2);
an HVR-H3 sequence comprising the amino acid sequence of ESTTYDLLAGPFDY (SEQ ID NO: 3);
an HVR-L1 sequence comprising the amino acid sequence of KSSQTVLYSSNNKKYLA (SEQ ID NO: 4);
an HVR-L2 sequence comprising the amino acid sequence of WASTRES (SEQ ID NO: 5); and
an HVR-L3 sequence comprising the amino acid sequence of QQYYSTPFT (SEQ ID NO: 6).

163. The use of embodiment 162, wherein the anti-TIGIT antagonist antibody further comprises the following light chain variable region framework regions (FRs):

an FR-L1 comprising the amino acid sequence of DIVMTQSPDSLAVSLGERATINC (SEQ ID NO: 7);
an FR-L2 comprising the amino acid sequence of WYQQKPGQPPNLLIY (SEQ ID NO: 8);
an FR-L3 comprising the amino acid sequence of GVPDRFSGSGSGTDFTLTISSLQAEDVAVYYC (SEQ ID NO: 9); and
an FR-L4 comprising the amino acid sequence of FGPGTKVEIK (SEQ ID NO: 10).

164. The use of embodiment 162, wherein the anti-TIGIT antagonist antibody further comprises the following heavy chain variable region FRs:

an FR-H1 comprising the amino acid sequence of $X_1$VQLQQSGPGLVKPSQTLSLTCAISGDSVS (SEQ ID NO: 11), wherein $X_1$ is Q or E;
an FR-H2 comprising the amino acid sequence of WIRQSPSRGLEWLG (SEQ ID NO: 12);
an FR-H3 comprising the amino acid sequence of RITINPDTSKNQFSLQLNSVTPEDTAVFYCTR (SEQ ID NO: 13); and
an FR-H4 comprising the amino acid sequence of WGQGTLVTVSS (SEQ ID NO: 14).

165. The use of embodiment 164, wherein $X_1$ is Q.

166. The use of embodiment 164, wherein $X_1$ is E.

167. The use of any one of embodiments 164-166, wherein the anti-TIGIT antagonist antibody comprises:

(a) a heavy chain variable (VH) domain comprising an amino acid sequence having at least 95% sequence identity to the amino acid sequence of SEQ ID NO: 17 or 18;
(b) a light chain variable (VL) domain comprising an amino acid sequence having at least 95% sequence identity to the amino acid sequence of SEQ ID NO: 19; or
(c) a VH domain as in (a) and a VL domain as in (b).

168. The use of any one of embodiments 157-167, wherein the anti-TIGIT antagonist antibody comprises:

a VH domain comprising the amino acid sequence of SEQ ID NO: 17 or 18; and
a VL domain comprising the amino acid sequence of SEQ ID NO: 19.

169. The use of any one of embodiments 157-168, wherein the anti-TIGIT antagonist antibody is a monoclonal antibody.

170. The use of embodiment 169, wherein the anti-TIGIT antagonist antibody is a human antibody.
171. The use of any one of embodiments 157-170, wherein the anti-TIGIT antagonist antibody is a full-length antibody.
172. The use of any one of embodiments 157-164 and 166-171, wherein the anti-TIGIT antagonist antibody is tiragolumab.
173. The use of any one of embodiments 157-170, wherein the anti-TIGIT antagonist antibody is an antibody fragment that binds TIGIT selected from the group consisting of Fab, Fab', Fab'-SH, Fv, single chain variable fragment (scFv), and $(Fab')_2$ fragments.
174. The use of any one of embodiments 157-173, wherein the anti-TIGIT antagonist antibody is an IgG class antibody.
175. The use of embodiment 174, wherein the IgG class antibody is an IgG1 subclass antibody.
176. The use of any one of embodiments 157-175, wherein the PD-1 axis binding antagonist is to be administered to the subject at a fixed dose of about 1200 mg every three weeks.
177. The use of any one of embodiment 176, wherein the PD-1 axis binding antagonist is a PD-L1 binding antagonist or a PD-1 binding antagonist.
178. The use of embodiment 177, wherein the PD-L1 binding antagonist is an anti-PD-L1 antagonist antibody.
179. The use of embodiment 178, wherein the anti-PD-L1 antagonist antibody is atezolizumab (MPDL3280A), MSB0010718C, MDX-1105, or MEDI4736.
180. The use of embodiment 179, wherein the anti-PD-L1 antagonist antibody is atezolizumab.
181. The use of embodiment 177, wherein the PD-1 binding antagonist is an anti-PD-1 antagonist antibody.
182. The use of embodiment 181, wherein the anti-PD-1 antagonist antibody is nivolumab (MDX-1106), pembrolizumab (MK-3475), MED1-0680, spartalizumab (PDR001), cemiplimab (REGN2810), BGB-108, prolgolimab, camrelizumab, sintilimab, tislelizumab, or toripalimab.
183. The use of embodiment 178, wherein the anti-PD-L1 antagonist antibody comprises the following HVRs:

an HVR-H1 sequence comprising the amino acid sequence of GFTFSDSWIH (SEQ ID NO: 20);
an HVR-H2 sequence comprising the amino acid sequence of AWISPYGGSTYYADSVKG (SEQ ID NO: 21);
an HVR-H3 sequence comprising the amino acid sequence of RHWPGGFDY (SEQ ID NO: 22);
an HVR-L1 sequence comprising the amino acid sequence of RASQDVSTAVA (SEQ ID NO: 23);
an HVR-L2 sequence comprising the amino acid sequence of SASFLYS (SEQ ID NO: 24); and
an HVR-L3 sequence comprising the amino acid sequence of QQYLYHPAT (SEQ ID NO: 25).

184. The use of embodiment 183, wherein the anti-PD-L1 antagonist antibody comprises:

(a) a heavy chain variable (VH) domain comprising an amino acid sequence having at least 95% sequence identity to the amino acid sequence of SEQ ID NO: 26;
(b) a light chain variable (VL) domain comprising an amino acid sequence having at least 95% sequence identity to the amino acid sequence of SEQ ID NO: 27; or
(c) a VH domain as in (a) and a VL domain as in (b).

185. The use of embodiment 184, wherein the anti-PD-L1 antagonist antibody comprises:

a VH domain comprising the amino acid sequence of SEQ ID NO: 26; and
a VL domain comprising the amino acid sequence of SEQ ID NO: 27.

186. The use of any one of embodiments 157-185, wherein the PD-1 axis binding antagonist is a monoclonal antibody.
187. The use of any one of embodiments 157-186, wherein the PD-1 axis binding antagonist is a humanized antibody.
188. The use of any one of embodiments 157-187, wherein the PD-1 axis binding antagonist is a full-length antibody.
189. The use of any one of embodiments 157-187, wherein the PD-1 axis binding antagonist is an antibody fragment that binds PD-L1 selected from the group consisting of Fab, Fab', Fab'-SH, Fv, single chain variable fragment (scFv), and $(Fab')_2$ fragments.
190. The use of embodiment 188, wherein the PD-1 axis binding antagonist is an IgG class antibody.
191. The use of embodiment 190, wherein the IgG class antibody is an IgG1 subclass antibody.
192. The use of any one of embodiments 157-191, wherein the anti-TIGIT antagonist antibody is to be administered to the subject at a fixed dose of about 600 mg of every three weeks and the PD-1 axis binding antagonist is to be administered to the subject at a fixed dose of about 1200 mg every three weeks.
193. The use of any one of embodiments 157-192, wherein the length of each of the one or more dosing cycles is 21 days.
194. The use of any one of embodiments 157-193, wherein the anti-TIGIT antagonist antibody and PD-1 axis binding antagonist are to be administered to the subject on about Day 1 of each of the one or more dosing cycles.

195. The use of any one of embodiments 157-194, wherein the anti-TIGIT antagonist antibody is to be administered to the subject before the PD-1 axis binding antagonist.

196. The use of embodiment 195, wherein a first observation period is to follow administration of the PD-1 axis binding antagonist and second observation period is to follow administration of the anti-TIGIT antagonist antibody.

197. The use of embodiment 196, wherein the first observation period and the second observation period are each between about 30 minutes to about 60 minutes in length.

198. The use of any one of embodiments 157-194, wherein the anti-TIGIT antagonist antibody is to be administered to the subject before the PD-1 axis binding antagonist.

199. The use of embodiment 198, wherein a first observation period is to follow administration of the anti-TIGIT antagonist antibody and second observation period is to follow administration of the PD-1 axis binding antagonist.

200. The use of embodiment 199, wherein the first observation period and the second observation period are each between about 30 minutes to about 60 minutes in length.

201. The use of any one of embodiments 157-194, wherein the anti-TIGIT antagonist antibody is to be administered to the subject simultaneously with the PD-1 axis binding antagonist.

202. The use of any one of embodiments 157-201, wherein the anti-TIGIT antagonist antibody and PD-1 axis binding antagonist are to be administered to the subject intravenously.

203. The use of embodiment 202, wherein the anti-TIGIT antagonist antibody is to be administered to the subject by intravenous infusion over 60 $\pm$ 10 minutes.

204. The use of embodiment 200 or 203, wherein the PD-1 axis binding antagonist is to be administered to the subject by intravenous infusion over 60 $\pm$ 15 minutes.

205. The use of any one of embodiments 157-201, wherein the anti-TIGIT antagonist antibody and PD-1 axis binding antagonist are to be administered to the subject subcutaneously.

206. The use of any one of embodiments 158-205, wherein the PD-L1-positive tumor cell fraction is determined by positive staining with an anti-PD-L1 antibody, wherein the anti-PD-L1 antibody is SP263, 22C3, SP142, or 28-8.

207. The use of embodiment 206, wherein the staining is part of an immunohistochemical (IHC) assay.

208. The use of embodiment 206 or 207, wherein the PD-L1-positive tumor cell fraction is greater than, or equal to, 50%, as determined by positive staining with the anti-PD-L1 antibody SP263, 22C3, or 28-8.

209. The use of embodiment 208, wherein the PD-L1-positive tumor cell fraction is determined by positive staining with the anti-PD-L1 antibody SP263 and using the Ventana SP263 IHC assay.

210. The use of embodiment 208, wherein the PD-L1-positive tumor cell fraction is determined by positive staining with the anti-PD-L1 antibody 22C3 and using the pharmDx 22C3 IHC assay.

211. The use of any one of embodiments 157-210, wherein a tumor sample obtained from the subject has been determined to have a detectable nucleic acid expression level of PD-L1.

212. The use of embodiment 211, wherein the detectable nucleic acid expression level of PD-L1 has been determined by RNA-seq, RT-qPCR, qPCR, multiplex qPCR or RT-qPCR, microarray analysis, SAGE, MassARRAY technique, ISH, or a combination thereof

213. The use of any one of embodiments 157-212, wherein the lung cancer is a non-small cell lung cancer.

214. The use of embodiment 213, wherein the NSCLC is a squamous NSCLC.

215. The use of embodiment 214, wherein the NSCLC is a non-squamous NSCLC.

216. The use of any one of embodiments 213-215, wherein the NSCLC is a locally advanced unresectable NSCLC.

217. The use of embodiment 216, wherein the NSCLC is a Stage IIIB NSCLC.

218. The use of any one of embodiments 213-216, wherein the NSCLC is a recurrent or metastatic NSCLC.

219. The use of embodiment 218, wherein the NSCLC is a Stage IV NSCLC.

220. The use of embodiment 218 or 219, wherein the subject has not been previously treated for Stage IV NSCLC.

221. The use of any one of embodiments 157-220, wherein the subject does not have a sensitizing epidermal growth factor receptor *(EGFR)* gene mutation or anaplastic lymphoma kinase *(ALK)* gene rearrangement.

222. The use of any one of embodiments 157-221, wherein the subject does not have a pulmonary lymphoepithelioma-like carcinoma subtype of NSCLC.

223. The use of any one of embodiments 157-222, wherein the subject does not have an active EBV infection or a known or suspected chronic active EBV infection.

224. The use of any one of embodiments 157-223, wherein the subject is negative for EBV IgM or negative by EBV PCR.

225. The use of embodiment 224, wherein the subject is negative for EBV IgM and negative by EBV PCR.

226. The use of embodiment 224 or 225, wherein the subject is positive for EBV IgG or positive for EBNA.

227. The use of embodiment 226, wherein the subject is positive for EBV IgG and positive for EBNA.

228. The use of any one of embodiments 157-227, wherein the subject is negative for EBV IgG or negative for EBNA.

229. The use of embodiment 228, wherein the subject is negative for EBV IgG and negative for EBNA.

230. The use of any one of embodiments 157-229, wherein the PFS is increased as compared to a reference PFS time.
231. The use of embodiment 230, wherein the reference PFS time is the median PFS time of a population of subjects who have received a treatment comprising an PD-1 axis binding antagonist without an anti-TIGIT antagonist antibody.
232. Use of an anti-TIGIT antagonist antibody and atezolizumab in the manufacture of a medicament for use in a method of treating a subject having a NSCLC, wherein the method comprises administering to the subject one or more dosing cycles of the medicament, wherein the medicament is formulated for administration of the anti-TIGIT antagonist antibody (e.g., at a fixed dose of 600 mg every three weeks) and atezolizumab (e.g., at a fixed dose of 1200 mg every three weeks), wherein the anti-TIGIT antagonist antibody comprises:

a VH domain comprising the amino acid sequence of SEQ ID NO: 17 or 18; and
a VL domain comprising the amino acid sequence of SEQ ID NO: 19, and wherein the subject has been determined to have a high PD-L1 expression (e.g., a PD-L1-positive tumor cell fraction of greater than, or equal to, 30%, or a PD-L1 TPS of greater than, or equal to, 50), and the treatment results in (a) a CR or a PR and/or (b) an increase in PFS as compared to treatment with atezolizumab without the anti-TIGIT antagonist antibody.

233. Use of tiragolumab and atezolizumab in the manufacture of a medicament for use in a method of treating a subject having a NSCLC, wherein the method comprises administering to the subject one or more dosing cycles of the medicament, wherein the medicament is formulated for administration of tiragolumab (e.g., at a fixed dose of 600 mg every three weeks) and atezolizumab (e.g., at a fixed dose of 1200 mg every three weeks), and wherein the subject has been determined to have a high PD-L1 expression (e.g., a PD-L1-positive tumor cell fraction of greater than, or equal to, 30%, or a PD-L1 TPS of greater than, or equal to, 50), and the treatment results in (a) a CR or a PR and/or (b) an increase in PFS as compared to treatment with atezolizumab without tiragolumab.
234. The method of any one of embodiments 1-78, the anti-TIGIT antagonist antibody of any one of embodiments 81-155, or the use of any one of embodiments 157-232, wherein the anti-TIGIT antagonist antibody is tiragolumab and the PD-1 axis binding antagonist is atezolizumab, and wherein the treatment results in an increase in PFS of at least about 3.1 months, as compared to treatment with atezolizumab without tiragolumab.
235. The method of embodiment 79, the tiragolumab and atezolizumab of embodiment 156, or the use of embodiment 233, wherein the treatment results in an increase in PFS of at least about 3.1 months, as compared to treatment with atezolizumab without tiragolumab.
236. The method of any one of embodiments 1-78, the anti-TIGIT antagonist antibody of any one of embodiments 81-155, or the use of any one of embodiments 157-232, wherein the anti-TIGIT antagonist antibody is tiragolumab and the PD-1 axis binding antagonist is atezolizumab, and wherein the treatment results in an increase in PFS of at least about 4.9 months, as compared to treatment with atezolizumab without tiragolumab.
237. The method of embodiment 79, the tiragolumab and atezolizumab of embodiment 156, or the use of embodiment 233, wherein the treatment results in an increase in PFS of at least about 4.9 months, as compared to treatment with atezolizumab without tiragolumab.
238. The method of any one of embodiments 1-78, the anti-TIGIT antagonist antibody of any one of embodiments 81-155, or the use of any one of embodiments 157-232, wherein the anti-TIGIT antagonist antibody is tiragolumab and the PD-1 axis binding antagonist is atezolizumab, and wherein the treatment results in an increase in OS of at least about 5.7 months, as compared to treatment with atezolizumab without tiragolumab.
239. The method of embodiment 79, the tiragolumab and atezolizumab of embodiment 156, or the use of embodiment 233, wherein the treatment results in an increase in OS of at least about 5.7 months, as compared to treatment with atezolizumab without tiragolumab.
240. The method of any one of embodiments 1-78, the anti-TIGIT antagonist antibody of any one of embodiments 81-155, or the use of any one of embodiments 157-232, wherein the anti-TIGIT antagonist antibody is tiragolumab and the PD-1 axis binding antagonist is atezolizumab, and wherein the treatment results in an increase in OS of at least about 9 months, as compared to treatment with atezolizumab without tiragolumab.
241. The method of embodiment 79, the tiragolumab and atezolizumab of embodiment 156, or the use of embodiment 233, wherein the treatment results in an increase in OS of at least about 9 months, as compared to treatment with atezolizumab without tiragolumab.

Although the foregoing invention has been described in some detail by way of illustration and example for purposes of clarity of understanding, the descriptions and examples should not be construed as limiting the scope of the invention. The disclosures of all patent and scientific literature cited herein are expressly incorporated in their entirety by reference.
The following numbered paragraphs (paras), describing aspects of the invention, are part of the description:

1. A method for treating a subject having a lung cancer, the method comprising administering to the subject one or more dosing cycles of an anti-TIGIT antagonist antibody and a PD-1 axis binding antagonist, wherein the subject has been determined to have a PD-L1-positive tumor cell fraction of greater than, or equal to, 30%, and the treatment results in (a) a complete response (CR) or a partial response (PR) and/or (b) an increase in progression-free survival (PFS) as compared to treatment with the PD-1 axis binding antagonist without the anti-TIGIT antagonist antibody.

2. The method of para 1, wherein the anti-TIGIT antagonist antibody comprises the following hypervariable regions (HVRs):

an HVR-H1 sequence comprising the amino acid sequence of SNSAAWN (SEQ ID NO: 1);
an HVR-H2 sequence comprising the amino acid sequence of KTYYRFKWYSDYAVSVKG (SEQ ID NO: 2);
an HVR-H3 sequence comprising the amino acid sequence of ESTTYDLLAGPFDY (SEQ ID NO: 3);
an HVR-L1 sequence comprising the amino acid sequence of KSSQTVLYSSNNKKYLA (SEQ ID NO: 4);
an HVR-L2 sequence comprising the amino acid sequence of WASTRES (SEQ ID NO: 5); and
an HVR-L3 sequence comprising the amino acid sequence of QQYYSTPFT (SEQ ID NO: 6).

3. The method of para 2, wherein the anti-TIGIT antagonist antibody further comprises the following light chain variable region framework regions (FRs):

an FR-L1 comprising the amino acid sequence of DIVMTQSPDSLAVSLGERATINC (SEQ ID NO: 7);
an FR-L2 comprising the amino acid sequence of WYQQKPGQPPNLLIY (SEQ ID NO: 8);
an FR-L3 comprising the amino acid sequence of GVPDRFSGSGSGTDFTLTISSLQAEDVAVYYC (SEQ ID NO: 9); and
an FR-L4 comprising the amino acid sequence of FGPGTKVEIK (SEQ ID NO: 10).

4. The method of para 2, wherein the anti-TIGIT antagonist antibody further comprises the following heavy chain variable region FRs:

an FR-H1 comprising the amino acid sequence of $X_1$VQLQQSGPGLVKPSQTLSLTCAISGDSVS (SEQ ID NO: 11), wherein $X_1$ is Q or E;
an FR-H2 comprising the amino acid sequence of WIRQSPSRGLEWLG (SEQ ID NO: 12);
an FR-H3 comprising the amino acid sequence of RITINPDTSKNQFSLQLNSVTPEDTAVFYCTR (SEQ ID NO: 13); and
an FR-H4 comprising the amino acid sequence of WGQGTLVTVSS (SEQ ID NO: 14).

5. The method of para 4, wherein $X_1$ is Q.

6. The method of para 4, wherein $X_1$ is E.

7. The method of any one of paras 2-6, wherein the anti-TIGIT antagonist antibody comprises:

(a) a heavy chain variable (VH) domain comprising an amino acid sequence having at least 95% sequence identity to the amino acid sequence of SEQ ID NO: 17 or 18;
(b) a light chain variable (VL) domain comprising an amino acid sequence having at least 95% sequence identity to the amino acid sequence of SEQ ID NO: 19; or
(c) a VH domain as in (a) and a VL domain as in (b).

8. The method of any one of paras 1-7, wherein the anti-TIGIT antagonist antibody comprises:

a VH domain comprising the amino acid sequence of SEQ ID NO: 17 or 18; and
a VL domain comprising the amino acid sequence of SEQ ID NO: 19.

9. The method of any one of paras 1-8, wherein the anti-TIGIT antagonist antibody is a monoclonal antibody.

10. The method of para 9, wherein the anti-TIGIT antagonist antibody is a human antibody.

11. The method of any one of paras 1-10, wherein the anti-TIGIT antagonist antibody is a full-length antibody.

12. The method of any one of paras 1-4 and 6-11, wherein the anti-TIGIT antagonist antibody is tiragolumab.

13. The method of any one of paras 1-10, wherein the anti-TIGIT antagonist antibody is an antibody fragment that binds TIGIT selected from the group consisting of Fab, Fab', Fab'-SH, Fv, single chain variable fragment (scFv), and $(Fab')_2$ fragments.

14. The method of any one of paras 1-13, wherein the anti-TIGIT antagonist antibody is an IgG class antibody.

15. The method of para 14, wherein the IgG class antibody is an IgG1 subclass antibody.

16. The method of any one of paras 1-15, wherein the PD-1 axis binding antagonist is a PD-L1 binding antagonist or a PD-1 binding antagonist.

17. The method of para 16, wherein the PD-L1 binding antagonist is an anti-PD-L1 antagonist antibody.

18. The method of para 17, wherein the anti-PD-L1 antagonist antibody is atezolizumab (MPDL3280A), MSB0010718C, MDX-1105, or MEDI4736.

19. The method of para 18, wherein the anti-PD-L1 antagonist antibody is atezolizumab.

20. The method of para 16, wherein the PD-1 binding antagonist is an anti-PD-1 antagonist antibody.

21. The method of para 20, wherein the anti-PD-1 antagonist antibody is nivolumab (MDX-1106), pembrolizumab (MK-3475), MED1-0680, spartalizumab (PDR001), cemiplimab (REGN2810), BGB-108, prolgolimab, camrelizumab, sintilimab, tislelizumab, or toripalimab.

22. The method of para 17, wherein the anti-PD-L1 antagonist antibody comprises the following HVRs:

an HVR-H1 sequence comprising the amino acid sequence of GFTFSDSWIH (SEQ ID NO: 20);
an HVR-H2 sequence comprising the amino acid sequence of AWISPYGGSTYYADSVKG (SEQ ID NO: 21);
an HVR-H3 sequence comprising the amino acid sequence of RHWPGGFDY (SEQ ID NO: 22);
an HVR-L1 sequence comprising the amino acid sequence of RASQDVSTAVA (SEQ ID NO: 23);
an HVR-L2 sequence comprising the amino acid sequence of SASFLYS (SEQ ID NO: 24); and
an HVR-L3 sequence comprising the amino acid sequence of QQYLYHPAT (SEQ ID NO: 25).

23. The method of para 22, wherein the anti-PD-L1 antagonist antibody comprises:

(a) a heavy chain variable (VH) domain comprising an amino acid sequence having at least 95% sequence identity to the amino acid sequence of SEQ ID NO: 26;
(b) a light chain variable (VL) domain comprising an amino acid sequence having at least 95% sequence identity to the amino acid sequence of SEQ ID NO: 27; or
(c) a VH domain as in (a) and a VL domain as in (b).

24. The method of para 23, wherein the anti-PD-L1 antagonist antibody comprises:

a VH domain comprising the amino acid sequence of SEQ ID NO: 26; and
a VL domain comprising the amino acid sequence of SEQ ID NO: 27.

25. The method of any one of paras 1-24, wherein the PD-1 axis binding antagonist is a monoclonal antibody.

26. The method of any one of paras 1-25, wherein the PD-1 axis binding antagonist is a humanized antibody.

27. The method of any one of paras 1-26, wherein the PD-1 axis binding antagonist is a full-length antibody.

28. The method of any one of paras 1-26, wherein the PD-1 axis binding antagonist is an antibody fragment that binds PD-L1 selected from the group consisting of Fab, Fab', Fab'-SH, Fv, single chain variable fragment (scFv), and $(Fab')_2$ fragments.

29. The method of any one of paras 1-27, wherein the PD-1 axis binding antagonist is an IgG class antibody.

30. The method of para 29, wherein the IgG class antibody is an IgG1 subclass antibody.

31. The method of any one of paras 1-30, wherein the method comprises administering to the subject the anti-TIGIT antagonist antibody at a fixed dose of between about 30 mg to about 1200 mg every three weeks.

32. The method of para 31, wherein the method comprises administering to the subject the anti-TIGIT antagonist antibody at a fixed dose of about 600 mg every three weeks.

33. The method of any one of paras 1-32, wherein the method comprises administering to the subject the PD-1 axis binding antagonist at a fixed dose of between about 80 mg to about 1600 mg every three weeks.

34. The method of para 33, wherein the method comprises administering to the subject the PD-1 axis binding antagonist at a fixed dose of about 1200 mg every three weeks.

35. The method of any one of paras 1-34, wherein the method comprises administering to the subject the anti-TIGIT antagonist antibody at a fixed dose of about 600 mg every three weeks and the PD-1 axis binding antagonist at a fixed dose of about 1200 mg every three weeks.

36. The method of any one of paras 1-35, wherein the length of each of the one or more dosing cycles is 21 days.

37. The method of any one of paras 1-36, wherein the method comprises administering to the subject the anti-TIGIT antagonist antibody and the PD-1 axis binding antagonist on about Day 1 of each of the one or more dosing cycles.

38. The method of any one of paras 1-30, wherein the method comprises administering to the subject the anti-TIGIT antagonist antibody at a fixed dose of between about 300 mg to about 800 mg every two weeks.

39. The method of para 38, wherein the method comprises administering to the subject the anti-TIGIT antagonist antibody at a fixed dose of between about 400 mg to about 500 mg every two weeks.

40. The method of para 39, wherein the method comprises administering to the subject the anti-TIGIT antagonist antibody at a fixed dose of about 420 mg every two weeks.

41. The method of any one of paras 1-30 and 38-40, wherein the method comprises administering to the subject the PD-1 axis binding antagonist at a fixed dose of between about 200 mg to about 1200 mg every two weeks.

42. The method of para 41, wherein the method comprises administering to the subject the PD-1 axis binding antagonist at a fixed dose of about 840 mg every two weeks.

43. The method of para 42, wherein the method comprises administering to the subject the anti-TIGIT antagonist antibody at a fixed dose of about 420 mg every two weeks and the PD-1 axis binding antagonist at a fixed dose of about 840 mg every two weeks.

44. The method of para 43, wherein the length of each of the one or more dosing cycles is 28 days.

45. The method of para 44, wherein the method comprises administering to the subject the anti-TIGIT antagonist antibody and the PD-1 axis binding antagonist on about Days 1 and 15 of each of the one or more dosing cycles.

46. The method of any one of paras 1-30, wherein the method comprises administering to the subject the anti-TIGIT antagonist antibody at a fixed dose of between about 700 mg to about 1000 mg every four weeks.

47. The method of para 46, wherein the method comprises administering to the subject the anti-TIGIT antagonist antibody at a fixed dose of between about 800 mg to about 900 mg every four weeks.

48. The method of para 47, wherein the method comprises administering to the subject the anti-TIGIT antagonist antibody at a fixed dose of about 840 mg every four weeks.

49. The method of any one of paras 1-30 and 46-48, wherein the method comprises administering to the subject the PD-1 axis binding antagonist at a fixed dose of between about 400 mg to about 2000 mg every four weeks.

50. The method of para 49, wherein the method comprises administering to the subject the PD-1 axis binding antagonist at a fixed dose of about 1680 mg every four weeks.

51. The method of para 50, wherein the method comprises administering to the subject the anti-TIGIT antagonist antibody at a fixed dose of about 840 mg every four weeks and the PD-1 axis binding antagonist at a fixed dose of about 1680 mg every four weeks.

52. The method of any one of paras 1-30 and 46-51, wherein the length of each of the one or more dosing cycles is 28 days.

53. The method of para 52, wherein the method comprises administering to the subject the anti-TIGIT antagonist antibody and the PD-1 axis binding antagonist on about Day 1 of each of the one or more dosing cycles.

54. The method of any one of paras 1-53, wherein the method comprises administering to the subject the PD-1 axis binding antagonist before the anti-TIGIT antagonist antibody.

55. The method of para 54, wherein the method comprises a first observation period following administration of the PD-1 axis binding antagonist and second observation period following administration of the anti-TIGIT antagonist antibody.

56. The method of para 55, wherein the first observation period and the second observation period are each between about 30 minutes to about 60 minutes in length.

57. The method of any one of paras 1-53, wherein the method comprises administering to the subject the anti-TIGIT antagonist antibody before the PD-1 axis binding antagonist.

58. The method of para 57, wherein the method comprises a first observation period following administration of the anti-TIGIT antagonist antibody and second observation period following administration of the PD-1 axis binding antagonist.

59. The method of para 58, wherein the first observation period and the second observation period are each between about 30 minutes to about 60 minutes in length.

60. The method of any one of paras 1-53, wherein the method comprises administering to the subject the anti-TIGIT antagonist antibody and the PD-1 axis binding antagonist simultaneously.

61. The method of any one of paras 1-60, wherein the method comprises administering to the subject the anti-TIGIT antagonist antibody and PD-1 axis binding antagonist intravenously.

62. The method of para 61, wherein the method comprises administering to the subject the anti-TIGIT antagonist antibody by intravenous infusion over 60 $\pm$ 10 minutes.

63. The method of para 61 or 62, wherein the method comprises administering to the subject the PD-1 axis binding antagonist by intravenous infusion over 60 $\pm$ 15 minutes.

64. The method of any one of paras 1-60, wherein the method comprises administering to the subject the anti-TIGIT antagonist antibody and PD-1 axis binding antagonist subcutaneously.

65. The method of any one of paras 1-64, wherein the PD-L1-positive tumor cell fraction has been determined by an immunohistochemical (IHC) assay.

66. The method of any one of paras 1-65, wherein the PD-L1-positive tumor cell fraction is determined by positive staining with an anti-PD-L1 antibody, wherein the anti-PD-L1 antibody is SP263, 22C3, SP142, or 28-8.

67. The method of para 66, wherein the PD-L1-positive tumor cell fraction is greater than, or equal to, 50%, as determined by positive staining with the anti-PD-L1 antibody SP263.

68. The method of para 67, wherein the PD-L1-positive tumor cell fraction is calculated using the Ventana SP263 IHC assay.

69. The method of para 66, wherein the PD-L1-positive tumor cell fraction is greater than, or equal to, 50%, as determined by positive staining with the anti-PD-L1 antibody 22C3.

70. The method of para 69, wherein the PD-L1-positive tumor cell fraction is calculated using the pharmDx 22C3 IHC assay.

71. The method of para 66, wherein the PD-L1-positive tumor cell fraction is greater than, or equal to, 30%, as determined by positive staining with the anti-PD-L1 antibody SP142.

72. The method of para 66, wherein the PD-L1-positive tumor cell fraction is greater than, or equal to, 50%, as determined by positive staining with the anti-PD-L1 antibody 28-8.

73. The method of any one of paras 1-72, wherein a tumor sample obtained from the subject has been determined to have a detectable nucleic acid expression level of PD-L1.

74. The method of para 73, wherein the detectable nucleic acid expression level of PD-L1 has been determined by RNA-seq, RT-qPCR, qPCR, multiplex qPCR or RT-qPCR, microarray analysis, SAGE, MassARRAY technique, ISH, or a combination thereof.

75. The method of any one of paras 1-74, wherein the lung cancer is a non-small cell lung cancer (NSCLC).

76. The method of para 75, wherein the NSCLC is a squamous NSCLC.

77. The method of para 76, wherein the NSCLC is a non-squamous NSCLC.

78. The method of any one of paras 75-77, wherein the NSCLC is a locally advanced unresectable NSCLC.

79. The method of para 78, wherein the NSCLC is a Stage IIIB NSCLC.

80. The method of any one of paras 75-77, wherein the NSCLC is a recurrent or metastatic NSCLC.

81. The method of para 80, wherein the NSCLC is a Stage IV NSCLC.

82. The method of para 80 or 81, wherein the subject has not been previously treated for Stage IV NSCLC.

83. The method of any one of paras 1-82, wherein the subject does not have a sensitizing epidermal growth factor receptor *(EGFR)* gene mutation or anaplastic lymphoma kinase *(ALK)* gene rearrangement.

84. The method of any one of paras 1-83, wherein the subject does not have a pulmonary lymphoepithelioma-like carcinoma subtype of NSCLC.

85. The method of any one of paras 1-84, wherein the subject does not have an active Epstein-Barr virus (EBV) infection or a known or suspected chronic active EBV infection.

86. The method of any one of paras 1-85, wherein the subject is negative for EBV IgM or negative by EBV PCR.

87. The method of para 86, wherein the subject is negative for EBV IgM and negative by EBV PCR.

88. The method of para 86 or 87, wherein the subject is positive for EBV IgG or positive for Epstein-Barr nuclear antigen (EBNA).

89. The method of para 88, wherein the subject is positive for EBV IgG and positive for EBNA.

90. The method of any one of paras 1-89, wherein the subject is negative for EBV IgG or negative for EBNA.

91. The method of para 90, wherein the subject is negative for EBV IgG and negative for EBNA.

92. The method of any one of paras 1-91, wherein the PFS is increased as compared to a reference PFS time.

93. The method of para 92, wherein the reference PFS time is the median PFS time of a population of subjects who have received a treatment comprising a PD-1 axis binding antagonist without an anti-TIGIT antagonist antibody.

94. A method for treating a subject having a NSCLC, the method comprising administering to the subject one or more dosing cycles of an anti-TIGIT antagonist antibody and atezolizumab, wherein the anti-TIGIT antagonist antibody comprises:

a VH domain comprising the amino acid sequence of SEQ ID NO: 17 or 18; and
a VL domain comprising the amino acid sequence of SEQ ID NO: 19, and wherein the subject has been determined to have a PD-L1-positive tumor cell fraction of greater than, or equal to, 30%, and the treatment results in (a) a CR or a PR and/or (b) an increase in PFS as compared to treatment with atezolizumab without the anti-TIGIT antagonist antibody.

95. The method of para 94, wherein the anti-TIGIT antagonist antibody is administered at a fixed dose of 600 mg every three weeks and atezolizumab is administered at a fixed dose of 1200 mg every three weeks.

96. The method of para 94, wherein the anti-TIGIT antagonist antibody is administered at a fixed dose of 420 mg every two weeks and atezolizumab is administered at a fixed dose of 840 mg every two weeks.

97. The method of para 94, wherein the anti-TIGIT antagonist antibody is administered at a fixed dose of 840 mg every four weeks and atezolizumab is administered at a fixed dose of 1680 mg every four weeks.

98. A method of treating a subject having a NSCLC, the method comprising:

(a) obtaining a tumor sample from the subject;
(b) detecting the protein expression level of PD-L1 in the tumor sample by staining tumor cells from the tumor sample with anti-PD-L1 antibody SP263 and determining a percentage of PD-L1-positive tumor cells therefrom, wherein 50% or more of the tumor cells stained with the anti-PD-L1 antibody SP263 are PD-L1-positive tumor cells; and
(c) administering to the subject a therapy comprising one or more dosing cycles of an anti-TIGIT antagonist antibody and atezolizumab, wherein the anti-TIGIT antagonist antibody comprises:

a VH domain comprising the amino acid sequence of SEQ ID NO: 17 or 18; and
a VL domain comprising the amino acid sequence of SEQ ID NO: 19, and
wherein the treatment results in (a) a CR or a PR and/or (b) an increase in PFS as compared to treatment with atezolizumab without the anti-TIGIT antagonist antibody.

99. The method of para 98, wherein the anti-TIGIT antagonist antibody is administered at a fixed dose of 600 mg every three weeks and atezolizumab is administered at a fixed dose of 1200 mg every three weeks.

100. The method of para 98, wherein the anti-TIGIT antagonist antibody is administered at a fixed dose of 420 mg every two weeks and atezolizumab is administered at a fixed dose of 840 mg every two weeks.

101. The method of para 98, wherein the anti-TIGIT antagonist antibody is administered at a fixed dose of 840 mg every four weeks and atezolizumab is administered at a fixed dose of 1680 mg every four weeks.

102. A method for treating a subject having a NSCLC, the method comprising administering to the subject one or more dosing cycles of tiragolumab and atezolizumab, wherein the subject has been determined to have a PD-L1-positive tumor cell fraction of greater than, or equal to, 30%, and the treatment results in (a) a CR or a PR and/or (b) an increase in PFS as compared to treatment with atezolizumab without tiragolumab.

103. The method of para 102, wherein tiragolumab is administered at a fixed dose of 600 mg every three weeks and atezolizumab is administered at a fixed dose of 1200 mg every three weeks.

104. The method of para 102, wherein tiragolumab is administered at a fixed dose of 420 mg every two weeks and atezolizumab is administered at a fixed dose of 840 mg every two weeks.

105. The method of para 102, wherein tiragolumab is administered at a fixed dose of 840 mg every four weeks and atezolizumab is administered at a fixed dose of 1680 mg every four weeks.

106. A method of treating a subject having a NSCLC, the method comprising:

(a) obtaining a tumor sample from the subject;
(b) detecting the protein expression level of PD-L1 in the tumor sample by an IHC assay using anti-PD-L1 antibody SP263 and determining a PD-L1-positive tumor cell fraction therefrom, wherein the PD-L1-positive tumor cell fraction is determined to be greater than, or equal to, 50%; and
(c) administering to the subject a therapy comprising one or more dosing cycles of an anti-TIGIT antagonist antibody and atezolizumab, wherein the anti-TIGIT antagonist antibody comprises:

a VH domain comprising the amino acid sequence of SEQ ID NO: 17 or 18; and
a VL domain comprising the amino acid sequence of SEQ ID NO: 19, and
wherein the treatment results in (a) a CR or a PR and/or (b) an increase in PFS as compared to treatment with atezolizumab without the anti-TIGIT antagonist antibody.

107. The method of para 106, wherein the anti-TIGIT antagonist antibody is administered at a fixed dose of 600 mg every three weeks and atezolizumab is administered at a fixed dose of 1200 mg every three weeks.

108. The method of para 106, wherein the anti-TIGIT antagonist antibody is administered at a fixed dose of 420 mg every two weeks and atezolizumab is administered at a fixed dose of 840 mg every two weeks.

109. The method of para 106, wherein the anti-TIGIT antagonist antibody is administered at a fixed dose of 840 mg every four weeks and atezolizumab is administered at a fixed dose of 1680 mg every four weeks.

110. An anti-TIGIT antagonist antibody and a PD-1 axis binding antagonist for use in a method of treating a subject having a lung cancer, wherein the method comprises administering to the subject one or more dosing cycles of the anti-TIGIT antagonist, wherein the subject has been determined to have a PD-L1-positive tumor cell fraction of greater than, or equal to, 30%, and the treatment results in (a) a CR or a PR and/or (b) an increase in PFS as compared to treatment with the PD-1 axis binding antagonist without the anti-TIGIT antagonist antibody.

111. The anti-TIGIT antagonist antibody and PD-1 axis binding antagonist for use of para 110, wherein the anti-TIGIT antagonist antibody comprises the following HVRs:

an HVR-H1 sequence comprising the amino acid sequence of SNSAAWN (SEQ ID NO: 1);
an HVR-H2 sequence comprising the amino acid sequence of KTYYRFKWYSDYAVSVKG (SEQ ID NO: 2);
an HVR-H3 sequence comprising the amino acid sequence of ESTTYDLLAGPFDY (SEQ ID NO: 3);
an HVR-L1 sequence comprising the amino acid sequence of KSSQTVLYSSNNKKYLA (SEQ ID NO: 4);
an HVR-L2 sequence comprising the amino acid sequence of WASTRES (SEQ ID NO: 5); and
an HVR-L3 sequence comprising the amino acid sequence of QQYYSTPFT (SEQ ID NO: 6).

112. The anti-TIGIT antagonist antibody and PD-1 axis binding antagonist for use of para 111, wherein the anti-TIGIT antagonist antibody further comprises the following light chain variable region FRs:

an FR-L1 comprising the amino acid sequence of DIVMTQSPDSLAVSLGERATINC (SEQ ID NO: 7);
an FR-L2 comprising the amino acid sequence of WYQQKPGQPPNLLIY (SEQ ID NO: 8);
an FR-L3 comprising the amino acid sequence of GVPDRFSGSGSGTDFTLTISSLQAEDVAVYYC (SEQ ID NO: 9); and
an FR-L4 comprising the amino acid sequence of FGPGTKVEIK (SEQ ID NO: 10).

113. The anti-TIGIT antagonist antibody and PD-1 axis binding antagonist for use of para 111, wherein the anti-TIGIT antagonist antibody further comprises the following heavy chain variable region FRs:

an FR-H1 comprising the amino acid sequence of $X_1$VQLQQSGPGLVKPSQTLSLTCAISGDSVS (SEQ ID NO: 11), wherein $X_1$ is Q or E;
an FR-H2 comprising the amino acid sequence of WIRQSPSRGLEWLG (SEQ ID NO: 12);
an FR-H3 comprising the amino acid sequence of RITINPDTSKNQFSLQLNSVTPEDTAVFYCTR (SEQ ID NO:

13); and
an FR-H4 comprising the amino acid sequence of WGQGTLVTVSS (SEQ ID NO: 14).

114. The anti-TIGIT antagonist antibody and PD-1 axis binding antagonist for use of para 113, wherein $X_1$ is Q.

115. The anti-TIGIT antagonist antibody and PD-1 axis binding antagonist of para 113, wherein $X_1$ is E.

116. The anti-TIGIT antagonist antibody and PD-1 axis binding antagonist for use of any one of paras 111-115, wherein the anti-TIGIT antagonist antibody comprises:

(a) a heavy chain variable (VH) domain comprising an amino acid sequence having at least 95% sequence identity to the amino acid sequence of SEQ ID NO: 17 or 18;
(b) a light chain variable (VL) domain comprising an amino acid sequence having at least 95% sequence identity to the amino acid sequence of SEQ ID NO: 19; or
(c) a VH domain as in (a) and a VL domain as in (b).

117. The anti-TIGIT antagonist antibody and PD-1 axis binding antagonist for use of any one of paras 110-116, wherein the anti-TIGIT antagonist antibody comprises:

a VH domain comprising the amino acid sequence of SEQ ID NO: 17 or 18; and
a VL domain comprising the amino acid sequence of SEQ ID NO: 19.

118. The anti-TIGIT antagonist antibody and PD-1 axis binding antagonist for use of any one of paras 110-117, wherein the anti-TIGIT antagonist antibody is a monoclonal antibody.

119. The anti-TIGIT antagonist antibody and PD-1 axis binding antagonist for use of para 118, wherein the anti-TIGIT antagonist antibody is a human antibody.

120. The anti-TIGIT antagonist antibody and PD-1 axis binding antagonist for use of any one of paras 110-119, wherein the anti-TIGIT antagonist antibody is a full-length antibody.

121. The anti-TIGIT antagonist antibody and PD-1 axis binding antagonist for use of any one of paras 110-113 and 115-120, wherein the anti-TIGIT antagonist antibody is tiragolumab.

122. The anti-TIGIT antagonist antibody and PD-1 axis binding antagonist for use of any one of paras 110-119, wherein the anti-TIGIT antagonist antibody is an antibody fragment that binds TIGIT selected from the group consisting of Fab, Fab', Fab'-SH, Fv, single chain variable fragment (scFv), and $(Fab')_2$ fragments.

123. The anti-TIGIT antagonist antibody and PD-1 axis binding antagonist for use of any one of paras 110-122, wherein the anti-TIGIT antagonist antibody is an IgG class antibody.

124. The anti-TIGIT antagonist antibody and PD-1 axis binding antagonist for use of para 123, wherein the IgG class antibody is an IgG1 subclass antibody.

125. The anti-TIGIT antagonist antibody and PD-1 axis binding antagonist for use of any one of paras 110-124, wherein the PD-1 axis binding antagonist is a PD-L1 binding antagonist or a PD-1 binding antagonist.

126. The anti-TIGIT antagonist antibody and PD-1 axis binding antagonist of para 125, wherein the PD-L1 binding antagonist is an anti-PD-L1 antagonist antibody.

127. The anti-TIGIT antagonist antibody and PD-1 axis binding antagonist of para 126, wherein the anti-PD-L1 antagonist antibody is atezolizumab (MPDL3280A), MSB0010718C, MDX-1105, or MEDI4736.

128. The anti-TIGIT antagonist antibody and anti-PD-L1 antagonist antibody for use of para 127, wherein the anti-PD-L1 antagonist antibody is atezolizumab.

129. The anti-TIGIT antagonist antibody and PD-1 axis binding antagonist of para 125, wherein the PD-1 binding antagonist is an anti-PD-1 antagonist antibody.

130. The anti-TIGIT antagonist antibody and PD-1 axis binding antagonist of para 129, wherein the anti-PD-1 antagonist antibody is nivolumab (MDX-1106), pembrolizumab (MK-3475), MED1-0680, spartalizumab (PDR001), cemiplimab (REGN2810), BGB-108, prolgolimab, camrelizumab, sintilimab, tislelizumab, or toripalimab.

131. The anti-TIGIT antagonist antibody and PD-1 axis binding antagonist for use of para 126, wherein the anti-PD-L1 antagonist antibody comprises the following HVRs:

an HVR-H1 sequence comprising the amino acid sequence of GFTFSDSWIH (SEQ ID NO: 20);
an HVR-H2 sequence comprising the amino acid sequence of AWISPYGGSTYYADSVKG (SEQ ID NO: 21);
an HVR-H3 sequence comprising the amino acid sequence of RHWPGGFDY (SEQ ID NO: 22);
an HVR-L1 sequence comprising the amino acid sequence of RASQDVSTAVA (SEQ ID NO: 23);
an HVR-L2 sequence comprising the amino acid sequence of SASFLYS (SEQ ID NO: 24); and
an HVR-L3 sequence comprising the amino acid sequence of QQYLYHPAT (SEQ ID NO: 25).

132. The anti-TIGIT antagonist antibody and PD-1 axis binding antagonist for use of para 131, wherein the anti-PD-L1 antagonist antibody comprises:

(a) a heavy chain variable (VH) domain comprising an amino acid sequence having at least 95% sequence identity to the amino acid sequence of SEQ ID NO: 26;
(b) a light chain variable (VL) domain comprising an amino acid sequence having at least 95% sequence identity to the amino acid sequence of SEQ ID NO: 27; or
(c) a VH domain as in (a) and a VL domain as in (b).

133. The anti-TIGIT antagonist antibody and PD-1 axis binding antagonist for use of para 132, wherein the anti-PD-L1 antagonist antibody comprises:

a VH domain comprising the amino acid sequence of SEQ ID NO: 26; and
a VL domain comprising the amino acid sequence of SEQ ID NO: 27.

134. The anti-TIGIT antagonist antibody and PD-1 axis binding antagonist for use of any one of paras 110-133, wherein the PD-1 axis binding antagonist is a monoclonal antibody.

135. The anti-TIGIT antagonist antibody and PD-1 axis binding antagonist for use of para 134, wherein the PD-1 axis binding antagonist is a humanized antibody.

136. The anti-TIGIT antagonist antibody and PD-1 axis binding antagonist for use of para 134 or 135, wherein the PD-1 axis binding antagonist is a full-length antibody.

137. The anti-TIGIT antagonist antibody and PD-1 axis binding antagonist for use of any one of paras 110-135, wherein the PD-1 axis binding antagonist is an antibody fragment that binds PD-L1 selected from the group consisting of Fab, Fab', Fab'-SH, Fv, single chain variable fragment (scFv), and $(Fab')_2$ fragments.

138. The anti-TIGIT antagonist antibody and PD-1 axis binding antagonist for use of any one of paras 110-136, wherein the PD-1 axis binding antagonist is an IgG class antibody.

139. The anti-TIGIT antagonist antibody and PD-1 axis binding antagonist for use of para 138, wherein the IgG class antibody is an IgG1 subclass antibody.

140. The anti-TIGIT antagonist antibody and PD-1 axis binding antagonist for use of any one of paras 110-139, wherein the method comprises administering to the subject the anti-TIGIT antagonist antibody at a fixed dose of between about 30 mg to about 1200 mg every three weeks.

141. The anti-TIGIT antagonist antibody and PD-1 axis binding antagonist for use of para 140, wherein the method comprises administering to the subject the anti-TIGIT antagonist antibody at a fixed dose of between about 30 mg to about 600 mg every three weeks.

142. The anti-TIGIT antagonist antibody and PD-1 axis binding antagonist for use of para 141, wherein the method comprises administering to the subject the anti-TIGIT antagonist antibody at a fixed dose of about 600 mg every three weeks.

143. The anti-TIGIT antagonist antibody and PD-1 axis binding antagonist for use of any one of paras 110-142, wherein the method comprises administering to the subject the PD-1 axis binding antagonist at a fixed dose of between about 80 mg to about 1600 mg every three weeks.

144. The anti-TIGIT antagonist antibody and PD-1 axis binding antagonist for use of para 143, wherein the method comprises administering to the subject the PD-1 axis binding antagonist at a fixed dose of about 1200 mg every three weeks.

145. The anti-TIGIT antagonist antibody and PD-1 axis binding antagonist for use of para 144, wherein the anti-TIGIT antagonist antibody is to be administered to the subject at a fixed dose of about 600 mg every three weeks and the PD-1 axis binding antagonist is to be administered to the subject at a fixed dose of about 1200 mg every three weeks.

146. The anti-TIGIT antagonist antibody and PD-1 axis binding antagonist for use of any one of paras 110-145, wherein the length of each of the one or more dosing cycles is 21 days.

147. The anti-TIGIT antagonist antibody and PD-1 axis binding antagonist for use of para 146, wherein the anti-TIGIT antagonist antibody and PD-1 axis binding antagonist are to be administered to the subject on about Day 1 of each of the one or more dosing cycles.

148. The anti-TIGIT antagonist antibody and PD-1 axis binding antagonist for use of any one of paras 110-139, wherein the method comprises administering to the subject the anti-TIGIT antagonist antibody at a fixed dose of between about 300 mg to about 800 mg every two weeks.

149. The anti-TIGIT antagonist antibody and PD-1 axis binding antagonist for use of para 148, wherein the method comprises administering to the subject the anti-TIGIT antagonist antibody at a fixed dose of between about 400 mg to about 500 mg every two weeks.

150. The anti-TIGIT antagonist antibody and PD-1 axis binding antagonist for use of para 149, wherein the method comprises administering to the subject the anti-TIGIT antagonist antibody at a fixed dose of about 420 mg every two weeks.

151. The anti-TIGIT antagonist antibody and PD-1 axis binding antagonist for use of any one of paras 110-139 and 148-150, wherein the method comprises administering to the subject the PD-1 axis binding antagonist at a fixed dose of between about 200 mg to about 1200 mg every two weeks.

152. The anti-TIGIT antagonist antibody and PD-1 axis binding antagonist for use of para 151, wherein the method comprises administering to the subject the PD-1 axis binding antagonist at a fixed dose of about 840 mg every two weeks.

153. The anti-TIGIT antagonist antibody and PD-1 axis binding antagonist for use of para 152, wherein the method comprises administering to the subject the anti-TIGIT antagonist antibody at a fixed dose of about 420 mg every two weeks and the PD-1 axis binding antagonist at a fixed dose of about 840 mg every two weeks.

154. The anti-TIGIT antagonist antibody and PD-1 axis binding antagonist for use of para 153, wherein the length of each of the one or more dosing cycles is 28 days.

155. The anti-TIGIT antagonist antibody and PD-1 axis binding antagonist for use of para 154, wherein the method comprises administering to the subject the anti-TIGIT antagonist antibody and the PD-1 axis binding antagonist on about Days 1 and 15 of each of the one or more dosing cycles.

156. The anti-TIGIT antagonist antibody and PD-1 axis binding antagonist for use of any one of paras 110-139, wherein the method comprises administering to the subject the anti-TIGIT antagonist antibody at a fixed dose of between about 700 mg to about 1000 mg every four weeks.

157. The anti-TIGIT antagonist antibody and PD-1 axis binding antagonist for use of para 156, wherein the method comprises administering to the subject the anti-TIGIT antagonist antibody at a fixed dose of between about 800 mg to about 900 mg every four weeks.

158. The anti-TIGIT antagonist antibody and PD-1 axis binding antagonist for use of para 157, wherein the method comprises administering to the subject the anti-TIGIT antagonist antibody at a fixed dose of about 840 mg every four weeks.

159. The anti-TIGIT antagonist antibody and PD-1 axis binding antagonist for use of any one of paras 110-139 and 156-158, wherein the method comprises administering to the subject the PD-1 axis binding antagonist at a fixed dose of between about 400 mg to about 2000 mg every four weeks.

160. The anti-TIGIT antagonist antibody and PD-1 axis binding antagonist for use of para 159, wherein the method comprises administering to the subject the PD-1 axis binding antagonist at a fixed dose of about 1680 mg every four weeks.

161. The anti-TIGIT antagonist antibody and PD-1 axis binding antagonist for use of para 160, wherein the method comprises administering to the subject the anti-TIGIT antagonist antibody at a fixed dose of about 840 mg every four weeks and the PD-1 axis binding antagonist at a fixed dose of about 1680 mg every four weeks.

162. The anti-TIGIT antagonist antibody and PD-1 axis binding antagonist for use of any one of paras 110-139 and 156-161, wherein the length of each of the one or more dosing cycles is 28 days.

163. The anti-TIGIT antagonist antibody and PD-1 axis binding antagonist for use of para 162, wherein the method comprises administering to the subject the anti-TIGIT antagonist antibody and the PD-1 axis binding antagonist on about Day 1 of each of the one or more dosing cycles.

164. The anti-TIGIT antagonist antibody and PD-1 axis binding antagonist for use of any one of paras 110-163, wherein the PD-1 axis binding antagonist is to be administered to the subject before the anti-TIGIT antagonist antibody.

165. The anti-TIGIT antagonist antibody and PD-1 axis binding antagonist for use of para 164, wherein a first observation period is to follow administration of the PD-1 axis binding antagonist and second observation period is to follow administration of the anti-TIGIT antagonist antibody.

166. The anti-TIGIT antagonist antibody and PD-1 axis binding antagonist for use of para 165, wherein the first observation period and the second observation period are each between about 30 minutes to about 60 minutes in length.

167. The anti-TIGIT antagonist antibody and PD-1 axis binding antagonist for use of any one of paras 110-163, wherein the anti-TIGIT antagonist antibody is to be administered to the subject before the PD-1 axis binding antagonist.

168. The anti-TIGIT antagonist antibody and PD-1 axis binding antagonist for use of para 167, wherein a first observation period is to follow administration of the anti-TIGIT antagonist antibody and second observation period is to follow administration of the PD-1 axis binding antagonist.

169. The anti-TIGIT antagonist antibody and PD-1 axis binding antagonist for use of para 168, wherein the first observation period and the second observation period are each between about 30 minutes to about 60 minutes in length.

170. The anti-TIGIT antagonist antibody and PD-1 axis binding antagonist for use of any one of paras 110-163, wherein the anti-TIGIT antagonist antibody is to be administered to the subject simultaneously with the PD-1 axis binding antagonist.

171. The anti-TIGIT antagonist antibody and PD-1 axis binding antagonist for use of any one of paras 110-170, wherein the anti-TIGIT antagonist antibody and PD-1 axis binding antagonist are to be administered to the subject intravenously.

172. The anti-TIGIT antagonist antibody and PD-1 axis binding antagonist for use of para 171, wherein the anti-TIGIT antagonist antibody is to be administered to the subject by intravenous infusion over 60 ± 10 minutes.

173. The anti-TIGIT antagonist antibody and PD-1 axis binding antagonist for use of para 171 or 172, wherein the PD-1 axis binding antagonist is to be administered to the subject by intravenous infusion over 60 ± 15 minutes.

174. The anti-TIGIT antagonist antibody and PD-1 axis binding antagonist for use of any one of paras 110-170, wherein the anti-TIGIT antagonist antibody and PD-1 axis binding antagonist are to be administered to the subject subcutaneously.

175. The anti-TIGIT antagonist antibody and PD-1 axis binding antagonist for use of any one of paras 110-174, wherein the PD-L1-positive tumor cell fraction is determined by positive staining with an anti-PD-L1 antibody, wherein the anti-PD-L1 antibody is SP263, 22C3, SP142, or 28-8.

176. The anti-TIGIT antagonist antibody and PD-1 axis binding antagonist for use of para 175, wherein the staining is part of an immunohistochemical (IHC) assay.

177. The anti-TIGIT antagonist antibody and PD-1 axis binding antagonist for use of para 175 or 176, wherein the PD-L1-positive tumor cell fraction is greater than, or equal to, 50%, as determined by positive staining with the anti-PD-L1 antibody SP263, 22C3, or 28-8.

178. The anti-TIGIT antagonist antibody and PD-1 axis binding antagonist for use of para 177, wherein the PD-L1-positive tumor cell fraction is determined by positive staining with the anti-PD-L1 antibody SP263 and using the Ventana SP263 IHC assay.

179. The anti-TIGIT antagonist antibody and PD-1 axis binding antagonist for use of para 177, wherein the PD-L1-positive tumor cell fraction is determined by positive staining with the anti-PD-L1 antibody 22C3 and using the pharmDx 22C3 IHC assay.

180. The anti-TIGIT antagonist antibody and PD-1 axis binding antagonist for use of any one of paras 110-179, wherein a tumor sample obtained from the subject has been determined to have a detectable nucleic acid expression level of PD-L1.

181. The anti-TIGIT antagonist antibody and PD-1 axis binding antagonist for use of para 180, wherein the detectable nucleic acid expression level of PD-L1 has been determined by RNA-seq, RT-qPCR, qPCR, multiplex qPCR or RT-qPCR, microarray analysis, SAGE, MassARRAY technique, ISH, or a combination thereof.

182. The anti-TIGIT antagonist antibody and PD-1 axis binding antagonist for use of any one of paras 110-181, wherein the subject has been determined to have a PD-L1-positive tumor cell fraction of greater than, or equal to, 50%, as determined by positive staining with the anti-PD-L1 antibody SP263, 22C3, or 28-8.

183. The anti-TIGIT antagonist antibody and PD-1 axis binding antagonist for use of any one of paras 110-182, wherein the lung cancer is a non-small cell lung cancer (NSCLC).

184. The anti-TIGIT antagonist antibody and PD-1 axis binding antagonist for use of para 183, wherein the NSCLC is a squamous NSCLC.

185. The anti-TIGIT antagonist antibody and PD-1 axis binding antagonist for use of para 184, wherein the NSCLC is a non-squamous NSCLC.

186. The anti-TIGIT antagonist antibody and PD-1 axis binding antagonist for use of any one of paras 183-185, wherein the NSCLC is a locally advanced unresectable NSCLC.

187. The anti-TIGIT antagonist antibody and PD-1 axis binding antagonist for use of para 186, wherein the NSCLC is a Stage IIIB NSCLC.

188. The anti-TIGIT antagonist antibody and PD-1 axis binding antagonist for use of any one of paras 183-185, wherein the NSCLC is a recurrent or metastatic NSCLC.

189. The anti-TIGIT antagonist antibody and PD-1 axis binding antagonist for use of para 188, wherein the NSCLC is a Stage IV NSCLC.

190. The anti-TIGIT antagonist antibody and PD-1 axis binding antagonist for use of para 188 or 189, wherein the subject has not been previously treated for Stage IV NSCLC.

191. The anti-TIGIT antagonist antibody and PD-1 axis binding antagonist for use of any one of paras 110-190, wherein the subject does not have a sensitizing epidermal growth factor receptor *(EGFR)* gene mutation or anaplastic lymphoma kinase *(ALK)* gene rearrangement.

192. The anti-TIGIT antagonist antibody and PD-1 axis binding antagonist for use of any one of paras 110-191, wherein the subject does not have a pulmonary lymphoepithelioma-like carcinoma subtype of NSCLC.

193. The anti-TIGIT antagonist antibody and PD-1 axis binding antagonist for use of any one of paras 110-192, wherein the subject does not have an active EBV infection or a known or suspected chronic active EBV infection.

194. The anti-TIGIT antagonist antibody and PD-1 axis binding antagonist for use of any one of paras 110-193, wherein the subject is negative for EBV IgM or negative by EBV PCR.

195. The anti-TIGIT antagonist antibody and PD-1 axis binding antagonist for use of para 194, wherein the subject is negative for EBV IgM and negative by EBV PCR.

196. The anti-TIGIT antagonist antibody and PD-1 axis binding antagonist for use of para 194 or 195, wherein the subject is positive for EBV IgG or positive for EBNA.

197. The anti-TIGIT antagonist antibody and PD-1 axis binding antagonist for use of para 196, wherein the subject is positive for EBV IgG and positive for EBNA.

198. The anti-TIGIT antagonist antibody and PD-1 axis binding antagonist for use of any one of paras 110-195, wherein the subject is negative for EBV IgG or negative for EBNA.

199. The anti-TIGIT antagonist antibody and PD-1 axis binding antagonist for use of para 198, wherein the subject is negative for EBV IgG and negative for EBNA.

200. The anti-TIGIT antagonist antibody and PD-1 axis binding antagonist for use of any one of paras 110-199, wherein the PFS is increased as compared to a reference PFS time.

201. The anti-TIGIT antagonist antibody and PD-1 axis binding antagonist for use of para 200, wherein the reference PFS time is the median PFS time of a population of subjects who have received a treatment comprising a PD-1 axis binding antagonist without an anti-TIGIT antagonist antibody.

202. An anti-TIGIT antagonist antibody and atezolizumab for use in a method of treating a subject having a NSCLC, wherein the method comprises administering to the subject one or more dosing cycles of an anti-TIGIT antagonist antibody and atezolizumab, wherein the anti-TIGIT antagonist antibody comprises:

a VH domain comprising the amino acid sequence of SEQ ID NO: 17 or 18; and
a VL domain comprising the amino acid sequence of SEQ ID NO: 19, and wherein the subject has been determined to have a PD-L1-positive tumor cell fraction of greater than, or equal to, 30%, and the treatment results in (a) a CR or a PR and/or (b) an increase in PFS as compared to treatment with atezolizumab without the anti-TIGIT antagonist antibody.

203. The anti-TIGIT antagonist antibody and atezolizumab for use of para 202, wherein the anti-TIGIT antagonist antibody is administered at a fixed dose of 600 mg every three weeks and atezolizumab is administered at a fixed dose of 1200 mg every three weeks.

204. The anti-TIGIT antagonist antibody and atezolizumab for use of para 202, wherein the anti-TIGIT antagonist antibody is administered at a fixed dose of 420 mg every two weeks and atezolizumab is administered at a fixed dose of 840 mg every two weeks.

205. The anti-TIGIT antagonist antibody and atezolizumab for use of para 202, wherein the anti-TIGIT antagonist antibody is administered at a fixed dose of 840 mg every four weeks and atezolizumab is administered at a fixed dose of 1680 mg every four weeks.

206. Tiragolumab and atezolizumab for use in a method of treating a subject having a NSCLC, wherein the method comprises administering to the subject one or more dosing cycles of tiragolumab and atezolizumab, and wherein the subject has been determined to have a PD-L1-positive tumor cell fraction of greater than, or equal to, 30%, and the treatment results in (a) a CR or a PR and/or (b) an increase in PFS as compared to treatment with atezolizumab without tiragolumab.

207. The tiragolumab and atezolizumab for use of para 206, wherein tiragolumab is administered at a fixed dose of 600 mg every three weeks and atezolizumab is administered at a fixed dose of 1200 mg every three weeks.

208. The tiragolumab and atezolizumab for use of para 206, wherein tiragolumab is administered at a fixed dose of 420 mg every two weeks and atezolizumab is administered at a fixed dose of 840 mg every two weeks.

209. The tiragolumab and atezolizumab for use of para 206, wherein tiragolumab is administered at a fixed dose of 840 mg every four weeks and atezolizumab is administered at a fixed dose of 1680 mg every four weeks.

210. Use of an anti-TIGIT antagonist antibody and a PD-1 axis binding antagonist in the manufacture of a medicament for use in a method of treating a subject having a lung cancer, wherein the method comprises administering to the subject one or more dosing cycles of the medicament, and wherein the medicament is formulated for administration of the anti-TIGIT antagonist antibody and the PD-1 axis binding antagonist, wherein the subject has been determined to have a PD-L1-positive tumor cell fraction of greater than, or equal to, 30%, and the treatment results in (a) a CR or a PR and/or (b) an increase in PFS as compared to treatment with the PD-1 axis binding antagonist without the anti-TIGIT antagonist antibody.

211. Use of an anti-TIGIT antagonist antibody in the manufacture of a medicament for use in a method of treating a subject having a lung cancer, wherein the method comprises administering to the subject one or more dosing cycles of the medicament and a PD-1 axis binding antagonist, and wherein the medicament is formulated for administration of the anti-TIGIT antagonist antibody and the PD-1 axis binding antagonist, wherein the subject has been determined to have a PD-L1-positive tumor cell fraction of greater than, or equal to, 30%, and the treatment results in (a) a CR or a PR and/or (b) an increase in PFS as compared to treatment with the PD-1 axis binding antagonist without the anti-TIGIT antagonist antibody.

212. Use of a PD-1 axis binding antagonist in the manufacture of a medicament for use in a method of treating a subject having a lung cancer, wherein the method comprises administering to the subject one or more dosing cycles of the medicament and an anti-TIGIT antagonist antibody, and wherein the medicament is formulated for administration of the PD-1 axis binding antagonist and the anti-TIGIT antagonist antibody, wherein the subject has been determined to have a PD-L1-positive tumor cell fraction of greater than, or equal to, 30%, and the treatment results in (a) a CR or a PR and/or (b) an increase in PFS as compared to treatment with the PD-1 axis binding antagonist without the anti-TIGIT antagonist antibody.

213. The use of any one of paras 210-212, wherein the anti-TIGIT antagonist antibody comprises the following hypervariable regions (HVRs):

an HVR-H1 sequence comprising the amino acid sequence of SNSAAWN (SEQ ID NO: 1);
an HVR-H2 sequence comprising the amino acid sequence of KTYYRFKWYSDYAVSVKG (SEQ ID NO: 2);
an HVR-H3 sequence comprising the amino acid sequence of ESTTYDLLAGPFDY (SEQ ID NO: 3);
an HVR-L1 sequence comprising the amino acid sequence of KSSQTVLYSSNNKKYLA (SEQ ID NO: 4);
an HVR-L2 sequence comprising the amino acid sequence of WASTRES (SEQ ID NO: 5); and
an HVR-L3 sequence comprising the amino acid sequence of QQYYSTPFT (SEQ ID NO: 6).

214. The use of para 213, wherein the anti-TIGIT antagonist antibody further comprises the following light chain variable region framework regions (FRs):

an FR-L1 comprising the amino acid sequence of DIVMTQSPDSLAVSLGERATINC (SEQ ID NO: 7);
an FR-L2 comprising the amino acid sequence of WYQQKPGQPPNLLIY (SEQ ID NO: 8);

an FR-L3 comprising the amino acid sequence of GVPDRFSGSGSGTDFTLTISSLQAEDVAVYYC (SEQ ID NO: 9); and
an FR-L4 comprising the amino acid sequence of FGPGTKVEIK (SEQ ID NO: 10).

215. The use of para 213, wherein the anti-TIGIT antagonist antibody further comprises the following heavy chain variable region FRs:

an FR-H1 comprising the amino acid sequence of $X_1$VQLQQSGPGLVKPSQTLSLTCAISGDSVS (SEQ ID NO: 11), wherein $X_1$ is Q or E;
an FR-H2 comprising the amino acid sequence of WIRQSPSRGLEWLG (SEQ ID NO: 12);
an FR-H3 comprising the amino acid sequence of RITINPDTSKNQFSLQLNSVTPEDTAVFYCTR (SEQ ID NO: 13); and
an FR-H4 comprising the amino acid sequence of WGQGTLVTVSS (SEQ ID NO: 14).

216. The use of para 215, wherein $X_1$ is Q.

217. The use of para 215, wherein $X_1$ is E.

218. The use of any one of paras 215-217, wherein the anti-TIGIT antagonist antibody comprises:

(a) a heavy chain variable (VH) domain comprising an amino acid sequence having at least 95% sequence identity to the amino acid sequence of SEQ ID NO: 17 or 18;
(b) a light chain variable (VL) domain comprising an amino acid sequence having at least 95% sequence identity to the amino acid sequence of SEQ ID NO: 19; or
(c) a VH domain as in (a) and a VL domain as in (b).

219. The use of any one of paras 210-218, wherein the anti-TIGIT antagonist antibody comprises:

a VH domain comprising the amino acid sequence of SEQ ID NO: 17 or 18; and
a VL domain comprising the amino acid sequence of SEQ ID NO: 19.

220. The use of any one of paras 210-219, wherein the anti-TIGIT antagonist antibody is a monoclonal antibody and/or a human antibody.

221. The use of any one of paras 210-220, wherein the anti-TIGIT antagonist antibody is a full-length antibody.

222. The use of any one of paras 210-215 and 217-221, wherein the anti-TIGIT antagonist antibody is tiragolumab.

223. The use of any one of paras 210-220, wherein the anti-TIGIT antagonist antibody is an antibody fragment that binds TIGIT selected from the group consisting of Fab, Fab', Fab'-SH, Fv, single chain variable fragment (scFv), and $(Fab')_2$ fragments.

224. The use of any one of paras 210-223, wherein the anti-TIGIT antagonist antibody is an IgG class antibody.

225. The use of para 224, wherein the IgG class antibody is an IgG1 subclass antibody.

226. The use of any one of paras 223-225, wherein the PD-1 axis binding antagonist is a PD-L1 binding antagonist or a PD-1 binding antagonist.

227. The use of para 226, wherein the PD-L1 binding antagonist is an anti-PD-L1 antagonist antibody.

228. The use of para 227, wherein the anti-PD-L1 antagonist antibody is atezolizumab (MPDL3280A), MSB0010718C, MDX-1105, or MEDI4736.

229. The use of para 228, wherein the anti-PD-L1 antagonist antibody is atezolizumab.

230. The use of para 226, wherein the PD-1 binding antagonist is an anti-PD-1 antagonist antibody.

231. The use of para 230, wherein the anti-PD-1 antagonist antibody is nivolumab (MDX-1106), pembrolizumab (MK-3475), MED1-0680, spartalizumab (PDR001), cemiplimab (REGN2810), BGB-108, prolgolimab, camrelizumab, sintilimab, tislelizumab, or toripalimab.

232. The use of para 227, wherein the anti-PD-L1 antagonist antibody comprises the following HVRs:

an HVR-H1 sequence comprising the amino acid sequence of GFTFSDSWIH (SEQ ID NO: 20);
an HVR-H2 sequence comprising the amino acid sequence of AWISPYGGSTYYADSVKG (SEQ ID NO: 21);
an HVR-H3 sequence comprising the amino acid sequence of RHWPGGFDY (SEQ ID NO: 22);
an HVR-L1 sequence comprising the amino acid sequence of RASQDVSTAVA (SEQ ID NO: 23);
an HVR-L2 sequence comprising the amino acid sequence of SASFLYS (SEQ ID NO: 24); and
an HVR-L3 sequence comprising the amino acid sequence of QQYLYHPAT (SEQ ID NO: 25).

233. The use of para 232, wherein the anti-PD-L1 antagonist antibody comprises:

(a) a heavy chain variable (VH) domain comprising an amino acid sequence having at least 95% sequence identity to the amino acid sequence of SEQ ID NO: 26;
(b) a light chain variable (VL) domain comprising an amino acid sequence having at least 95% sequence identity to the amino acid sequence of SEQ ID NO: 27; or
(c) a VH domain as in (a) and a VL domain as in (b).

234. The use of para 233, wherein the anti-PD-L1 antagonist antibody comprises:

a VH domain comprising the amino acid sequence of SEQ ID NO: 26; and
a VL domain comprising the amino acid sequence of SEQ ID NO: 27.

235. The use of any one of paras 210-234, wherein the PD-1 axis binding antagonist is a monoclonal antibody.

236. The use of any one of paras 210-235, wherein the PD-1 axis binding antagonist is a humanized antibody.

237. The use of any one of paras 210-236, wherein the PD-1 axis binding antagonist is a full-length antibody.

238. The use of any one of paras 210-236, wherein the PD-1 axis binding antagonist is an antibody fragment that binds PD-L1 selected from the group consisting of Fab, Fab', Fab'-SH, Fv, single chain variable fragment (scFv), and $(Fab')_2$ fragments.

239. The use of para 237, wherein the PD-1 axis binding antagonist is an IgG class antibody.

240. The use of para 239, wherein the IgG class antibody is an IgG1 subclass antibody.

241. The use of any one of paras 210-240, wherein the anti-TIGIT antagonist antibody is to be administered at a fixed dose of between about 30 mg to about 1200 mg every three weeks.

242. The use of para 241, wherein the anti-TIGIT antagonist antibody is to be administered at a fixed dose of between about 30 mg to about 600 mg every three weeks.

243. The use of para 242, wherein the anti-TIGIT antagonist antibody is to be administered at a fixed dose of about 600 mg every three weeks.

244. The use of any one of paras 210-243, wherein the PD-1 axis binding antagonist is to be administered at a fixed dose of between about 80 mg to about 1600 mg every three weeks.

245. The use of para 244, wherein the PD-1 axis binding antagonist is to be administered at a fixed dose of about 1200 mg every three weeks.

246. The use of any one of paras 210-245, wherein the anti-TIGIT antagonist antibody is to be administered to the subject at a fixed dose of about 600 mg of every three weeks and the PD-1 axis binding antagonist is to be administered to the subject at a fixed dose of about 1200 mg every three weeks.

247. The use of any one of paras 210-246, wherein the length of each of the one or more dosing cycles is 21 days.

248. The use of any one of paras 210-247, wherein the anti-TIGIT antagonist antibody and PD-1 axis binding antagonist are to be administered to the subject on about Day 1 of each of the one or more dosing cycles.

249. The use of any one of paras 210-240, wherein the anti-TIGIT antagonist antibody is to be administered at a fixed dose of between about 300 mg to about 800 mg every two weeks.

250. The use of para 249, wherein the anti-TIGIT antagonist antibody is to be administered at a fixed dose of between about 400 mg to about 500 mg every two weeks.

251. The use of para 250, wherein the anti-TIGIT antagonist antibody is to be administered at a fixed dose of about 420 mg every two weeks.

252. The use of any one of paras 210-240 and 249-251, wherein the PD-1 axis binding antagonist is to be administered at a fixed dose of between about 200 mg to about 1200 mg every two weeks.

253. The use of para 252, wherein the PD-1 axis binding antagonist is to be administered at a fixed dose of about 840 mg every two weeks.

254. The use of para 253, wherein the anti-TIGIT antagonist antibody is to be administered at a fixed dose of about 420 mg every two weeks and the PD-1 axis binding antagonist is to be administered at a fixed dose of about 840 mg every two weeks.

255. The use of any one of paras 210-240 and 249-254, wherein the length of each of the one or more dosing cycles is 28 days.

256. The use of para 255, wherein the anti-TIGIT antagonist antibody and the PD-1 axis binding antagonist are each to be administered on about Days 1 and 15 of each of the one or more dosing cycles.

257. The use of any one of paras 210-240, wherein the anti-TIGIT antagonist antibody is to be administered at a fixed dose of between about 700 mg to about 1000 mg every four weeks.

258. The use of para 257, wherein the anti-TIGIT antagonist antibody is to be administered at a fixed dose of between about 800 mg to about 900 mg every four weeks.

259. The use of para 258, wherein the anti-TIGIT antagonist antibody is to be administered at a fixed dose of about 840 mg every four weeks.

260. The use of any one of paras 210-240 and 257-259, wherein the PD-1 axis binding antagonist is to be administered at a fixed dose of between about 400 mg to about 2000 mg every four weeks.

261. The use of para 260, wherein the PD-1 axis binding antagonist is to be administered at a fixed dose of about 1680 mg every four weeks.

262. The use of para 261, wherein the anti-TIGIT antagonist antibody is to be administered at a fixed dose of about 840 mg every four weeks and the PD-1 axis binding antagonist is to be administered at a fixed dose of about 1680 mg every four weeks.

263. The use of any one of paras 210-240 and 257-262, wherein the length of each of the one or more dosing cycles is 28 days.

264. The use of para 263, wherein the anti-TIGIT antagonist antibody and the PD-1 axis binding antagonist are each to be administered on about Day 1 of each of the one or more dosing cycles.

265. The use of any one of paras 210-264, wherein the anti-TIGIT antagonist antibody is to be administered to the subject before the PD-1 axis binding antagonist.

266. The use of para 265, wherein a first observation period is to follow administration of the PD-1 axis binding antagonist and second observation period is to follow administration of the anti-TIGIT antagonist antibody.

267. The use of para 266, wherein the first observation period and the second observation period are each between about 30 minutes to about 60 minutes in length.

268. The use of any one of paras 210-264, wherein the anti-TIGIT antagonist antibody is to be administered to the subject before the PD-1 axis binding antagonist.

269. The use of para 268, wherein a first observation period is to follow administration of the anti-TIGIT antagonist antibody and second observation period is to follow administration of the PD-1 axis binding antagonist.

270. The use of para 269, wherein the first observation period and the second observation period are each between about 30 minutes to about 60 minutes in length.

271. The use of any one of paras 210-264, wherein the anti-TIGIT antagonist antibody is to be administered to the subject simultaneously with the PD-1 axis binding antagonist.

272. The use of any one of paras 210-271, wherein the anti-TIGIT antagonist antibody and PD-1 axis binding antagonist are to be administered to the subject intravenously.

273. The use of para 272, wherein the anti-TIGIT antagonist antibody is to be administered to the subject by intravenous infusion over 60 $\pm$ 10 minutes.

274. The use of para 270 or 273, wherein the PD-1 axis binding antagonist is to be administered to the subject by intravenous infusion over 60 $\pm$ 15 minutes.

275. The use of any one of paras 210-271, wherein the anti-TIGIT antagonist antibody and PD-1 axis binding antagonist are to be administered to the subject subcutaneously.

276. The use of any one of paras 210-275, wherein the PD-L1-positive tumor cell fraction is determined by positive staining with an anti-PD-L1 antibody, wherein the anti-PD-L1 antibody is SP263, 22C3, SP142, or 28-8.

277. The use of para 276, wherein the staining is part of an immunohistochemical (IHC) assay.

278. The use of para 276 or 277, wherein the PD-L1-positive tumor cell fraction is greater than, or equal to, 50%, as determined by positive staining with the anti-PD-L1 antibody SP263, 22C3, or 28-8.

279. The use of para 278, wherein the PD-L1-positive tumor cell fraction is determined by positive staining with the anti-PD-L1 antibody SP263 and using the Ventana SP263 IHC assay.

280. The use of para 278, wherein the PD-L1-positive tumor cell fraction is determined by positive staining with the anti-PD-L1 antibody 22C3 and using the pharmDx 22C3 IHC assay.

281. The use of any one of paras 210-280, wherein a tumor sample obtained from the subject has been determined to have a detectable nucleic acid expression level of PD-L1.

282. The use of para 281, wherein the detectable nucleic acid expression level of PD-L1 has been determined by RNA-seq, RT-qPCR, qPCR, multiplex qPCR or RT-qPCR, microarray analysis, SAGE, MassARRAY technique, ISH, or a combination thereof

283. The use of any one of paras 210-282, wherein the lung cancer is a non-small cell lung cancer.

284. The use of para 283, wherein the NSCLC is a squamous NSCLC.

285. The use of para 284, wherein the NSCLC is a non-squamous NSCLC.

286. The use of any one of paras 283-285, wherein the NSCLC is a locally advanced unresectable NSCLC.

287. The use of para 286, wherein the NSCLC is a Stage IIIB NSCLC.

288. The use of any one of paras 283-286, wherein the NSCLC is a recurrent or metastatic NSCLC.

289. The use of para 288, wherein the NSCLC is a Stage IV NSCLC.

290. The use of para 288 or 289, wherein the subject has not been previously treated for Stage IV NSCLC.

291. The use of any one of paras 210-290, wherein the subject does not have a sensitizing epidermal growth factor receptor *(EGFR)* gene mutation or anaplastic lymphoma kinase *(ALK)* gene rearrangement.

292. The use of any one of paras 210-291, wherein the subject does not have a pulmonary lymphoepithelioma-like carcinoma subtype of NSCLC.

293. The use of any one of paras 210-292, wherein the subject does not have an active EBV infection or a known or suspected chronic active EBV infection.

294. The use of any one of paras 210-293, wherein the subject is negative for EBV IgM or negative by EBV PCR.

295. The use of para 294, wherein the subject is negative for EBV IgM and negative by EBV PCR.

296. The use of para 294 or 295, wherein the subject is positive for EBV IgG or positive for EBNA.

297. The use of para 296, wherein the subject is positive for EBV IgG and positive for EBNA.

298. The use of any one of paras 210-297, wherein the subject is negative for EBV IgG or negative for EBNA.

299. The use of para 298, wherein the subject is negative for EBV IgG and negative for EBNA.

300. The use of any one of paras 210-299, wherein the PFS is increased as compared to a reference PFS time.

301. The use of para 300, wherein the reference PFS time is the median PFS time of a population of subjects who have received a treatment comprising an PD-1 axis binding antagonist without an anti-TIGIT antagonist antibody.

302. Use of an anti-TIGIT antagonist antibody and atezolizumab in the manufacture of a medicament for use in a method of treating a subject having a NSCLC, wherein the method comprises administering to the subject one or more dosing cycles of the medicament, wherein the medicament is formulated for administration of the anti-TIGIT antagonist antibody and atezolizumab, wherein the anti-TIGIT antagonist antibody comprises:

a VH domain comprising the amino acid sequence of SEQ ID NO: 17 or 18; and
a VL domain comprising the amino acid sequence of SEQ ID NO: 19, and wherein the subject has been determined to have a PD-L1-positive tumor cell fraction of greater than, or equal to, 30%, and the treatment results in (a) a CR or a PR and/or (b) an increase in PFS as compared to treatment with atezolizumab without the anti-TIGIT antagonist antibody.

303. The use of para 302, wherein the anti-TIGIT antagonist antibody is to be administered at a fixed dose of 600 mg every three weeks and atezolizumab is to be administered at a fixed dose of 1200 mg every three weeks.

304. The use of para 302, wherein the anti-TIGIT antagonist antibody is to be administered at a fixed dose of 420 mg every two weeks and atezolizumab is to be administered at a fixed dose of 840 mg every two weeks.

305. The use of para 302, wherein the anti-TIGIT antagonist antibody is to be administered at a fixed dose of 840 mg every four weeks and atezolizumab is to be administered at a fixed dose of 1680 mg every four weeks.

306. Use of tiragolumab and atezolizumab in the manufacture of a medicament for use in a method of treating a subject having a NSCLC, wherein the method comprises administering to the subject one or more dosing cycles of the medicament, wherein the medicament is formulated for administration of tiragolumab and atezolizumab, and wherein the subject has been determined to have a PD-L1-positive tumor cell fraction of greater than, or equal to, 30%, and the treatment results in (a) a CR or a PR and/or (b) an increase in PFS as compared to treatment with atezolizumab without tiragolumab.

307. The use of para 306, wherein tiragolumab is to be administered at a fixed dose of 600 mg every three weeks and atezolizumab is to be administered at a fixed dose of 1200 mg every three weeks.

308. The use of para 306, wherein tiragolumab is to be administered at a fixed dose of 420 mg every two weeks and atezolizumab is to be administered at a fixed dose of 840 mg every two weeks.

309. The use of para 306, wherein tiragolumab is to be administered at a fixed dose of 840 mg every four weeks and atezolizumab is to be administered at a fixed dose of 1680 mg every four weeks.

310. The method of any one of paras 1-109, the anti-TIGIT antagonist antibody of any one of paras 110-209, or the use of any one of paras 210-309, wherein the anti-TIGIT antagonist antibody is tiragolumab and the PD-1 axis binding antagonist is atezolizumab, and wherein the treatment results in an increase in PFS of at least about 3.1 months, as compared to treatment with atezolizumab without tiragolumab.

311. The method of any one of paras 102-105, the tiragolumab and atezolizumab of any one of paras 206-209, or the use of any one of paras 306-309, wherein the treatment results in an increase in PFS of at least about 3.1 months, as compared to treatment with atezolizumab without tiragolumab.

312. The method of any one of paras 1-109, the anti-TIGIT antagonist antibody of any one of paras 110-209, or the use of any one of paras 210-309, wherein the anti-TIGIT antagonist antibody is tiragolumab and the PD-1 axis binding antagonist is atezolizumab, and wherein the treatment results in an increase in PFS of at least about 4.9 months, as compared to treatment with atezolizumab without tiragolumab.

313. The method of any one of paras 102-105, the tiragolumab and atezolizumab of any one of paras 206-209, or the use of any one of paras 306-309, wherein the treatment results in an increase in PFS of at least about 4.9 months, as compared to treatment with atezolizumab without tiragolumab.

314. The method of any one of paras 1-109, the anti-TIGIT antagonist antibody of any one of paras 110-209, or the use of any one of paras 210-309, wherein the anti-TIGIT antagonist antibody is tiragolumab and the PD-1 axis binding antagonist is atezolizumab, and wherein the treatment results in an increase in OS of at least about 5.7 months, as compared to treatment with atezolizumab without tiragolumab.

315. The method of any one of paras 102-105, the tiragolumab and atezolizumab of any one of paras 206-209, or the use of any one of paras 306-309, wherein the treatment results in an increase in OS of at least about 5.7 months, as compared to treatment with atezolizumab without tiragolumab.

316. The method of any one of paras 1-109, the anti-TIGIT antagonist antibody of any one of paras 110-209, or the use of any one of paras 210-309, wherein the anti-TIGIT antagonist antibody is tiragolumab and the PD-1 axis binding antagonist is atezolizumab, and wherein the treatment results in an increase in OS of at least about 9 months, as compared to treatment with atezolizumab without tiragolumab.

317. The method of any one of paras 102-105, the tiragolumab and atezolizumab of any one of paras 206-209, or the use of any one of paras 306-309, wherein the treatment results in an increase in OS of at least about 9 months, as compared to treatment with atezolizumab without tiragolumab.

318. A method for treating a subject having a lung cancer, the method comprising administering to the subject one or more dosing cycles of an anti-TIGIT antagonist antibody and a PD-1 axis binding antagonist, wherein the subject previously received concurrent chemoradiotherapy (cCRT) for lung cancer, and wherein the subject has not had disease progression after the cCRT.

319. The method of para 318, wherein the subject previously received at least two cycles of the cCRT.

320. The method of para 318 or 319, wherein the cCRT comprises a platinum-based chemotherapy.

321. The method of any one of paras 318-320, wherein the cCRT comprises a thoracic radiotherapy.

322. The method of para 321, wherein the thoracic radiotherapy was administered to the subject at 60-66 Gy in 30-33 fractions.

323. The method of any one of paras 318-322, wherein the cCRT was administered with curative intent.

324. The method of any one of paras 318-323, wherein the cCRT was administered as a consolidation therapy.

325. The method of any one of paras 318-324, wherein the anti-TIGIT antagonist antibody comprises the following hypervariable regions (HVRs):

an HVR-H1 sequence comprising the amino acid sequence of SNSAAWN (SEQ ID NO: 1);
an HVR-H2 sequence comprising the amino acid sequence of KTYYRFKWYSDYAVSVKG (SEQ ID NO: 2);
an HVR-H3 sequence comprising the amino acid sequence of ESTTYDLLAGPFDY (SEQ ID NO: 3);
an HVR-L1 sequence comprising the amino acid sequence of KSSQTVLYSSNNKKYLA (SEQ ID NO: 4);
an HVR-L2 sequence comprising the amino acid sequence of WASTRES (SEQ ID NO: 5); and
an HVR-L3 sequence comprising the amino acid sequence of QQYYSTPFT (SEQ ID NO: 6).

326. The method of para 325, wherein the anti-TIGIT antagonist antibody further comprises the following light chain variable region framework regions (FRs):

an FR-L1 comprising the amino acid sequence of DIVMTQSPDSLAVSLGERATINC (SEQ ID NO: 7);
an FR-L2 comprising the amino acid sequence of WYQQKPGQPPNLLIY (SEQ ID NO: 8);
an FR-L3 comprising the amino acid sequence of GVPDRFSGSGSGTDFTLTISSLQAEDVAVYYC (SEQ ID NO: 9); and
an FR-L4 comprising the amino acid sequence of FGPGTKVEIK (SEQ ID NO: 10).

327. The method of para 325, wherein the anti-TIGIT antagonist antibody further comprises the following heavy chain variable region FRs:

an FR-H1 comprising the amino acid sequence of $X_1$VQLQQSGPGLVKPSQTLSLTCAISGDSVS (SEQ ID NO: 11), wherein $X_1$ is Q or E;
an FR-H2 comprising the amino acid sequence of WIRQSPSRGLEWLG (SEQ ID NO: 12);
an FR-H3 comprising the amino acid sequence of RITINPDTSKNQFSLQLNSVTPEDTAVFYCTR (SEQ ID NO: 13); and
an FR-H4 comprising the amino acid sequence of WGQGTLVTVSS (SEQ ID NO: 14).

328. The method of para 327, wherein $X_1$ is Q.

329. The method of para 327, wherein $X_1$ is E.

330. The method of any one of paras 325-329, wherein the anti-TIGIT antagonist antibody comprises:

(a) a heavy chain variable (VH) domain comprising an amino acid sequence having at least 95% sequence identity to the amino acid sequence of SEQ ID NO: 17 or 18;
(b) a light chain variable (VL) domain comprising an amino acid sequence having at least 95% sequence identity to the amino acid sequence of SEQ ID NO: 19; or
(c) a VH domain as in (a) and a VL domain as in (b).

331. The method of any one of paras 318-330, wherein the anti-TIGIT antagonist antibody comprises:

a VH domain comprising the amino acid sequence of SEQ ID NO: 17 or 18; and
a VL domain comprising the amino acid sequence of SEQ ID NO: 19.

332. The method of any one of paras 318-331, wherein the anti-TIGIT antagonist antibody is a monoclonal antibody.

333. The method of para 332, wherein the anti-TIGIT antagonist antibody is a human antibody.

334. The method of any one of paras 318-333, wherein the anti-TIGIT antagonist antibody is a full-length antibody.

335. The method of any one of paras 318-327 and 329-334, wherein the anti-TIGIT antagonist antibody is tiragolumab.

336. The method of any one of paras 318-333, wherein the anti-TIGIT antagonist antibody is an antibody fragment that binds TIGIT selected from the group consisting of Fab, Fab', Fab'-SH, Fv, single chain variable fragment (scFv), and $(Fab')_2$ fragments.

337. The method of any one of paras 318-335, wherein the anti-TIGIT antagonist antibody is an IgG class antibody.

338. The method of para 337, wherein the IgG class antibody is an IgG1 subclass antibody.

339. The method of any one of paras 318-338, wherein the PD-1 axis binding antagonist is a PD-L1 binding antagonist or a PD-1 binding antagonist.

340. The method of para 339, wherein the PD-L1 binding antagonist is an anti-PD-L1 antagonist antibody.

341. The method of para 340, wherein the anti-PD-L1 antagonist antibody is atezolizumab (MPDL3280A), MSB0010718C, MDX-1105, or MEDI4736.

342. The method of para 341, wherein the anti-PD-L1 antagonist antibody is atezolizumab.

343. The method of para 342, wherein the PD-1 binding antagonist is an anti-PD-1 antagonist antibody.

344. The method of para 343, wherein the anti-PD-1 antagonist antibody is nivolumab (MDX-1106), pembrolizumab (MK-3475), MED1-0680, spartalizumab (PDR001), cemiplimab (REGN2810), BGB-108, prolgolimab, camrelizumab, sintilimab, tislelizumab, or toripalimab.

345. The method of para 340, wherein the anti-PD-L1 antagonist antibody comprises the following HVRs:

an HVR-H1 sequence comprising the amino acid sequence of GFTFSDSWIH (SEQ ID NO: 20);
an HVR-H2 sequence comprising the amino acid sequence of AWISPYGGSTYYADSVKG (SEQ ID NO: 21);
an HVR-H3 sequence comprising the amino acid sequence of RHWPGGFDY (SEQ ID NO: 22);
an HVR-L1 sequence comprising the amino acid sequence of RASQDVSTAVA (SEQ ID NO: 23);
an HVR-L2 sequence comprising the amino acid sequence of SASFLYS (SEQ ID NO: 24); and
an HVR-L3 sequence comprising the amino acid sequence of QQYLYHPAT (SEQ ID NO: 25).

346. The method of para 345, wherein the anti-PD-L1 antagonist antibody comprises:

(a) a heavy chain variable (VH) domain comprising an amino acid sequence having at least 95% sequence identity to the amino acid sequence of SEQ ID NO: 26;
(b) a light chain variable (VL) domain comprising an amino acid sequence having at least 95% sequence identity to the amino acid sequence of SEQ ID NO: 27; or
(c) a VH domain as in (a) and a VL domain as in (b).

347. The method of para 346, wherein the anti-PD-L1 antagonist antibody comprises:

a VH domain comprising the amino acid sequence of SEQ ID NO: 26; and
a VL domain comprising the amino acid sequence of SEQ ID NO: 27.

348. The method of any one of paras 318-347, wherein the PD-1 axis binding antagonist is a monoclonal antibody.

349. The method of any one of paras 1-348, wherein the PD-1 axis binding antagonist is a humanized antibody.

350. The method of any one of paras 1-349, wherein the PD-1 axis binding antagonist is a full-length antibody.

351. The method of any one of paras 1-349, wherein the PD-1 axis binding antagonist is an antibody fragment that binds PD-L1 selected from the group consisting of Fab, Fab', Fab'-SH, Fv, single chain variable fragment (scFv), and $(Fab')_2$ fragments.

352. The method of para 350, wherein the PD-1 axis binding antagonist is an IgG class antibody.

353. The method of para 352, wherein the IgG class antibody is an IgG1 subclass antibody.

354. The method of any one of paras 318-353, wherein the method comprises administering to the subject the anti-TIGIT antagonist antibody at a fixed dose of between about 30 mg to about 1200 mg every three weeks.

355. The method of para 354, wherein the method comprises administering to the subject the anti-TIGIT antagonist antibody at a fixed dose of between about 30 mg to about 600 mg every three weeks.

356. The method of para 355, wherein the method comprises administering to the subject the anti-TIGIT antagonist antibody at a fixed dose of about 600 mg every three weeks.

357. The method of any one of paras 318-356, wherein the method comprises administering to the subject the PD-1 axis binding antagonist at a fixed dose of between about 80 mg to about 1600 mg every three weeks.

358. The method of para 357, wherein the method comprises administering to the subject the PD-1 axis binding antagonist at a fixed dose of about 1200 mg every three weeks.

359. The method of any one of paras 318-358, wherein the method comprises administering to the subject the anti-TIGIT antagonist antibody at a fixed dose of about 600 mg every three weeks and the PD-1 axis binding antagonist at a fixed dose of about 1200 mg every three weeks.

360. The method of any one of paras 318-359, wherein the length of each of the one or more dosing cycles is 21 days.

361. The method of para 360, wherein the method comprises administering to the subject the anti-TIGIT antagonist antibody and the PD-1 axis binding antagonist on about Day 1 of each of the one or more dosing cycles.

362. The method of any one of paras 318-353, wherein the method comprises administering to the subject the anti-TIGIT antagonist antibody at a fixed dose of between about 300 mg to about 800 mg every two weeks.

363. The method of para 362, wherein the method comprises administering to the subject the anti-TIGIT antagonist antibody at a fixed dose of between about 400 mg to about 500 mg every two weeks.

364. The method of para 363, wherein the method comprises administering to the subject the anti-TIGIT antagonist antibody at a fixed dose of about 420 mg every two weeks.

365. The method of any one of paras 318-353 and 362-364, wherein the method comprises administering to the subject the PD-1 axis binding antagonist at a fixed dose of between about 200 mg to about 1200 mg every two weeks.

366. The method of para 365, wherein the method comprises administering to the subject the PD-1 axis binding antagonist at a fixed dose of about 840 mg every two weeks.

367. The method of para 366, wherein the method comprises administering to the subject the anti-TIGIT antagonist antibody at a fixed dose of about 420 mg every two weeks and the PD-1 axis binding antagonist at a fixed dose of about 840 mg every two weeks.

368. The method of any one of paras 318-353 and 362-367, wherein the length of each of the one or more dosing cycles is 28 days.

369. The method of para 368, wherein the method comprises administering to the subject the anti-TIGIT antagonist antibody and the PD-1 axis binding antagonist on about Days 1 and 15 of each of the one or more dosing cycles.

370. The method of any one of paras 318-353, wherein the method comprises administering to the subject the anti-TIGIT antagonist antibody at a fixed dose of between about 700 mg to about 1000 mg every four weeks.

371. The method of para 370, wherein the method comprises administering to the subject the anti-TIGIT antagonist antibody at a fixed dose of between about 800 mg to about 900 mg every four weeks.

372. The method of para 371, wherein the method comprises administering to the subject the anti-TIGIT antagonist antibody at a fixed dose of about 840 mg every four weeks.

373. The method of any one of paras 318-353 and 370-372, wherein the method comprises administering to the subject the PD-1 axis binding antagonist at a fixed dose of between about 400 mg to about 2000 mg every four weeks.

374. The method of para 373, wherein the method comprises administering to the subject the PD-1 axis binding antagonist at a fixed dose of about 1680 mg every four weeks.

375. The method of para 374, wherein the method comprises administering to the subject the anti-TIGIT antagonist antibody at a fixed dose of about 840 mg every four weeks and the PD-1 axis binding antagonist at a fixed dose of about 1680 mg every four weeks.

376. The method of any one of paras 318-353 and 370-375, wherein the length of each of the one or more dosing cycles is 28 days.

377. The method of para 376, wherein the method comprises administering to the subject the anti-TIGIT antagonist antibody and the PD-1 axis binding antagonist on about Day 1 of each of the one or more dosing cycles.

378. The method of any one of paras 318-377, wherein the method comprises administering to the subject the PD-1 axis binding antagonist before the anti-TIGIT antagonist antibody.

379. The method of para 378, wherein the method comprises a first observation period following administration of the PD-1 axis binding antagonist and second observation period following administration of the anti-TIGIT antagonist antibody.

380. The method of para 379, wherein the first observation period and the second observation period are each between about 30 minutes to about 60 minutes in length.

381. The method of any one of paras 318-377, wherein the method comprises administering to the subject the anti-TIGIT antagonist antibody before the PD-1 axis binding antagonist.

382. The method of para 381, wherein the method comprises a first observation period following administration of the anti-TIGIT antagonist antibody and second observation period following administration of the PD-1 axis binding antagonist.

383. The method of para 382, wherein the first observation period and the second observation period are each between about 30 minutes to about 60 minutes in length.

384. The method of any one of paras 318-377, wherein the method comprises administering to the subject the anti-TIGIT antagonist antibody and the PD-1 axis binding antagonist simultaneously.

385. The method of any one of paras 318-384, wherein the method comprises administering to the subject the anti-TIGIT antagonist antibody and PD-1 axis binding antagonist intravenously.

386. The method of para 385, wherein the method comprises administering to the subject the anti-TIGIT antagonist antibody by intravenous infusion over 60 $\pm$ 10 minutes.

387. The method of para 385 or 386, wherein the method comprises administering to the subject the PD-1 axis binding antagonist by intravenous infusion over 60 $\pm$ 15 minutes.

388. The method of any one of paras 318-384, wherein the method comprises administering to the subject the anti-TIGIT antagonist antibody and PD-1 axis binding antagonist subcutaneously.

389. The method of any one of paras 318-388, wherein a PD-L1-positive tumor cell fraction of the subject is determined.

390. The method of para 389, wherein the PD-L1-positive tumor cell fraction is determined by positive staining with an anti-PD-L1 antibody, wherein the anti-PD-L1 antibody is SP263, 22C3, SP142, or 28-8.

391. The method of para 389 or 390, wherein the staining is part of an IHC assay.

392. The method of para 391, wherein the PD-L1-positive tumor cell fraction is greater than or equal to 1% tumor cell (TC), as determined by positive staining with an anti-PD-L1 antibody SP263 or 22C3.

393. The method of para 392, wherein the PD-L1-positive tumor cell fraction is less than 1% TC, as determined by positive staining with an anti-PD-L1 antibody SP263 or 22C3.

394. The method of para 393, wherein the PD-L1 expression is calculated using the Ventana SP263 IHC assay.

395. The method of para 393, wherein the PD-L1 expression is calculated using the pharmDx 22C3 IHC assay.

396. The method of any one of paras 318-395, wherein a detectable nucleic acid expression level of PD-L1 has been determined by RNA-seq, RT-qPCR, qPCR, multiplex qPCR or RT-qPCR, microarray analysis, SAGE, MassARRAY technique, ISH, or a combination thereof.

397. The method of any one of paras 318-396, wherein the lung cancer is a non-small cell lung cancer (NSCLC).

398. The method of para 397, wherein the NSCLC is a squamous NSCLC.

399. The method of para 397, wherein the NSCLC is a non-squamous NSCLC.

400. The method of any one of paras 394-396, wherein the NSCLC is a locally advanced unresectable NSCLC.

401. The method of any one of paras 397-400, wherein the NSCLC is a Stage III NSCLC.

402. The method of any one of paras 397-401, wherein the NSCLC is not a Stage IV NSCLC.

403. The method of any one of paras 318-402, wherein the subject does not have a sensitizing epidermal growth factor receptor *(EGFR)* gene mutation or anaplastic lymphoma kinase *(ALK)* gene rearrangement.

404. The method of any one of paras 318-403, wherein the subject does not have an active Epstein-Barr virus (EBV) infection or a known or suspected chronic active EBV infection.

405. The method of any one of paras 318-404, wherein the subject is negative for EBV IgM or negative by EBV PCR.

406. The method of para 405, wherein the subject is negative for EBV IgM and negative by EBV PCR.

407. The method of para 405 or 406, wherein the subject is positive for EBV IgG or positive for Epstein-Barr nuclear antigen (EBNA).

408. The method of para 407, wherein the subject is positive for EBV IgG and positive for EBNA.

409. The method of any one of paras 318-408, wherein the subject is negative for EBV IgG or negative for EBNA.

410. The method of para 409, wherein the subject is negative for EBV IgG and negative for EBNA.

411. The method of any one of paras 318-410, wherein the PFS is increased as compared to a reference PFS time.

412. The method of para 411, wherein the reference PFS time is the median PFS time of a population of subjects who have received a treatment comprising a PD-1 axis binding antagonist without an anti-TIGIT antagonist antibody.

413. A method for treating a subject having a NSCLC, the method comprising administering to the subject one or more dosing cycles of an anti-TIGIT antagonist antibody and atezolizumab, wherein the anti-TIGIT antagonist antibody comprises:

a VH domain comprising the amino acid sequence of SEQ ID NO: 17 or 18; and
a VL domain comprising the amino acid sequence of SEQ ID NO: 19, and wherein the subject previously received cCRT for lung cancer, and wherein the subject has not had disease progression after the cCRT, and wherein the treatment results in (a) a CR or a PR and/or (b) an increase in PFS as compared to treatment with durvalumab without the anti-TIGIT antagonist antibody.

414. The method of para 413, wherein the anti-TIGIT antagonist antibody is administered at a fixed dose of 600 mg every three weeks and atezolizumab is administered at a fixed dose of 1200 mg every three weeks.

415. The method of para 413, wherein the anti-TIGIT antagonist antibody is administered at a fixed dose of 420 mg every two weeks and atezolizumab is administered at a fixed dose of 840 mg every two weeks.

416. The method of para 413, wherein the anti-TIGIT antagonist antibody is administered at a fixed dose of 840 mg every four weeks and atezolizumab is administered at a fixed dose of 1680 mg every four weeks.

417. A method for treating a subject having a NSCLC, the method comprising administering to the subject one or more dosing cycles of tiragolumab and atezolizumab, wherein the subject previously received cCRT for lung cancer, and wherein the subject has not had disease progression after the cCRT, and the treatment results in (a) a CR or a PR and/or (b) an increase in PFS as compared to treatment with durvalumab without tiragolumab.

418. The method of para 417, wherein tiragolumab is administered at a fixed dose of 600 mg every three weeks and atezolizumab is administered at a fixed dose of 1200 mg every three weeks.

419. The method of para 417, wherein tiragolumab is administered at a fixed dose of 420 mg every two weeks and atezolizumab is administered at a fixed dose of 840 mg every two weeks.

420. The method of para 417, wherein tiragolumab is administered at a fixed dose of 840 mg every four weeks and atezolizumab is administered at a fixed dose of 1680 mg every four weeks.

421. The method of any one of para 413-420, wherein the subject previously received at least two cycles of the cCRT.

422. The method of any one of paras 413-421, wherein the cCRT comprises a platinum-based chemotherapy.

423. The method of any one of paras 413-422, wherein the cCRT comprises a thoracic radiotherapy.

424. The method of para 423, wherein the thoracic radiotherapy was administered to the subject at 60-66 Gy in 30-33 fractions.

425. The method of any one of paras 413-424, wherein the cCRT was administered with curative intent.

426. The method of any one of paras 413-425, wherein the cCRT was administered as a consolidation therapy.

427. The method of any one of paras 1-109 and 318-426, wherein the subject is a human.

428. An anti-TIGIT antagonist antibody and an anti-PD-L1 antagonist antibody for use in a method of treating a subject having a lung cancer, wherein the method is according to any one of paras 318-427.

429. Use of an anti-TIGIT antagonist antibody in the manufacture of a medicament for treating a subject having a lung cancer in combination with an anti-PD-L1 antagonist antibody, wherein the treatment is according to the method of any one of paras 318-427.

430. Use of an anti-PD-L1 antagonist antibody in the manufacture of a medicament for treating a subject having a lung cancer in combination with an anti-TIGIT antagonist antibody, wherein the treatment is according to the method of any one of paras 318-427.

431. The use of para 429 or 430, wherein the anti-TIGIT antagonist antibody and the anti-PD-L1 antagonist antibody are formulated separately.

432. The use of para 429 or 430, wherein the anti-TIGIT antagonist antibody and the anti-PD-L1 antagonist antibody are formulated together.

## Claims

1. Tiragolumab and atezolizumab for use in a method of treating a subject having a lung cancer, wherein the method comprises administering to the subject one or more dosing cycles of the tiragolumab and the atezolizumab, wherein the subject has been determined to have a PD-L1-positive tumor cell fraction of greater than, or equal to, 30%, and the treatment results in an increase in progression-free survival (PFS) as compared to treatment with the atezolizumab without the tiragolumab, and wherein the tiragolumab is to be administered to the subject at a fixed dose of about 600 mg every three weeks and the atezolizumab is to be administered to the subject at a fixed dose of about 1200 mg every three weeks.

2. The tiragolumab and atezolizumab for use of claim, wherein the length of each of the one or more dosing cycles is 21 days.

3. The tiragolumab and atezolizumab for use of claim 2, wherein the tiragolumab and atezolizumab are to be administered to the subject on about Day 1 of each of the one or more dosing cycles.

4. The tiragolumab and atezolizumab for use of any one of claims 1-3, wherein the atezolizumab is to be administered to the subject before the tiragolumab.

5. The tiragolumab and atezolizumab for use of any one of claims 1-3, wherein the tiragolumab is to be administered to the subject before the atezolizumab.

6. The tiragolumab and atezolizumab for use of any one of claims 1-5, wherein the tiragolumab and atezolizumab are to be administered to the subject intravenously.

7. The tiragolumab and atezolizumab for use of any one of claims 1-6, wherein the PD-L1-positive tumor cell fraction is determined by positive staining with an anti-PD-L1 antibody, wherein the anti-PD-L1 antibody is SP263, 22C3, SP142, or 28-8.

8. The tiragolumab and atezolizumab for use of claim 7, wherein the staining is part of an immunohistochemical (IHC) assay.

9. The tiragolumab and atezolizumab for use of claim 7 or 8, wherein the PD-L1-positive tumor cell fraction is greater than, or equal to, 50%, as determined by positive staining with the anti-PD-L1 antibody SP263, 22C3, or 28-8.

10. The tiragolumab and atezolizumab for use of any one of claims 1-9, wherein the lung cancer is a non-small cell lung cancer (NSCLC).

11. The tiragolumab and atezolizumab for use of claim 10, wherein the NSCLC is a squamous NSCLC.

FIG. 1

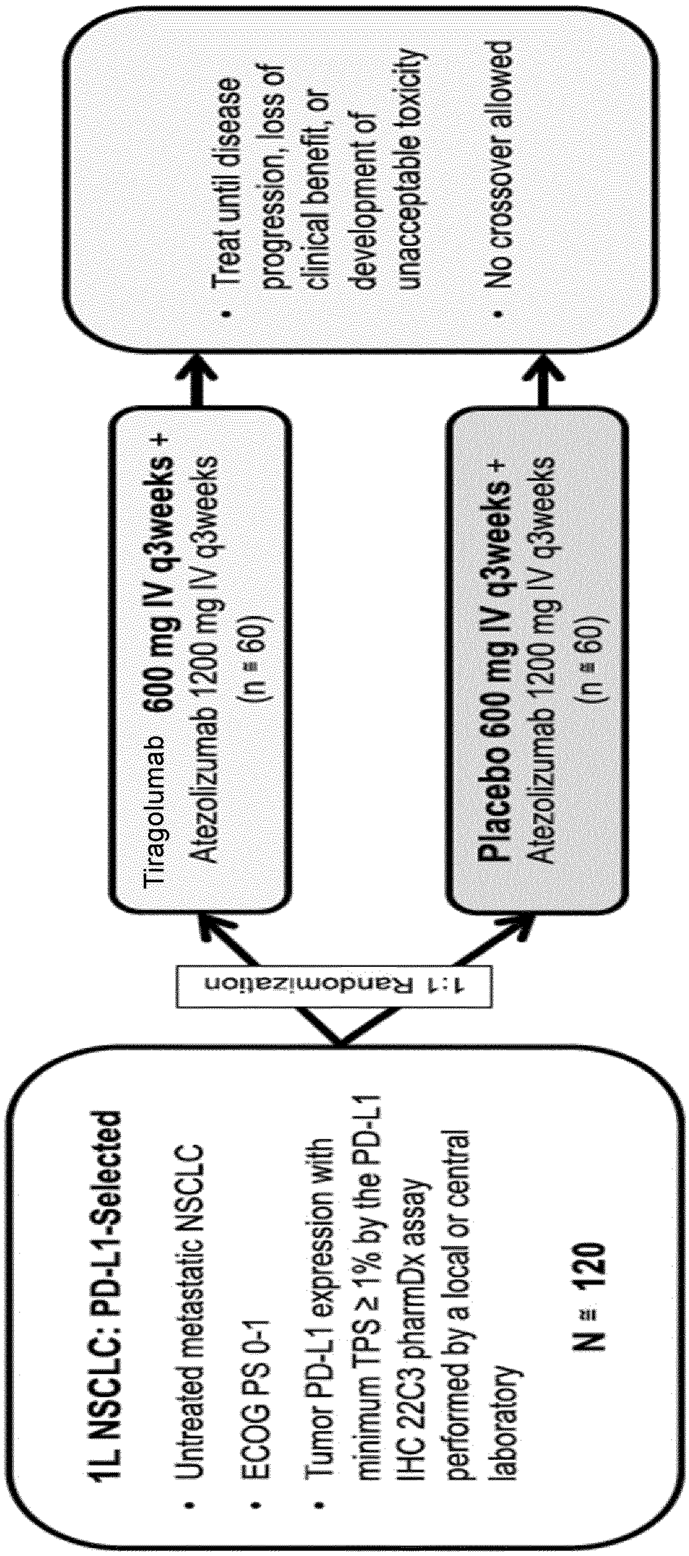
1L NSCLC: PD-L1-Selected
• Untreated metastatic NSCLC
• ECOG PS 0-1
• Tumor PD-L1 expression with minimum TPS ≥ 1% by the PD-L1 IHC 22C3 pharmDx assay performed by a local or central laboratory
N = 120
1:1 Randomization
Tiragolumab 600 mg IV q3weeks + Atezolizumab 1200 mg IV q3weeks (n = 60)
Placebo 600 mg IV q3weeks + Atezolizumab 1200 mg IV q3weeks (n = 60)
• Treat until disease progression, loss of clinical benefit, or development of unacceptable toxicity
• No crossover allowed

# FIG. 2

## Local TPS ≥ 50% (n = 58; 43%)

| | Atezo (N=29) | Tira + Atezo (N=29) | All Pts (N=58) |
|---|---|---|---|
| **Age (yr)** | | | |
| Median | 65 | 69 | 65.5 |
| Min - Max | 40 - 83 | 49 - 84 | 40 - 84 |
| **Sex** | | | |
| Male | 21 (72.4%) | 15 (51.7%) | 36 (62.1%) |
| Female | 8 (27.6%) | 14 (48.3%) | 22 (37.9%) |
| **Race** | | | |
| Asian | 9 (31.0%) | 9 (31.0%) | 18 (31.0%) |
| White | 18 (62.1%) | 18 (62.1%) | 36 (62.1%) |
| Multiple | 0 (0%) | 0 (0%) | 0 (0%) |
| Unknown | 2 (6.9%) | 2 (6.9%) | 4 (6.9%) |
| **ECOG at screening** | | | |
| 0 | 7 (24.1%) | 11 (37.9%) | 18 (31.0%) |
| 1 | 22 (75.9%) | 18 (62.1%) | 40 (69.0%) |
| **Tobacco History** | | | |
| N | 2 (6.9%) | 3 (10.3%) | 5 (8.6%) |
| Y | 27 (93.1%) | 26 (89.7%) | 53 (91.4%) |
| **Tumor Histology** | | | |
| Non-Squamous | 20 (69.0%) | 21 (72.4%) | 41 (70.7%) |
| Squamous | 9 (31.0%) | 8 (27.6%) | 17 (29.3%) |

## Local TPS 1 - 49% (n = 77; 57%)

| | Atezo (N=39) | Tira + Atezo (N=38) | All Pts (N=77) |
|---|---|---|---|
| **Age (yr)** | | | |
| Median | 69 | 68 | 68 |
| Min - Max | 44 - 83 | 42 - 82 | 42 - 83 |
| **Sex** | | | |
| Male | 27 (69.2%) | 24 (63.2%) | 51 (66.2%) |
| Female | 12 (30.8%) | 14 (36.8%) | 26 (33.8%) |
| **Race** | | | |
| Asian | 14 (35.9%) | 9 (23.7%) | 23 (29.9%) |
| White | 22 (56.4%) | 24 (63.2%) | 46 (59.7%) |
| Multiple | 1 (2.6%) | 0 | 1 (1.3%) |
| Unknown | 2 (5.1%) | 5 (13.2%) | 7 (9.1%) |
| **ECOG at screening** | | | |
| 0 | 7 (17.9%) | 12 (31.6%) | 19 (24.7%) |
| 1 | 32 (82.1%) | 26 (68.4%) | 58 (75.3%) |
| **Tobacco History** | | | |
| N | 5 (12.8%) | 4 (10.5%) | 9 (11.7%) |
| Y | 34 (87.2%) | 34 (89.5%) | 68 (88.3%) |
| **Tumor Histology** | | | |
| Non-Squamous | 20 (51.3%) | 19 (50.0%) | 39 (50.6%) |
| Squamous | 19 (48.7%) | 19 (50.0%) | 38 (49.4%) |

# FIG. 3

Local TPS ≥ 50%

| | Atezo (N=29) | Tira+Atezo (N=29) | All Pts (N=58) |
|---|---|---|---|
| **On Study** | 22 (75.9%) | 26 (89.7%) | 48 (82.8%) |
| **Continuing Treatment** | 14 (48.3%) | 22 (75.9%) | 36 (62.1%) |
| **Discontinued Treatment** | 8 (27.6%) | 4 (13.8%) | 12 (20.7%) |
| Adverse event | 1 (3.4%) | 1 (3.4%) | 2 (3.4%) |
| Physician decision | 1 (3.4%) | 0 | 1 (1.7%) |
| Radiographic PD | 5 (17.2%) | 2 (6.9%) | 7 (12.1%) |
| Other | 1 (3.4%) | 1 (3.4%) | 2 (3.4%) |
| **Discontinued study** | 7 (24.1%) | 3 (10.3%) | 10 (17.2%) |
| Death | 6 (20.7%) | 2 (6.9%) | 8 (13.8%) |
| Withdrawal by subject | 1 (3.4%) | 1 (3.4%) | 2 (3.4%) |

Local TPS 1 - 49%

| | Atezo (N=39) | Tira+Atezo (N=38) | All Pts (N=77) |
|---|---|---|---|
| **On Study** | 33 (84.6%) | 32 (84.2%) | 65 (84.4%) |
| **Continuing Treatment** | 18 (46.1%) | 20 (52.6%) | 38 (49.4%) |
| **Discontinued Treatment** | 15 (38.5%) | 12 (31.6%) | 27 (35.1%) |
| Adverse event | 1 (2.6%) | 2 (5.3%) | 3 (3.9%) |
| Physician decision | 0 | 2 (5.3%) | 2 (2.6%) |
| Radiographic PD | 13 (33.3%) | 7 (18.4%) | 20 (26.0%) |
| Symptomatic deterioration | 1 (2.6%) | 1 (2.6%) | 2 (2.6%) |
| **Discontinued study** | 6 (15.4%) | 6 (15.8%) | 12 (15.6%) |
| Death | 5 (12.8%) | 6 (15.8%) | 11 (14.3%) |
| Withdrawal by subject | 1 (2.6%) | 0 | 1 (1.3%) |

# FIG. 4

| | ITT: Local TPS ≥ 1% | | Local TPS ≥ 50% | | Local TPS 1 - 49% | |
|---|---|---|---|---|---|---|
| | Atezo (N=63) | Tira + Atezo (N=64) | Atezo (N=27) | Tira + Atezo (N=27) | Atezo (N=36) | Tira + Atezo (N=37) |
| Responders (n) | 10 | 20 | 5 | 14 | 5 | 6 |
| BOR % (95% CI) | **15.9** (6.06 to 25.69) | **31.3** (19.11 to 43.39) | **18.5** (2.01 to 35.02) | **51.9** (31.15 to 72.55) | **13.9** (1.20 to 26.57) | **16.2** (2.99 to 29.44) |
| Difference in BOR % (95% CI, p-value) | **15.38** (-0.70 to 31.46, 0.0538) | | **33.33** (5.76 to 60.91, 0.0164) | | **2.33** (-16.80 to 21.46, 0.9079) | |
| CR | 2 ( 3.2%) | 0 | 1 ( 3.7%) | 0 | 1 ( 2.8%) | 0 |
| PR | 8 (12.7%) | 20 (31.3%) | 4 (14.8%) | 14 (51.9%) | 4 (11.1%) | 6 (16.2%) |
| SD | 34 (54.0%) | 28 (43.8%) | 15 (55.6%) | 10 (37.0%) | 19 (52.8%) | 18 (48.6%) |
| PD | 19 (30.2%) | 16 (25.0%) | 7 (25.9%) | 3 (11.1%) | 12 (33.3%) | 13 (35.1%) |

FIG. 5

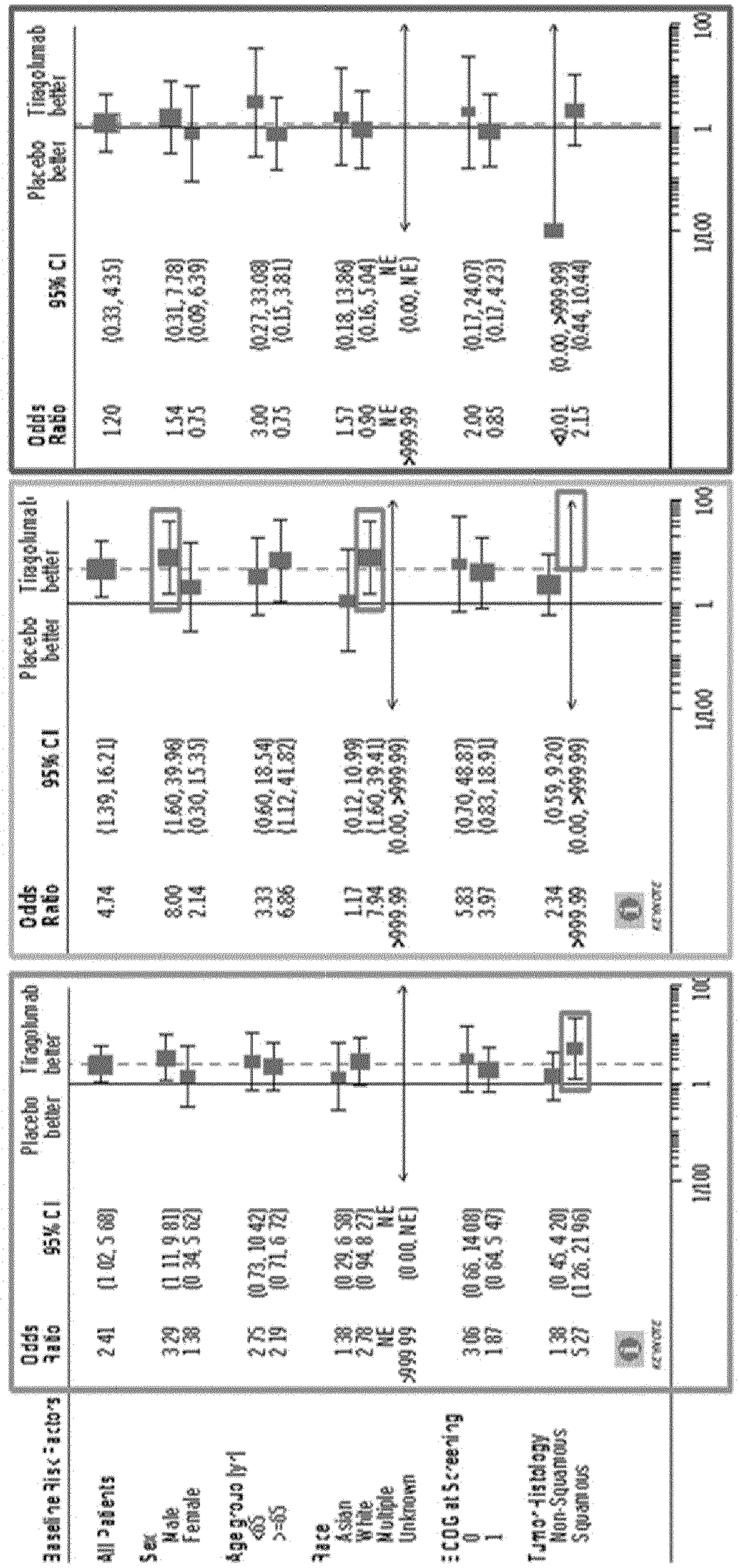
Baseline Risk Factors
All Patients
Sex
Male
Female
Age group (yr)
<65
>=65
Race
Asian
White
Multiple
Unknown
ECOG at Screening
0
1
Tumor Histology
Non-Squamous
Squamous
ITT: TPS ≥ 1%
Odds Ratio
95% CI
Placebo better
Tiragolumab better
2.41 (1.02, 5.68)
3.29 (1.11, 9.81)
1.38 (0.34, 5.62)
2.75 (0.73, 10.42)
2.19 (0.71, 6.72)
1.38 (0.29, 6.58)
2.78 (0.94, 8.27)
NE NE
>999.99 (0.00, NE)
3.06 (0.66, 14.08)
1.87 (0.64, 5.47)
1.38 (0.45, 4.20)
5.27 (1.26, 21.96)
1/100
1
100
Local TPS ≥ 50%
Odds Ratio
95% CI
Placebo better
Tiragolumab better
4.74 (1.39, 16.21)
8.00 (1.60, 39.96)
2.14 (0.30, 15.35)
3.33 (0.60, 18.54)
6.86 (1.12, 41.82)
1.17 (0.12, 10.99)
7.94 (1.60, 39.41)
>999.99 (0.00, >999.99)
5.83 (0.70, 48.87)
3.97 (0.83, 18.91)
2.34 (0.59, 9.20)
>999.99 (0.00, >999.99)
1/100
1
100
Local TPS 1 - 49%
Odds Ratio
95% CI
Placebo better
Tiragolumab better
1.20 (0.33, 4.35)
1.54 (0.31, 7.78)
0.75 (0.09, 6.39)
3.00 (0.27, 33.08)
0.75 (0.15, 3.81)
1.57 (0.18, 13.86)
0.90 (0.16, 5.04)
NE NE
>999.99 (0.00, NE)
2.00 (0.17, 24.07)
0.85 (0.17, 4.23)
<0.01 (0.00, >999.99)
2.15 (0.44, 10.44)
1/100
1
100

# FIG. 6

**All Adverse Events (>5%) Independent of Relationship to Study**

| | Atezolizumab (N=68) | Tiragolumab + Atezolizumab (N=67) |
|---|---|---|
| **Any AE (regardess of attribution)** | | |
| Serious | 19 (27.9%) | 22 (32.8%) |
| Grade 3-5 | 24 (35.3%) | 25 (37.3%) |
| Led to discontinuation | 4 ( 5.9%) | 4 ( 6.0%) |
| Led to death | 3 ( 4.4%) | 2 ( 3.0%) |

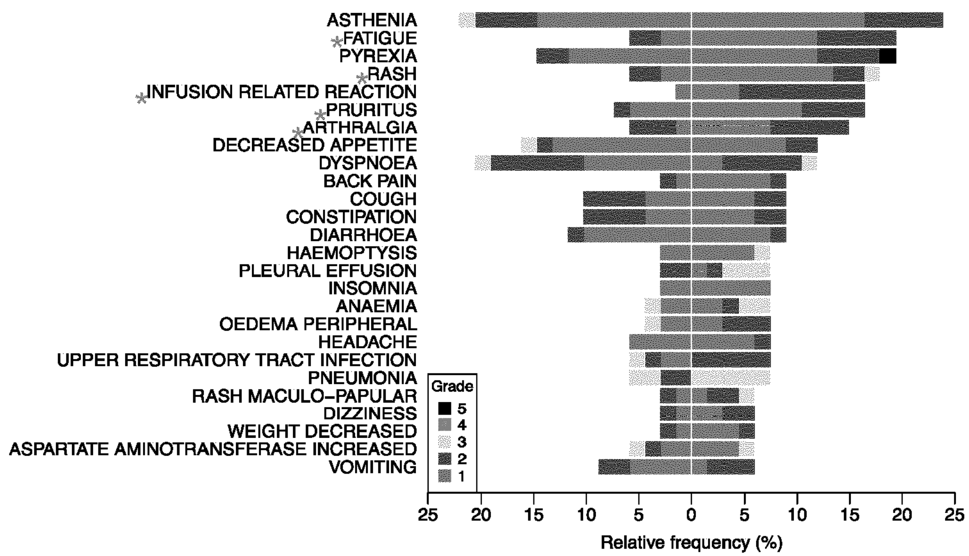

# FIG. 7

| | Atezo (N=68) | Tira + Atezo (N=67) |
|---|---|---|
| **Treatment related AE** | | |
| Any | **38 (55.9%)** | **51 (76.1%)** |
| Serious | 7 (10.3%) | 7 (10.4%) |
| Grade 3-5 | 10 (14.7%) | 9 (13.4%) |
| Led to discontinuation | 4 (5.9%) | 3 (4.5%) |
| **Immune-related AE*** | | |
| **Any** | **23 (33.8%)** | **35 (52.2%)** |
| Rash | 7 (10.3%) | 20 (29.9%) |
| Hepatitis (diagnosis and lab) | 10 (14.7%) | 6 (9.0%) |
| IRR | 1 (1.5%) | 11 (16.4%) |
| Hypothyroidism | 4 (5.9%) | 3 (4.5%) |
| Pancreatitis (diagnosis and lab) | 2 (2.9%) | 5 (7.5%) |
| Hyperthyroidism | 2 (2.9%) | 2 (3.0%) |
| Colitis | 1 (1.5%) | 1 (1.5%) |
| Diabetes Mellitus | 1 (1.5%) | 1 (1.5%) |
| Pneumonitis | 1 (1.5%) | 1 (1.5%) |
| Ocular Inflammatory Toxicity | 1 (1.5%) | 1 (1.5%) |
| Myocarditis | 1 (1.5%) | 0 |
| Nephritis | 0 | 1 (1.5%) |

# FIG. 8A

| Baseline Risk Factors | Total n | Placebo + Atezolizumab (N=68) n | Placebo + Atezolizumab (N=68) n Responder | Placebo + Atezolizumab (N=68) Response (%) | Tiragolumab + Atezolizumab (N=67) n | Tiragolumab + Atezolizumab (N=67) n Responder | Tiragolumab + Atezolizumab (N=67) Response (%) | Odds Ratio | 95% CI |
|---|---|---|---|---|---|---|---|---|---|
| All Patients | 135 | 68 | 11 | 16.2 | 67 | 21 | 31.3 | 2.37 | (1.04, 5.41) |
| Sex | | | | | | | | | |
| Male | 87 | 48 | 7 | 14.6 | 39 | 13 | 33.3 | 2.93 | (1.03, 8.30) |
| Female | 48 | 20 | 4 | 20.0 | 28 | 8 | 28.6 | 1.60 | (0.41, 6.29) |
| Age group (yr) | | | | | | | | | |
| <65 | 56 | 28 | 5 | 17.9 | 28 | 11 | 39.3 | 2.98 | (0.87, 10.17) |
| >=65 | 79 | 40 | 6 | 15.0 | 39 | 10 | 25.6 | 1.95 | (0.63, 6.03) |
| Race | | | | | | | | | |
| Asian | 41 | 23 | 4 | 17.4 | 18 | 5 | 27.8 | 1.83 | (0.41, 8.12) |
| White | 82 | 40 | 7 | 17.5 | 42 | 13 | 31.0 | 2.11 | (0.74, 6.01) |
| Multiple | 1 | 1 | 0 | NE | | | | NE | NE |
| Unknown | 11 | 4 | 0 | NE | 7 | 3 | 42.9 | >999.99 | (0.00, >999.99) |
| ECOG | | | | | | | | | |
| 0 | 39 | 19 | 3 | 15.8 | 20 | 7 | 35.0 | 2.87 | (0.62, 13.37) |
| 1 | 95 | 48 | 7 | 14.6 | 47 | 14 | 29.8 | 2.48 | (0.90, 6.87) |
| 2 | 1 | 1 | 1 | 100 | | | | NE | NE |
| Tumor histology | | | | | | | | | |
| Non-Squamous | 80 | 40 | 7 | 17.5 | 40 | 12 | 30.0 | 2.02 | (0.70, 5.83) |
| Squamous | 55 | 28 | 4 | 14.3 | 27 | 9 | 33.3 | 3.00 | (0.80, 11.31) |
| Region | | | | | | | | | |
| East Asia | 42 | 24 | 4 | 16.7 | 18 | 5 | 27.8 | 1.92 | (0.43, 8.52) |
| Europe | 57 | 29 | 5 | 17.2 | 28 | 12 | 42.9 | 3.60 | (1.06, 12.19) |
| North America | 36 | 15 | 2 | 13.3 | 21 | 4 | 19.0 | 1.53 | (0.24, 9.67) |
| PD-L1 IHC 22C3 pharmDX result | | | | | | | | | |
| TPS 1-49% | 77 | 39 | 6 | 15.4 | 38 | 5 | 13.2 | 0.83 | (0.23, 3.00) |
| TPS >= 50% | 58 | 29 | 5 | 17.2 | 29 | 16 | 55.2 | 5.91 | (1.76, 19.81) |
| HistoGeneX IHC 22C3 membrane staining intensity | | | | | | | | | |
| <1% | 12 | 5 | 0 | NE | 7 | 0 | NE | NE | NE |
| 1-49% | 59 | 35 | 7 | 20.0 | 24 | 4 | 16.7 | 0.80 | (0.21, 3.10) |
| >=50% | 56 | 25 | 4 | 16.0 | 31 | 17 | 54.8 | 6.37 | (1.77, 22.97) |
| NA | 8 | 3 | 0 | NE | 5 | 0 | NE | NE | NE |
| HistoGeneX IHC 22C3 membrane staining intensity | | | | | | | | | |
| <25% | 57 | 30 | 4 | 13.3 | 27 | 3 | 11.1 | 0.81 | (0.16, 4.01) |
| >=25% | 70 | 35 | 7 | 20.0 | 35 | 18 | 51.4 | 4.24 | (1.47, 12.23) |
| NA | 8 | 3 | 0 | NE | 5 | 0 | NE | NE | NE |

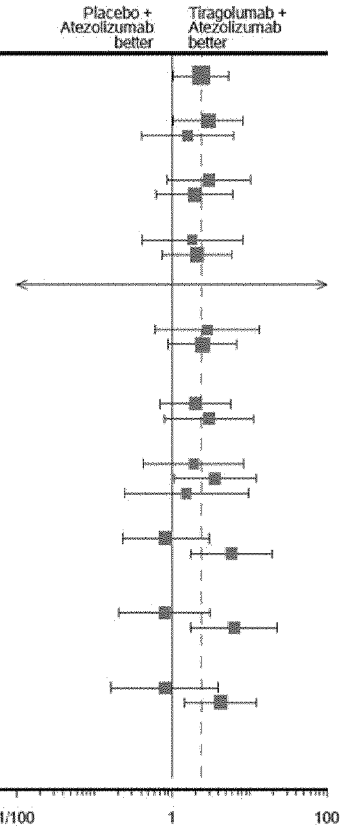

# FIG. 8B

| Baseline Risk Factors | Total n | Placebo + Atezolizumab (N=68) n | Placebo + Atezolizumab (N=68) n Responder | Placebo + Atezolizumab (N=68) Response (%) | Tiragolumab + Atezolizumab (N=67) n | Tiragolumab + Atezolizumab (N=67) n Responder | Tiragolumab + Atezolizumab (N=67) Response (%) | Odds Ratio | 95% CI |
|---|---|---|---|---|---|---|---|---|---|
| All Patients | 135 | 68 | 11 | 16.2 | 67 | 21 | 31.3 | 2.37 | (1.04, 5.41) |
| HistoGeneX IHC CLONE SP263 membrane staining intensity | | | | | | | | | |
| <1% | 17 | 10 | 1 | 10.0 | 7 | 0 | NE | <0.01 | (0.00, >999.99) |
| 1-49% | 51 | 28 | 6 | 21.4 | 23 | 3 | 13.0 | 0.55 | (0.12, 2.50) |
| >=50% | 45 | 20 | 4 | 20.0 | 25 | 13 | 52.0 | 4.33 | (1.13, 16.68) |
| NA | 22 | 10 | 0 | NE | 12 | 5 | 41.7 | >999.99 | (0.00, NE) |
| HistoGeneX IHC CLONE SP263 percent infiltrating immune cells | | | | | | | | | |
| <1% | 17 | 7 | 0 | NE | 10 | 1 | 10.0 | >999.99 | (0.00, >999.99) |
| >=1% | 96 | 51 | 11 | 21.6 | 45 | 15 | 33.3 | 1.82 | (0.73, 4.52) |
| NA | 22 | 10 | 0 | NE | 12 | 5 | 41.7 | >999.99 | (0.00, NE) |
| HistoGeneX IHC CLONE SP263 percent infiltrating immune cells | | | | | | | | | |
| <5% | 51 | 23 | 2 | 8.7 | 28 | 4 | 14.3 | 1.75 | (0.29, 10.54) |
| >=5% | 62 | 35 | 9 | 25.7 | 27 | 12 | 44.4 | 2.31 | (0.79, 6.76) |
| NA | 22 | 10 | 0 | NE | 12 | 5 | 41.7 | >999.99 | (0.00, NE) |
| HistoGeneX IHC CLONE SP263 percent infiltrating immune cells | | | | | | | | | |
| <10% | 91 | 45 | 8 | 17.8 | 46 | 13 | 28.3 | 1.82 | (0.67, 4.94) |
| >=10% | 22 | 13 | 3 | 23.1 | 9 | 3 | 33.3 | 1.67 | (0.25, 11.07) |
| NA | 22 | 10 | 0 | NE | 12 | 5 | 41.7 | >999.99 | (0.00, NE) |
| Spring TIGIT IHC (Ventana) | | | | | | | | | |
| <1% | 16 | 10 | 0 | NE | 6 | 2 | 33.3 | >999.99 | (0.00, NE) |
| >=1% | 89 | 45 | 9 | 20.0 | 44 | 17 | 38.6 | 2.52 | (0.97, 6.51) |
| NA | 30 | 13 | 2 | 15.4 | 17 | 2 | 11.8 | 0.73 | (0.09, 6.04) |
| Spring TIGIT IHC (Ventana) | | | | | | | | | |
| <5% | 56 | 33 | 5 | 15.2 | 23 | 8 | 34.8 | 2.99 | (0.83, 10.76) |
| >=5% | 49 | 22 | 4 | 18.2 | 27 | 11 | 40.7 | 3.09 | (0.82, 11.67) |
| NA | 30 | 13 | 2 | 15.4 | 17 | 2 | 11.8 | 0.73 | (0.09, 6.04) |
| C-reactive protein (Covance) | | | | | | | | | |
| < 3 mg/L | 21 | 11 | 2 | 18.2 | 10 | 3 | 30.0 | 1.93 | (0.25, 14.89) |
| >= 3 mg/L | 106 | 54 | 9 | 16.7 | 52 | 16 | 30.8 | 2.22 | (0.88, 5.61) |
| NA | 8 | 3 | 0 | NE | 5 | 2 | 40.0 | >999.99 | (0.00, NE) |
| Lactate dehydrogenase | | | | | | | | | |
| < ULN | 99 | 49 | 7 | 14.3 | 50 | 12 | 24.0 | 1.89 | (0.68, 5.31) |
| >= ULN | 36 | 19 | 4 | 21.1 | 17 | 9 | 52.9 | 4.22 | (0.98, 18.12) |
| Tobacco history | | | | | | | | | |
| No | 14 | 7 | 2 | 28.6 | 7 | 0 | NE | <0.01 | (0.00, >999.99) |
| Yes | 121 | 61 | 9 | 14.8 | 60 | 21 | 35.0 | 3.11 | (1.28, 7.53) |

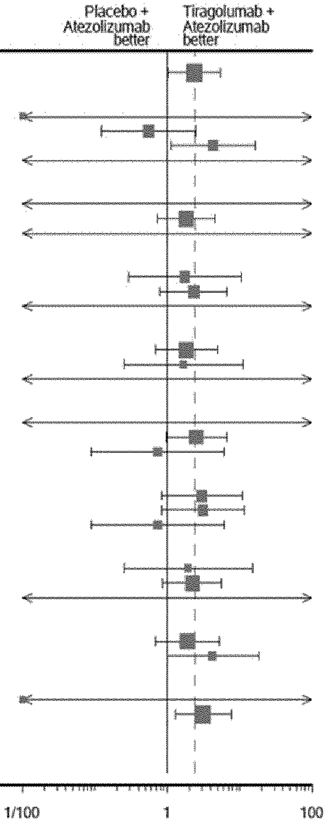

# FIG. 9A

| | Placebo + atezolizumab (*n* = 68) | Tiragolumab + atezolizumab (*n* = 67) |
|---|---|---|
| ORR | 16.2% (11 responders) | 31.3% (21 responders) |
| Difference in ORR (95% CI) | **15.17% (-0.46%, 30.79%)** | |
| Stratified odds ratio (95% CI) | 2.57 (1.07, 6.14) | |

# FIG. 9B

PD-L1 IHC 22C3 TPS ≥ 50%

| | Placebo + atezolizumab ($n$ = 29) | Tiragolumab + atezolizumab ($n$ = 29) |
|---|---|---|
| ORR | 17.2% (5 responders) | **55.2%** (16 responders) |
| Difference in ORR (95% CI) | **37.93% (11.75%, 64.11%)** | |
| Unstratified odds ratio (95% CI) | 5.91 (1.76, 19.81) | |

PD-L1 IHC 22C3 TPS 1 - 49%

| | Placebo + atezolizumab ($n$ = 39) | Tiragolumab + atezolizumab ($n$ = 38) |
|---|---|---|
| ORR | 15.4% (6 responders) | 13.2% (5 responders) |
| Difference in ORR (95% CI) | **-2.23% (-20.44%, 15.98%)** | |
| Unstratified odds ratio (95% CI) | 0.83 (0.23, 3.00) | |

# FIG. 10A

| Baseline Risk Factors | Total n | Placebo + Atezolizumab (N=68) n | Events | Median (Months) | Tiragolumab + Atezolizumab (N=67) n | Events | Median (Months) | Hazard Ratio | 95% Wald CI |
|---|---|---|---|---|---|---|---|---|---|
| All Patients | 135 | 68 | 47 | 3.6 | 67 | 35 | 5.4 | 0.59 | (0.38, 0.92) |
| Sex | | | | | | | | | |
| Male | 87 | 48 | 35 | 4.1 | 39 | 20 | 5.5 | 0.54 | (0.31, 0.94) |
| Female | 48 | 20 | 12 | 3.1 | 28 | 15 | 5.4 | 0.74 | (0.34, 1.59) |
| Age group (yr) | | | | | | | | | |
| <65 | 56 | 28 | 20 | 3.5 | 28 | 10 | NE | 0.29 | (0.13, 0.64) |
| >=65 | 79 | 40 | 27 | 4.2 | 39 | 25 | 4.1 | 0.92 | (0.53, 1.59) |
| Race | | | | | | | | | |
| Asian | 41 | 23 | 19 | 2.7 | 18 | 11 | 4.1 | 0.66 | (0.30, 1.42) |
| White | 82 | 40 | 23 | 4.4 | 42 | 21 | 5.4 | 0.73 | (0.40, 1.32) |
| Multiple | 1 | 1 | 1 | 4.2 | | | | NE | NE |
| Unknown | 11 | 4 | 4 | 3.0 | 7 | 3 | NE | 0.16 | (0.03, 0.92) |
| ECOG | | | | | | | | | |
| 0 | 39 | 19 | 12 | 4.7 | 20 | 9 | 5.6 | 0.63 | (0.26, 1.51) |
| 1 | 95 | 48 | 35 | 2.8 | 47 | 26 | 5.4 | 0.54 | (0.32, 0.90) |
| 2 | 1 | 1 | 0 | NE | | | | NE | NE |
| Tumor histology | | | | | | | | | |
| Non-Squamous | 80 | 40 | 28 | 3.5 | 40 | 24 | 5.4 | 0.59 | (0.34, 1.04) |
| Squamous | 55 | 28 | 19 | 4.1 | 27 | 11 | NE | 0.54 | (0.25, 1.13) |
| Region | | | | | | | | | |
| East Asia | 42 | 24 | 20 | 2.7 | 18 | 11 | 4.1 | 0.66 | (0.31, 1.41) |
| Europe | 57 | 29 | 16 | 4.1 | 28 | 11 | NE | 0.57 | (0.26, 1.23) |
| North America | 36 | 15 | 11 | 4.2 | 21 | 13 | 5.4 | 0.63 | (0.28, 1.41) |
| PD-L1 IHC 22C3 pharmDX result | | | | | | | | | |
| TPS 1-49% | 77 | 39 | 27 | 3.6 | 38 | 25 | 4.1 | 0.85 | (0.49, 1.48) |
| TPS >= 50% | 58 | 29 | 20 | 3.9 | 29 | 10 | NE | 0.33 | (0.15, 0.72) |
| HistoGeneX IHC 22C3 membrane staining intensity | | | | | | | | | |
| <1% | 12 | 5 | 3 | 2.7 | 7 | 6 | 1.4 | 1.67 | (0.41, 6.91) |
| 1-49% | 59 | 35 | 24 | 4.2 | 24 | 14 | 4.1 | 0.86 | (0.44, 1.67) |
| >=50% | 56 | 25 | 17 | 3.9 | 31 | 10 | NE | 0.28 | (0.13, 0.63) |
| NA | 8 | 3 | 3 | 1.3 | 5 | 5 | 5.5 | <0.01 | (0.00, NE) |
| HistoGeneX IHC 22C3 membrane staining intensity | | | | | | | | | |
| <25% | 57 | 30 | 21 | 2.8 | 27 | 18 | 3.9 | 0.94 | (0.50, 1.78) |
| >=25% | 70 | 35 | 23 | 4.1 | 35 | 12 | NE | 0.38 | (0.19, 0.77) |
| NA | 8 | 3 | 3 | 1.3 | 5 | 5 | 5.5 | <0.01 | (0.00, NE) |

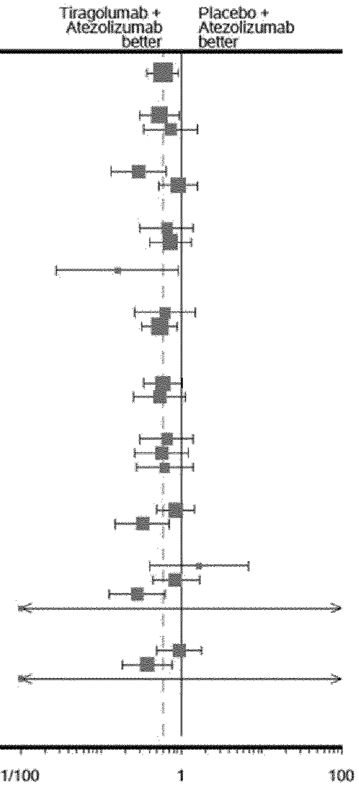

# FIG. 10B

| Baseline Risk Factors | Total n | Placebo + Atezolizumab (N=68) n | Events | Median (Months) | Tiragolumab + Atezolizumab (N=67) n | Events | Median (Months) | Hazard Ratio | 95% Wald CI |
|---|---|---|---|---|---|---|---|---|---|
| All Patients | 135 | 68 | 47 | 3.6 | 67 | 35 | 5.4 | 0.59 | (0.38, 0.92) |
| HistoGeneX IHC CLONE SP263 membrane staining intensity | | | | | | | | | |
| <1% | 17 | 10 | 9 | 1.4 | 7 | 6 | 1.4 | 0.88 | (0.31, 2.51) |
| 1-49% | 51 | 28 | 19 | 4.2 | 23 | 14 | 4.2 | 0.96 | (0.48, 1.93) |
| >=50% | 45 | 20 | 14 | 3.6 | 25 | 8 | NE | 0.25 | (0.10, 0.60) |
| NA | 22 | 10 | 5 | 7.0 | 12 | 7 | 5.5 | 0.82 | (0.22, 3.05) |
| HistoGeneX IHC CLONE SP263 percent infiltrating immune cells | | | | | | | | | |
| <1% | 17 | 7 | 6 | 3.4 | 10 | 4 | NE | 0.25 | (0.07, 0.94) |
| >=1% | 96 | 51 | 36 | 3.6 | 45 | 24 | 5.1 | 0.65 | (0.38, 1.09) |
| NA | 22 | 10 | 5 | 7.0 | 12 | 7 | 5.5 | 0.82 | (0.22, 3.05) |
| HistoGeneX IHC CLONE SP263 percent infiltrating immune cells | | | | | | | | | |
| <5% | 51 | 23 | 19 | 2.7 | 28 | 14 | 5.4 | 0.43 | (0.21, 0.86) |
| >=5% | 62 | 35 | 23 | 4.2 | 27 | 14 | 5.1 | 0.70 | (0.36, 1.38) |
| NA | 22 | 10 | 5 | 7.0 | 12 | 7 | 5.5 | 0.82 | (0.22, 3.05) |
| HistoGeneX IHC CLONE SP263 percent infiltrating immune cells | | | | | | | | | |
| <10% | 91 | 45 | 36 | 3.3 | 46 | 23 | 5.4 | 0.47 | (0.28, 0.80) |
| >=10% | 22 | 13 | 6 | 4.2 | 9 | 5 | 4.2 | 1.26 | (0.38, 4.18) |
| NA | 22 | 10 | 5 | 7.0 | 12 | 7 | 5.5 | 0.82 | (0.22, 3.05) |
| Spring TIGIT IHC (Ventana) | | | | | | | | | |
| <1% | 16 | 10 | 10 | 2.2 | 6 | 2 | NE | 0.24 | (0.05, 1.14) |
| >=1% | 89 | 45 | 28 | 4.2 | 44 | 19 | NE | 0.55 | (0.31, 0.99) |
| NA | 30 | 13 | 9 | 1.4 | 17 | 14 | 3.9 | 0.87 | (0.36, 2.06) |
| Spring TIGIT IHC (Ventana) | | | | | | | | | |
| <5% | 56 | 33 | 25 | 3.1 | 23 | 9 | NE | 0.33 | (0.15, 0.71) |
| >=5% | 49 | 22 | 13 | 4.5 | 27 | 12 | 5.5 | 0.80 | (0.36, 1.77) |
| NA | 30 | 13 | 9 | 1.4 | 17 | 14 | 3.9 | 0.87 | (0.36, 2.06) |
| C-reactive protein (Covance) | | | | | | | | | |
| < 3 mg/L | 21 | 11 | 8 | 3.6 | 10 | 4 | NE | 0.37 | (0.11, 1.26) |
| >= 3 mg/L | 106 | 54 | 36 | 4.1 | 52 | 29 | 5.4 | 0.72 | (0.44, 1.17) |
| NA | 8 | 3 | 3 | 0.8 | 5 | 2 | NE | 0.13 | (0.01, 1.35) |
| Lactate dehydrogenase | | | | | | | | | |
| < ULN | 99 | 49 | 34 | 4.1 | 50 | 28 | 5.4 | 0.65 | (0.39, 1.09) |
| >= ULN | 36 | 19 | 13 | 2.1 | 17 | 7 | NE | 0.44 | (0.17, 1.12) |
| Tobacco history | | | | | | | | | |
| No | 14 | 7 | 5 | 2.7 | 7 | 4 | 4.1 | 0.80 | (0.21, 3.03) |
| Yes | 121 | 61 | 42 | 3.9 | 60 | 31 | 5.5 | 0.57 | (0.35, 0.91) |

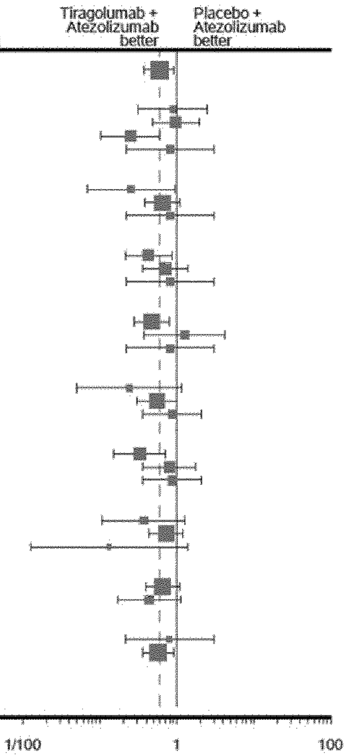

# FIG. 11A

| | Placebo + Atezo (*n* = 68) | Tira + Atezo (*n* = 67) |
|---|---|---|
| **Patients with event (%)** | 47 (69.1%) | 35 (52.2%) |
| **Median (months) (95% CI)** | 3.58 (2.73, 4.44) | 5.42 (4.21, NE) |
| **Stratified HR (95% CI)** | 0.57 (0.37, 0.90) | |

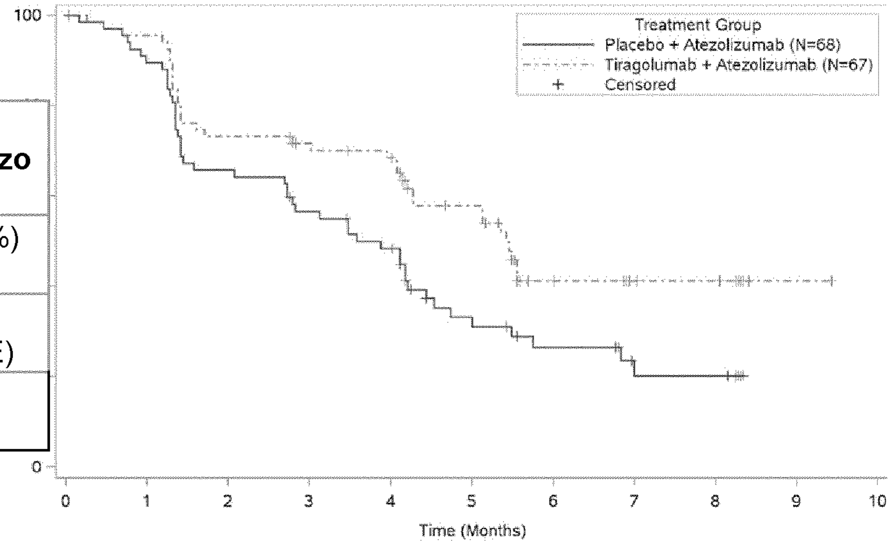


| Patients remaining at risk | | | | | | | | | | | |
|---|---|---|---|---|---|---|---|---|---|---|---|
| Placebo + Atezolizumab (N=68) | 68 | 60 | 44 | 35 | 29 | 15 | 11 | 6 | 6 | NE | NE |
| Tiragolumab + Atezolizumab (N=67) | 67 | 64 | 49 | 45 | 42 | 30 | 14 | 9 | 8 | 1 | NE |

Only deaths for which a complete date is available are considered events.

# FIG. 11B

| | Placebo + Atezo ($n$ = 29) | Tira + Atezo ($n$ = 29) |
|---|---|---|
| **Patients with event (%)** | 20 (69.0%) | 10 (34.5%) |
| **Median (months) (95% CI)** | 3.88 (2.07, 4.73) | NE (5.42, NE) |
| **Unstratified HR (95% CI)** | 0.33 (0.15, 0.72) | |

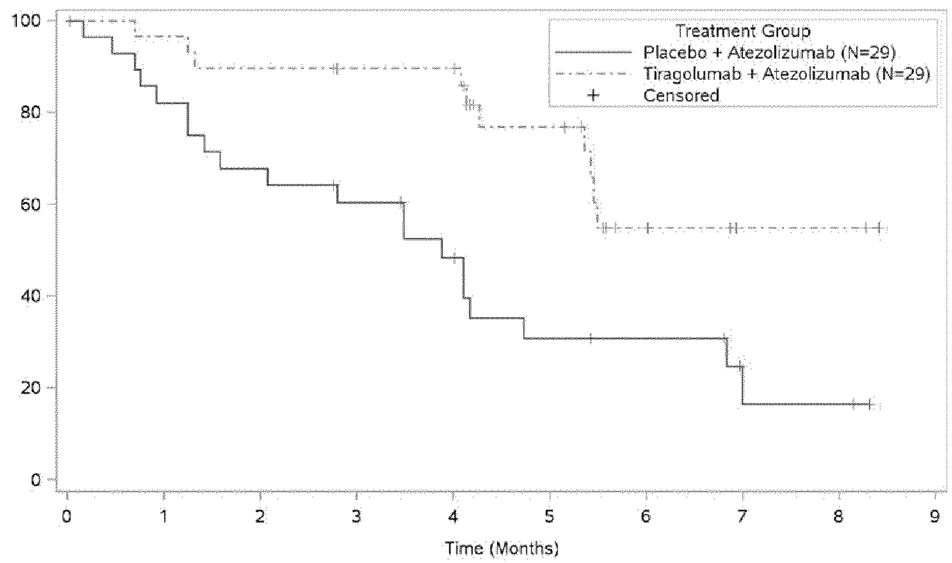


| Patients remaining at risk | | | | | | | | | | |
|---|---|---|---|---|---|---|---|---|---|---|
| Placebo + Atezolizumab (N=29) | 29 | 23 | 19 | 16 | 12 | 7 | 6 | 2 | 2 | NE |
| Tiragolumab + Atezolizumab (N=29) | 29 | 28 | 26 | 24 | 24 | 16 | 6 | 2 | 2 | NE |

Only deaths for which a complete date is available are considered events.

# FIG. 11C

| | Placebo + Atezo (*n* = 39) | Tira + Atezo (*n* = 38) |
|---|---|---|
| **Patients with event (%)** | 27 (69.2%) | 25 (65.8%) |
| **Median (months) (95% CI)** | 3.58 (1.45, 4.99) | 4.07 (1.61, 5.55) |
| **Unstratified HR (95% CI)** | 0.85 (0.49, 1.48) | |

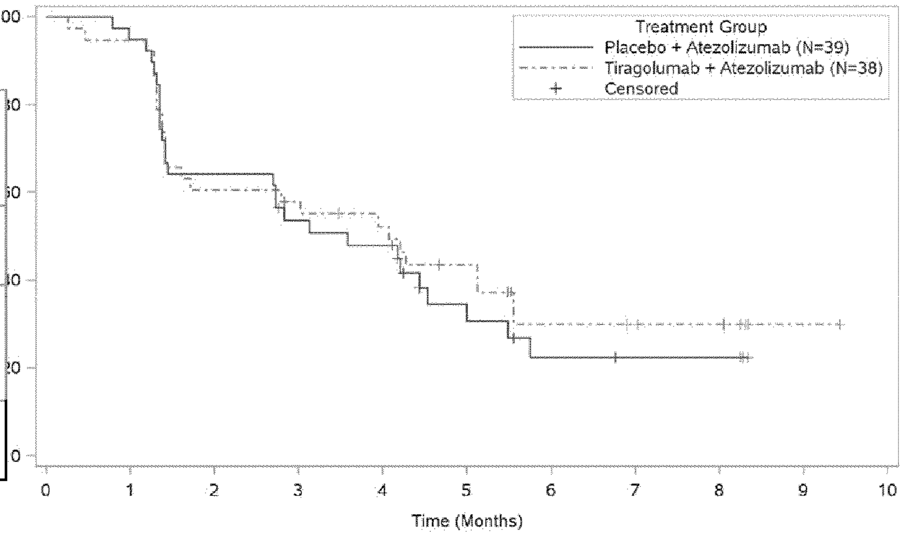


| Patients remaining at risk | | | | | | | | | | | |
|---|---|---|---|---|---|---|---|---|---|---|---|
| Placebo + Atezolizumab (N=39) | 39 | 37 | 25 | 19 | 17 | 8 | 5 | 4 | 4 | NE | NE |
| Tiragolumab + Atezolizumab (N=38) | 38 | 36 | 23 | 21 | 18 | 14 | 8 | 7 | 6 | 1 | NE |

Only deaths for which a complete date is available are considered events.

# FIG. 12A

| Baseline Risk Factors | Total n | Placebo + Atezolizumab (N=68) n | Events | Median (Months) | Tiragolumab + Atezolizumab (N=67) n | Events | Median (Months) | Hazard Ratio | 95% Wald CI |
|---|---|---|---|---|---|---|---|---|---|
| **All Patients** | 135 | 68 | 20 | NE | 67 | 14 | NE | 0.61 | (0.31, 1.21) |
| **Sex** | | | | | | | | | |
| Male | 87 | 48 | 16 | 8.3 | 39 | 6 | NE | 0.41 | (0.16, 1.06) |
| Female | 48 | 20 | 4 | NE | 28 | 8 | NE | 1.23 | (0.37, 4.09) |
| **Age group (yr)** | | | | | | | | | |
| <65 | 56 | 28 | 4 | NE | 28 | 4 | NE | 0.67 | (0.17, 2.70) |
| >=65 | 79 | 40 | 16 | 8.2 | 39 | 10 | NE | 0.69 | (0.31, 1.53) |
| **Race** | | | | | | | | | |
| Asian | 41 | 23 | 6 | 8.2 | 18 | 5 | NE | 1.22 | (0.35, 4.23) |
| White | 82 | 40 | 12 | NE | 42 | 9 | NE | 0.62 | (0.26, 1.48) |
| Multiple | 1 | 1 | 0 | NE | | | | NE | NE |
| Unknown | 11 | 4 | 2 | 4.7 | 7 | 0 | NE | <0.01 | (0.00, NE) |
| **ECOG** | | | | | | | | | |
| 0 | 39 | 19 | 2 | NE | 20 | 4 | NE | 1.66 | (0.30, 9.07) |
| 1 | 95 | 48 | 18 | 8.2 | 47 | 10 | NE | 0.47 | (0.22, 1.02) |
| 2 | 1 | 1 | 0 | NE | | | | NE | NE |
| **Tumor histology** | | | | | | | | | |
| Non-Squamous | 80 | 40 | 12 | NE | 40 | 9 | NE | 0.63 | (0.26, 1.50) |
| Squamous | 55 | 28 | 8 | NE | 27 | 5 | NE | 0.57 | (0.19, 1.76) |
| **Region** | | | | | | | | | |
| East Asia | 42 | 24 | 6 | 8.2 | 18 | 5 | NE | 1.31 | (0.38, 4.55) |
| Europe | 57 | 29 | 6 | NE | 28 | 3 | NE | 0.45 | (0.11, 1.81) |
| North America | 36 | 15 | 8 | 6.0 | 21 | 6 | NE | 0.44 | (0.15, 1.26) |
| **PD-L1 IHC 22C3 pharmDX result** | | | | | | | | | |
| TPS 1-49% | 77 | 39 | 11 | NE | 38 | 10 | NE | 0.87 | (0.37, 2.05) |
| TPS >= 50% | 58 | 29 | 9 | NE | 29 | 4 | NE | 0.35 | (0.11, 1.14) |
| **HistoGeneX IHC 22C3 membrane staining intensity** | | | | | | | | | |
| <1% | 12 | 5 | 1 | NE | 7 | 4 | 6.1 | 2.57 | (0.29, 23.23) |
| 1-49% | 59 | 35 | 11 | NE | 24 | 5 | NE | 0.68 | (0.23, 1.96) |
| >=50% | 56 | 25 | 8 | 8.3 | 31 | 2 | NE | 0.13 | (0.03, 0.64) |
| NA | 8 | 3 | 0 | NE | 5 | 3 | 5.6 | >999.99 | (0.00, NE) |
| **HistoGeneX IHC 22C3 membrane staining intensity** | | | | | | | | | |
| <25% | 57 | 30 | 11 | 8.2 | 27 | 7 | NE | 0.62 | (0.24, 1.59) |
| >=25% | 70 | 35 | 9 | NE | 35 | 4 | NE | 0.38 | (0.12, 1.24) |
| NA | 8 | 3 | 0 | NE | 5 | 3 | 5.6 | >999.99 | (0.00, NE) |

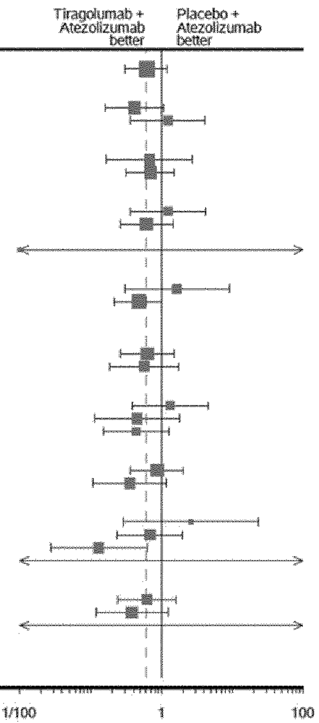

# FIG. 12B

| Baseline Risk Factors | Total n | Placebo + Atezolizumab (N=68) n | Events | Median (Months) | Tiragolumab + Atezolizumab (N=67) n | Events | Median (Months) | Hazard Ratio | 95% Wald CI |
|---|---|---|---|---|---|---|---|---|---|
| All Patients | 135 | 68 | 20 | NE | 67 | 14 | NE | 0.61 | (0.31, 1.21) |
| HistoGeneX IHC CLONE SP263 membrane staining intensity | | | | | | | | | |
| <1% | 17 | 10 | 6 | 6.0 | 7 | 2 | NE | 0.40 | (0.08, 2.03) |
| 1-49% | 51 | 28 | 9 | NE | 23 | 5 | NE | 0.71 | (0.24, 2.13) |
| >=50% | 45 | 20 | 5 | NE | 25 | 3 | NE | 0.34 | (0.08, 1.44) |
| NA | 22 | 10 | 0 | NE | 12 | 4 | NE | >999.99 | (0.00, NE) |
| HistoGeneX IHC CLONE SP263 percent infiltrating immune cells | | | | | | | | | |
| <1% | 17 | 7 | 3 | 8.2 | 10 | 2 | NE | 0.35 | (0.06, 2.12) |
| >=1% | 96 | 51 | 17 | NE | 45 | 8 | NE | 0.48 | (0.21, 1.12) |
| NA | 22 | 10 | 0 | NE | 12 | 4 | NE | >999.99 | (0.00, NE) |
| HistoGeneX IHC CLONE SP263 percent infiltrating immune cells | | | | | | | | | |
| <5% | 51 | 23 | 10 | 8.2 | 28 | 6 | NE | 0.38 | (0.14, 1.06) |
| >=5% | 62 | 35 | 10 | NE | 27 | 4 | NE | 0.50 | (0.16, 1.59) |
| NA | 22 | 10 | 0 | NE | 12 | 4 | NE | >999.99 | (0.00, NE) |
| HistoGeneX IHC CLONE SP263 percent infiltrating immune cells | | | | | | | | | |
| <10% | 91 | 45 | 18 | 8.2 | 46 | 8 | NE | 0.34 | (0.15, 0.79) |
| >=10% | 22 | 13 | 2 | NE | 9 | 2 | NE | 1.55 | (0.22, 11.05) |
| NA | 22 | 10 | 0 | NE | 12 | 4 | NE | >999.99 | (0.00, NE) |
| Spring TIGIT IHC (Ventana) | | | | | | | | | |
| <1% | 16 | 10 | 5 | 4.7 | 6 | 2 | NE | 0.57 | (0.11, 2.99) |
| >=1% | 89 | 45 | 14 | 8.3 | 44 | 4 | NE | 0.25 | (0.08, 0.76) |
| NA | 30 | 13 | 1 | NE | 17 | 8 | 6.1 | 5.26 | (0.66, 42.06) |
| Spring TIGIT IHC (Ventana) | | | | | | | | | |
| <5% | 56 | 33 | 13 | 8.2 | 23 | 3 | NE | 0.25 | (0.07, 0.90) |
| >=5% | 49 | 22 | 6 | NE | 27 | 3 | NE | 0.44 | (0.11, 1.79) |
| NA | 30 | 13 | 1 | NE | 17 | 8 | 6.1 | 5.26 | (0.66, 42.06) |
| C-reactive protein (Covance) | | | | | | | | | |
| < 3 mg/L | 21 | 11 | 1 | NE | 10 | 3 | NE | 3.45 | (0.35, 34.18) |
| >= 3 mg/L | 106 | 54 | 17 | NE | 52 | 10 | NE | 0.53 | (0.24, 1.17) |
| NA | 8 | 3 | 2 | 2.6 | 5 | 1 | NE | 0.18 | (0.02, 2.05) |
| Lactate dehydrogenase | | | | | | | | | |
| < ULN | 99 | 49 | 14 | NE | 50 | 10 | NE | 0.66 | (0.29, 1.50) |
| >= ULN | 36 | 19 | 6 | 5.9 | 17 | 4 | NE | 0.45 | (0.12, 1.66) |
| Tobacco history | | | | | | | | | |
| No | 14 | 7 | 1 | 8.2 | 7 | 1 | NE | 0.91 | (0.06, 14.63) |
| Yes | 121 | 61 | 19 | NE | 60 | 13 | NE | 0.59 | (0.29, 1.19) |

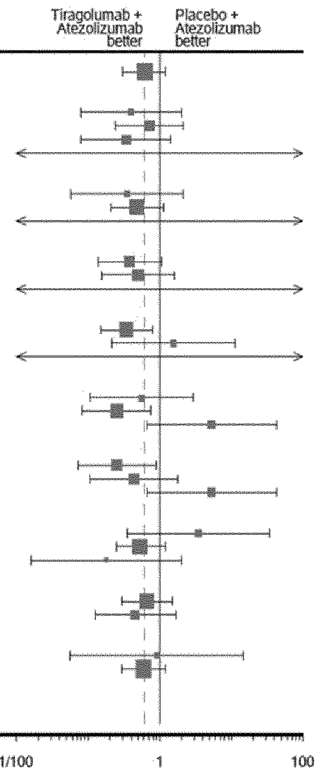

# FIG. 13A

| | Placebo + Atezo (*n* = 68) | Tira + Atezo (*n* = 67) |
|---|---|---|
| Patients with event (%) | 20 (29.4%) | 14 (20.9%) |
| Median (months) (95% CI) | NE (8.21, NE) | NE (NE, NE) |
| Stratified HR (95% CI) | 0.61 (0.31, 1.22) | |
| 6-mo event-free rate (%) | 68.71% | 79.02% |
| Difference (95% CI) | 10.31% (-6.49%, 27.12%) | |

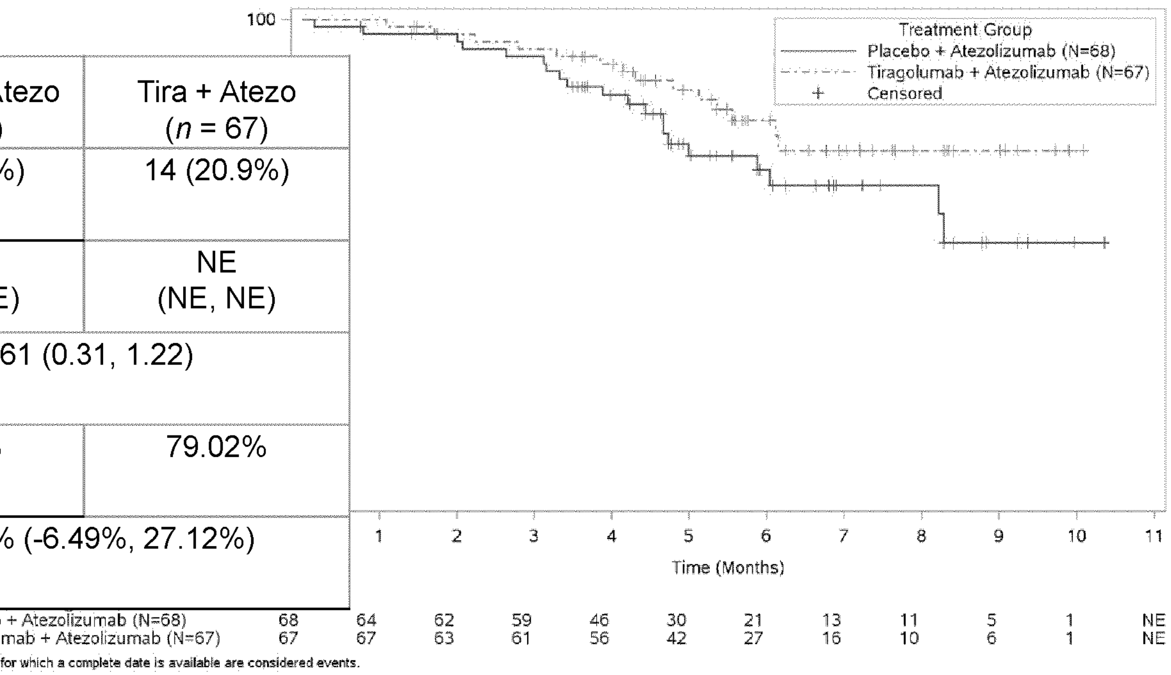

# FIG. 13B

| | Placebo + Atezo (*n* = 29) | Tira + Atezo (*n* = 29) |
|---|---|---|
| Patients with event (%) | 9 (31.0%) | 4 (13.8%) |
| Median (months) (95% CI) | NE (4.73, NE) | NE (NE, NE) |
| Unstratified HR (95% CI) | 0.35 (0.11, 1.14) | |
| 6-mo event-free rate (%) | 67.51% | 87.72% |
| Difference (95% CI) | 20.21% (-2.85%, 43.26%) | |

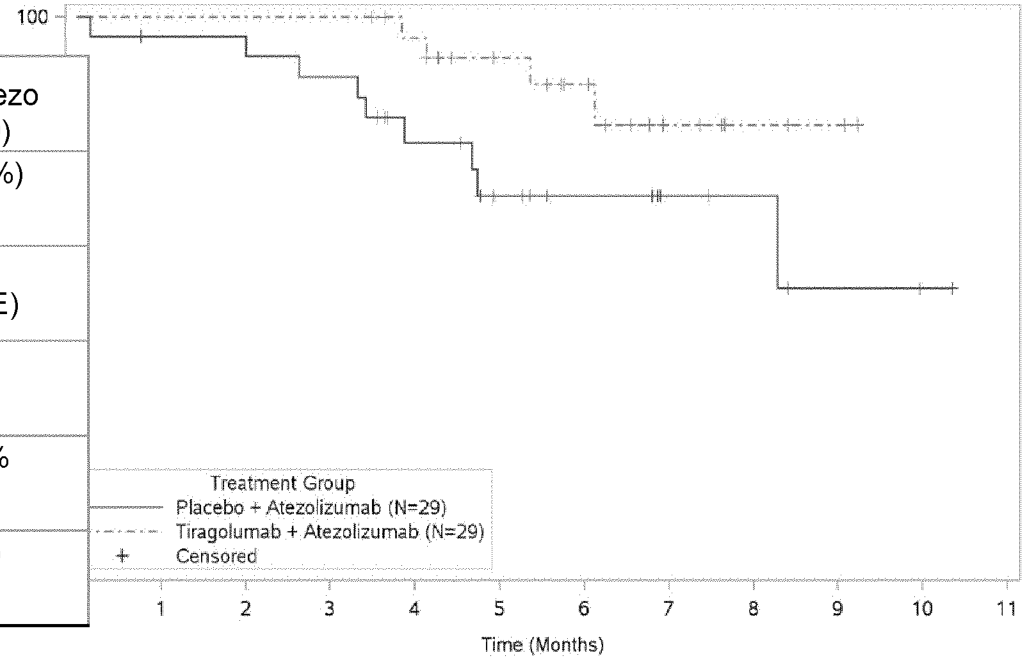


| Patients remaining at risk | | | | | | | | | | | | |
|---|---|---|---|---|---|---|---|---|---|---|---|---|
| Placebo + Atezolizumab (N=29) | 29 | 26 | 26 | 24 | 17 | 12 | 8 | 5 | 4 | 2 | 1 | NE |
| Tiragolumab + Atezolizumab (N=29) | 29 | 29 | 29 | 29 | 26 | 19 | 13 | 6 | 3 | 2 | NE | NE |

Only deaths for which a complete date is available are considered events.

# FIG. 13C

| | Placebo + Atezo (*n* = 39) | Tira + Atezo (*n* = 38) |
|---|---|---|
| Patients with event (%) | 11 (28.2%) | 10 (26.3%) |
| Median (months) (95% CI) | NE (6.05, NE) | NE (6.14, NE) |
| Unstratified HR (95% CI) | 0.87 (0.37, 2.05) | |
| 6-mo event-free rate (%) | 70.1% | 72.2% |
| Difference (95% CI) | 2.12% (-21.18%, 25.41%) | |

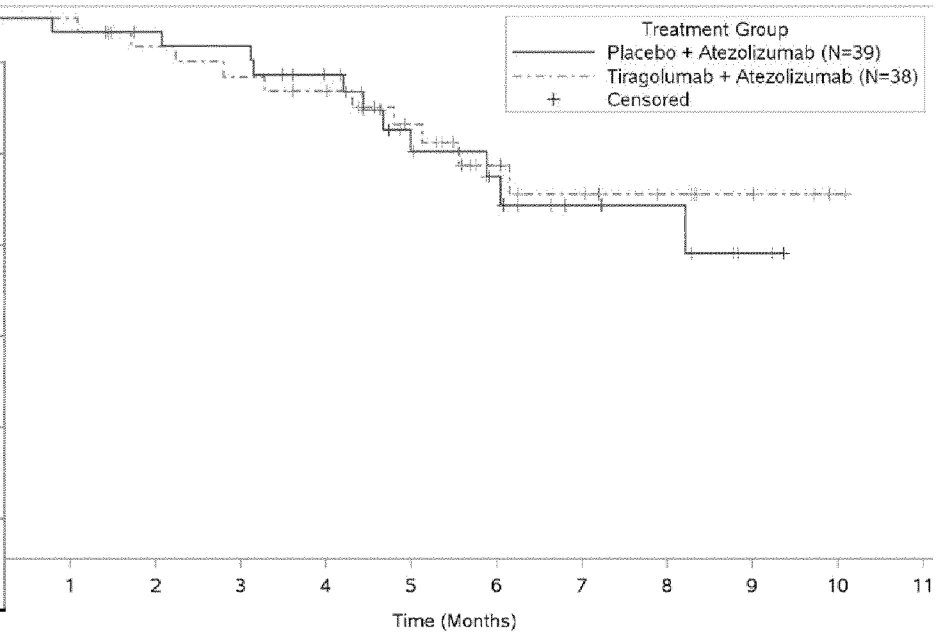


| Patients remaining at risk | | | | | | | | | | | |
|---|---|---|---|---|---|---|---|---|---|---|---|
| Placebo + Atezolizumab (N=39) | 39 | 38 | 36 | 35 | 29 | 18 | 13 | 8 | 7 | 3 | NE | NE |
| Tiragolumab + Atezolizumab (N=38) | 38 | 38 | 34 | 32 | 30 | 23 | 14 | 10 | 7 | 4 | 1 | NE |

Only deaths for which a complete date is available are considered events.

FIG. 14A

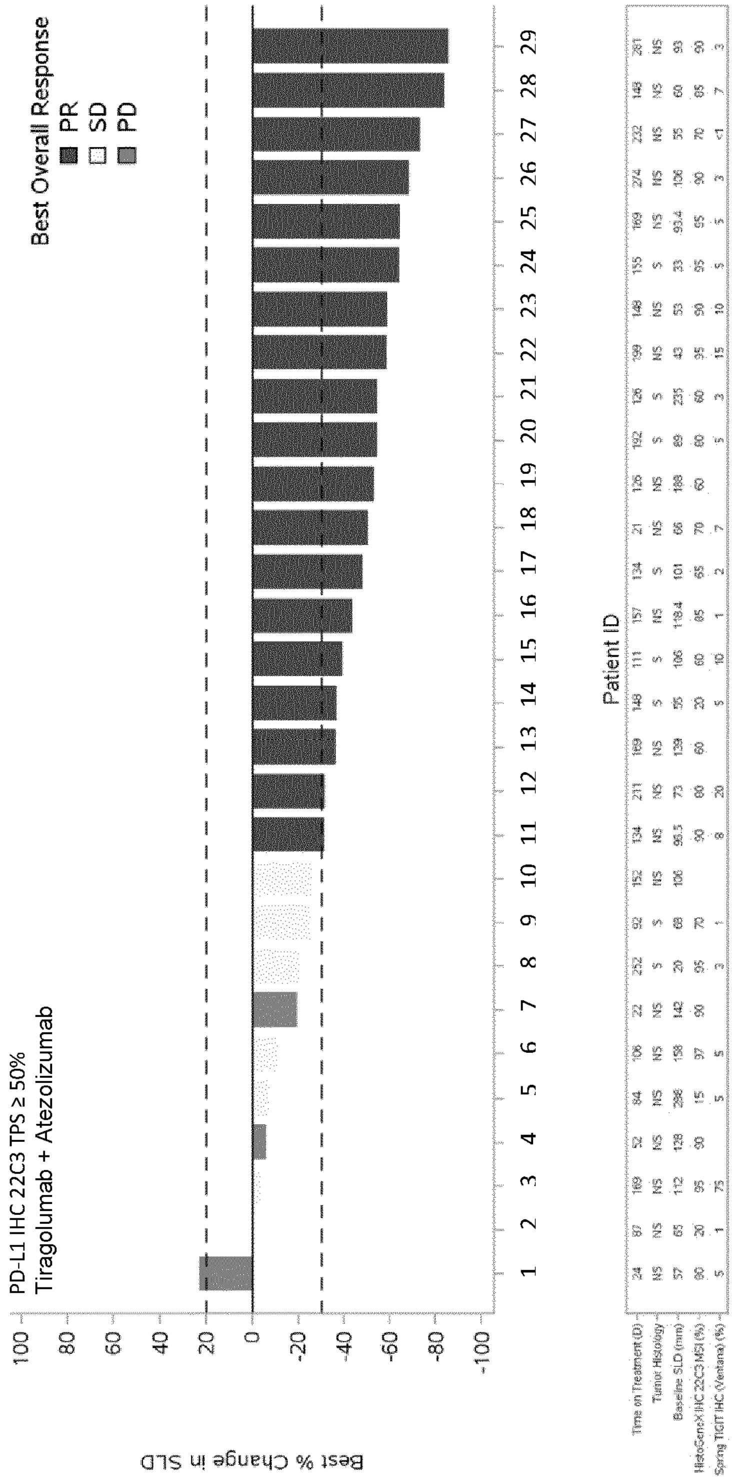
PD-L1 IHC 22C3 TPS ≥ 50%
Tiragolumab + Atezolizumab
Best Overall Response
PR
SD
PD
Best % Change in SLD
100
80
60
40
20
0
-20
-40
-60
-80
-100
1 2 3 4 5 6 7 8 9 10 11 12 13 14 15 16 17 18 19 20 21 22 23 24 25 26 27 28 29
Patient ID
Time on Treatment (D)
Tumor Histology
Baseline SLD (mm)
HistoGeneX IHC 22C3 MSI (%)
Spring TIGIT IHC (Ventana) (%)

# FIG. 14B

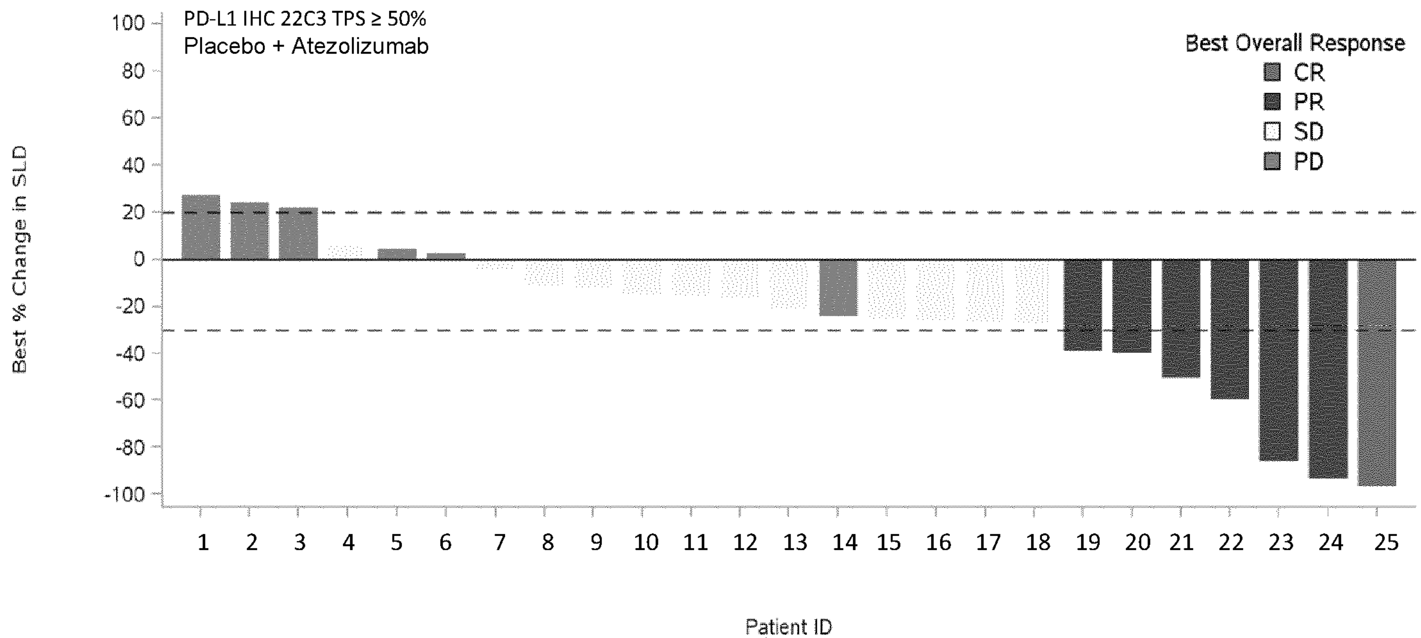


| | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 |
|---|---|---|---|---|---|---|---|---|---|---|---|---|---|---|---|---|---|---|---|---|---|---|---|---|---|
| Time on Treatment (D) | 1 | 1 | 43 | 46 | 63 | 94 | 70 | 225 | 106 | 89 | 232 | 126 | 85 | 134 | 85 | 104 | 262 | 128 | 84 | 150 | 107 | 189 | 86 | 190 | 253 |
| Tumor Histology | NS | NS | NS | NS | S | NS | S | S | NS | NS | NS | NS | S | NS | NS | S | S | S | NS | S | NS | NS | NS | NS | NS |
| Baseline SLD (mm) | 160 | 46 | 284 | 35 | 159.9 | 88 | 122 | 72 | 118 | 164 | 52 | 73 | 183 | 42 | 18.01 | 97 | 83 | 22 | 13 | 41 | 30 | 156 | 61 | 124 | 15 |
| HistoGeneX IHC 22C3 MSI (%) | 90 | 30 | 80 | 90 | 90 | 60 | 100 | 90 | 95 | 95 | 60 | 90 | 80 | 50 | 80 | 95 | 10 | 100 | 50 | 40 | 0.5 | 90 | 90 | 85 | 20 |
| Spring TIGIT IHC (Ventana) (%) | 2 | | <1 | 2 | 10 | <1 | 8 | 15 | | <1 | 5 | | 5 | 2 | 5 | 15 | 10 | | | 1 | 3 | 25 | 1 | 8 | |

# FIG. 14C

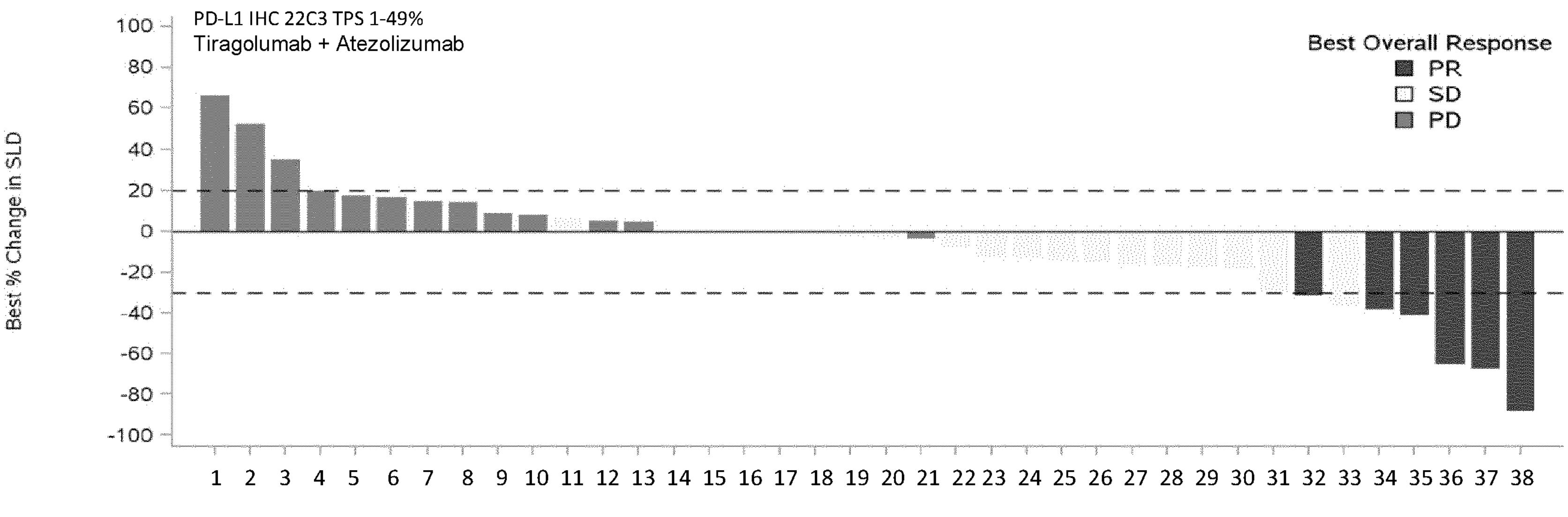


| | 1 | 2 | 3 | 4 | 5 | 6 | 7 | 8 | 9 | 10 | 11 | 12 | 13 | 14 | 15 | 16 | 17 | 18 | 19 |
|---|---|---|---|---|---|---|---|---|---|---|---|---|---|---|---|---|---|---|---|
| Time on Treatment (D) | 22 | 1 | 1 | 23 | 27 | 22 | 43 | 22 | 22 | 22 | 130 | 23 | 64 | 143 | 125 | 64 | 154 | 22 | 78 |
| Tumor Histology | S | S | S | S | NS | NS | NS | S | NS | NS | NS | S | NS | S | NS | NS | NS | S | S |
| Baseline SLD (mm) | 47 | 19 | 49 | 67 | 67.5 | 151 | 137.7 | 129 | 91 | 152 | 47.1 | 141 | 22 | 25 | 19 | 15 | 43 | 49.5 | 75 |
| HistoGeneX IHC 22C3 MSI (%) | 0.5 | 0.5 | 0.5 | 10 | 90 | 35 | 0.5 | 1 | 1 | 15 | 0.5 | 15 | 1 | 50 | | 20 | 70 | 30 | |
| Spring TIGIT IHC (Ventana) (%) | 5 | <1 | <1 | 7 | 5 | 5 | | 1 | 5 | 7 | | 2 | | 3 | | | 2 | | |

| | 20 | 21 | 22 | 23 | 24 | 25 | 26 | 27 | 28 | 29 | 30 | 31 | 32 | 33 | 34 | 35 | 36 | 37 | 38 |
|---|---|---|---|---|---|---|---|---|---|---|---|---|---|---|---|---|---|---|---|
| Time on Treatment (D) | 150 | 19 | 141 | 253 | 43 | 252 | 132 | 1 | 70 | 113 | 259 | 210 | 167 | 239 | 306 | 190 | 274 | 110 | 212 |
| Tumor Histology | NS | NS | NS | S | NS | NS | S | S | S | NS | NS | S | NS | NS | S | S | S | S | S |
| Baseline SLD (mm) | 110 | 192 | 39 | 97 | 126 | 36 | 34 | 117 | 122 | 47 | 51 | 78 | 84 | 25 | 53 | 110 | 60 | 97 | 98 |
| HistoGeneX IHC 22C3 MSI (%) | | 1 | | 2 | 0.5 | 15 | 95 | 30 | 15 | 5 | 15 | 8 | 20 | 0 | 90 | 60 | 35 | 20 | 1 |
| Spring TIGIT IHC (Ventana) (%) | | | | <1 | | 1 | 15 | <1 | 5 | 2 | 3 | 5 | 2 | | 20 | <1 | 3 | 15 | 10 |

FIG. 14D

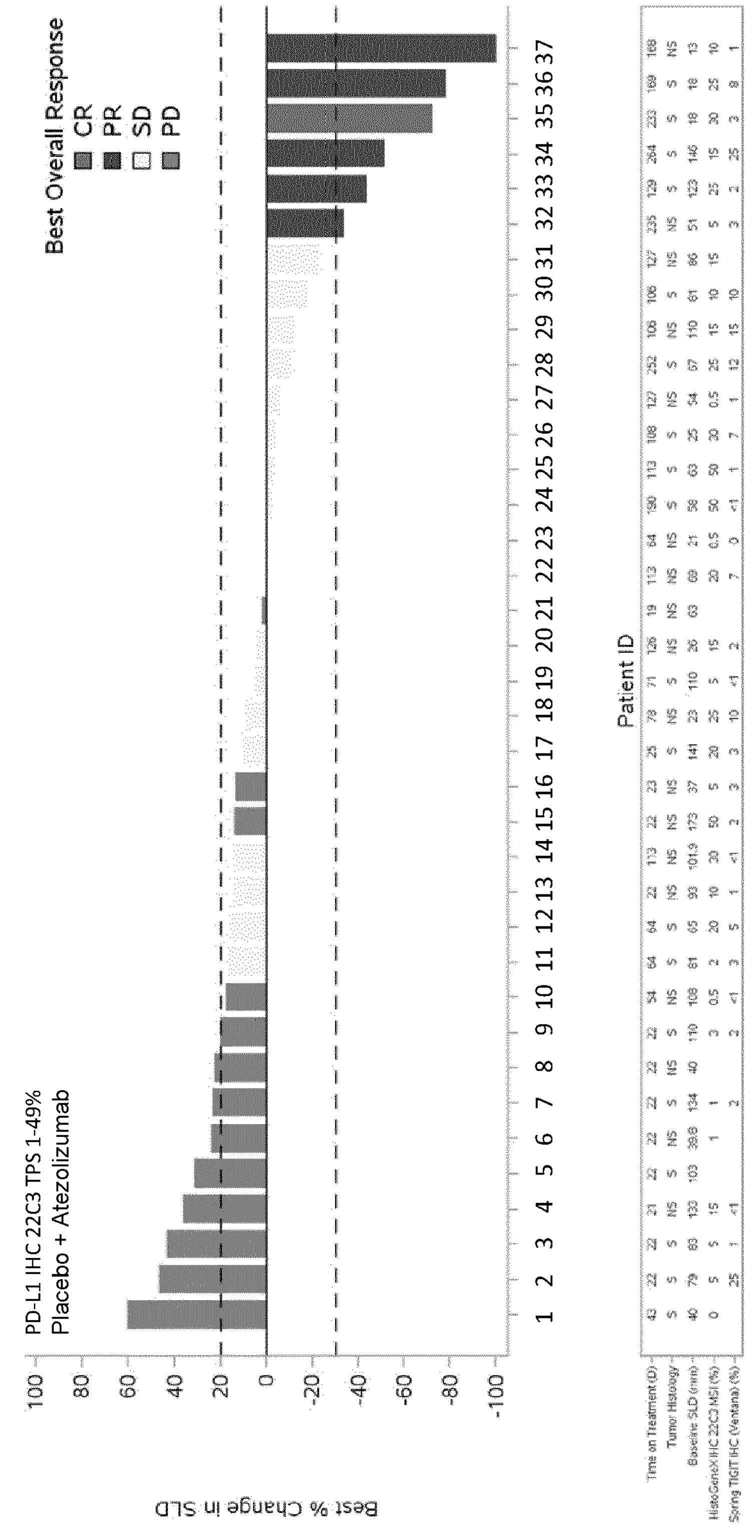
PD-L1 IHC 22C3 TPS 1-49%
Placebo + Atezolizumab
Best Overall Response
CR
PR
SD
PD
Best % Change in SLD
100
80
60
40
20
0
-20
-40
-60
-80
-100
1 2 3 4 5 6 7 8 9 10 11 12 13 14 15 16 17 18 19 20 21 22 23 24 25 26 27 28 29 30 31 32 33 34 35 36 37
Patient ID
Time on Treatment (D)
Tumor Histology
Baseline SLD (mm)
HistoGeneX IHC 22C3 MSI (%)
Spring TIGIT IHC (Ventana) (%)

FIG. 15A

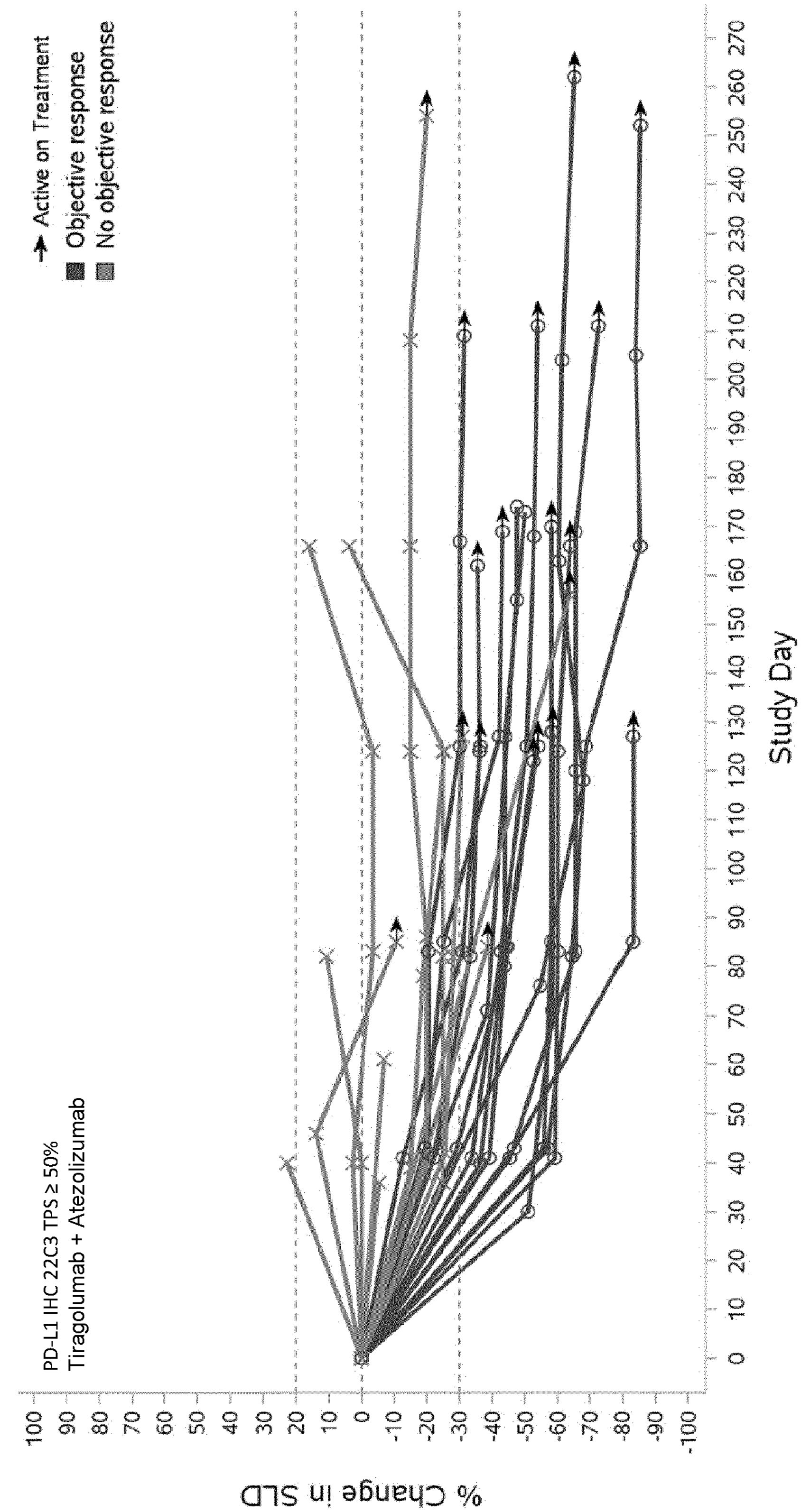
PD-L1 IHC 22C3 TPS ≥ 50%
Tiragolumab + Atezolizumab
Active on Treatment
Objective response
No objective response
% Change in SLD
100
90
80
70
60
50
40
30
20
10
0
-10
-20
-30
-40
-50
-60
-70
-80
-90
-100
0
10
20
30
40
50
60
70
80
90
100
110
120
130
140
150
160
170
180
190
200
210
220
230
240
250
260
270
Study Day

FIG. 15B

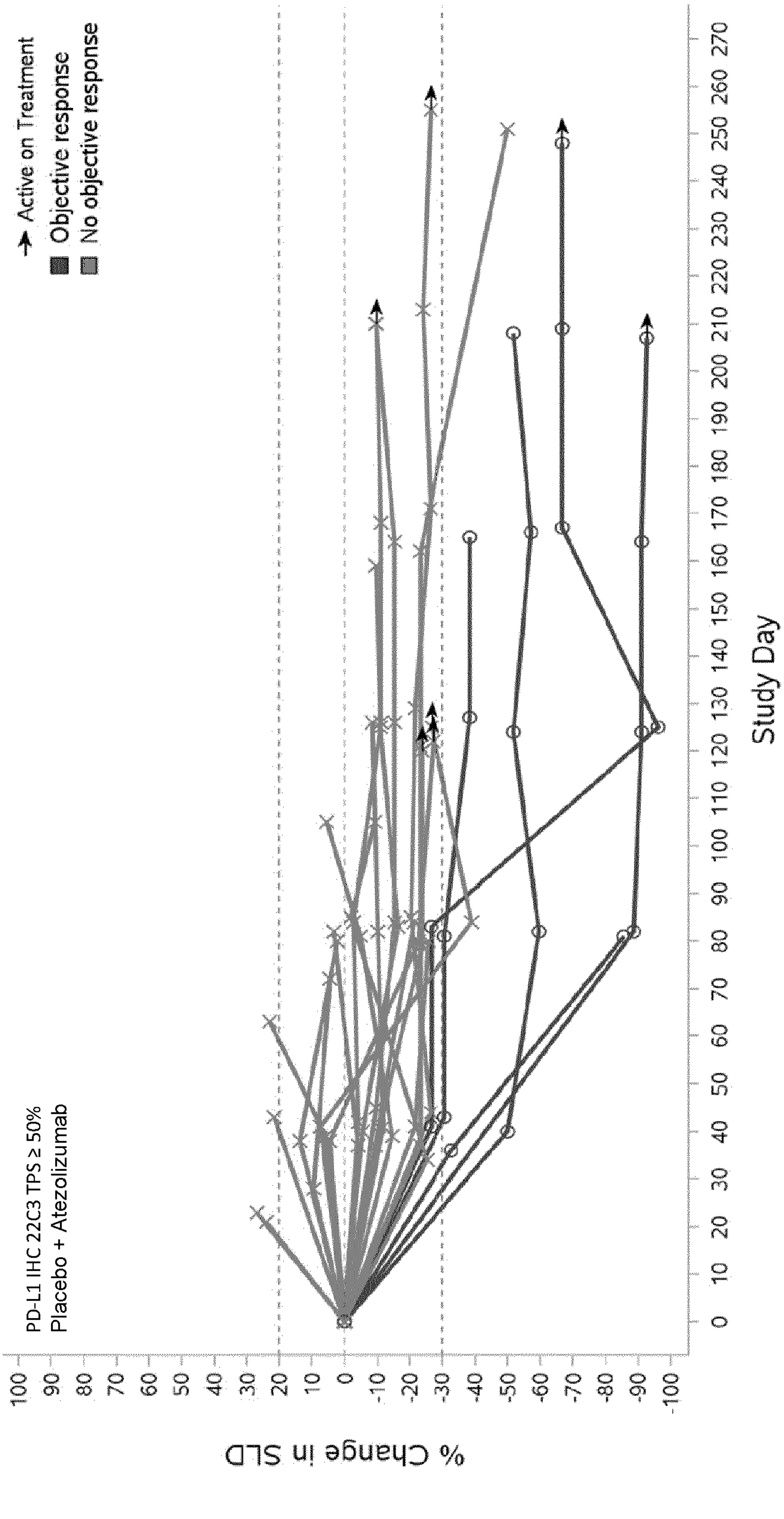
PD-L1 IHC 22C3 TPS ≥ 50%
Placebo + Atezolizumab
Active on Treatment
Objective response
No objective response
% Change in SLD
Study Day
100 90 80 70 60 50 40 30 20 10 0 -10 -20 -30 -40 -50 -60 -70 -80 -90 -100
0 10 20 30 40 50 60 70 80 90 100 110 120 130 140 150 160 170 180 190 200 210 220 230 240 250 260 270

FIG. 15C

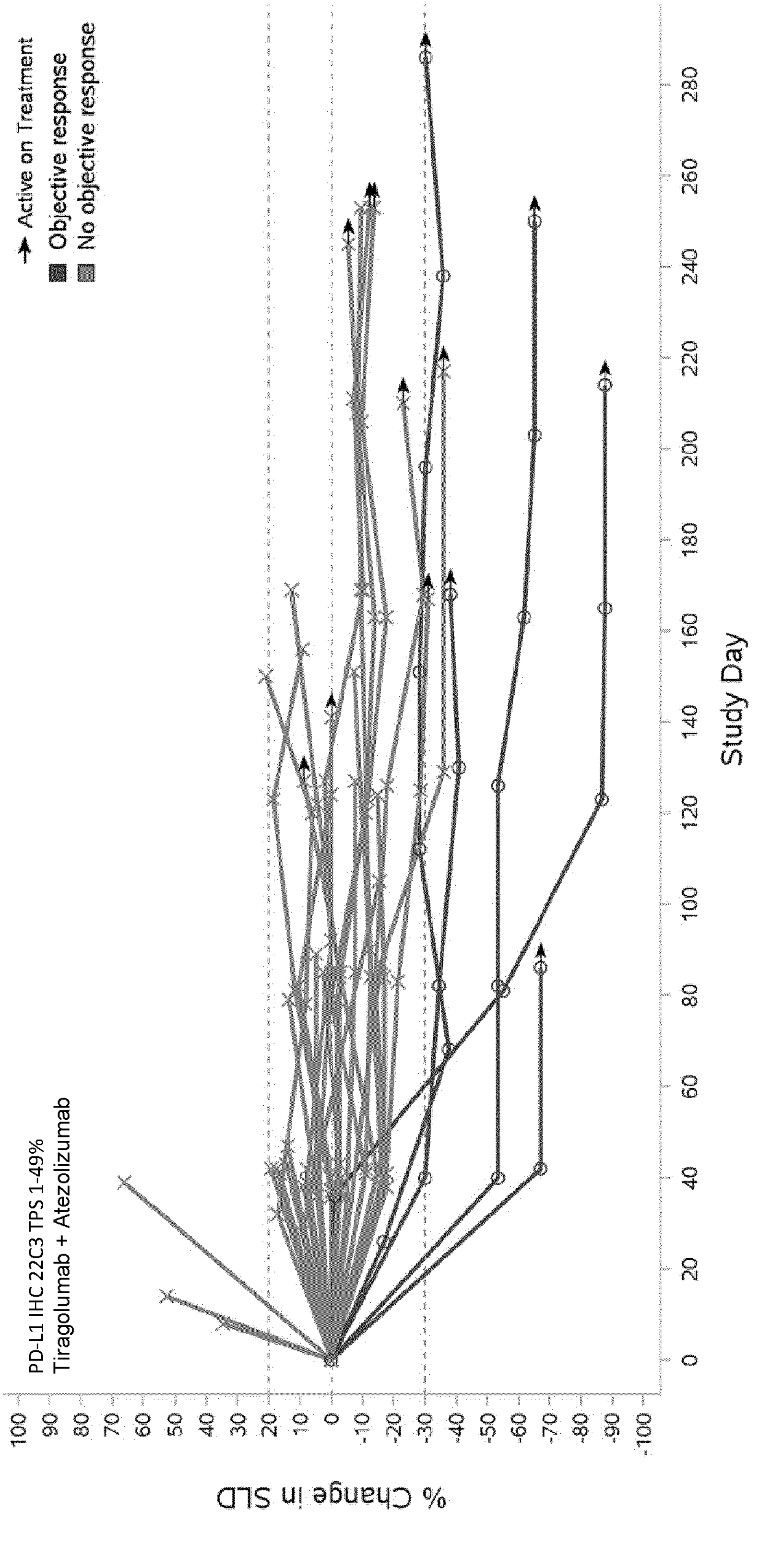
PD-L1 IHC 22C3 TPS 1-49%
Tiragolumab + Atezolizumab
Active on Treatment
Objective response
No objective response
% Change in SLD
100
90
80
70
60
50
40
30
20
10
0
-10
-20
-30
-40
-50
-60
-70
-80
-90
-100
Study Day
0
20
40
60
80
100
120
140
160
180
200
220
240
260
280

# FIG. 15D

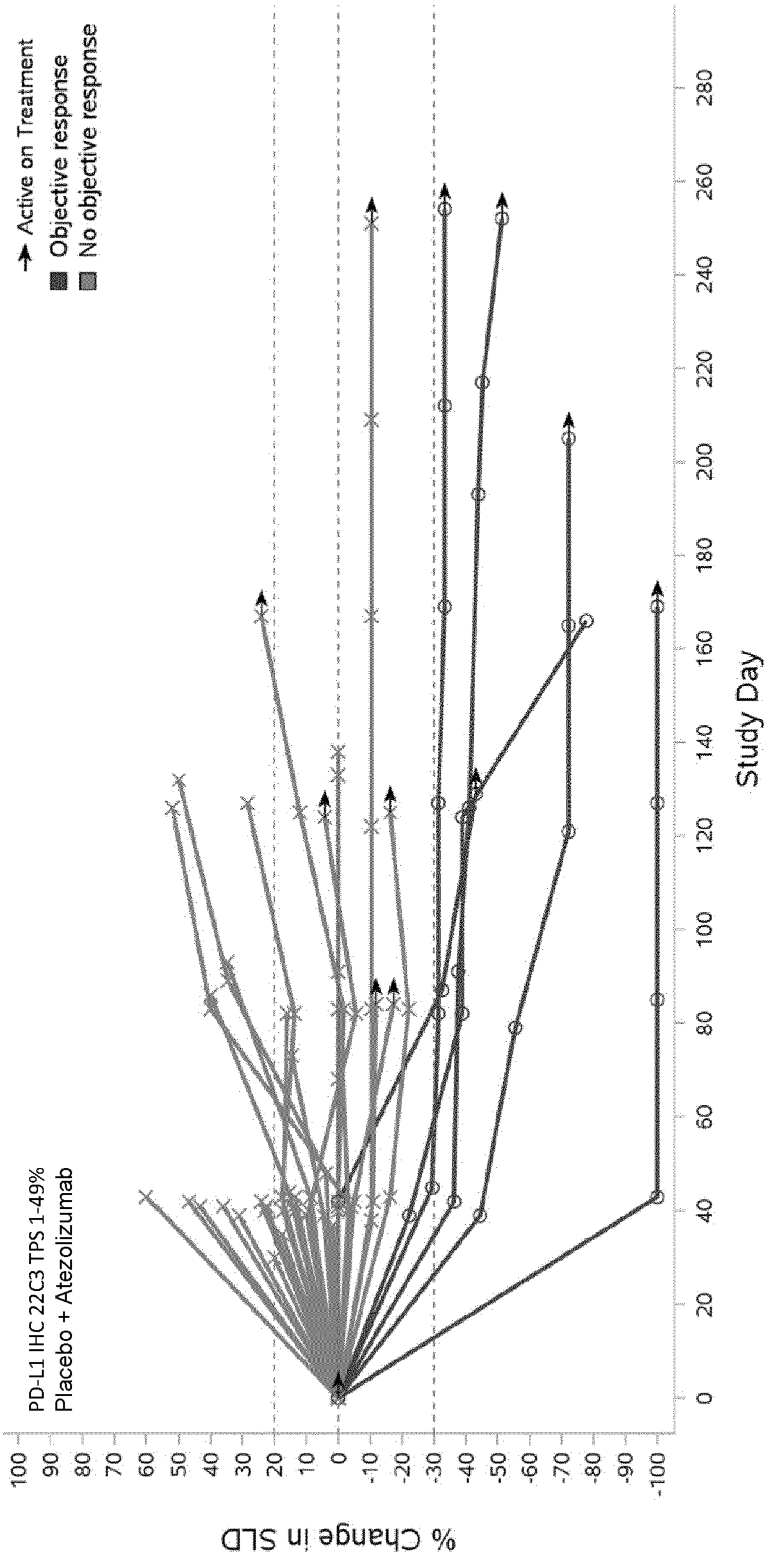
PD-L1 IHC 22C3 TPS 1-49%
Placebo + Atezolizumab
Active on Treatment
Objective response
No objective response
% Change in SLD
100
90
80
70
60
50
40
30
20
10
0
-10
-20
-30
-40
-50
-60
-70
-80
-90
-100
Study Day
0
20
40
60
80
100
120
140
160
180
200
220
240
260
280

FIG. 16

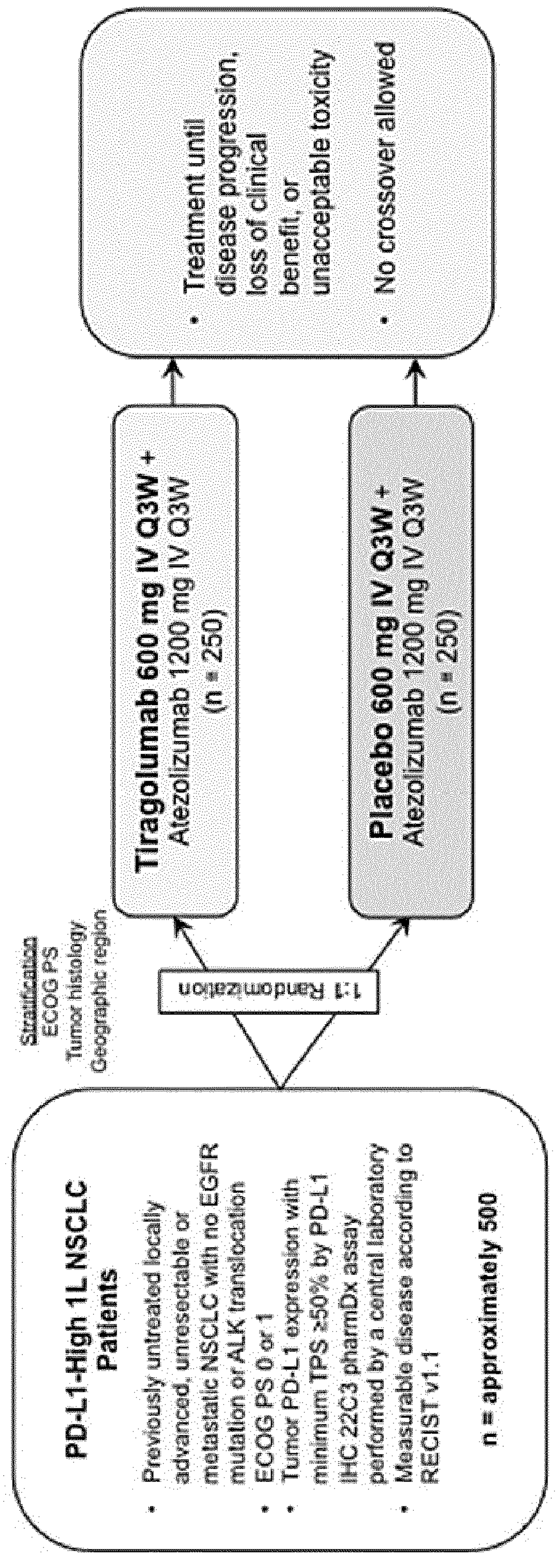
PD-L1-High 1L NSCLC Patients
• Previously untreated locally advanced, unresectable or metastatic NSCLC with no EGFR mutation or ALK translocation
• ECOG PS 0 or 1
• Tumor PD-L1 expression with minimum TPS ≥50% by PD-L1 IHC 22C3 pharmDx assay performed by a central laboratory
• Measurable disease according to RECIST v1.1
n = approximately 500
Stratification
ECOG PS
Tumor histology
Geographic region
1:1 Randomization
Tiragolumab 600 mg IV Q3W +
Atezolizumab 1200 mg IV Q3W
(n = 250)
Placebo 600 mg IV Q3W +
Atezolizumab 1200 mg IV Q3W
(n = 250)
• Treatment until disease progression, loss of clinical benefit, or unacceptable toxicity
• No crossover allowed

FIG. 17

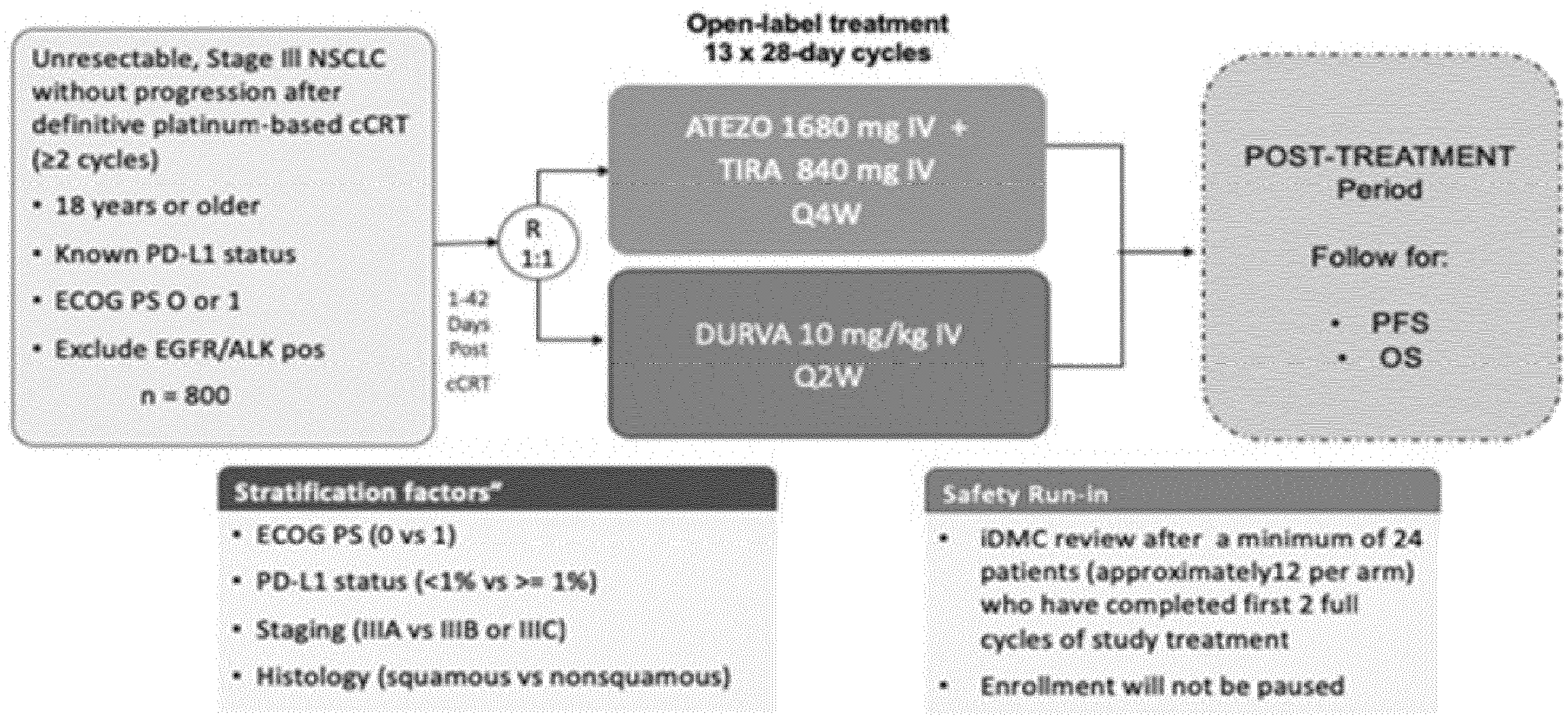
Unresectable, Stage III NSCLC without progression after definitive platinum-based cCRT (≥2 cycles)
• 18 years or older
• Known PD-L1 status
• ECOG PS O or 1
• Exclude EGFR/ALK pos
n = 800
1-42 Days Post cCRT
R 1:1
Open-label treatment
13 x 28-day cycles
ATEZO 1680 mg IV + TIRA 840 mg IV Q4W
DURVA 10 mg/kg IV Q2W
POST-TREATMENT Period
Follow for:
• PFS
• OS
Stratification factors*
• ECOG PS (0 vs 1)
• PD-L1 status (<1% vs >= 1%)
• Staging (IIIA vs IIIB or IIIC)
• Histology (squamous vs nonsquamous)
Safety Run-in
• iDMC review after a minimum of 24 patients (approximately12 per arm) who have completed first 2 full cycles of study treatment
• Enrollment will not be paused

FIG. 18

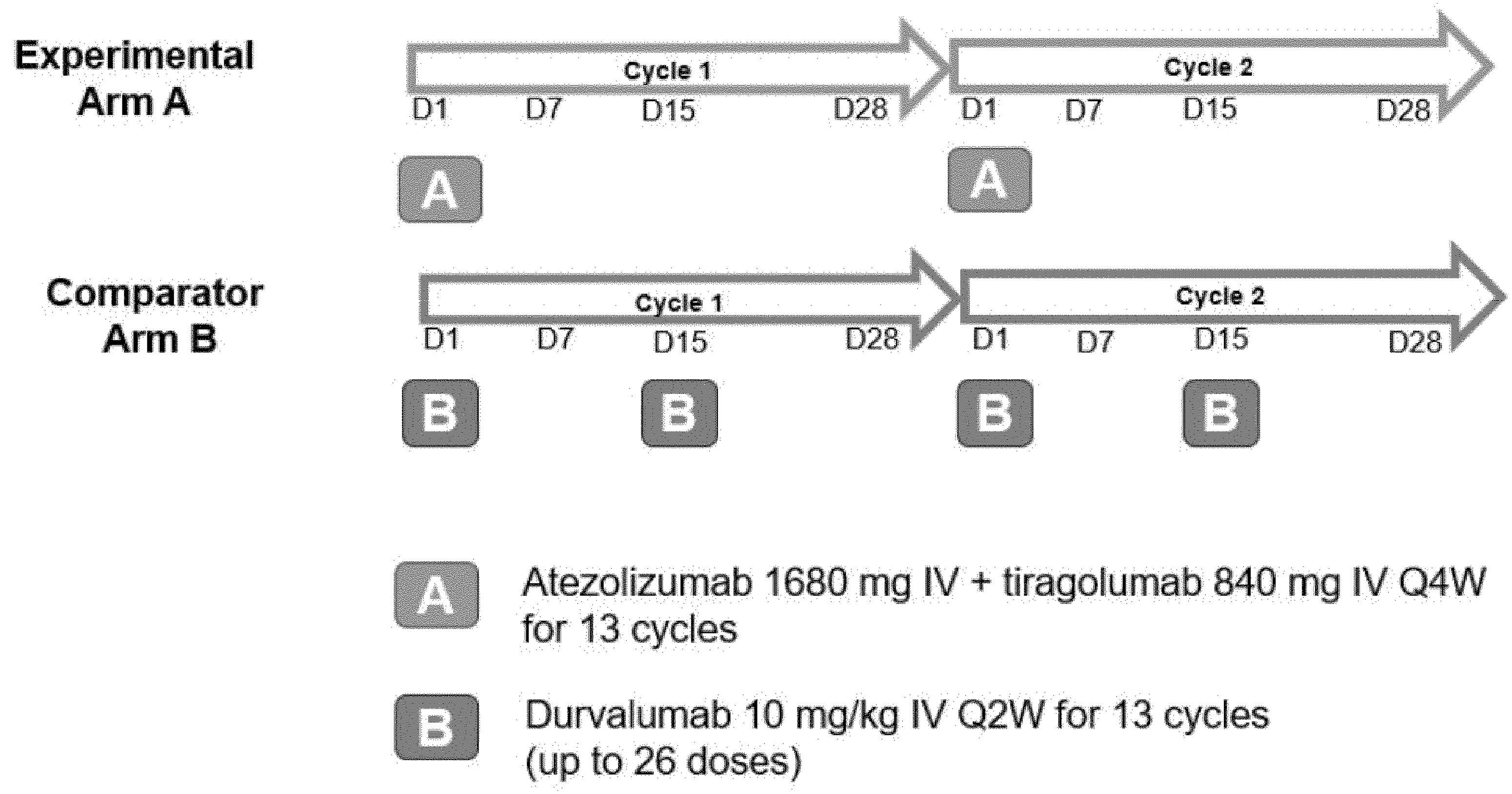
Experimental
Arm A
Cycle 1
D1
D7
D15
D28
Cycle 2
D1
D7
D15
D28
A
A
Comparator
Arm B
Cycle 1
D1
D7
D15
D28
Cycle 2
D1
D7
D15
D28
B
B
B
B
A
Atezolizumab 1680 mg IV + tiragolumab 840 mg IV Q4W for 13 cycles
B
Durvalumab 10 mg/kg IV Q2W for 13 cycles (up to 26 doses)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4943 A
- US 533 A, Mendelsohn
- WO 9640210 A
- US 5212290 A
- US 5891996 A
- WO 9850433 A
- US 6235883 B
- EP 659439 A2
- US 5616582 A
- US 5457105 A
- US 5475001 A
- US 5654307 A
- US 5679683 A
- US 6084095 A
- US 6265410 B
- US 6455534 B
- US 6521620 B
- US 6596726 B
- US 6713484 B
- US 5770599 A
- US 6140332 A
- US 5866572 A
- US 6399602 B
- US 6344459 B
- US 6602863 B
- US 6391874 B
- US 6344455 B
- US 5760041 A
- US 6002008 A
- US 5747498 A
- WO 9814451 A
- WO 9850038 A
- WO 9909016 A
- WO 9924037 A
- US 5804396 A
- WO 199909016 A
- WO 199843960 A
- WO 199738983 A, Warner Lambert
- WO 199906378 A, Warner Lambert
- WO 199906396 A, Warner Lambert
- WO 199630347 A
- WO 199633978 A
- WO 19963397 A
- WO 199633980 A
- US 5641870 A
- EP 404097 A
- WO 9311161 A
- US 4816567 A
- US 6075181 A
- US 6150584 A
- US 6982321 B
- US 7087409 B
- WO 199824893 A
- WO 199634096 A
- WO 199633735 A
- WO 199110741 A
- US 5545807 A
- US 5545806 A
- US 5569825 A
- US 5625126 A
- US 5633425 A
- US 5661016 A
- US 9651555 B
- US 20180235968 A
- WO 2010077634 A
- WO 2007005874 A
- WO 2011066389 A
- US 2013034559 A
- US 8217149 B
- WO 9316185 A
- US 5571894 A
- US 5587458 A
- US 5869046 A
- WO 199301161 A
- US 6248516 B1
- US 5821337 A
- US 7527791 B
- US 5770429 A
- US 7041870 B
- US 20070061900 A
- US 7189826 B
- US 5750373 A
- US 20050079574 A
- US 20050119455 A
- US 20050266000 A
- US 20070117126 A
- US 20070160598 A
- US 20070237764 A
- US 20070292936 A
- US 20090002360 A
- WO 2008077546 A
- US 20030157108 A, Presta, L.
- US 20040093621 A
- WO 200061739 A
- WO 200129246 A
- US 20030115614 A
- US 20020164328 A
- US 20040132140 A

- US 20040110704 A
- US 20040110282 A
- US 20040109865 A
- WO 2003085119 A
- WO 2003084570 A
- WO 2005035586 A
- WO 2005035778 A
- WO 2005053742 A
- WO 2002031140 A
- US 20030157108 A1, Presta, L
- WO 2004056312 A1, Adams
- WO 2003085107 A
- WO 2003011878 A, Jean-Mairet
- US 6602684 B, Umana
- US 20050123546 A, Umana
- WO 199730087 A, Patel
- WO 199858964 A, Raju, S
- WO 199922764 A, Raju, S
- US 20120251531 A
- US 5500362 A
- WO 2006029879 A
- WO 2005100402 A
- US 6737056 B
- US 8219149 B
- US 7332581 B
- WO 2004056312 A
- US 6194551 B
- WO 9951642 A
- US 20050014934 A1, Hinton
- US 7371826 B
- US 5648260 A
- US 5624821 A
- WO 9429351 A
- US 7521541 B
- US 5648237 A
- US 5789199 A
- US 5840523 A
- US 5959177 A
- US 6040498 A
- US 6420548 B
- US 7125978 B
- US 6417429 B
- US 5208020 A
- US 5416064 A
- EP 0425235 B1
- US 5635483 A
- US 5780588 A
- US 7498298 B
- US 5712374 A
- US 5714586 A
- US 5739116 A
- US 5767285 A
- US 5770701 A
- US 5770710 A
- US 5773001 A
- US 5877296 A
- US 6630579 B
- WO 9411026 A
- US 20050260186 A
- US 20060104968 A
- US 6267958 B
- US 6171586 B
- WO 2006044908 A

**Non-patent literature cited in the description**


- **SAMBROOK et al.** Molecular Cloning: A Laboratory Manual. Cold Spring Harbor Laboratory Press, 2001
- Current Protocols in Molecular Biology. 2003
- Methods in Enzymology. PCR 2: A Practical Approach. Academic Press, Inc, 1995
- Antibodies, A Laboratory Manual, and Animal Cell Culture. 1987
- Oligonucleotide Synthesis. 1984
- Methods in Molecular Biology. Humana Press
- Cell Biology: A Laboratory Notebook. Academic Press, 1998
- Animal Cell Culture. 1987
- Introduction to Cell and Tissue Culture. Plenum Press, 1998
- Cell and Tissue Culture: Laboratory Procedures. J. Wiley and Sons, August 1993
- Gene Transfer Vectors for Mammalian Cells. 1987
- PCR: The Polymerase Chain Reaction. 1994
- Current Protocols in Immunology. 1991
- Short Protocols in Molecular Biology. Wiley and Sons, 1999
- Immunobiology. 1997
- Antibodies. 1997
- Antibodies: A Practical Approach. IRL Press, 1988
- Monoclonal Antibodies: A Practical Approach. Oxford University Press, 2000
- Using Antibodies: A Laboratory Manual. Cold Spring Harbor Laboratory Press, 1999
- The Antibodies. Harwood Academic Publishers, 1995
- Cancer: Principles and Practice of Oncology. J.B. Lippincott Company, 1993
- Current Protocols In Molecular Biology. 1995
- International Nonproprietary Names for Pharmaceutical Substances. *Recommended INN: List 74,* 2015, vol. 29 (3), 387
- *Angew Chem. Intl. Ed. Engl.,* 1994, vol. 33, 183-186
- **STRAGLIOTTO et al.** *Eur. J. Cancer,* 1996, vol. 32A, 636-640

- **JOHNS et al.** *J. Biol. Chem.,* 2004, vol. 279 (29), 30375-30384
- **HIRSCH et al.** *Journal of Thoracic Oncology,* 2016, vol. 12 (2), 208-222
- Basic and Clinical Immunology. Appleton & Lange, 1994, 71
- **XU et al.** *Immunity,* 2000, vol. 13, 37-45
- **JOHNSON ; WU.** Methods in Molecular Biology. Human Press, 2003, vol. 248, 1-25
- **HAMERS-CASTERMAN et al.** *Nature,* 1993, vol. 363, 446-448
- **SHERIFF et al.** *Nature Struct. Biol.,* 1996, vol. 3, 733-736
- **KABAT et al.** Sequences of Proteins of Immunological Interest. Public Health Service, National Institutes of Health, 1991
- **CHOTHIA ; LESK.** *J. Mol. Biol.,* 1987, vol. 196, 901-917
- **KABAT et al.** Sequences of Immunological Interest. National Institute of Health, 1991
- **ZAPATA et al.** *Protein Eng.,* 1995, vol. 8 (10), 1057-1062
- **PLUCKTHUN.** The Pharmacology of Monoclonal Antibodies. Springer-Verlag, 1994, vol. 113, 269-315
- **HOLLINGER et al.** *Proc. Natl. Acad. Sci. USA,* 1993, vol. 90, 6444-6448
- **MORRISON et al.** *Proc. Natl. Acad. Sci. USA,* 1984, vol. 81, 6851-6855
- **M. DAËRON.** *Annu. Rev. Immunol.,* 1997, vol. 15, 203-234
- **RAVETCH ; KINET.** *Annu. Rev. Immunol.,* 1991, vol. 9, 457-92
- **CAPEL et al.** *Immunomethods,* 1994, vol. 4, 25-34
- **DE HAAS et al.** *J. Lab. Clin. Med.,* 1995, vol. 126, 330-41
- **HOOGENBOOM ; WINTER.** *J. Mol. Biol.,* 1991, vol. 227, 381
- **MARKS et al.** *J. Mol. Biol.,* 1991, vol. 222, 581
- **COLE et al.** *Monoclonal Antibodies and Cancer Therapy,* 1985, vol. 77
- **BOERNER et al.** *J. Immunol.,* 1991, vol. 147 (1), 86-95
- **VAN DIJK ; VAN DE WINKEL.** *Curr. Opin. Pharmacol.,* 2001, vol. 5, 368-74
- **LI et al.** *Proc. Natl. Acad. Sci. USA,* 2006, vol. 103, 3557-3562
- **JONES et al.** *Nature,* 1986, vol. 321, 522-525
- **RIECHMANN et al.** *Nature,* 1988, vol. 332, 323-329
- **PRESTA.** *Curr. Op. Struct. Biol.,* 1992, vol. 2, 593-596
- **VASWANI ; HAMILTON.** *Ann. Allergy, Asthma & Immunol.,* 1998, vol. 1, 105-115
- **HARRIS.** *Biochem. Soc. Transactions,* 1995, vol. 23, 1035-1038
- **HURLE ; GROSS.** *Curr. Op. Biotech.,* 1994, vol. 5, 428-433
- **FLATMAN et al.** *J. Chromatogr. B,* 2007, vol. 848, 79-87
- **KOHLER ; MILSTEIN.** *Nature,* 1975, vol. 256, 495-97
- **HONGO et al.** *Hybridoma,* 1995, vol. 14 (3), 253-260
- **HARLOW et al.** Antibodies: A Laboratory Manual. Cold Spring Harbor Laboratory Press, 1988
- **HAMMERLING et al.** Monoclonal Antibodies and T-Cell Hybridomas. Elsevier, 1981, 563-681
- **CLACKSON et al.** *Nature,* 1991, vol. 352, 624-628
- **MARKS et al.** *J. Mol. Biol.,* 1992, vol. 222, 581-597
- **SIDHU et al.** *J. Mol. Biol.,* 2004, vol. 338 (2), 299-310
- **LEE et al.** *J. Mol. Biol.,* 2004, vol. 340 (5), 1073-1093
- **FELLOUSE.** *Proc. Natl. Acad. Sci. USA,* 2004, vol. 101 (34), 12467-12472
- **LEE et al.** *J. Immunol. Methods,* 2004, vol. 284 (1-2), 119-132
- **JAKOBOVITS et al.** *Proc. Natl. Acad. Sci. USA,* 1993, vol. 90, 2551
- **JAKOBOVITS et al.** *Nature,* 1993, vol. 362, 255-258
- **BRUGGEMANN et al.** *Year in Immunol.,* 1993, vol. 7, 33
- **MARKS et al.** *BiolTechnology,* 1992, vol. 10, 779-783
- **LONBERG et al.** *Nature,* 1994, vol. 368, 856-859
- **MORRISON.** *Nature,* 1994, vol. 368, 812-813
- **FISHWILD et al.** *Nature Biotechnol.,* 1996, vol. 14, 845-851
- **NEUBERGER.** *Nature Biotechnol.,* 1996, vol. 14, 826
- **LONBERG ; HUSZAR.** *Intern. Rev. Immunol.,* 1995, vol. 13, 65-93
- **FRAMPTON et al.** *Nature Biotechnology,* 2013, vol. 31 (11), 1023-1033
- **JUAN et al.** *Therapeutic Advances in Medical Oncology.,* 2017, vol. 9 (3), 201-216
- **DU et al.** *Thoracic Cancer.,* 2018, vol. 9, 423-430
- **EISENHAUER et al.** *Eur. J. Cancer.,* 2009, vol. 45, 228-47
- *CHEMICAL ABSTRACTS,* 1918185-84-8
- *CHEMICAL ABSTRACTS,* 1422185-06-5
- **CHEN et al.** *J. Mol. Biol.,* 1999, vol. 293, 865-881
- **PRESTA et al.** *Cancer Res.,* 1997, vol. 57, 4593-4599
- **HUDSON et al.** *Nat. Med.,* 2003, vol. 9, 129-134

- **PLUCKTHÜN.** The Pharmacology of Monoclonal Antibodies. Springer-Verlag, 1994, vol. 113, 269-315
- **ALMAGRO ; FRANSSON.** *Front. Biosci.,* 2008, vol. 13, 1619-1633
- **QUEEN et al.** *Proc. Natl Acad. Sci. USA,* 1989, vol. 86, 10029-10033
- **KASHMIRI et al.** *Methods,* 2005, vol. 36, 25-34
- **PADLAN.** *Mol. Immunol.,* 1991, vol. 28, 489-498
- **DALL'ACQUA et al.** *Methods,* 2005, vol. 36, 43-60
- **OSBOURN et al.** *Methods,* 2005, vol. 36, 61-68
- **KLIMKA et al.** *Br. J. Cancer,* 2000, vol. 83, 252-260
- **SIMS et al.** *J. Immunol.,* 1993, vol. 151, 2296
- **CARTER et al.** *Proc. Natl. Acad. Sci. USA,* 1992, vol. 89, 4285
- **PRESTA et al.** *J. Immunol.,* 1993, vol. 151, 2623
- **BACA et al.** *J. Biol. Chem.,* 1997, vol. 272, 10678-10684
- **ROSOK et al.** *J. Biol. Chem.,* 1996, vol. 271, 22611-22618
- **LONBERG.** *Curr. Opin. Immunol.,* 2008, vol. 20, 450-459
- **LONBERG.** *Nat. Biotech.,* 2005, vol. 23, 1117-1125
- **KOZBOR.** *J. Immunol.,* 1984, vol. 133, 3001
- **BRODEUR et al.** Monoclonal Antibody Production Techniques and Applications. Marcel Dekker, Inc, 1987, 51-63
- **BOERNER et al.** *J. Immunol.,* 1991, vol. 147, 86
- **NI.** *Xiandai Mianyixue,* 2006, vol. 26 (4), 265-268
- **VOLLMERS ; BRANDLEIN.** *Histology and Histopathology,* 2005, vol. 20 (3), 927-937
- **VOLLMERS ; BRANDLEIN.** *Methods and Findings in Experimental and Clinical Pharmacology,* 2005, vol. 27 (3), 185-91
- **HOOGENBOOM et al.** Methods in Molecular Biology. Human Press, 2001, vol. 178, 1-37
- **MCCAFFERTY et al.** *Nature,* vol. 348, 552-554
- **MARKS ; BRADBURY.** Methods in Molecular Biology. Human Press, 2003, vol. 248, 161-175
- **WINTER et al.** *Ann. Rev. Immunol.,* 1994, vol. 12, 433-455
- **GRIFFITHS et al.** *EMBO J,* 1993, vol. 12, 725-734
- **HOOGENBOOM ; WINTER.** *J. Mol. Biol.,* 1992, vol. 227, 381-388
- **CHOWDHURY.** *Methods Mol. Biol.,* 2008, vol. 207, 179-196
- **CUNNINGHAM ; WELLS.** *Science,* 1989, vol. 244, 1081-1085
- **WRIGHT et al.** *TIBTECH,* 1997, vol. 15, 26-32
- **OKAZAKI et al.** *J. Mol. Biol.,* 2004, vol. 336, 1239-1249
- **YAMANE-OHNUKI et al.** *Biotech. Bioeng.,* 2004, vol. 87, 614
- **RIPKA et al.** *Arch. Biochem. Biophys.,* 1986, vol. 249, 533-545
- **KANDA, Y. et al.** *Biotechnol. Bioeng.,* 2006, vol. 94 (4), 680-688
- **RAVETCH ; KINET.** *Annu. Rev. Immunol.,* 1991, vol. 9, 457-492
- **HELLSTROM, I. et al.** *Proc. Nat'l Acad. Sci. USA,* 1986, vol. 83, 7059-7063
- **HELLSTROM, I et al.** *Proc. Nat'l Acad. Sci. USA,* 1985, vol. 82, 1499-1502
- **BRUGGEMANN, M et al.** *J. Exp. Med.,* 1987, vol. 166, 1351-1361
- **CLYNES et al.** *Proc. Nat'l Acad. Sci. USA,* 1998, vol. 95, 652-656
- **GAZZANO-SANTORO et al.** *J. Immunol. Methods,* 1996, vol. 202, 163
- **CRAGG, M.S. et al.** *Blood,* 2003, vol. 101, 1045-1052
- **CRAGG, M.S. ; M.J. GLENNIE.** *Blood,* 2004, vol. 103, 2738-2743
- **PETKOVA, S.B. et al.** *Int'l. Immunol.,* 2006, vol. 18 (12), 1759-1769
- **SONDERMANN et al.** *Nature,* 20 July 2000, vol. 406, 267-273
- **SHIELDS et al.** *J. Biol. Chem.,* 2001, vol. 9 (2), 6591-6604
- **IDUSOGIE et al.** *J. Immunol.,* 2000, vol. 164, 4178-4184
- **GUYER et al.** *J. Immunol.,* 1976, vol. 117, 587
- **KIM et al.** *J. Immunol.,* 1994, vol. 24, 249
- **DUNCAN ; WINTER.** *Nature,* 1988, vol. 322, 738-40
- **KAM et al.** *Proc. Natl. Acad. Sci. USA,* 2005, vol. 102, 11600-11605
- **CHARLTON.** Methods in Molecular Biology. Humana Press, 2003, vol. 248, 245-254
- **GERNGROSS.** *Nat. Biotech.,* 2004, vol. 22, 1409-1414
- **LI et al.** *Nat. Biotech.,* 2006, vol. 24, 210-215
- **GRAHAM et al.** *J. Gen Virol.,* 1977, vol. 36, 59
- **MATHER.** *Biol. Reprod.,* 1980, vol. 23, 243-251
- **MATHER et al.** *Annals N.Y. Acad. Sci.,* 1982, vol. 383, 44-68
- **URLAUB et al.** *Proc. Natl. Acad. Sci. USA,* 1980, vol. 77, 4216
- **YAZAKI ; WU.** Methods in Molecular Biology. Humana Press, 2003, vol. 248, 255-268
- **HINMAN et al.** *Cancer Res.,* 1993, vol. 53, 3336-3342

- **LODE et al.** *Cancer Res.,* 1998, vol. 58, 2925-2928
- **KRATZ et al.** *Current Med. Chem.,* 2006, vol. 13, 477-523
- **JEFFREY et al.** *Bioorganic & Med. Chem. Letters,* 2006, vol. 16, 358-362
- **TORGOV et al.** *Bioconj. Chem.,* 2005, vol. 16, 717-721
- **NAGY et al.** *Proc. Natl. Acad. Sci. USA,* 2000, vol. 97, 829-834
- **DUBOWCHIK et al.** *Bioorg. & Med. Chem. Letters,* 2002, vol. 12, 1529-1532
- **KING et al.** *J. Med. Chem.,* 2002, vol. 45, 4336-4343
- **VITETTA et al.** *Science,* 1987, vol. 238, 1098
- **CHARI et al.** *Cancer Res.,* 1992, vol. 52, 127-131
- Remington's Pharmaceutical Sciences. 1980
- **HODI et al.** *J. Clin. Oncol.,* 17 January 2018
- **WOLCHOK et al.** *Clin. Can. Res.,* 2009, vol. 15 (23), 7412-20
- **AARONSON et al.** *J. Natl. Cancer Inst.,* 1993, vol. 85, 365-76
- **FITZSIMMONS et al.** *Eur. J. Cancer,* 1999, vol. 35, 939-41
- **BASCH et al.** *J. Natl Cancer Inst.,* 2014, vol. 106, 1-11
- **DUECK et al.** *JAMA Oncol.,* 2015, vol. 1, 1051-52
- **AMIN et al.** AJCC cancer staging manual. Springer, 2017
- **POSTMUS et al.** *Ann. Oncol.,* 2017, vol. 28 (4), iv1-iv21
- **COCKCROFT ; GAULT.** *Nephron,* 1976, vol. 16, 31-41
- **JOLLER et al.** *J. Immunol.,* 2011, vol. 186, 1338-42
- **BROOKMEYER ; CROWLEY.** *Biometrics,* 1982, vol. 38, 29-41